# EUROPEAN PATENT APPLICATION

(11) **EP 4 834 928 A1**
(43) Date of publication of application: **04.11.2026**
(21) Application number: 24911817.5
(22) Date of filing: 09.07.2024
(51) Int. Cl.: C07K 16/00, C07K 7/06, C12N 15/10, C12N 15/13, C12Q 1/37, G01N 27/62, G01N 33/68

(54) **PEPTIDE CAPABLE OF BEING ANALYZED AND/OR EVALUATED BY MASS SPECTROMETRY AND USE THEREOF**

(30) Priority: 28.12.2023 JP 2023222138
(71) Applicant: CHUGAI SEIYAKU KABUSHIKI KAISHA, Tokyo 115-8543 (JP)
(72) Inventor: HIRONIWA, Naoka, Synaps, 138623 (SG); MURAO, Naoaki, Yokohama-shi, Kanagawa 244-8602 (JP); ICHIKAWA, Takuya, Yokohama-shi, Kanagawa 244-8602 (JP)
(74) Representative: Vossius & Partner Patentanwälte Rechtsanwälte mbB
(86) International application number: PCT/JP2024/024773
(87) International publication number: WO 2025/141918

(57) **Abstract**

The present inventors invented a technique for designing unique sequences by modifying a portion of the sequence of an antigen-binding molecule and analyzing and/or evaluating the sequences with a mass spectrometer. Based on that invention, the present application provides antigen-binding molecules, methods for detecting and/or quantifying antigen-binding molecules in biological samples, methods for designing, selecting, or producing modified antigen-binding molecules, modified antigen-binding molecules designed, selected, or produced by the methods, peptides that are derived from antigen-binding molecules and can be analyzed and/or evaluated by mass spectrometry, sets comprising two or more such peptides, and the like.

## Description

### [Technical Field]

The present application relates to peptides that can be analyzed and/or evaluated by mass spectrometry and uses thereof.

### [Background Art]

One technique for detecting, quantifying, or investigating the *in vivo* kinetics and distribution of antigen-binding molecules such as antibodies is mass spectrometry of peptides with specific sequences comprised in the antigen-binding molecules (Patent Literature (PTL) 1). An example of measurement using the unique sequence comprised in each antibody is described in Non-Patent Literature (NPL) 1. There is also a method for identifying antibodies and antigen-binding molecules by adding an amino acid sequence that serves as a barcode to the antigen-binding molecules such as antibodies, and measuring the barcode portion.

Cassette dosing study (cocktail administration study) is known as a technique to simultaneously administer multiple drugs or samples to an animal and evaluate their *in vivo* kinetics and distribution in the same animal at the same time. Since it allows multiple samples to be evaluated in one animal at the same time, cassette dosing is advantageous in that it facilitates comparison with a standard and reduces the number of animals used. To date, the following four cassette dosing studies have been performed on antibodies:
(1) a method of mixing three types of antibodies each recognizing different antigens, and measuring them by an ECL method using three different antigens (NPL 1);
(2) a method of labeling antibodies with different radioactive metals (NPL 2);
(3) a method of measuring sequences comprising antibody-specific sequences present in the variable regions of the antibodies by using a mass spectrometer (NPL 3); and
(4) a method of adding an amino acid sequence that serves as a barcode to the end of antibodies and measuring the barcode site.

### [Citation List]

### [Non-Patent Literature]

[NPL 1] Pharmaceutical Research volume 38, 583-592 (2021)
[NPL 2] Methods Mol Biol. 2014; 1141: 147-157
[NPL 3] Clin Vaccine Immunol. 2017 May; 24(5): e00545-16

### [Patent Literature]

[PTL 1] WO2012/155019
[PTL 2] WO2008/093762

### [Summary of the Invention]

### [Technical Problem]

An objective of the present application is to provide novel techniques for analyzing and/or evaluating antigen-binding molecules with a mass spectrometer.

### [Solution to Problem]

The present inventors invented a technique for designing unique sequences by modifying a portion of the sequence of an antigen-binding molecule and analyzing and/or evaluating the sequences with a mass spectrometer. Based on such an invention, the present application provides antigen-binding molecules, methods for detecting and/or quantifying the antigen-binding molecules in biological samples, methods for designing, selecting, or producing modified antigen-binding molecules, and peptides derived from the antigen-binding molecules that can be analyzed and/or evaluated by mass spectrometry. For example, the present application provides the following.

[A1] An antigen-binding molecule comprising an antigen-binding domain, a CL domain, and a non-native human IgG CH1 domain, wherein the amino acid sequence between positions 121 and 133 according to the EU index in the non-native human IgG CH1 domain is different from a corresponding sequence in a native human IgG of the same isotype as the non-native human IgG.
[A2] The antigen-binding molecule of [A1], wherein the amino acid sequence between positions 121 and 133 according to the EU index in the non-native human IgG CH1 domain has any one of the following features:
   (1) an amino acid at any one or more sites of positions 122 to 132 is different from an amino acid present at corresponding sites in a native human IgG of the same isotype, and the amino acid at said sites is an amino acid other than Lys, Arg, and Met (provided that the amino acid at position 122 is not Pro);
   (2) an amino acid other than Lys, Arg, and Met has been inserted at one or more sites selected from a site between positions 121 and 122, a site between positions 122 and 123, a site between positions 123 and 124, a site between positions 124 and 125, a site between positions 125 and 126, a site between positions 126 and 127, a site between positions 127 and 128, a site between positions 128 and 129, a site between positions 129 and 130, a site between positions 130 and 131, a site between positions 131 and 132, and a site between positions 132 and 133 (provided that the amino acid at the site between positions 121 and 122 is not Pro).
[A3] The antigen-binding molecule of [A1] or [A2], wherein the amino acid sequence between positions 121 and 133 according to the EU index in the non-native human IgG CH1 domain has any one of the following features:
   (1) an amino acid at any one or more sites of positions 122 to 128 and positions 130 to 132 is Ala;
   (2) an amino acid at any one or more sites of positions 122 to 132 is Ile;
   (3) an amino acid at any one or more sites of positions 122 to 124 and positions 126 to 132 is Val;
   (4) an amino acid at any one or more sites of positions 122 to 127 and positions 129 to 132 is Leu;
   (5) an amino acid at any one or more sites of positions 122 to 125 and positions 127 to 132 is Phe;
   (6) Ala, Ile, Val, Leu, Phe, Ala-Ala, Ile-Ile, Val-Val, Leu-Leu, Phe-Phe, Ala-Ile, Ala-Val, Ala-Leu, Ala-Phe, Ile-Val, Ile-Leu, Ile-Phe, Val-Leu, Val-Phe, Leu-Phe, or Ala-Ala-Ala has been inserted at one or more sites selected from a site between positions 121 and 122, a site between positions 122 and 123, a site between positions 123 and 124, a site between positions 124 and 125, a site between positions 125 and 126, a site between positions 126 and 127, a site between positions 127 and 128, a site between positions 128 and 129, a site between positions 129 and 130, a site between positions 130 and 131, a site between positions 131 and 132, and a site between positions 132 and 133.
[A4] The antigen-binding molecule of [A1] or [A2], wherein the amino acid sequence between positions 121 and 133 according to the EU index in the non-native human IgG CH1 domain has any one of the following features:
   (1) an amino acid at any one site of positions 122 to 128 and positions 130 to 132 is Ala;
   (2) an amino acid at any one site of positions 122 to 132 is Ile;
   (3) an amino acid at any one site of positions 122 to 124 and positions 126 to 132 is Val;
   (4) an amino acid at any one site of positions 122 to 127 and positions 129 to 132 is Leu;
   (5) an amino acid at any one site of positions 122 to 125 and positions 127 to 132 is Phe;
   (6) Ala, Ile, Val, Leu, Phe, Ala-Ala, Ile-Ile, Val-Val, Leu-Leu, Phe-Phe, Ala-Ile, Ala-Val, Ala-Leu, Ala-Phe, Ile-Val, Ile-Leu, Ile-Phe, Val-Leu, Val-Phe, Leu-Phe, or Ala-Ala-Ala has been inserted at one site selected from a site between positions 121 and 122, a site between positions 122 and 123, a site between positions 123 and 124, a site between positions 124 and 125, a site between positions 125 and 126, a site between positions 126 and 127, a site between positions 127 and 128, a site between positions 128 and 129, a site between positions 129 and 130, a site between positions 130 and 131, a site between positions 131 and 132, and a site between positions 132 and 133.
[A5] The antigen-binding molecule of any one of [A1] to [A4], wherein the non-native human IgG CH1 domain is a non-native human IgG1 CH1 domain, a non-native human IgG2 CH1 domain, a non-native human IgG3 CH1 domain, or a non-native human IgG4 CH1 domain.
[A6] The antigen-binding molecule of any one of [A1] to [A5], wherein the amino acid sequence between positions 121 and 133 according to the EU index in the non-native human IgG CH1 domain is the amino acid sequence of any one of SEQ ID NOs: 6-294 and 1565-1833.
[A7] The antigen-binding molecule of any one of [A1] to [A6], wherein the antigen-binding domain comprises a VH domain and a VL domain.
[A8] The antigen-binding molecule of [A7], wherein the VH domain and the VL domain are a humanized VH domain and a humanized VL domain.
[A9] The antigen-binding molecule of [A7] or [A8], wherein the C-terminus of the VH domain is linked with the N-terminus of the non-native human IgG CH1 domain, and the C-terminus of the VL domain is linked with the N-terminus of the CL domain.
[A10] The antigen-binding molecule of [A7] or [A8], wherein the C-terminus of the VL domain is linked with the N-terminus of the non-native human IgG CH1 domain, and the C-terminus of the VH domain is linked with the N-terminus of the CL domain.
[A11] The antigen-binding molecule of any one of [A1] to [A10], which further comprises an antibody Fc region.
[A12] The antigen-binding molecule of any one of [A1] to [A11], which is a non-native IgG antibody.
[A13] The antigen-binding molecule of [A12], wherein the non-native IgG antibody is a non-native IgG1 antibody, a non-native IgG2 antibody, a non-native IgG3 antibody, or a non-native IgG4 antibody.
[A14] The antigen-binding molecule of any one of [A1] to [A13], which is conjugated to a drug moiety.

[B1] A method for detecting a peptide, comprising:
   (a) preparing a sample containing a peptide consisting of an amino acid sequence between positions 121 and 133 according to the EU index in a non-native human IgG CH1 domain,
      wherein the amino acid sequence between positions 121 and 133 according to the EU index in the non-native human IgG CH1 domain is different from a corresponding sequence in a native human IgG of the same isotype as the non-native human IgG; and
   (b) analyzing the sample prepared in (a) by mass spectrometry to detect the peptide.
[B2] A method for quantifying a peptide, comprising:
   (a) preparing a sample comprising a peptide consisting of an amino acid sequence between positions 121 and 133 according to the EU index in a non-native human IgG CH1 domain,
      wherein the amino acid sequence between positions 121 and 133 according to the EU index in the non-native human IgG CH1 domain is different from a corresponding sequence in a native human IgG of the same isotype as the non-native human IgG;
   (b) analyzing the sample prepared in (a) by mass spectrometry to detect the peptide; and
   (c) quantifying the peptide based on the result of the analysis of (b).
[B3] A method for detecting and/or quantifying an antigen-binding molecule in a biological sample, comprising:
   (a) treating a biological sample containing an antigen-binding molecule with a digestive enzyme to generate a peptide from the antigen-binding molecule, wherein
      the antigen-binding molecule comprises an antigen-binding domain, a CL domain, and a non-native human IgG CH1 domain,
      an amino acid sequence between positions 121 and 133 according to the EU index in the non-native human IgG CH1 domain is different from a corresponding sequence in a native human IgG of the same isotype as the non-native human IgG, and
      the peptide consists of the amino acid sequence between positions 121 and 133 according to the EU index in the non-native human IgG CH1 domain;
   (b) analyzing the biological sample treated in (a) by mass spectrometry to detect the peptide; and
   (c) detecting and/or quantifying the antigen-binding molecule based on the result of the analysis of (b).
[B4] The method of any one of [B1] to [B3], wherein the amino acid sequence between positions 121 and 133 according to the EU index in the non-native human IgG CH1 domain has any one of the following features:
   (1) an amino acid at any one or more sites of positions 122 to 132 is different from an amino acid present at corresponding sites in a native human IgG of the same isotype, and the amino acid at said sites is an amino acid other than Lys, Arg, and Met (provided that the amino acid at position 122 is not Pro);
   (2) an amino acid other than Lys, Arg, and Met has been inserted at one or more sites selected from a site between positions 121 and 122, a site between positions 122 and 123, a site between positions 123 and 124, a site between positions 124 and 125, a site between positions 125 and 126, a site between positions 126 and 127, a site between positions 127 and 128, a site between positions 128 and 129, a site between positions 129 and 130, a site between positions 130 and 131, a site between positions 131 and 132, and a site between positions 132 and 133 (provided that the amino acid at the site between positions 121 and 122 is not Pro).
[B5] The method of any one of [B1] to [B3], wherein the amino acid sequence between positions 121 and 133 according to the EU index in the non-native human IgG CH1 domain has any one of the following features:
   (1) an amino acid at any one or more sites of positions 122 to 128 and positions 130 to 132 is Ala;
   (2) an amino acid at any one or more sites of positions 122 to 132 is Ile;
   (3) an amino acid at any one or more sites of positions 122 to 124 and positions 126 to 132 is Val;
   (4) an amino acid at any one or more sites of positions 122 to 127 and positions 129 to 132 is Leu;
   (5) an amino acid at any one or more sites of positions 122 to 125 and positions 127 to 132 is Phe;
   (6) Ala, Ile, Val, Leu, Phe, Ala-Ala, Ile-Ile, Val-Val, Leu-Leu, Phe-Phe, Ala-Ile, Ala-Val, Ala-Leu, Ala-Phe, Ile-Val, Ile-Leu, Ile-Phe, Val-Leu, Val-Phe, Leu-Phe, or Ala-Ala-Ala has been inserted at one or more sites selected from a site between positions 121 and 122, a site between positions 122 and 123, a site between positions 123 and 124, a site between positions 124 and 125, a site between positions 125 and 126, a site between positions 126 and 127, a site between positions 127 and 128, a site between positions 128 and 129, a site between positions 129 and 130, a site between positions 130 and 131, a site between positions 131 and 132, and a site between positions 132 and 133.
[B6] The method of any one of [B1] to [B3], wherein the amino acid sequence between positions 121 and 133 according to the EU index in the non-native human IgG CH1 domain has any one of the following features:
   (1) an amino acid at any one site of positions 122 to 128 and positions 130 to 132 is Ala;
   (2) an amino acid at any one site of positions 122 to 132 is Ile;
   (3) an amino acid at any one site of positions 122 to 124 and positions 126 to 132 is Val;
   (4) an amino acid at any one site of positions 122 to 127 and positions 129 to 132 is Leu;
   (5) an amino acid at any one site of positions 122 to 125 and positions 127 to 132 is Phe;
   (6) Ala, Ile, Val, Leu, Phe, Ala-Ala, Ile-Ile, Val-Val, Leu-Leu, Phe-Phe, Ala-Ile, Ala-Val, Ala-Leu, Ala-Phe, Ile-Val, Ile-Leu, Ile-Phe, Val-Leu, Val-Phe, Leu-Phe, or Ala-Ala-Ala has been inserted at one site selected from a site between positions 121 and 122, a site between positions 122 and 123, a site between positions 123 and 124, a site between positions 124 and 125, a site between positions 125 and 126, a site between positions 126 and 127, a site between positions 127 and 128, a site between positions 128 and 129, a site between positions 129 and 130, a site between positions 130 and 131, a site between positions 131 and 132, and a site between positions 132 and 133.
[B7] The method of any one of [B1] to [B6], wherein the non-native human IgG CH1 domain is a non-native human IgG1 CH1 domain, a non-native human IgG2 CH1 domain, a non-native human IgG3 CH1 domain, or a non-native human IgG4 CH1 domain.
[B8] The method of any one of [B1] to [B6], wherein the amino acid sequence between positions 121 and 133 according to the EU index in the non-native human IgG CH1 domain is the amino acid sequence of any one of SEQ ID NOs: 6-294 and 1565-1833.
[B9] The method of any one of [B3] to [B8], wherein the digestive enzyme is a protease.
[B10] The method of any one of [B3] to [B8], wherein the digestive enzyme is trypsin.
[B11] The method of any one of [B3] to [B10], wherein the step (b) comprises contacting the biological sample treated in the step (a) with an affinity capture medium or a chromatographic adsorbent and eluting a biological sample enriched with the peptide, and analyzing the enriched biological sample by mass spectrometry.
[B12] The method of [B1] or [B2], wherein the step (b) comprises contacting the sample prepared in the step (a) with an affinity capture medium or a chromatographic adsorbent and eluting a sample enriched with the peptide, and analyzing the enriched sample by mass spectrometry.
[B13] The method of any one of [B3] to [B10], wherein the step (a) comprises contacting the biological sample with an affinity capture medium or a chromatographic adsorbent and eluting a biological sample enriched with the antigen-binding molecule, and treating the enriched biological sample with a digestive enzyme.
[B14] The method of any one of [B11] to [B13], wherein the affinity capture medium is Protein A or Protein G.
[B15] The method of any one of [B11] to [B13], wherein the chromatographic adsorbent is a solid phase extraction (SPE) adsorbent.
[B16] The method of any one of [B1] to [B10], wherein the step (b) comprises analyzing the biological sample treated in the step (a) or the sample prepared in the step (a) by mass spectrometry in combination with liquid chromatography.
[B17] The method of any one of [B1] to [B10], wherein the step (b) comprises separating a fraction containing the peptide from the biological sample treated in the step (a) or the sample prepared in the step (a) by liquid chromatography, and analyzing the separated fraction by mass spectrometry.
[B18] The method of any one of [B3] to [B10], wherein the step (a) comprises separating a fraction containing the antigen-binding molecule from the biological sample by liquid chromatography, and treating the separated fraction with a digestive enzyme.
[B19] The method of any one of [B1] to [B18], wherein the mass spectrometry is performed by tandem mass spectrometry or multiple-stage mass spectrometry.
[B20] The method of any one of [B1] to [B18], wherein the mass spectrometry is performed by tandem mass spectrometry or multiple-stage mass spectrometry in a selected reaction monitoring (SRM) mode.
[B21] The method of any one of [B3] to [B20], wherein the biological sample is a serum, plasma, a tissue, or a cell from an animal treated with the antigen-binding molecule.
[B22] The method of any one of [B3] to [B21], wherein the antigen-binding domain comprises a VH domain and a VL domain.
[B23] The method of [B22], wherein the VH domain and the VL domain are a humanized VH domain and a humanized VL domain.
[B24] The method of [B22] or [B23], wherein the C-terminus of the VH domain is linked with the N-terminus of the non-native human IgG CH1 domain, and the C-terminus of the VL domain is linked with the N-terminus of the CL domain.
[B25] The method of [B22] or [B23], wherein the C-terminus of the VL domain is linked with the N-terminus of the non-native human IgG CH1 domain, and the C-terminus of the VH domain is linked with the N-terminus of the CL domain.
[B26] The method of any one of [B3] to [B25], wherein the antigen-binding molecule further comprises an antibody Fc region.
[B27] The method of [B11] or [B13], wherein the antigen-binding molecule further comprises an antibody Fc region, and the affinity capture medium is an anti-Fc antibody.
[B28] The method of any one of [B3] to [B27], wherein the antigen-binding molecule is a non-native IgG antibody.
[B29] The method of [B28], wherein the non-native IgG antibody is a non-native IgG1 antibody, a non-native IgG2 antibody, a non-native IgG3 antibody, or a non-native IgG4 antibody.
[B30] The method of any one of [B3] to [B29], wherein the antigen-binding molecule is conjugated to a drug moiety.

[C1] A method for detecting each of a plurality of types of peptides in a sample, comprising:
   (a) preparing a sample containing a plurality of types of peptides, wherein
      the plurality of types of peptides consist of an amino acid sequence between positions 121 and 133 according to the EU index in a native or non-native human IgG CH1 domain, and the amino acid sequence is different among the plurality of types of peptides; and
   (b) analyzing the sample prepared in (a) by mass spectrometry to detect each of the plurality of types of peptides.
[C2] A method for quantifying each of a plurality of types of peptides in a sample, comprising:
   (a) preparing a sample containing a plurality of types of peptides, wherein
      the plurality of types of peptides consist of an amino acid sequence between positions 121 and 133 according to the EU index in a native or non-native human IgG CH1 domain, and the amino acid sequence is different among the plurality of types of peptides;
   (b) analyzing the sample prepared in (a) by mass spectrometry to detect each of the plurality of types of peptides; and
   (c) quantifying each of the plurality of types of peptides based on the result of the analysis of (b).
[C3] A method for detecting and/or quantifying each of a plurality of types of antigen-binding molecules in a biological sample, comprising:
   (a) treating a biological sample containing a plurality of types of antigen-binding molecules with a digestive enzyme to generate a plurality of types of peptides from the plurality of types of antigen-binding molecules, wherein
      the plurality of types of antigen-binding molecules comprise antigen-binding molecules comprising an antigen-binding domain, a CL domain, and a native or non-native human IgG CH1 domain,
      the amino acid sequence between positions 121 and 133 according to the EU index in the human IgG CH1 domain of the plurality of types of antigen-binding molecules is different among the plurality of types of antigen-binding molecules,
      the plurality of types of peptides consist of an amino acid sequence between positions 121 and 133 according to the EU index in a native or non-native human IgG CH1 domain, and the amino acid sequence is different among the plurality of types of peptides;
   (b) analyzing the biological sample treated in (a) by mass spectrometry to detect each of the plurality of types of peptides; and
   (c) detecting and/or quantifying each of the plurality or types of antigen-binding molecules based on the result of the analysis of (b).
[C4] The method of any one of [C1] to [C3], wherein the plurality of types of peptides comprise:
   (I) two or more types of peptides which consist of an amino acid sequence between positions 121 and 133 according to the EU index in a non-native human IgG CH1 domain, wherein the amino acid sequence is different from a corresponding sequence in a native human IgG of the same isotype as the non-native human IgG, or
   (II) a peptide consisting of an amino acid sequence between positions 121 and 133 according to the EU index in a native human IgG CH1 domain, and one or more types of peptides consisting of an amino acid sequence between positions 121 and 133 according to the EU index in a non-native human IgG CH1 domain, wherein the amino acid sequence is different from a corresponding sequence in a native human IgG of the same isotype as the non-native human IgG.
[C5] The method of any one of [C1] to [C4], wherein the amino acid sequence between positions 121 and 133 according to the EU index in the non-native human IgG CH1 domain has any one of the following features:
   (1) an amino acid at any one or more sites of positions 122 to 132 is different from an amino acid present at corresponding sites in a native human IgG of the same isotype, and the amino acid at said sites is an amino acid other than Lys, Arg, and Met (provided that the amino acid at position 122 is not Pro);
   (2) an amino acid other than Lys, Arg, and Met has been inserted at one or more sites selected from a site between positions 121 and 122, a site between positions 122 and 123, a site between positions 123 and 124, a site between positions 124 and 125, a site between positions 125 and 126, a site between positions 126 and 127, a site between positions 127 and 128, a site between positions 128 and 129, a site between positions 129 and 130, a site between positions 130 and 131, a site between positions 131 and 132, and a site between positions 132 and 133 (provided that the amino acid at the site between positions 121 and 122 is not Pro).
[C6] The method of any one of [C1] to [C4], wherein the amino acid sequence between positions 121 and 133 according to the EU index in the non-native human IgG CH1 domain has any one of the following features:
   (1) an amino acid at any one or more sites of positions 122 to 128 and positions 130 to 132 is Ala;
   (2) an amino acid at any one or more sites of positions 122 to 132 is Ile;
   (3) an amino acid at any one or more sites of positions 122 to 124 and positions 126 to 132 is Val;
   (4) an amino acid at any one or more sites of positions 122 to 127 and positions 129 to 132 is Leu;
   (5) an amino acid at any one or more sites of positions 122 to 125 and positions 127 to 132 is Phe;
   (6) Ala, Ile, Val, Leu, Phe, Ala-Ala, Ile-Ile, Val-Val, Leu-Leu, Phe-Phe, Ala-Ile, Ala-Val, Ala-Leu, Ala-Phe, Ile-Val, Ile-Leu, Ile-Phe, Val-Leu, Val-Phe, Leu-Phe, or Ala-Ala-Ala has been inserted at one or more sites selected from a site between positions 121 and 122, a site between positions 122 and 123, a site between positions 123 and 124, a site between positions 124 and 125, a site between positions 125 and 126, a site between positions 126 and 127, a site between positions 127 and 128, a site between positions 128 and 129, a site between positions 129 and 130, a site between positions 130 and 131, a site between positions 131 and 132, and a site between positions 132 and 133.
[C7] The method of any one of [C1] to [C4], wherein the amino acid sequence between positions 121 and 133 according to the EU index in the non-native human IgG CH1 domain has any one of the following features:
   (1) an amino acid at any one site of positions 122 to 128 and positions 130 to 132 is Ala;
   (2) an amino acid at any one site of positions 122 to 132 is Ile;
   (3) an amino acid at any one site of positions 122 to 124 and positions 126 to 132 is Val;
   (4) an amino acid at any one site of positions 122 to 127 and positions 129 to 132 is Leu;
   (5) an amino acid at any one site of positions 122 to 125 and positions 127 to 132 is Phe;
   (6) Ala, Ile, Val, Leu, Phe, Ala-Ala, Ile-Ile, Val-Val, Leu-Leu, Phe-Phe, Ala-Ile, Ala-Val, Ala-Leu, Ala-Phe, Ile-Val, Ile-Leu, Ile-Phe, Val-Leu, Val-Phe, Leu-Phe, or Ala-Ala-Ala has been inserted at one site selected from a site between positions 121 and 122, a site between positions 122 and 123, a site between positions 123 and 124, a site between positions 124 and 125, a site between positions 125 and 126, a site between positions 126 and 127, a site between positions 127 and 128, a site between positions 128 and 129, a site between positions 129 and 130, a site between positions 130 and 131, a site between positions 131 and 132, and a site between positions 132 and 133.
[C8] The method of any one of [C1] to [C7], wherein the human IgG CH1 domain is a human IgG1 CH1 domain, a human IgG2 CH1 domain, a human IgG3 CH1 domain, or a human IgG4 CH1 domain.
[C9] The method of any one of [C1] to [C7], wherein the plurality of types of peptides comprise two or more types of peptides consisting of an amino acid sequence between positions 121 and 133 according to the EU index in a non-native human IgG CH1 domain, wherein the amino acid sequence between positions 121 and 133 according to the EU index in the non-native human IgG CH1 domain is selected from the group consisting of SEQ ID NOs: 6-294 and 1565-1833.
[C10] The method of any one of [C1] to [C7], wherein the plurality of types of peptides comprise two or more types of peptides consisting of an amino acid sequence between positions 121 and 133 according to the EU index in a non-native human IgG CH1 domain, wherein the amino acid sequence between positions 121 and 133 according to the EU index in the non-native human IgG CH1 domain is selected from the group consisting of SEQ ID NOs: 6-294.
[C11] The method of any one of [C1] to [C7], wherein the plurality of types of peptides comprise two or more types of peptides consisting of an amino acid sequence between positions 121 and 133 according to the EU index in a non-native human IgG CH1 domain, wherein the amino acid sequence between positions 121 and 133 according to the EU index in the non-native human IgG CH1 domain is selected from the group consisting of SEQ ID NOs: 1565-1833.
[C12] The method of any one of [C1] to [C7], wherein the plurality of types of peptides comprise a peptide consisting of an amino acid sequence between positions 121 and 133 according to the EU index in a native human IgG CH1 domain, and one or more types of peptides consisting of an amino acid sequence between positions 121 and 133 according to the EU index in a non-native human IgG CH1 domain, wherein
   when the amino acid sequence between positions 121 and 133 according to the EU index in the native human IgG CH1 domain is SEQ ID NO: 5, the amino acid sequence between positions 121 and 133 according to the EU index in the non-native human IgG CH1 domain is selected from the group consisting of SEQ ID NOs: 6-294, and
   when the amino acid sequence between positions 121 and 133 according to the EU index in the native human IgG CH1 domain is SEQ ID NO: 945, the amino acid sequence between positions 121 and 133 according to the EU index in the non-native human IgG CH1 domain is selected from the group consisting of SEQ ID NOs: 1565-1833.
[C13] The method of any one of [C1] to [C7], wherein the plurality of types of peptides are two or more types of peptides selected from the group consisting of (1) to (26) below:
   (1) a peptide consisting of the amino acid sequence of SEQ ID NO: 5;
   (2) a peptide consisting of the amino acid sequence of SEQ ID NO: 6;
   (3) a peptide consisting of the amino acid sequence of SEQ ID NO: 7;
   (4) a peptide consisting of the amino acid sequence of SEQ ID NO: 8;
   (5) a peptide consisting of the amino acid sequence of SEQ ID NO: 9;
   (6) a peptide consisting of the amino acid sequence of SEQ ID NO: 10;
   (7) a peptide consisting of the amino acid sequence of SEQ ID NO: 12;
   (8) a peptide consisting of the amino acid sequence of SEQ ID NO: 13;
   (9) a peptide consisting of the amino acid sequence of SEQ ID NO: 14;
   (10) a peptide consisting of the amino acid sequence of SEQ ID NO: 17;
   (11) a peptide consisting of the amino acid sequence of SEQ ID NO: 24;
   (12) a peptide consisting of the amino acid sequence of SEQ ID NO: 27;
   (13) a peptide consisting of the amino acid sequence of SEQ ID NO: 36;
   (14) a peptide consisting of the amino acid sequence of SEQ ID NO: 38;
   (15) a peptide consisting of the amino acid sequence of SEQ ID NO: 40;
   (16) a peptide consisting of the amino acid sequence of SEQ ID NO: 42;
   (17) a peptide consisting of the amino acid sequence of SEQ ID NO: 45;
   (18) a peptide consisting of the amino acid sequence of SEQ ID NO: 46;
   (19) a peptide consisting of the amino acid sequence of SEQ ID NO: 47;
   (20) a peptide consisting of the amino acid sequence of SEQ ID NO: 73;
   (21) a peptide consisting of the amino acid sequence of SEQ ID NO: 75;
   (22) a peptide consisting of the amino acid sequence of SEQ ID NO: 76;
   (23) a peptide consisting of the amino acid sequence of SEQ ID NO: 78;
   (24) a peptide consisting of the amino acid sequence of SEQ ID NO: 79;
   (25) a peptide consisting of the amino acid sequence of SEQ ID NO: 80; and
   (26) a peptide consisting of the amino acid sequence of SEQ ID NO: 82.
[C14] The method of any one of [C1] to [C7], wherein the plurality of types of peptides are two or more types of peptides selected from the group consisting of (1) to (13) below:
   (1) a peptide consisting of the amino acid sequence of SEQ ID NO: 5;
   (2) a peptide consisting of the amino acid sequence of SEQ ID NO: 6;
   (3) a peptide consisting of the amino acid sequence of SEQ ID NO: 7;
   (4) a peptide consisting of the amino acid sequence of SEQ ID NO: 8;
   (5) a peptide consisting of the amino acid sequence of SEQ ID NO: 9;
   (6) a peptide consisting of the amino acid sequence of SEQ ID NO: 10;
   (7) a peptide consisting of the amino acid sequence of SEQ ID NO: 12;
   (8) a peptide consisting of the amino acid sequence of SEQ ID NO: 13;
   (9) a peptide consisting of the amino acid sequence of SEQ ID NO: 14;
   (10) a peptide consisting of the amino acid sequence of SEQ ID NO: 17;
   (11) a peptide consisting of the amino acid sequence of SEQ ID NO: 24;
   (12) a peptide consisting of the amino acid sequence of SEQ ID NO: 27; and
   (13) a peptide consisting of the amino acid sequence of SEQ ID NO: 36.
[C15] The method of any one of [C1] to [C7], wherein the plurality of types of peptides are two types of peptides, and the two types of peptides are selected from the group consisting of (1) to (15) below:
   (1) a peptide consisting of the amino acid sequence of SEQ ID NO: 5 and a peptide consisting of the amino acid sequence of SEQ ID NO: 7;
   (2) a peptide consisting of the amino acid sequence of SEQ ID NO: 5 and a peptide consisting of the amino acid sequence of SEQ ID NO: 12;
   (3) a peptide consisting of the amino acid sequence of SEQ ID NO: 5 and a peptide consisting of the amino acid sequence of SEQ ID NO: 10;
   (4) a peptide consisting of the amino acid sequence of SEQ ID NO: 5 and a peptide consisting of the amino acid sequence of SEQ ID NO: 17;
   (5) a peptide consisting of the amino acid sequence of SEQ ID NO: 7 and a peptide consisting of the amino acid sequence of SEQ ID NO: 12;
   (6) a peptide consisting of the amino acid sequence of SEQ ID NO: 7 and a peptide consisting of the amino acid sequence of SEQ ID NO: 10;
   (7) a peptide consisting of the amino acid sequence of SEQ ID NO: 7 and a peptide consisting of the amino acid sequence of SEQ ID NO: 17;
   (8) a peptide consisting of the amino acid sequence of SEQ ID NO: 12 and a peptide consisting of the amino acid sequence of SEQ ID NO: 10;
   (9) a peptide consisting of the amino acid sequence of SEQ ID NO: 12 and a peptide consisting of the amino acid sequence of SEQ ID NO: 17;
   (10) a peptide consisting of the amino acid sequence of SEQ ID NO: 10 and a peptide consisting of the amino acid sequence of SEQ ID NO: 17;
   (11) a peptide consisting of the amino acid sequence of SEQ ID NO: 5 and a peptide consisting of the amino acid sequence of SEQ ID NO: 27;
   (12) a peptide consisting of the amino acid sequence of SEQ ID NO: 7 and a peptide consisting of the amino acid sequence of SEQ ID NO: 27;
   (13) a peptide consisting of the amino acid sequence of SEQ ID NO: 10 and a peptide consisting of the amino acid sequence of SEQ ID NO: 27;
   (14) a peptide consisting of the amino acid sequence of SEQ ID NO: 12 and a peptide consisting of the amino acid sequence of SEQ ID NO: 27; and
   (15) a peptide consisting of the amino acid sequence of SEQ ID NO: 17 and a peptide consisting of the amino acid sequence of SEQ ID NO: 27.
[C16] The method of any one of [C1] to [C7], wherein the plurality of types of peptides are three types of peptides, and the three types of peptides are selected from the group consisting of (1) to (20) below:
   (1) a peptide consisting of the amino acid sequence of SEQ ID NO: 5, a peptide consisting of the amino acid sequence of SEQ ID NO: 7, and a peptide consisting of the amino acid sequence of SEQ ID NO: 12;
   (2) a peptide consisting of the amino acid sequence of SEQ ID NO: 5, a peptide consisting of the amino acid sequence of SEQ ID NO: 7, and a peptide consisting of the amino acid sequence of SEQ ID NO: 10;
   (3) a peptide consisting of the amino acid sequence of SEQ ID NO: 5, a peptide consisting of the amino acid sequence of SEQ ID NO: 7, and a peptide consisting of the amino acid sequence of SEQ ID NO: 17;
   (4) a peptide consisting of the amino acid sequence of SEQ ID NO: 5, a peptide consisting of the amino acid sequence of SEQ ID NO: 12, and a peptide consisting of the amino acid sequence of SEQ ID NO: 10;
   (5) a peptide consisting of the amino acid sequence of SEQ ID NO: 5, a peptide consisting of the amino acid sequence of SEQ ID NO: 12, and a peptide consisting of the amino acid sequence of SEQ ID NO: 17;
   (6) a peptide consisting of the amino acid sequence of SEQ ID NO: 5, a peptide consisting of the amino acid sequence of SEQ ID NO: 10, and a peptide consisting of the amino acid sequence of SEQ ID NO: 17;
   (7) a peptide consisting of the amino acid sequence of SEQ ID NO: 7, a peptide consisting of the amino acid sequence of SEQ ID NO: 12, and a peptide consisting of the amino acid sequence of SEQ ID NO: 10;
   (8) a peptide consisting of the amino acid sequence of SEQ ID NO: 7, a peptide consisting of the amino acid sequence of SEQ ID NO: 12, and a peptide consisting of the amino acid sequence of SEQ ID NO: 17;
   (9) a peptide consisting of the amino acid sequence of SEQ ID NO: 12, a peptide consisting of the amino acid sequence of SEQ ID NO: 10, and a peptide consisting of the amino acid sequence of SEQ ID NO: 17;
   (10) a peptide consisting of the amino acid sequence of SEQ ID NO: 7, a peptide consisting of the amino acid sequence of SEQ ID NO: 10, and a peptide consisting of the amino acid sequence of SEQ ID NO: 17;
   (11) a peptide consisting of the amino acid sequence of SEQ ID NO: 5, a peptide consisting of the amino acid sequence of SEQ ID NO: 7, and a peptide consisting of the amino acid sequence of SEQ ID NO: 27;
   (12) a peptide consisting of the amino acid sequence of SEQ ID NO: 5, a peptide consisting of the amino acid sequence of SEQ ID NO: 12, and a peptide consisting of the amino acid sequence of SEQ ID NO: 27;
   (13) a peptide consisting of the amino acid sequence of SEQ ID NO: 5, a peptide consisting of the amino acid sequence of SEQ ID NO: 10, and a peptide consisting of the amino acid sequence of SEQ ID NO: 27;
   (14) a peptide consisting of the amino acid sequence of SEQ ID NO: 5, a peptide consisting of the amino acid sequence of SEQ ID NO: 17, and a peptide consisting of the amino acid sequence of SEQ ID NO: 27;
   (15) a peptide consisting of the amino acid sequence of SEQ ID NO: 7, a peptide consisting of the amino acid sequence of SEQ ID NO: 10, and a peptide consisting of the amino acid sequence of SEQ ID NO: 27;
   (16) a peptide consisting of the amino acid sequence of SEQ ID NO: 7, a peptide consisting of the amino acid sequence of SEQ ID NO: 12, and a peptide consisting of the amino acid sequence of SEQ ID NO: 27;
   (17) a peptide consisting of the amino acid sequence of SEQ ID NO: 7, a peptide consisting of the amino acid sequence of SEQ ID NO: 17, and a peptide consisting of the amino acid sequence of SEQ ID NO: 27;
   (18) a peptide consisting of the amino acid sequence of SEQ ID NO: 12, a peptide consisting of the amino acid sequence of SEQ ID NO: 10, and a peptide consisting of the amino acid sequence of SEQ ID NO: 27;
   (19) a peptide consisting of the amino acid sequence of SEQ ID NO: 10, a peptide consisting of the amino acid sequence of SEQ ID NO: 17, and a peptide consisting of the amino acid sequence of SEQ ID NO: 27; and
   (20) a peptide consisting of the amino acid sequence of SEQ ID NO: 12, a peptide consisting of the amino acid sequence of SEQ ID NO: 17, and a peptide consisting of the amino acid sequence of SEQ ID NO: 27.
[C17] The method of any one of [C1] to [C7], wherein the plurality of types of peptides are four types of peptides, and the four types of peptides are selected from the group consisting of (1) to (15) below:
   (1) a peptide consisting of the amino acid sequence of SEQ ID NO: 5, a peptide consisting of the amino acid sequence of SEQ ID NO: 7, a peptide consisting of the amino acid sequence of SEQ ID NO: 12, and a peptide consisting of the amino acid sequence of SEQ ID NO: 10;
   (2) a peptide consisting of the amino acid sequence of SEQ ID NO: 5, a peptide consisting of the amino acid sequence of SEQ ID NO: 7, a peptide consisting of the amino acid sequence of SEQ ID NO: 12, and a peptide consisting of the amino acid sequence of SEQ ID NO: 17;
   (3) a peptide consisting of the amino acid sequence of SEQ ID NO: 5, a peptide consisting of the amino acid sequence of SEQ ID NO: 12, a peptide consisting of the amino acid sequence of SEQ ID NO: 10, and a peptide consisting of the amino acid sequence of SEQ ID NO: 17;
   (4) a peptide consisting of the amino acid sequence of SEQ ID NO: 5, a peptide consisting of the amino acid sequence of SEQ ID NO: 7, a peptide consisting of the amino acid sequence of SEQ ID NO: 10, and a peptide consisting of the amino acid sequence of SEQ ID NO: 17;
   (5) a peptide consisting of the amino acid sequence of SEQ ID NO: 7, a peptide consisting of the amino acid sequence of SEQ ID NO: 12, a peptide consisting of the amino acid sequence of SEQ ID NO: 10, and a peptide consisting of the amino acid sequence of SEQ ID NO: 17;
   (6) a peptide consisting of the amino acid sequence of SEQ ID NO: 5, a peptide consisting of the amino acid sequence of SEQ ID NO: 7, a peptide consisting of the amino acid sequence of SEQ ID NO: 10, and a peptide consisting of the amino acid sequence of SEQ ID NO: 27;
   (7) a peptide consisting of the amino acid sequence of SEQ ID NO: 5, a peptide consisting of the amino acid sequence of SEQ ID NO: 7, a peptide consisting of the amino acid sequence of SEQ ID NO: 12, and a peptide consisting of the amino acid sequence of SEQ ID NO: 27;
   (8) a peptide consisting of the amino acid sequence of SEQ ID NO: 5, a peptide consisting of the amino acid sequence of SEQ ID NO: 7, a peptide consisting of the amino acid sequence of SEQ ID NO: 17, and a peptide consisting of the amino acid sequence of SEQ ID NO: 27;
   (9) a peptide consisting of the amino acid sequence of SEQ ID NO: 5, a peptide consisting of the amino acid sequence of SEQ ID NO: 10, a peptide consisting of the amino acid sequence of SEQ ID NO: 12, and a peptide consisting of the amino acid sequence of SEQ ID NO: 27;
   (10) a peptide consisting of the amino acid sequence of SEQ ID NO: 5, a peptide consisting of the amino acid sequence of SEQ ID NO: 10, a peptide consisting of the amino acid sequence of SEQ ID NO: 17, and a peptide consisting of the amino acid sequence of SEQ ID NO: 27;
   (11) a peptide consisting of the amino acid sequence of SEQ ID NO: 5, a peptide consisting of the amino acid sequence of SEQ ID NO: 12, a peptide consisting of the amino acid sequence of SEQ ID NO: 17, and a peptide consisting of the amino acid sequence of SEQ ID NO: 27;
   (12) a peptide consisting of the amino acid sequence of SEQ ID NO: 7, a peptide consisting of the amino acid sequence of SEQ ID NO: 10, a peptide consisting of the amino acid sequence of SEQ ID NO: 12, and a peptide consisting of the amino acid sequence of SEQ ID NO: 27;
   (13) a peptide consisting of the amino acid sequence of SEQ ID NO: 7, a peptide consisting of the amino acid sequence of SEQ ID NO: 10, a peptide consisting of the amino acid sequence of SEQ ID NO: 17, and a peptide consisting of the amino acid sequence of SEQ ID NO: 27;
   (14) a peptide consisting of the amino acid sequence of SEQ ID NO: 7, a peptide consisting of the amino acid sequence of SEQ ID NO: 12, a peptide consisting of the amino acid sequence of SEQ ID NO: 17, and a peptide consisting of the amino acid sequence of SEQ ID NO: 27; and
   (15) a peptide consisting of the amino acid sequence of SEQ ID NO: 10, a peptide consisting of the amino acid sequence of SEQ ID NO: 12, a peptide consisting of the amino acid sequence of SEQ ID NO: 17, and a peptide consisting of the amino acid sequence of SEQ ID NO: 27.
[C18] The method of any one of [C1] to [C7], wherein the plurality of types of peptides are five types of peptides, and the five types of peptides are selected from the group consisting of (1) to (6) below:
   (1) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, and a peptide consisting of the amino acid sequence of SEQ ID NO: 17;
   (2) a peptide consisting of the amino acid sequence of SEQ ID NO: 5, a peptide consisting of the amino acid sequence of SEQ ID NO: 7, a peptide consisting of the amino acid sequence of SEQ ID NO: 10, a peptide consisting of the amino acid sequence of SEQ ID NO: 12, and a peptide consisting of the amino acid sequence of SEQ ID NO: 27;
   (3) a peptide consisting of the amino acid sequence of SEQ ID NO: 5, a peptide consisting of the amino acid sequence of SEQ ID NO: 7, a peptide consisting of the amino acid sequence of SEQ ID NO: 10, a peptide consisting of the amino acid sequence of SEQ ID NO: 17, and a peptide consisting of the amino acid sequence of SEQ ID NO: 27;
   (4) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 17, and a peptide consisting of the amino acid sequence of SEQ ID NO: 27;
   (5) a peptide consisting of the amino acid sequence of SEQ ID NO: 5, a peptide consisting of the amino acid sequence of SEQ ID NO: 10, a peptide consisting of the amino acid sequence of SEQ ID NO: 12, a peptide consisting of the amino acid sequence of SEQ ID NO: 17, and a peptide consisting of the amino acid sequence of SEQ ID NO: 27; and
   (6) a peptide consisting of the amino acid sequence of SEQ ID NO: 7, a peptide consisting of the amino acid sequence of SEQ ID NO: 10, a peptide consisting of the amino acid sequence of SEQ ID NO: 12, a peptide consisting of the amino acid sequence of SEQ ID NO: 17, and a peptide consisting of the amino acid sequence of SEQ ID NO: 27.
[C19] The method of any one of [C1] to [C7], wherein the plurality of types of peptides are the following six types of peptides:
   (1) a peptide consisting of the amino acid sequence of SEQ ID NO: 5;
   (2) a peptide consisting of the amino acid sequence of SEQ ID NO: 7;
   (3) a peptide consisting of the amino acid sequence of SEQ ID NO: 12;
   (4) a peptide consisting of the amino acid sequence of SEQ ID NO: 10;
   (5) a peptide consisting of the amino acid sequence of SEQ ID NO: 17; and
   (6) a peptide consisting of SEQ ID NO: 27.
[C20] The method of any one of [C1] to [C7], wherein the plurality of types of peptides are seven types of peptides, and the seven types of peptides are selected from the group consisting of (1) to (4) below:
   (1) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, and a peptide consisting of the amino acid sequence of SEQ ID NO: 13;
   (2) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, and a peptide consisting of the amino acid sequence of SEQ ID NO: 14;
   (3) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, and a peptide consisting of the amino acid sequence of SEQ ID NO: 24; and
   (4) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, and a peptide consisting of the amino acid sequence of SEQ ID NO: 36.
[C21] The method of any one of [C1] to [C7], wherein the plurality of types of peptides are eight types of peptides, and the eight types of peptides are selected from the group consisting of (1) to (6) below:
   (1) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, and a peptide consisting of the amino acid sequence of SEQ ID NO: 14;
   (2) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, and a peptide consisting of the amino acid sequence of SEQ ID NO: 24;
   (3) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, and a peptide consisting of the amino acid sequence of SEQ ID NO: 36;
   (4) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 14, and a peptide consisting of the amino acid sequence of SEQ ID NO: 24;
   (5) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 14, and a peptide consisting of the amino acid sequence of SEQ ID NO: 36; and
   (6) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 24, and a peptide consisting of the amino acid sequence of SEQ ID NO: 36.
[C22] The method of any one of [C1] to [C7], wherein the plurality of types of peptides are nine types of peptides, and the nine types of peptides are selected from the group consisting of (1) to (4) below:
   (1) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, a peptide consisting of the amino acid sequence of SEQ ID NO: 14, and a peptide consisting of the amino acid sequence of SEQ ID NO: 24;
   (2) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, a peptide consisting of the amino acid sequence of SEQ ID NO: 14, and a peptide consisting of the amino acid sequence of SEQ ID NO: 36;
   (3) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, a peptide consisting of the amino acid sequence of SEQ ID NO: 24, and a peptide consisting of the amino acid sequence of SEQ ID NO: 36; and
   (4) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 14, a peptide consisting of the amino acid sequence of SEQ ID NO: 24, and a peptide consisting of the amino acid sequence of SEQ ID NO: 36.
[C23] The method of any one of [C1] to [C7], wherein the plurality of types of peptides are the following ten types of peptides:
   (1) a peptide consisting of the amino acid sequence of SEQ ID NO: 5;
   (2) a peptide consisting of the amino acid sequence of SEQ ID NO: 7;
   (3) a peptide consisting of the amino acid sequence of SEQ ID NO: 12;
   (4) a peptide consisting of the amino acid sequence of SEQ ID NO: 10;
   (5) a peptide consisting of the amino acid sequence of SEQ ID NO: 17;
   (6) a peptide consisting of the amino acid sequence of SEQ ID NO: 27;
   (7) a peptide consisting of the amino acid sequence of SEQ ID NO: 13;
   (8) a peptide consisting of the amino acid sequence of SEQ ID NO: 14;
   (9) a peptide consisting of the amino acid sequence of SEQ ID NO: 24; and
   (10) a peptide consisting of the amino acid sequence of SEQ ID NO: 36.
[C24] The method of any one of [C1] to [C7], wherein the plurality of types of peptides are eleven types of peptides, and the eleven types of peptides are selected from the group consisting of (1) to (2) below:
   (1) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, a peptide consisting of the amino acid sequence of SEQ ID NO: 14, a peptide consisting of the amino acid sequence of SEQ ID NO: 24, a peptide consisting of the amino acid sequence of SEQ ID NO: 36, and a peptide consisting of the amino acid sequence of SEQ ID NO: 6; and
   (2) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, a peptide consisting of the amino acid sequence of SEQ ID NO: 14, a peptide consisting of the amino acid sequence of SEQ ID NO: 24, a peptide consisting of the amino acid sequence of SEQ ID NO: 36, and a peptide consisting of the amino acid sequence of SEQ ID NO: 8.
[C25] The method of any one of [C1] to [C7], wherein the plurality of types of peptides are the following twelve types of peptides:
   (1) a peptide consisting of the amino acid sequence of SEQ ID NO: 5;
   (2) a peptide consisting of the amino acid sequence of SEQ ID NO: 6;
   (3) a peptide consisting of the amino acid sequence of SEQ ID NO: 7;
   (4) a peptide consisting of the amino acid sequence of SEQ ID NO: 8;
   (5) a peptide consisting of the amino acid sequence of SEQ ID NO: 10;
   (6) a peptide consisting of the amino acid sequence of SEQ ID NO: 12;
   (7) a peptide consisting of the amino acid sequence of SEQ ID NO: 13;
   (8) a peptide consisting of the amino acid sequence of SEQ ID NO: 14;
   (9) a peptide consisting of the amino acid sequence of SEQ ID NO: 17;
   (10) a peptide consisting of the amino acid sequence of SEQ ID NO: 24;
   (11) a peptide consisting of the amino acid sequence of SEQ ID NO: 27; and
   (12) a peptide consisting of the amino acid sequence of SEQ ID NO: 36.
[C26] The method of any one of [C3] to [C25], wherein the digestive enzyme is a protease.
[C27] The method of any one of [C3] to [C25], wherein the digestive enzyme is trypsin.
[C28] The method of any one of [C3] to [C27], wherein the step (b) comprises contacting the biological sample treated in the step (a) with an affinity capture medium or a chromatographic adsorbent and eluting a biological sample enriched with the peptide, and analyzing the enriched biological sample by mass spectrometry.
[C29] The method of [C1] or [C2], wherein the step (b) comprises contacting the sample prepared in the step (a) with an affinity capture medium or a chromatographic adsorbent and eluting a sample enriched with the peptide, and analyzing the enriched sample by mass spectrometry.
[C30] The method of any one of [C3] to [C27], wherein the step (a) comprises contacting the biological sample with an affinity capture medium or a chromatographic adsorbent and eluting a biological sample enriched with the antigen-binding molecule, and treating the enriched biological sample with a digestive enzyme.
[C31] The method of any one of [C28] to [C30], wherein the affinity capture medium is Protein A or Protein G.
[C32] The method of any one of [C28] to [C30], wherein the chromatographic adsorbent is a solid phase extraction (SPE) adsorbent.
[C33] The method of any one of [C1] to [C27], wherein the step (b) comprises analyzing the biological sample treated in the step (a) or the sample prepared in the step (a) by mass spectrometry in combination with liquid chromatography.
[C34] The method of any one of [C1] to [C27], wherein the step (b) comprises separating a fraction containing the peptide from the biological sample treated in the step (a) or the sample prepared in the step (a) by liquid chromatography, and analyzing the separated fraction by mass spectrometry.
[C35] The method of any one of [C3] to [C27], wherein the step (a) comprises separating a fraction containing the antigen-binding molecule from the biological sample by liquid chromatography, and treating the separated fraction with a digestive enzyme.
[C36] The method of any one of [C1] to [C35], wherein the mass spectrometry is performed by tandem mass spectrometry or multiple-stage mass spectrometry.
[C37] The method of any one of [C1] to [C35], wherein the mass spectrometry is performed by tandem mass spectrometry or multiple-stage mass spectrometry in a selected reaction monitoring (SRM) mode.
[C38] The method of any one of [C3] to [C37], wherein the biological sample is a serum, plasma, a tissue, or a cell from an animal treated with the antigen-binding molecule.
[C39] The method of any one of [C3] to [C38], wherein the antigen-binding domain comprises a VH domain and a VL domain.
[C40] The method of [C39], wherein the VH domain and the VL domain are a humanized VH domain and a humanized VL domain.
[C41] The method of [C39] or [C40], wherein the C-terminus of the VH domain is linked with the N-terminus of the non-native human IgG CH1 domain, and the C-terminus of the VL domain is linked with the N-terminus of the CL domain.
[C42] The method of [C39] or [C40], wherein the C-terminus of the VL domain is linked with the N-terminus of the non-native human IgG CH1 domain, and the C-terminus of the VH domain is linked with the N-terminus of the CL domain.
[C43] The method of any one of [C3] to [C42], wherein the antigen-binding molecule further comprises an antibody Fc region.
[C44] The method of [C28] or [C30], wherein the antigen-binding molecule further comprises an antibody Fc region, and the affinity capture medium is an anti-Fc antibody.
[C45] The antigen-binding molecule of any one of [C3] to [C44], wherein the antigen-binding molecule is a non-native IgG antibody.
[C46] The method of [C45], wherein the non-native IgG antibody is a non-native IgG1 antibody, a non-native IgG2 antibody, a non-native IgG3 antibody, or a non-native IgG4 antibody.
[C47] The method of any one of [C3] to [C46], wherein the antigen-binding molecule is conjugated to a drug moiety.

[D1] A method for designing, selecting, or producing a modified antigen-binding molecule comprising an artificially modified peptide portion that is detectable by mass spectrometry, wherein the method comprises:
   (I) identifying a peptide which is obtained after treating one antigen-binding molecule with a digestive enzyme and detectable by mass spectrometry;
   (II) designing an artificially modified peptide by any of the following:
      (II-1) deleting or substituting one or more amino acid residues in the peptide identified in (I) with other amino acid residues;
      (II-2) inserting one or more amino acid residues into the peptide identified in (I);
      (II-3) adding an amino acid residue to the N-terminus or C-terminus of the peptide identified in (I);
      (II-4) a combination of (II-1) and (II-2);
      (II-5) a combination of (II-1) and (II-3);
      (II-6) a combination of (II-2) and (II-3);
      (II-7) a combination of (II-1), (II-2), and (II-3);
   (III) performing the modification of (II) on the peptide portion identified in (I) in the antigen-binding molecule of (I) to produce a modified antigen-binding molecule;
   (IV) selecting the modified antigen-binding molecule produced in (III) if the modified antigen-binding molecule satisfies any of the following:
      (IV-1) the Tm value of the modified antigen-binding molecule is 50°C or higher;
      (IV-2) the antigen-binding activity of the modified antigen-binding molecule is 10% or more of the antigen-binding activity of the antigen-binding molecule of (I);
      (IV-3) both (IV-1) and (IV-2) above;
   (V) selecting the modified antigen-binding molecule selected in (IV) if a portion contained in the modified antigen-binding molecule that corresponds to the peptide designed in (II) is detectable by mass spectrometry after treating a biological sample containing the modified antigen-binding molecule with the digestive enzyme of (I).
[D2] The method of [D1], which further comprises:
   (VI) treating a biological sample containing the antigen-binding molecule of (I) with the digestive enzyme of (I), and then performing a pharmacokinetic test utilizing mass spectrometry to obtain a pharmacokinetic profile or a pharmacokinetic parameter;
   (VII) treating a biological sample containing the modified antigen-binding molecule selected in
   (V) with the digestive enzyme of (I), and then performing a pharmacokinetic test utilizing mass spectrometry to obtain a pharmacokinetic profile or a pharmacokinetic parameter; and
   (VIII) selecting the modified antigen-binding molecule if the pharmacokinetic profile or pharmacokinetic parameter obtained in (VI) is comparable to the pharmacokinetic profile or pharmacokinetic parameter obtained in (VII).
[D3] The method of [D1] or [D2], wherein, in the step (I), the antigen-binding molecule comprises an antigen-binding domain, a CL domain, and a human IgG CH1 domain, and the peptide detectable by mass spectrometry is derived from the human IgG CH1 domain.
[D4] The method of [D3], wherein the peptide detectable by mass spectrometry consists of an amino acid sequence from positions 120 to 133 according to the EU index in a human IgG CH1 domain.
[D5] The method of [D3], wherein the peptide detectable by mass spectrometry consists of an amino acid sequence between positions 121 and 133 according to the EU index in a human IgG CH1 domain.
[D6] The method of any one of [D1] to [D5], wherein the other amino acid residues in (II-1) are Ala, Ile, Val, Leu, or Phe.
[D7] The method of any one of [D1] to [D6], wherein, in (II-2), the amino acid residues inserted are one, two, or three residues.
[D8] The method of any one of [D1] to [D7], wherein, in (II-2), the amino acid residues inserted are Ala, Ile, Val, Leu, Phe, Ala-Ala, Ile-Ile, Val-Val, Leu-Leu, Phe-Phe, Ala-Ile, Ala-Val, Ala-Leu, Ala-Phe, Ile-Val, Ile-Leu, Ile-Phe, Val-Leu, Val-Phe, Leu-Phe, or Ala-Ala-Ala.
[D9] The method of any one of [D1] to [D7], wherein, in (II-2), the amino acid residues are inserted at one, two, or three sites in the peptides identified in (I).
[D10] The method of any one of [D1] to [D9], wherein, in (II-3), the amino acid residues added are one, two, or three residues.
[D11] The method of any one of [D1] to [D10], wherein the peptide designed in (II) does not comprise a site cleaved by the digestive enzyme of (I).
[D12] The method of any one of [D1] to [D11], wherein the digestive enzyme is a protease.
[D13] The method of any one of [D1] to [D11], wherein the digestive enzyme is trypsin.
[D14] The method of any one of [D1] to [D13], wherein the mass spectrometry is mass spectrometry combined with liquid chromatography.
[D15] The method of any one of [D1] to [D14], wherein the mass spectrometry is mass spectrometry by tandem mass spectrometry.
[D16] The method of any one of [D1] to [D15], wherein the biological sample in the steps (V) and (VII) is a serum, plasma, a tissue, or a cell from an animal treated with the modified antigen-binding molecule, and the biological sample in the step (VI) is a serum, plasma, a tissue, or a cell from an animal treated with the antigen-binding molecule.
[D17] The method of any one of [D1] to [D16], wherein the antigen-binding molecule of the step (I) comprises an antigen-binding domain, a CL domain, and a human IgG CH1 domain, and the antigen-binding domain comprises a VH domain and a VL domain.
[D18] The method of [D17], wherein the VH domain and the VL domain are a humanized VH domain and a humanized VL domain.
[D19] The method of [D17] or [D18], wherein the C-terminus of the VH domain is linked with the N-terminus of the non-native human IgG CH1 domain, and the C-terminus of the VL domain is linked with the N-terminus of the CL domain.
[D20] The method of [D17] or [D18], wherein the C-terminus of the VL domain is linked with the N-terminus of the non-native human IgG CH1 domain, and the C-terminus of the VH domain is linked with the N-terminus of the CL domain.
[D21] The method of any one of [D1] to [D20], wherein the antigen-binding molecule of the step (I) further comprises an antibody Fc region.
[D22] The method of any one of [D1] to [D21], wherein the antigen-binding molecule of the step (I) is an IgG antibody.
[D23] The method of [D22], wherein the IgG antibody is an IgG1 antibody, an IgG2 antibody, an IgG3 antibody, or an IgG4 antibody.
[D24] The method of any one of [D1] to [D23], wherein the antigen-binding molecule of the step (I) is conjugated to a drug moiety.

[E1] A modified antigen-binding molecule which is designed, selected, or produced by the method of any one of [D1] to [D24].
[E2] A peptide which is designed by step (II) in the method of any one of [D1] to [D24].

[F1] A peptide consisting of an amino acid sequence between positions 121 and 133 according to the EU index in a non-native human IgG CH1 domain, wherein the amino acid sequence is different from a corresponding sequence in a native human IgG of the same isotype as the non-native human IgG.
[F2] The peptide of [F1], wherein the amino acid sequence between positions 121 and 133 according to the EU index in the non-native human IgG CH1 domain has any one of the following features:
   (1) an amino acid at any one or more sites of positions 122 to 132 is different from an amino acid present at corresponding sites in a native human IgG of the same isotype, and the amino acid at said sites is an amino acid other than Lys, Arg, and Met (provided that the amino acid at position 122 is not Pro);
   (2) an amino acid other than Lys, Arg, and Met has been inserted at one or more sites selected from a site between positions 121 and 122, a site between positions 122 and 123, a site between positions 123 and 124, a site between positions 124 and 125, a site between positions 125 and 126, a site between positions 126 and 127, a site between positions 127 and 128, a site between positions 128 and 129, a site between positions 129 and 130, a site between positions 130 and 131, a site between positions 131 and 132, and a site between positions 132 and 133 (provided that the amino acid at the site between positions 121 and 122 is not Pro).
[F3] The peptide of [F1] or [F2], wherein the amino acid sequence between positions 121 and 133 according to the EU index in the non-native human IgG CH1 domain has any one of the following features:
   (1) an amino acid at any one or more sites of positions 122 to 128 and positions 130 to 132 is Ala;
   (2) an amino acid at any one or more sites of positions 122 to 132 is Ile;
   (3) an amino acid at any one or more sites of positions 122 to 124 and positions 126 to 132 is Val;
   (4) an amino acid at any one or more sites of positions 122 to 127 and positions 129 to 132 is Leu;
   (5) an amino acid at any one or more sites of positions 122 to 125 and positions 127 to 132 is Phe;
   (6) Ala, Ile, Val, Leu, Phe, Ala-Ala, Ile-Ile, Val-Val, Leu-Leu, Phe-Phe, Ala-Ile, Ala-Val, Ala-Leu, Ala-Phe, Ile-Val, Ile-Leu, Ile-Phe, Val-Leu, Val-Phe, Leu-Phe, or Ala-Ala-Ala has been inserted at one or more sites selected from a site between positions 121 and 122, a site between positions 122 and 123, a site between positions 123 and 124, a site between positions 124 and 125, a site between positions 125 and 126, a site between positions 126 and 127, a site between positions 127 and 128, a site between positions 128 and 129, a site between positions 129 and 130, a site between positions 130 and 131, a site between positions 131 and 132, and a site between positions 132 and 133.
[F4] The peptide of [F1] or [F2], wherein the amino acid sequence between positions 121 and 133 according to the EU index in the non-native human IgG CH1 domain has any one of the following features:
   (1) an amino acid at any one site of positions 122 to 128 and positions 130 to 132 is Ala;
   (2) an amino acid at any one site of positions 122 to 132 is Ile;
   (3) an amino acid at any one site of positions 122 to 124 and positions 126 to 132 is Val;
   (4) an amino acid at any one site of positions 122 to 127 and positions 129 to 132 is Leu;
   (5) an amino acid at any one site of positions 122 to 125 and positions 127 to 132 is Phe;
   (6) Ala, Ile, Val, Leu, Phe, Ala-Ala, Ile-Ile, Val-Val, Leu-Leu, Phe-Phe, Ala-Ile, Ala-Val, Ala-Leu, Ala-Phe, Ile-Val, Ile-Leu, Ile-Phe, Val-Leu, Val-Phe, Leu-Phe, or Ala-Ala-Ala has been inserted at one site selected from a site between positions 121 and 122, a site between positions 122 and 123, a site between positions 123 and 124, a site between positions 124 and 125, a site between positions 125 and 126, a site between positions 126 and 127, a site between positions 127 and 128, a site between positions 128 and 129, a site between positions 129 and 130, a site between positions 130 and 131, a site between positions 131 and 132, and a site between positions 132 and 133.
[F5] The peptide of any one of [F1] to [F4], wherein the non-native human IgG CH1 domain is a non-native human IgG1 CH1 domain, a non-native human IgG2 CH1 domain, a non-native human IgG3 CH1 domain, or a non-native human IgG4 CH1 domain.
[F6] The peptide of any one of [F1] to [F5], wherein the amino acid sequence between positions 121 and 133 according to the EU index in the non-native human IgG CH1 domain is the amino acid sequence of any one of SEQ ID NOs: 6-294 and 1565-1833.

[G1] A set comprising two or more types of peptides consisting of an amino acid sequence between positions 121 and 133 according to the EU index in a native or non-native human IgG CH1 domain, wherein the amino acid sequence is different among the two or more types of peptides.
[G2] The set of [G1], which comprises:
   (I) two or more types of peptides which consist of an amino acid sequence between positions 121 and 133 according to the EU index in a non-native human IgG CH1 domain, wherein the amino acid sequence is different from a corresponding sequence in a native human IgG of the same isotype as the non-native human IgG, or
   (II) a peptide consisting of an amino acid sequence between positions 121 and 133 according to the EU index in a native human IgG CH1 domain, and one or more types of peptides consisting of an amino acid sequence between positions 121 and 133 according to the EU index in a non-native human IgG CH1 domain, wherein the amino acid sequence is different from a corresponding sequence in a native human IgG of the same isotype as the non-native human IgG.
[G3] The set of [G1] or [G2], wherein the amino acid sequence between positions 121 and 133 according to the EU index in the non-native human IgG CH1 domain has any one of the following features:
   (1) an amino acid at any one or more sites of positions 122 to 132 is different from an amino acid present at corresponding sites in a native human IgG of the same isotype, and the amino acid at said sites is an amino acid other than Lys, Arg, and Met (provided that the amino acid at position 122 is not Pro);
   (2) an amino acid other than Lys, Arg, and Met has been inserted at one or more sites selected from a site between positions 121 and 122, a site between positions 122 and 123, a site between positions 123 and 124, a site between positions 124 and 125, a site between positions 125 and 126, a site between positions 126 and 127, a site between positions 127 and 128, a site between positions 128 and 129, a site between positions 129 and 130, a site between positions 130 and 131, a site between positions 131 and 132, and a site between positions 132 and 133 (provided that the amino acid at the site between positions 121 and 122 is not Pro).
[G4] The set of [G1] or [G2], wherein the amino acid sequence between positions 121 and 133 according to the EU index in the non-native human IgG CH1 domain has any one of the following features:
   (1) an amino acid at any one or more sites of positions 122 to 128 and positions 130 to 132 is Ala;
   (2) an amino acid at any one or more sites of positions 122 to 132 is Ile;
   (3) an amino acid at any one or more sites of positions 122 to 124 and positions 126 to 132 is Val;
   (4) an amino acid at any one or more sites of positions 122 to 127 and positions 129 to 132 is Leu;
   (5) an amino acid at any one or more sites of positions 122 to 125 and positions 127 to 132 is Phe;
   (6) Ala, Ile, Val, Leu, Phe, Ala-Ala, Ile-Ile, Val-Val, Leu-Leu, Phe-Phe, Ala-Ile, Ala-Val, Ala-Leu, Ala-Phe, Ile-Val, Ile-Leu, Ile-Phe, Val-Leu, Val-Phe, Leu-Phe, or Ala-Ala-Ala has been inserted at one or more sites selected from a site between positions 121 and 122, a site between positions 122 and 123, a site between positions 123 and 124, a site between positions 124 and 125, a site between positions 125 and 126, a site between positions 126 and 127, a site between positions 127 and 128, a site between positions 128 and 129, a site between positions 129 and 130, a site between positions 130 and 131, a site between positions 131 and 132, and a site between positions 132 and 133.
[G5] The set of [G1] or [G2], wherein the amino acid sequence between positions 121 and 133 according to the EU index in the non-native human IgG CH1 domain has any one of the following features:
   (1) an amino acid at any one site of positions 122 to 128 and positions 130 to 132 is Ala;
   (2) an amino acid at any one site of positions 122 to 132 is Ile;
   (3) an amino acid at any one site of positions 122 to 124 and positions 126 to 132 is Val;
   (4) an amino acid at any one site of positions 122 to 127 and positions 129 to 132 is Leu;
   (5) an amino acid at any one site of positions 122 to 125 and positions 127 to 132 is Phe;
   (6) Ala, Ile, Val, Leu, Phe, Ala-Ala, Ile-Ile, Val-Val, Leu-Leu, Phe-Phe, Ala-Ile, Ala-Val, Ala-Leu, Ala-Phe, Ile-Val, Ile-Leu, Ile-Phe, Val-Leu, Val-Phe, Leu-Phe, or Ala-Ala-Ala has been inserted at one site selected from a site between positions 121 and 122, a site between positions 122 and 123, a site between positions 123 and 124, a site between positions 124 and 125, a site between positions 125 and 126, a site between positions 126 and 127, a site between positions 127 and 128, a site between positions 128 and 129, a site between positions 129 and 130, a site between positions 130 and 131, a site between positions 131 and 132, and a site between positions 132 and 133.
[G6] The set of any one of [G1] to [G5], wherein the human IgG CH1 domain is a human IgG1 CH1 domain, a human IgG2 CH1 domain, a human IgG3 CH1 domain, or a human IgG4 CH1 domain.
[G7] The set of any one of [G1] to [G5], wherein the set comprises two or more types of peptides consisting of an amino acid sequence between positions 121 and 133 according to the EU index in a non-native human IgG CH1 domain, wherein the amino acid sequence between positions 121 and 133 according to the EU index in the non-native human IgG CH1 domain is selected from the group consisting of SEQ ID NOs: 6-294 and 1565-1833.
[G8] The set of any one of [G1] to [G5], wherein the set comprises two or more types of peptides consisting of an amino acid sequence between positions 121 and 133 according to the EU index in a non-native human IgG CH1 domain, wherein the amino acid sequence between positions 121 and 133 according to the EU index in the non-native human IgG CH1 domain is selected from the group consisting of SEQ ID NOs: 6-294.
[G9] The set of any one of [G1] to [G5], wherein the set comprises two or more types of peptides consisting of an amino acid sequence between positions 121 and 133 according to the EU index in a non-native human IgG CH1 domain, wherein the amino acid sequence between positions 121 and 133 according to the EU index in the non-native human IgG CH1 domain is selected from the group consisting of SEQ ID NOs: 1565-1833.
[G10] The set of any one of [G1] to [G5], wherein the set comprises a peptide consisting of an amino acid sequence between positions 121 and 133 according to the EU index in a native human IgG CH1 domain, and one or more types of peptides consisting of an amino acid sequence between positions 121 and 133 according to the EU index in a non-native human IgG CH1 domain, wherein
   when the amino acid sequence between positions 121 and 133 according to the EU index in the native human IgG CH1 domain is SEQ ID NO: 5, the amino acid sequence between positions 121 and 133 according to the EU index in the non-native human IgG CH1 domain is selected from the group consisting of SEQ ID NOs: 6-294, and
   when the amino acid sequence between positions 121 and 133 according to the EU index in the native human IgG CH1 domain is SEQ ID NO: 945, the amino acid sequence between positions 121 and 133 according to the EU index in the non-native human IgG CH1 domain is selected from the group consisting of SEQ ID NOs: 1565-1833.
[G11] The set of any one of [G1] to [G5], which is a set of two or more types of peptides selected from the group consisting of (1) to (26) below:
   (1) a peptide consisting of the amino acid sequence of SEQ ID NO: 5;
   (2) a peptide consisting of the amino acid sequence of SEQ ID NO: 6;
   (3) a peptide consisting of the amino acid sequence of SEQ ID NO: 7;
   (4) a peptide consisting of the amino acid sequence of SEQ ID NO: 8;
   (5) a peptide consisting of the amino acid sequence of SEQ ID NO: 9;
   (6) a peptide consisting of the amino acid sequence of SEQ ID NO: 10;
   (7) a peptide consisting of the amino acid sequence of SEQ ID NO: 12;
   (8) a peptide consisting of the amino acid sequence of SEQ ID NO: 13;
   (9) a peptide consisting of the amino acid sequence of SEQ ID NO: 14;
   (10) a peptide consisting of the amino acid sequence of SEQ ID NO: 17;
   (11) a peptide consisting of the amino acid sequence of SEQ ID NO: 24;
   (12) a peptide consisting of the amino acid sequence of SEQ ID NO: 27;
   (13) a peptide consisting of the amino acid sequence of SEQ ID NO: 36;
   (14) a peptide consisting of the amino acid sequence of SEQ ID NO: 38;
   (15) a peptide consisting of the amino acid sequence of SEQ ID NO: 40;
   (16) a peptide consisting of the amino acid sequence of SEQ ID NO: 42;
   (17) a peptide consisting of the amino acid sequence of SEQ ID NO: 45;
   (18) a peptide consisting of the amino acid sequence of SEQ ID NO: 46;
   (19) a peptide consisting of the amino acid sequence of SEQ ID NO: 47;
   (20) a peptide consisting of the amino acid sequence of SEQ ID NO: 73;
   (21) a peptide consisting of the amino acid sequence of SEQ ID NO: 75;
   (22) a peptide consisting of the amino acid sequence of SEQ ID NO: 76;
   (23) a peptide consisting of the amino acid sequence of SEQ ID NO: 78;
   (24) a peptide consisting of the amino acid sequence of SEQ ID NO: 79;
   (25) a peptide consisting of the amino acid sequence of SEQ ID NO: 80; and
   (26) a peptide consisting of the amino acid sequence of SEQ ID NO: 82.
[G12] The set of any one of [G1] to [G5], which is a set of two or more types of peptides selected from the group consisting of (1) to (13) below:
   (1) a peptide consisting of the amino acid sequence of SEQ ID NO: 5;
   (2) a peptide consisting of the amino acid sequence of SEQ ID NO: 6;
   (3) a peptide consisting of the amino acid sequence of SEQ ID NO: 7;
   (4) a peptide consisting of the amino acid sequence of SEQ ID NO: 8;
   (5) a peptide consisting of the amino acid sequence of SEQ ID NO: 9;
   (6) a peptide consisting of the amino acid sequence of SEQ ID NO: 10;
   (7) a peptide consisting of the amino acid sequence of SEQ ID NO: 12;
   (8) a peptide consisting of the amino acid sequence of SEQ ID NO: 13;
   (9) a peptide consisting of the amino acid sequence of SEQ ID NO: 14;
   (10) a peptide consisting of the amino acid sequence of SEQ ID NO: 17;
   (11) a peptide consisting of the amino acid sequence of SEQ ID NO: 24;
   (12) a peptide consisting of the amino acid sequence of SEQ ID NO: 27; and
   (13) a peptide consisting of the amino acid sequence of SEQ ID NO: 36.
[G13] The set of any one of [G1] to [G5], wherein the set is a set of two types of peptides, and the two types of peptides are selected from the group consisting of (1) to (15) below:
   (1) a peptide consisting of the amino acid sequence of SEQ ID NO: 5 and a peptide consisting of the amino acid sequence of SEQ ID NO: 7;
   (2) a peptide consisting of the amino acid sequence of SEQ ID NO: 5 and a peptide consisting of the amino acid sequence of SEQ ID NO: 12;
   (3) a peptide consisting of the amino acid sequence of SEQ ID NO: 5 and a peptide consisting of the amino acid sequence of SEQ ID NO: 10;
   (4) a peptide consisting of the amino acid sequence of SEQ ID NO: 5 and a peptide consisting of the amino acid sequence of SEQ ID NO: 17;
   (5) a peptide consisting of the amino acid sequence of SEQ ID NO: 7 and a peptide consisting of the amino acid sequence of SEQ ID NO: 12;
   (6) a peptide consisting of the amino acid sequence of SEQ ID NO: 7 and a peptide consisting of the amino acid sequence of SEQ ID NO: 10;
   (7) a peptide consisting of the amino acid sequence of SEQ ID NO: 7 and a peptide consisting of the amino acid sequence of SEQ ID NO: 17;
   (8) a peptide consisting of the amino acid sequence of SEQ ID NO: 12 and a peptide consisting of the amino acid sequence of SEQ ID NO: 10;
   (9) a peptide consisting of the amino acid sequence of SEQ ID NO: 12 and a peptide consisting of the amino acid sequence of SEQ ID NO: 17;
   (10) a peptide consisting of the amino acid sequence of SEQ ID NO: 10 and a peptide consisting of the amino acid sequence of SEQ ID NO: 17;
   (11) a peptide consisting of the amino acid sequence of SEQ ID NO: 5 and a peptide consisting of the amino acid sequence of SEQ ID NO: 27;
   (12) a peptide consisting of the amino acid sequence of SEQ ID NO: 7 and a peptide consisting of the amino acid sequence of SEQ ID NO: 27;
   (13) a peptide consisting of the amino acid sequence of SEQ ID NO: 10 and a peptide consisting of the amino acid sequence of SEQ ID NO: 27;
   (14) a peptide consisting of the amino acid sequence of SEQ ID NO: 12 and a peptide consisting of the amino acid sequence of SEQ ID NO: 27; and
   (15) a peptide consisting of the amino acid sequence of SEQ ID NO: 17 and a peptide consisting of the amino acid sequence of SEQ ID NO: 27.
[G14] The set of any one of [G1] to [G5], wherein the set is a set of three types of peptides, and the three types of peptides are selected from the group consisting of (1) to (20) below:
   (1) a peptide consisting of the amino acid sequence of SEQ ID NO: 5, a peptide consisting of the amino acid sequence of SEQ ID NO: 7, and a peptide consisting of the amino acid sequence of SEQ ID NO: 12;
   (2) a peptide consisting of the amino acid sequence of SEQ ID NO: 5, a peptide consisting of the amino acid sequence of SEQ ID NO: 7, and a peptide consisting of the amino acid sequence of SEQ ID NO: 10;
   (3) a peptide consisting of the amino acid sequence of SEQ ID NO: 5, a peptide consisting of the amino acid sequence of SEQ ID NO: 7, and a peptide consisting of the amino acid sequence of SEQ ID NO: 17;
   (4) a peptide consisting of the amino acid sequence of SEQ ID NO: 5, a peptide consisting of the amino acid sequence of SEQ ID NO: 12, and a peptide consisting of the amino acid sequence of SEQ ID NO: 10;
   (5) a peptide consisting of the amino acid sequence of SEQ ID NO: 5, a peptide consisting of the amino acid sequence of SEQ ID NO: 12, and a peptide consisting of the amino acid sequence of SEQ ID NO: 17;
   (6) a peptide consisting of the amino acid sequence of SEQ ID NO: 5, a peptide consisting of the amino acid sequence of SEQ ID NO: 10, and a peptide consisting of the amino acid sequence of SEQ ID NO: 17;
   (7) a peptide consisting of the amino acid sequence of SEQ ID NO: 7, a peptide consisting of the amino acid sequence of SEQ ID NO: 12, and a peptide consisting of the amino acid sequence of SEQ ID NO: 10;
   (8) a peptide consisting of the amino acid sequence of SEQ ID NO: 7, a peptide consisting of the amino acid sequence of SEQ ID NO: 12, and a peptide consisting of the amino acid sequence of SEQ ID NO: 17;
   (9) a peptide consisting of the amino acid sequence of SEQ ID NO: 12, a peptide consisting of the amino acid sequence of SEQ ID NO: 10, and a peptide consisting of the amino acid sequence of SEQ ID NO: 17;
   (10) a peptide consisting of the amino acid sequence of SEQ ID NO: 7, a peptide consisting of the amino acid sequence of SEQ ID NO: 10, and a peptide consisting of the amino acid sequence of SEQ ID NO: 17;
   (11) a peptide consisting of the amino acid sequence of SEQ ID NO: 5, a peptide consisting of the amino acid sequence of SEQ ID NO: 7, and a peptide consisting of the amino acid sequence of SEQ ID NO: 27;
   (12) a peptide consisting of the amino acid sequence of SEQ ID NO: 5, a peptide consisting of the amino acid sequence of SEQ ID NO: 12, and a peptide consisting of the amino acid sequence of SEQ ID NO: 27;
   (13) a peptide consisting of the amino acid sequence of SEQ ID NO: 5, a peptide consisting of the amino acid sequence of SEQ ID NO: 10, and a peptide consisting of the amino acid sequence of SEQ ID NO: 27;
   (14) a peptide consisting of the amino acid sequence of SEQ ID NO: 5, a peptide consisting of the amino acid sequence of SEQ ID NO: 17, and a peptide consisting of the amino acid sequence of SEQ ID NO: 27;
   (15) a peptide consisting of the amino acid sequence of SEQ ID NO: 7, a peptide consisting of the amino acid sequence of SEQ ID NO: 10, and a peptide consisting of the amino acid sequence of SEQ ID NO: 27;
   (16) a peptide consisting of the amino acid sequence of SEQ ID NO: 7, a peptide consisting of the amino acid sequence of SEQ ID NO: 12, and a peptide consisting of the amino acid sequence of SEQ ID NO: 27;
   (17) a peptide consisting of the amino acid sequence of SEQ ID NO: 7, a peptide consisting of the amino acid sequence of SEQ ID NO: 17, and a peptide consisting of the amino acid sequence of SEQ ID NO: 27;
   (18) a peptide consisting of the amino acid sequence of SEQ ID NO: 12, a peptide consisting of the amino acid sequence of SEQ ID NO: 10, and a peptide consisting of the amino acid sequence of SEQ ID NO: 27;
   (19) a peptide consisting of the amino acid sequence of SEQ ID NO: 10, a peptide consisting of the amino acid sequence of SEQ ID NO: 17, and a peptide consisting of the amino acid sequence of SEQ ID NO: 27; and
   (20) a peptide consisting of the amino acid sequence of SEQ ID NO: 12, a peptide consisting of the amino acid sequence of SEQ ID NO: 17, and a peptide consisting of the amino acid sequence of SEQ ID NO: 27.
[G15] The set of any one of [G1] to [G5], wherein the set is a set of four types of peptides, and the four types of peptides are selected from the group consisting of (1) to (15) below:
   (1) a peptide consisting of the amino acid sequence of SEQ ID NO: 5, a peptide consisting of the amino acid sequence of SEQ ID NO: 7, a peptide consisting of the amino acid sequence of SEQ ID NO: 12, and a peptide consisting of the amino acid sequence of SEQ ID NO: 10;
   (2) a peptide consisting of the amino acid sequence of SEQ ID NO: 5, a peptide consisting of the amino acid sequence of SEQ ID NO: 7, a peptide consisting of the amino acid sequence of SEQ ID NO: 12, and a peptide consisting of the amino acid sequence of SEQ ID NO: 17;
   (3) a peptide consisting of the amino acid sequence of SEQ ID NO: 5, a peptide consisting of the amino acid sequence of SEQ ID NO: 12, a peptide consisting of the amino acid sequence of SEQ ID NO: 10, and a peptide consisting of the amino acid sequence of SEQ ID NO: 17;
   (4) a peptide consisting of the amino acid sequence of SEQ ID NO: 5, a peptide consisting of the amino acid sequence of SEQ ID NO: 7, a peptide consisting of the amino acid sequence of SEQ ID NO: 10, and a peptide consisting of the amino acid sequence of SEQ ID NO: 17;
   (5) a peptide consisting of the amino acid sequence of SEQ ID NO: 7, a peptide consisting of the amino acid sequence of SEQ ID NO: 12, a peptide consisting of the amino acid sequence of SEQ ID NO: 10, and a peptide consisting of the amino acid sequence of SEQ ID NO: 17;
   (6) a peptide consisting of the amino acid sequence of SEQ ID NO: 5, a peptide consisting of the amino acid sequence of SEQ ID NO: 7, a peptide consisting of the amino acid sequence of SEQ ID NO: 10, and a peptide consisting of the amino acid sequence of SEQ ID NO: 27;
   (7) a peptide consisting of the amino acid sequence of SEQ ID NO: 5, a peptide consisting of the amino acid sequence of SEQ ID NO: 7, a peptide consisting of the amino acid sequence of SEQ ID NO: 12, and a peptide consisting of the amino acid sequence of SEQ ID NO: 27;
   (8) a peptide consisting of the amino acid sequence of SEQ ID NO: 5, a peptide consisting of the amino acid sequence of SEQ ID NO: 7, a peptide consisting of the amino acid sequence of SEQ ID NO: 17, and a peptide consisting of the amino acid sequence of SEQ ID NO: 27;
   (9) a peptide consisting of the amino acid sequence of SEQ ID NO: 5, a peptide consisting of the amino acid sequence of SEQ ID NO: 10, a peptide consisting of the amino acid sequence of SEQ ID NO: 12, and a peptide consisting of the amino acid sequence of SEQ ID NO: 27;
   (10) a peptide consisting of the amino acid sequence of SEQ ID NO: 5, a peptide consisting of the amino acid sequence of SEQ ID NO: 10, a peptide consisting of the amino acid sequence of SEQ ID NO: 17, and a peptide consisting of the amino acid sequence of SEQ ID NO: 27;
   (11) a peptide consisting of the amino acid sequence of SEQ ID NO: 5, a peptide consisting of the amino acid sequence of SEQ ID NO: 12, a peptide consisting of the amino acid sequence of SEQ ID NO: 17, and a peptide consisting of the amino acid sequence of SEQ ID NO: 27;
   (12) a peptide consisting of the amino acid sequence of SEQ ID NO: 7, a peptide consisting of the amino acid sequence of SEQ ID NO: 10, a peptide consisting of the amino acid sequence of SEQ ID NO: 12, and a peptide consisting of the amino acid sequence of SEQ ID NO: 27;
   (13) a peptide consisting of the amino acid sequence of SEQ ID NO: 7, a peptide consisting of the amino acid sequence of SEQ ID NO: 10, a peptide consisting of the amino acid sequence of SEQ ID NO: 17, and a peptide consisting of the amino acid sequence of SEQ ID NO: 27;
   (14) a peptide consisting of the amino acid sequence of SEQ ID NO: 7, a peptide consisting of the amino acid sequence of SEQ ID NO: 12, a peptide consisting of the amino acid sequence of SEQ ID NO: 17, and a peptide consisting of the amino acid sequence of SEQ ID NO: 27; and
   (15) a peptide consisting of the amino acid sequence of SEQ ID NO: 10, a peptide consisting of the amino acid sequence of SEQ ID NO: 12, a peptide consisting of the amino acid sequence of SEQ ID NO: 17, and a peptide consisting of the amino acid sequence of SEQ ID NO: 27.
[G16] The set of any one of [G1] to [G5], wherein the set is a set of five types of peptides, and the five types of peptides are selected from the group consisting of (1) to (6) below:
   (1) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, and a peptide consisting of the amino acid sequence of SEQ ID NO: 17;
   (2) a peptide consisting of the amino acid sequence of SEQ ID NO: 5, a peptide consisting of the amino acid sequence of SEQ ID NO: 7, a peptide consisting of the amino acid sequence of SEQ ID NO: 10, a peptide consisting of the amino acid sequence of SEQ ID NO: 12, and a peptide consisting of the amino acid sequence of SEQ ID NO: 27;
   (3) a peptide consisting of the amino acid sequence of SEQ ID NO: 5, a peptide consisting of the amino acid sequence of SEQ ID NO: 7, a peptide consisting of the amino acid sequence of SEQ ID NO: 10, a peptide consisting of the amino acid sequence of SEQ ID NO: 17, and a peptide consisting of the amino acid sequence of SEQ ID NO: 27;
   (4) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 17, and a peptide consisting of the amino acid sequence of SEQ ID NO: 27;
   (5) a peptide consisting of the amino acid sequence of SEQ ID NO: 5, a peptide consisting of the amino acid sequence of SEQ ID NO: 10, a peptide consisting of the amino acid sequence of SEQ ID NO: 12, a peptide consisting of the amino acid sequence of SEQ ID NO: 17, and a peptide consisting of the amino acid sequence of SEQ ID NO: 27; and
   (6) a peptide consisting of the amino acid sequence of SEQ ID NO: 7, a peptide consisting of the amino acid sequence of SEQ ID NO: 10, a peptide consisting of the amino acid sequence of SEQ ID NO: 12, a peptide consisting of the amino acid sequence of SEQ ID NO: 17, and a peptide consisting of the amino acid sequence of SEQ ID NO: 27.
[G17] The set of any one of [G1] to [G5], which is a set of the following six types of peptides:
   (1) a peptide consisting of the amino acid sequence of SEQ ID NO: 5;
   (2) a peptide consisting of the amino acid sequence of SEQ ID NO: 7;
   (3) a peptide consisting of the amino acid sequence of SEQ ID NO: 12;
   (4) a peptide consisting of the amino acid sequence of SEQ ID NO: 10;
   (5) a peptide consisting of the amino acid sequence of SEQ ID NO: 17; and
   (6) a peptide consisting of SEQ ID NO: 27.
[G18] The set of any one of [G1] to [G5], wherein the set is a set of seven types of peptides, and the seven types of peptides are selected from the group consisting of (1) to (4) below:
   (1) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, and a peptide consisting of the amino acid sequence of SEQ ID NO: 13;
   (2) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, and a peptide consisting of the amino acid sequence of SEQ ID NO: 14;
   (3) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, and a peptide consisting of the amino acid sequence of SEQ ID NO: 24; and
   (4) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, and a peptide consisting of the amino acid sequence of SEQ ID NO: 36.
[G19] The set of any one of [G1] to [G5], wherein the set is a set of eight types of peptides, and the eight types of peptides are selected from the group consisting of (1) to (6) below:
   (1) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, and a peptide consisting of the amino acid sequence of SEQ ID NO: 14;
   (2) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, and a peptide consisting of the amino acid sequence of SEQ ID NO: 24;
   (3) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, and a peptide consisting of the amino acid sequence of SEQ ID NO: 36;
   (4) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 14, and a peptide consisting of the amino acid sequence of SEQ ID NO: 24;
   (5) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 14, and a peptide consisting of the amino acid sequence of SEQ ID NO: 36; and
   (6) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 24, and a peptide consisting of the amino acid sequence of SEQ ID NO: 36.
[G20] The set of any one of [G1] to [G5], wherein the set is a set of nine types of peptides, and the nine types of peptides are selected from the group consisting of (1) to (4) below:
   (1) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, a peptide consisting of the amino acid sequence of SEQ ID NO: 14, and a peptide consisting of the amino acid sequence of SEQ ID NO: 24;
   (2) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, a peptide consisting of the amino acid sequence of SEQ ID NO: 14, and a peptide consisting of the amino acid sequence of SEQ ID NO: 36;
   (3) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, a peptide consisting of the amino acid sequence of SEQ ID NO: 24, and a peptide consisting of the amino acid sequence of SEQ ID NO: 36; and
   (4) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 14, a peptide consisting of the amino acid sequence of SEQ ID NO: 24, and a peptide consisting of the amino acid sequence of SEQ ID NO: 36.
[G21] The set of any one of [G1] to [G5], which is a set of the following ten types of peptides:
   (1) a peptide consisting of the amino acid sequence of SEQ ID NO: 5;
   (2) a peptide consisting of the amino acid sequence of SEQ ID NO: 7;
   (3) a peptide consisting of the amino acid sequence of SEQ ID NO: 12;
   (4) a peptide consisting of the amino acid sequence of SEQ ID NO: 10;
   (5) a peptide consisting of the amino acid sequence of SEQ ID NO: 17;
   (6) a peptide consisting of the amino acid sequence of SEQ ID NO: 27;
   (7) a peptide consisting of the amino acid sequence of SEQ ID NO: 13;
   (8) a peptide consisting of the amino acid sequence of SEQ ID NO: 14;
   (9) a peptide consisting of the amino acid sequence of SEQ ID NO: 24; and
   (10) a peptide consisting of the amino acid sequence of SEQ ID NO: 36.
[G22] The set of any one of [G1] to [G5], wherein the set is a set of eleven types of peptides, and the eleven types of peptides are selected from the group consisting of (1) to (2) below:
   (1) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, a peptide consisting of the amino acid sequence of SEQ ID NO: 14, a peptide consisting of the amino acid sequence of SEQ ID NO: 24, a peptide consisting of the amino acid sequence of SEQ ID NO: 36, and a peptide consisting of the amino acid sequence of SEQ ID NO: 6; and
   (2) a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, a peptide consisting of the amino acid sequence of SEQ ID NO: 14, a peptide consisting of the amino acid sequence of SEQ ID NO: 24, a peptide consisting of the amino acid sequence of SEQ ID NO: 36, and a peptide consisting of the amino acid sequence of SEQ ID NO: 8.
[G23] The set of any one of [G1] to [G5], which is a set of the following twelve types of peptides:
   (1) a peptide consisting of the amino acid sequence of SEQ ID NO: 5;
   (2) a peptide consisting of the amino acid sequence of SEQ ID NO: 6;
   (3) a peptide consisting of the amino acid sequence of SEQ ID NO: 7;
   (4) a peptide consisting of the amino acid sequence of SEQ ID NO: 8;
   (5) a peptide consisting of the amino acid sequence of SEQ ID NO: 10;
   (6) a peptide consisting of the amino acid sequence of SEQ ID NO: 12;
   (7) a peptide consisting of the amino acid sequence of SEQ ID NO: 13;
   (8) a peptide consisting of the amino acid sequence of SEQ ID NO: 14;
   (9) a peptide consisting of the amino acid sequence of SEQ ID NO: 17;
   (10) a peptide consisting of the amino acid sequence of SEQ ID NO: 24;
   (11) a peptide consisting of the amino acid sequence of SEQ ID NO: 27; and
   (12) a peptide consisting of the amino acid sequence of SEQ ID NO: 36.
[G24] The set of any one of [G1] to [G23], which is for use in a method for detecting each of a plurality of types of peptides derived from a plurality of types of antigen-binding molecules in a biological sample by mass spectrometry, or a method for detecting and/or quantifying each of a plurality of types of antigen-binding molecules in a biological sample.
[G25] Use of the set of any one of [G1] to [G23] for detecting each of a plurality of types of peptides derived from a plurality of types of antigen-binding molecules in a biological sample by mass spectrometry, or for detecting and/or quantifying each of a plurality of types of antigen-binding molecules in a biological sample.
[G26] A method for administering to an individual or a subject a mixture of a plurality of types of antigen-binding molecules each of which comprise a respective peptide of the set of any one of [G1] to [G23].
[G27] A method for selecting an antigen-binding molecule, comprising:
   (a) detecting and/or quantifying each of a plurality of types of antigen-binding molecules in a biological sample derived from an individual or a subject that a mixture of the plurality of types of antigen-binding molecules has been administered to, wherein the plurality of types of antigen-binding molecules each of which comprise a respective peptide of the set of any one of [G1] to [G23]; and
   (b) selecting an antigen-binding molecule based on the result of the detection and/or quantification of (a).
[G28] An antigen-binding molecule selected by the method of [G27].
[G29] An antigen-binding molecule which results from altering the amino acid sequence between positions 121 and 133 according to the EU index in the IgG CH1 domain of an antigen-binding molecule selected by the method of [G27] to a native sequence between positions 121 and 133 according to the EU index in the CH1 domain of an IgG antibody of the same isotype.

### [Brief Description of the Drawings]

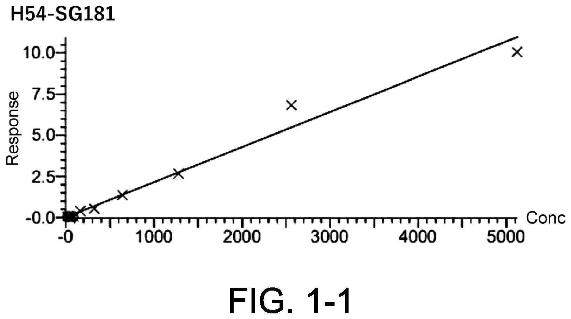

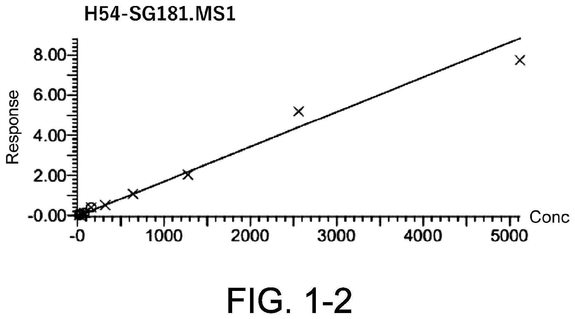

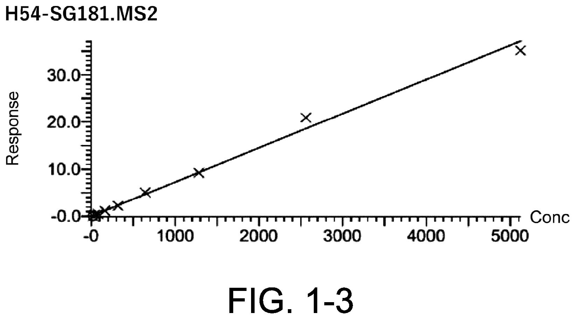

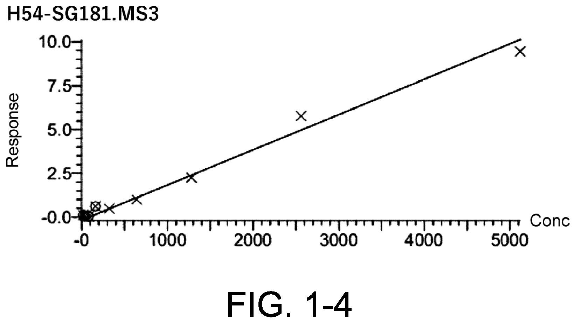

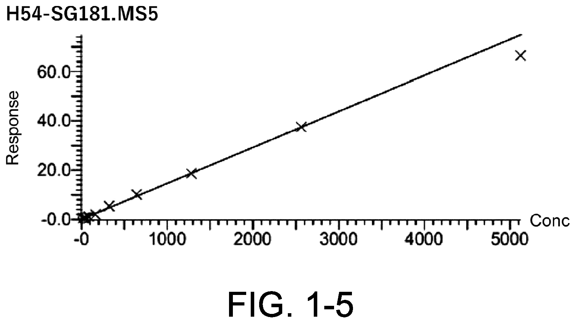

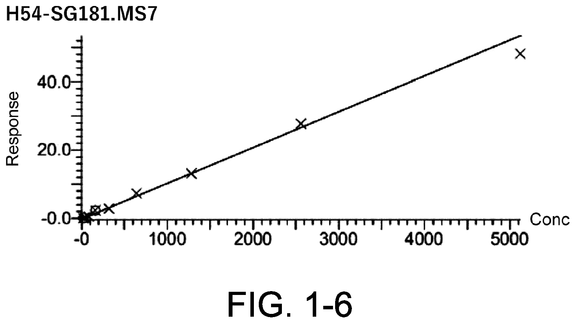

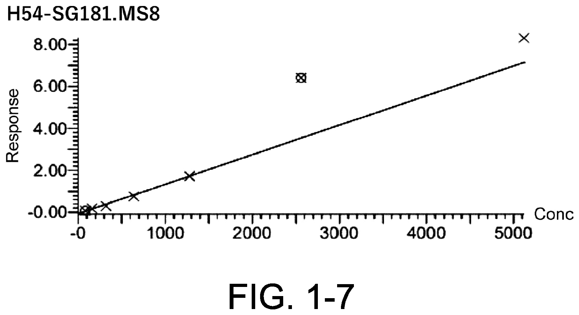

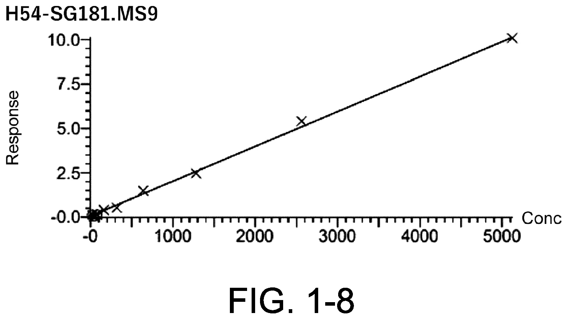

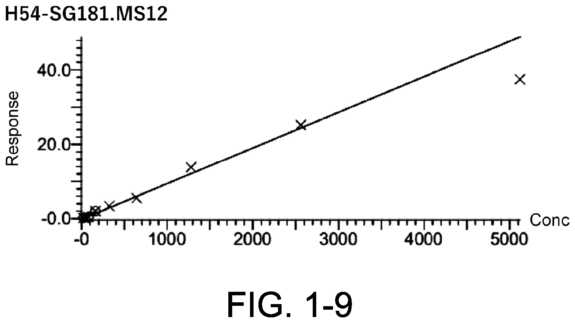

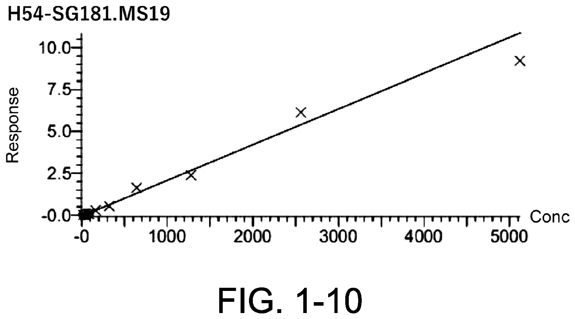

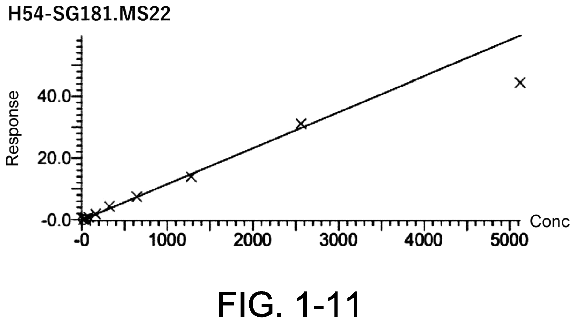

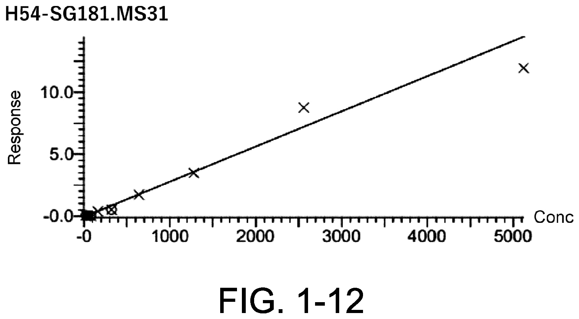

[Fig. 1-1] Fig. 1-1 shows an internal calibration curve generated from mass spectrometry on an antibody sample that was prepared so that the mouse plasma concentration of the antibody ranged from 0.0200 to 5.12 µg/mL. The name of the target antibody is indicated in the upper left of the figure. The vertical axis shows the peak area ratio (peak area of the target antibody sample / peak area of the internal standard), and the horizontal axis shows the concentration of the target antibody (ng/mL).
[Fig. 1-2] Fig. 1-2 shows an internal calibration curve generated from mass spectrometry on an antibody sample that was prepared so that the mouse plasma concentration of the antibody ranged from 0.0200 to 5.12 µg/mL. The name of the target antibody is indicated in the upper left of the figure. The vertical axis shows the peak area ratio (peak area of the target antibody sample / peak area of the internal standard), and the horizontal axis shows the concentration of the target antibody (ng/mL).
[Fig. 1-3] Fig. 1-3 shows an internal calibration curve generated from mass spectrometry on an antibody sample that was prepared so that the mouse plasma concentration of the antibody ranged from 0.0200 to 5.12 µg/mL. The name of the target antibody is indicated in the upper left of the figure. The vertical axis shows the peak area ratio (peak area of the target antibody sample / peak area of the internal standard), and the horizontal axis shows the concentration of the target antibody (ng/mL).
[Fig. 1-4] Fig. 1-4 shows an internal calibration curve generated from mass spectrometry on an antibody sample that was prepared so that the mouse plasma concentration of the antibody ranged from 0.0200 to 5.12 µg/mL. The name of the target antibody is indicated in the upper left of the figure. The vertical axis shows the peak area ratio (peak area of the target antibody sample / peak area of the internal standard), and the horizontal axis shows the concentration of the target antibody (ng/mL).
[Fig. 1-5] Fig. 1-5 shows an internal calibration curve generated from mass spectrometry on an antibody sample that was prepared so that the mouse plasma concentration of the antibody ranged from 0.0200 to 5.12 µg/mL. The name of the target antibody is indicated in the upper left of the figure. The vertical axis shows the peak area ratio (peak area of the target antibody sample / peak area of the internal standard), and the horizontal axis shows the concentration of the target antibody (ng/mL).
[Fig. 1-6] Fig. 1-6 shows an internal calibration curve generated from mass spectrometry on an antibody sample that was prepared so that the mouse plasma concentration of the antibody ranged from 0.0200 to 5.12 µg/mL. The name of the target antibody is indicated in the upper left of the figure. The vertical axis shows the peak area ratio (peak area of the target antibody sample / peak area of the internal standard), and the horizontal axis shows the concentration of the target antibody (ng/mL).
[Fig. 1-7] Fig. 1-7 shows an internal calibration curve generated from mass spectrometry on an antibody sample that was prepared so that the mouse plasma concentration of the antibody ranged from 0.0200 to 5.12 µg/mL. The name of the target antibody is indicated in the upper left of the figure. The vertical axis shows the peak area ratio (peak area of the target antibody sample / peak area of the internal standard), and the horizontal axis shows the concentration of the target antibody (ng/mL).
[Fig. 1-8] Fig. 1-8 shows an internal calibration curve generated from mass spectrometry on an antibody sample that was prepared so that the mouse plasma concentration of the antibody ranged from 0.0200 to 5.12 µg/mL. The name of the target antibody is indicated in the upper left of the figure. The vertical axis shows the peak area ratio (peak area of the target antibody sample / peak area of the internal standard), and the horizontal axis shows the concentration of the target antibody (ng/mL).
[Fig. 1-9] Fig. 1-9 shows an internal calibration curve generated from mass spectrometry on an antibody sample that was prepared so that the mouse plasma concentration of the antibody ranged from 0.0200 to 5.12 µg/mL. The name of the target antibody is indicated in the upper left of the figure. The vertical axis shows the peak area ratio (peak area of the target antibody sample / peak area of the internal standard), and the horizontal axis shows the concentration of the target antibody (ng/mL).
[Fig. 1-10] Fig. 1-10 shows an internal calibration curve generated from mass spectrometry on an antibody sample that was prepared so that the mouse plasma concentration of the antibody ranged from 0.0200 to 5.12 µg/mL. The name of the target antibody is indicated in the upper left of the figure. The vertical axis shows the peak area ratio (peak area of the target antibody sample / peak area of the internal standard), and the horizontal axis shows the concentration of the target antibody (ng/mL).
[Fig. 1-11] Fig. 1-11 shows an internal calibration curve generated from mass spectrometry on an antibody sample that was prepared so that the mouse plasma concentration of the antibody ranged from 0.0200 to 5.12 µg/mL. The name of the target antibody is indicated in the upper left of the figure. The vertical axis shows the peak area ratio (peak area of the target antibody sample / peak area of the internal standard), and the horizontal axis shows the concentration of the target antibody (ng/mL).
[Fig. 1-12] Fig. 1-12 shows an internal calibration curve generated from mass spectrometry on an antibody sample that was prepared so that the mouse plasma concentration of the antibody ranged from 0.0200 to 5.12 µg/mL. The name of the target antibody is indicated in the upper left of the figure. The vertical axis shows the peak area ratio (peak area of the target antibody sample / peak area of the internal standard), and the horizontal axis shows the concentration of the target antibody (ng/mL).

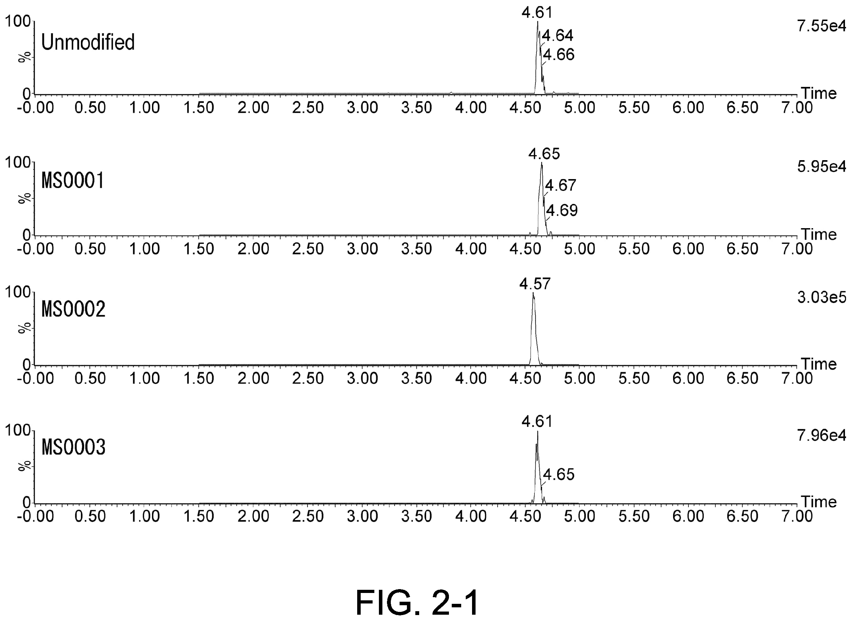

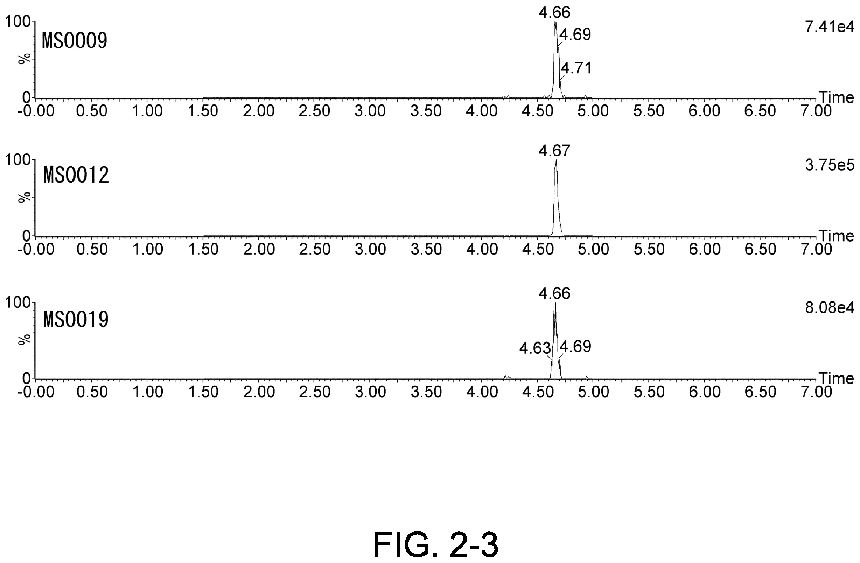

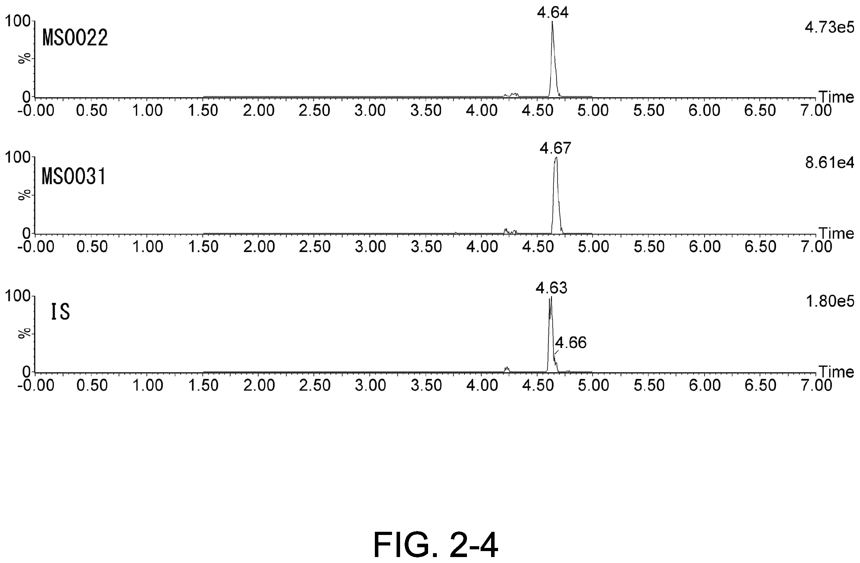

[Fig. 2-1] Fig. 2-1 shows SRM chromatograms of the mixed administration group 5 minutes after administration. The name of the measured target peptide is indicated in the upper left of each chromatogram. The numerical value in the upper right of each diagram indicates peak intensity. The peak intensity indicated in the upper right of each diagram is set as 100% in the vertical axis of each chromatogram. The horizontal axis shows the elution time (min) in LC.

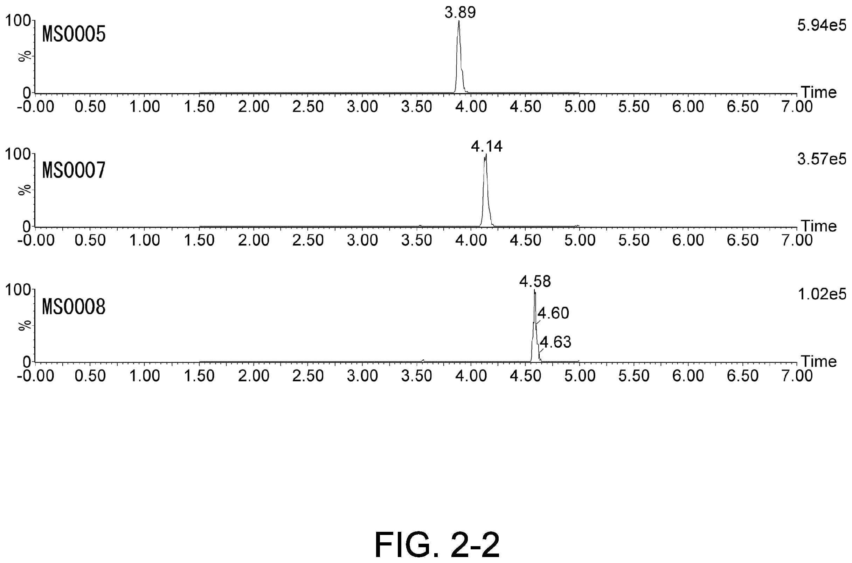

[Fig. 2-2] Fig. 2-2 shows SRM chromatograms of the mixed administration group 5 minutes after administration. The name of the measured target peptide is indicated in the upper left of each chromatogram. The numerical value in the upper right of each diagram indicates peak intensity. The peak intensity indicated in the upper right of each diagram is set as 100% in the vertical axis of each chromatogram. The horizontal axis shows the elution time (min) in LC.
[Fig. 2-3] Fig. 2-3 shows SRM chromatograms of the mixed administration group 5 minutes after administration. The name of the measured target peptide is indicated in the upper left of each chromatogram. The numerical value in the upper right of each diagram indicates peak intensity. The peak intensity indicated in the upper right of each diagram is set as 100% in the vertical axis of each chromatogram. The horizontal axis shows the elution time (min) in LC.
[Fig. 2-4] Fig. 2-4 shows SRM chromatograms of the mixed administration group 5 minutes after administration. The name of the measured target peptide is indicated in the upper left of each chromatogram. IS stands for the internal standard. The numerical value in the upper right of each diagram indicates peak intensity. The peak intensity indicated in the upper right of each diagram is set as 100% in the vertical axis of each chromatogram. The horizontal axis shows the elution time (min) in LC.

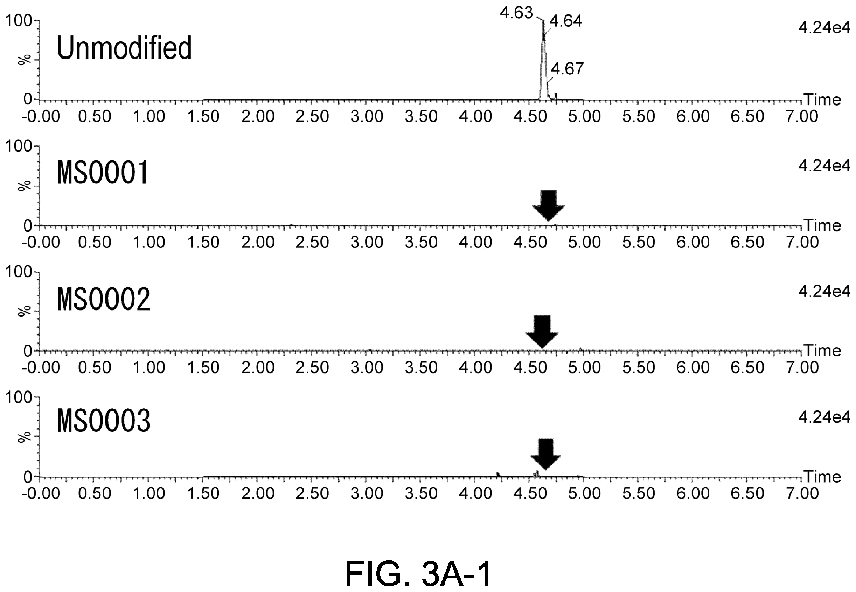

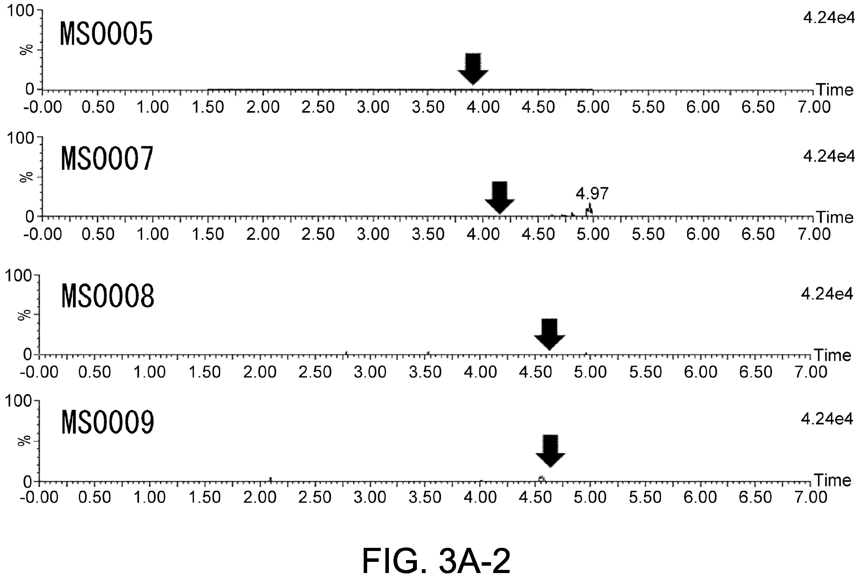

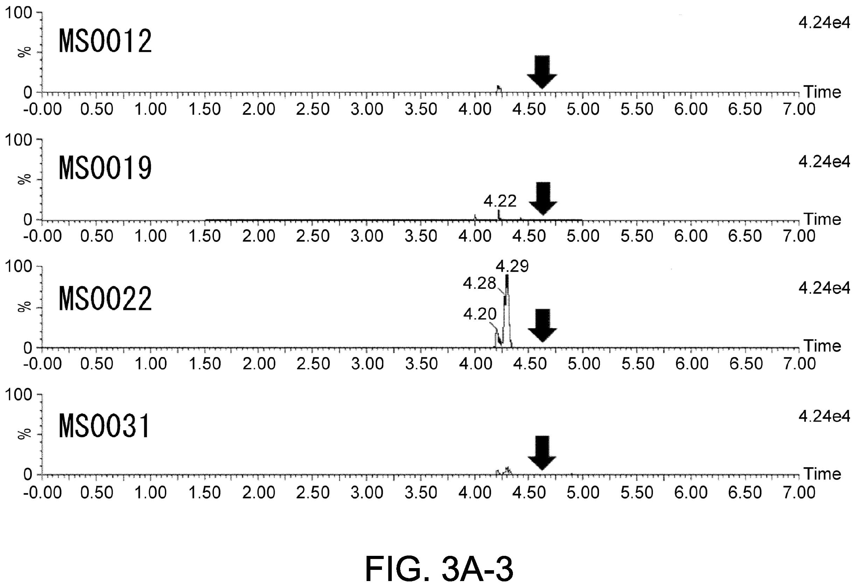

[Fig. 3A-1] Fig. 3A-1 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3A-1 shows the measurement results of a plasma sample to which an unmodified antibody alone was added. The peak intensity of the unmodified peptide, *i.e.*, 4.24e4, is set as 100% in the vertical axis of each chromatogram in Fig. 3A-1. The name of the measured target peptide is indicated in the upper left of each chromatogram. Each black arrow indicates the expected position of the peak for the measured target peptide.
[Fig. 3A-2] Fig. 3A-2 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3A-2 shows the measurement results of a plasma sample to which an unmodified antibody alone was added. The peak intensity of the unmodified peptide, *i.e.*, 4.24e4, is set as 100% in the vertical axis of each chromatogram in Fig. 3A-2. The name of the measured target peptide is indicated in the upper left of each chromatogram. Each black arrow indicates the expected position of the peak for the measured target peptide.
[Fig. 3A-3] Fig. 3A-3 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3A-3 shows the measurement results of a plasma sample to which an unmodified antibody alone was added. The peak intensity of the unmodified peptide, *i*.*e*.*,* 4.24e4, is set as 100% in the vertical axis of each chromatogram in Fig. 3A-3. The name of the measured target peptide is indicated in the upper left of each chromatogram. Each black arrow indicates the expected position of the peak for the measured target peptide.

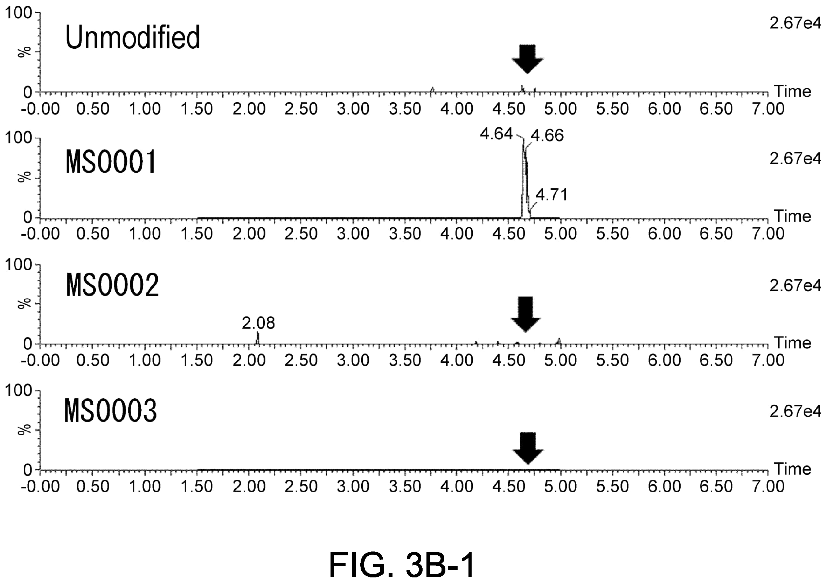

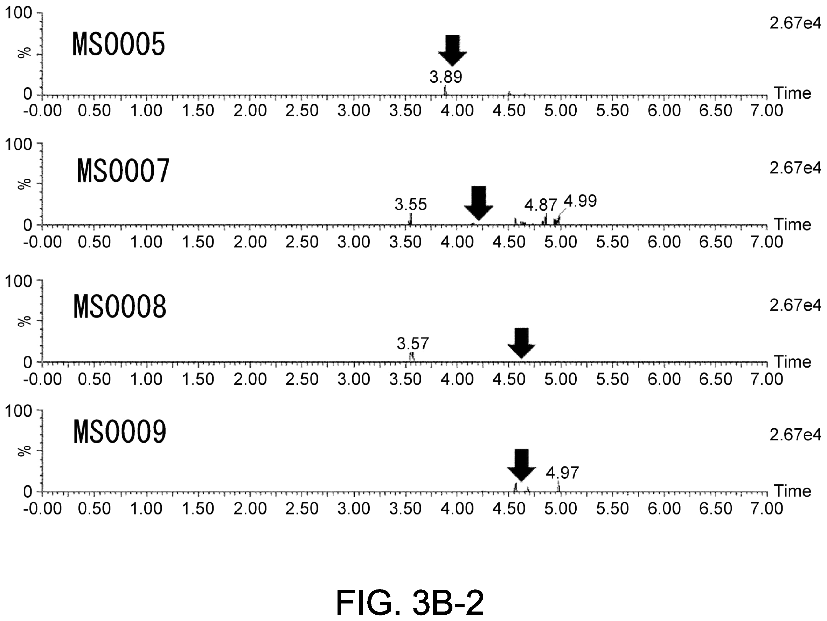

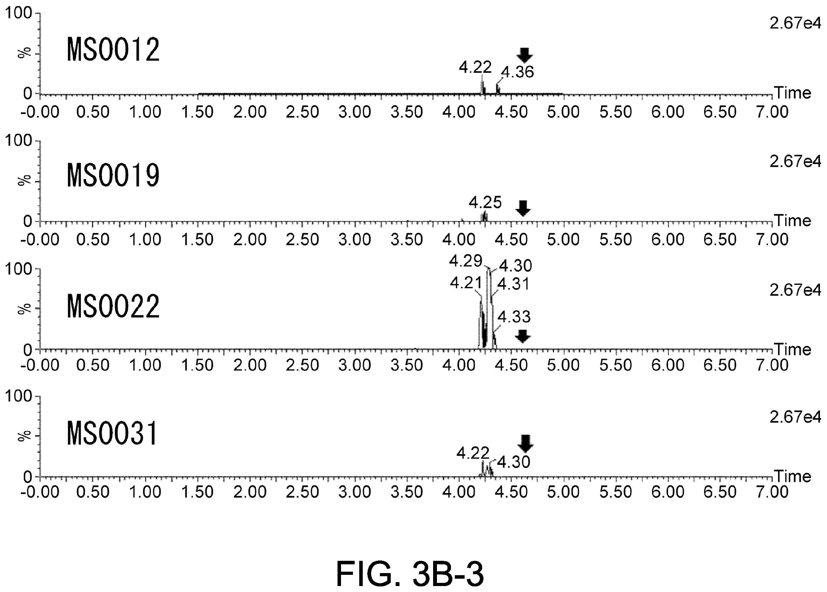

[Fig. 3B-1] Fig. 3B-1 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3B-1 shows the measurement results of a plasma sample to which a modified antibody corresponding to MS0001 alone was added. The peak intensity of MS0001, *i.e.*, 2.67e4, is set as 100% in the vertical axis of each chromatogram in Fig. 3B-1. The name of the measured target peptide is indicated in the upper left of each chromatogram. Each black arrow indicates the expected position of the peak for the measured target peptide.
[Fig. 3B-2] Fig. 3B-2 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3B-2 shows the measurement results of a plasma sample to which a modified antibody corresponding to MS0001 alone was added. The peak intensity of MS0001, *i.e.*, 2.67e4, is set as 100% in the vertical axis of each chromatogram in Fig. 3B-2. The name of the measured target peptide is indicated in the upper left of each chromatogram. Each black arrow indicates the expected position of the peak for the measured target peptide.
[Fig. 3B-3] Fig. 3B-3 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3B-3 shows the measurement results of a plasma sample to which a modified antibody corresponding to MS0001 alone was added. The peak intensity of MS0001, *i.e.*, 2.67e4, is set as 100% in the vertical axis of each chromatogram in Fig. 3B-3. The name of the measured target peptide is indicated in the upper left of each chromatogram. Each black arrow indicates the expected position of the peak for the measured target peptide.

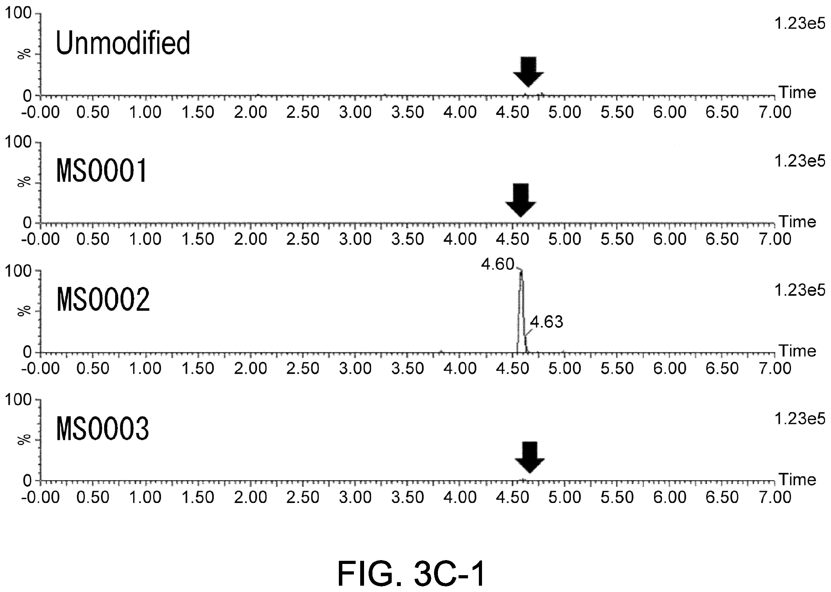

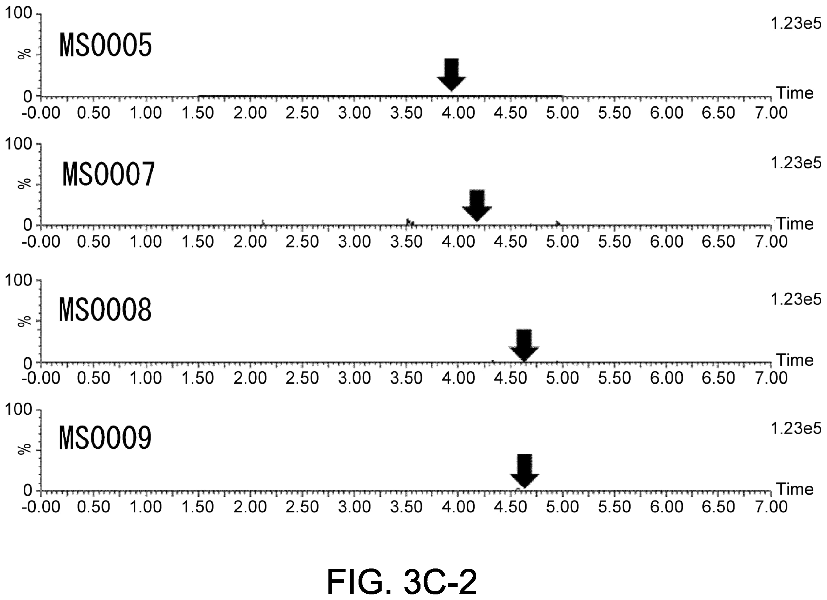

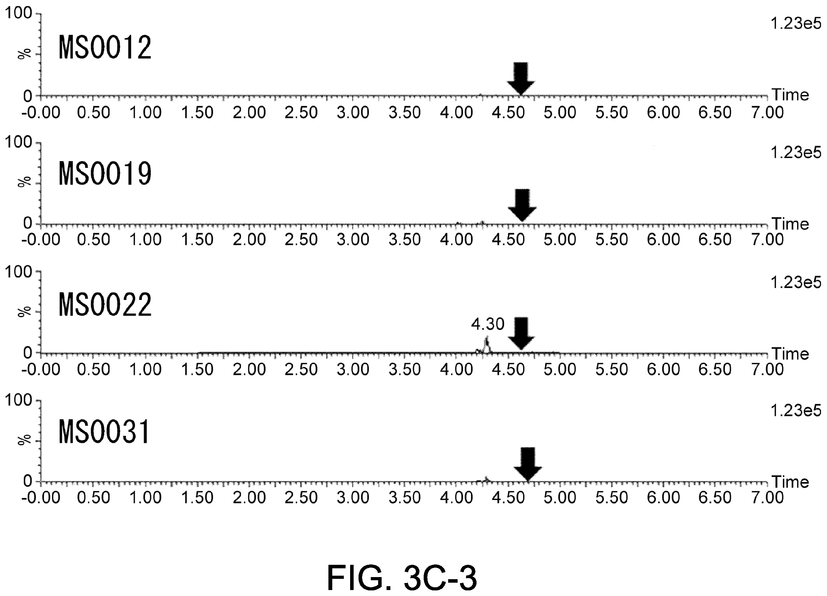

[Fig. 3C-1] Fig. 3C-1 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3C-1 shows the measurement results of a plasma sample to which a modified antibody corresponding to MS0002 alone was added. The peak intensity of MS0002, *i.e.*, 1.23e5, is set as 100% in the vertical axis of each chromatogram in Fig. 3C-1. The name of the measured target peptide is indicated in the upper left of each chromatogram. Each black arrow indicates the expected position of the peak for the measured target peptide.
[Fig. 3C-2] Fig. 3C-2 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3C-2 shows the measurement results of a plasma sample to which a modified antibody corresponding to MS0002 alone was added. The peak intensity of MS0002, *i.e.*, 1.23e5, is set as 100% in the vertical axis of each chromatogram in Fig. 3C-2. The name of the measured target peptide is indicated in the upper left of each chromatogram. Each black arrow indicates the expected position of the peak for the measured target peptide.
[Fig. 3C-3] Fig. 3C-3 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3C-3 shows the measurement results of a plasma sample to which a modified antibody corresponding to MS0002 alone was added. The peak intensity of MS0002, *i*.*e*.*,* 1.23e5, is set as 100% in the vertical axis of each chromatogram in Fig. 3C-3. The name of the measured target peptide is indicated in the upper left of each chromatogram. Each black arrow indicates the expected position of the peak for the measured target peptide.

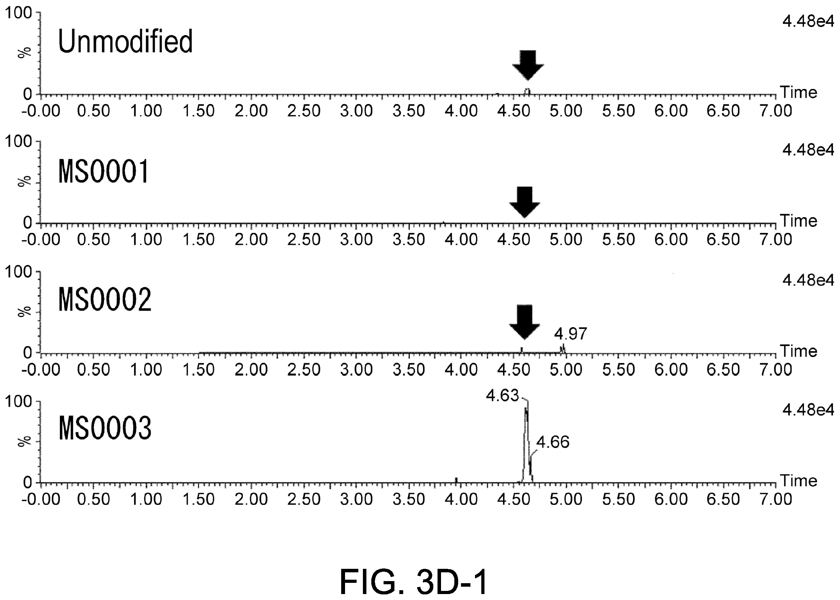

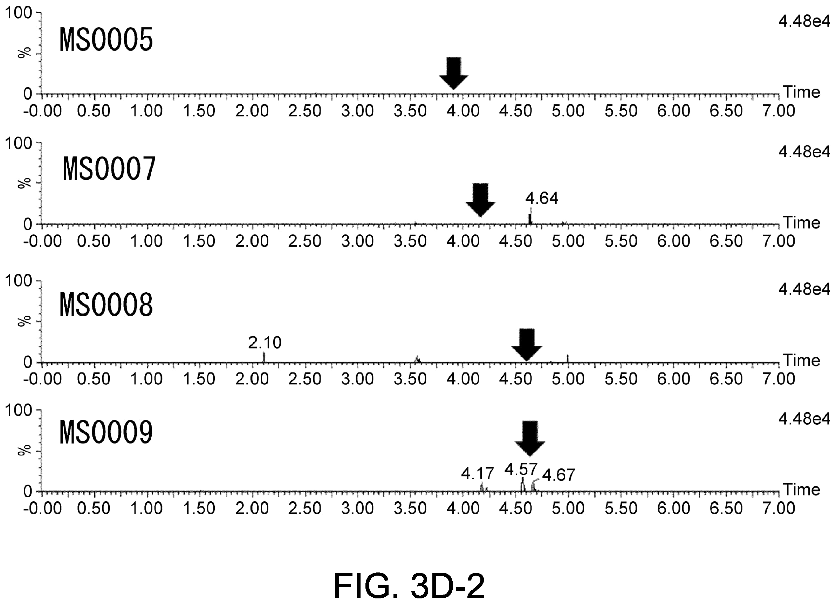

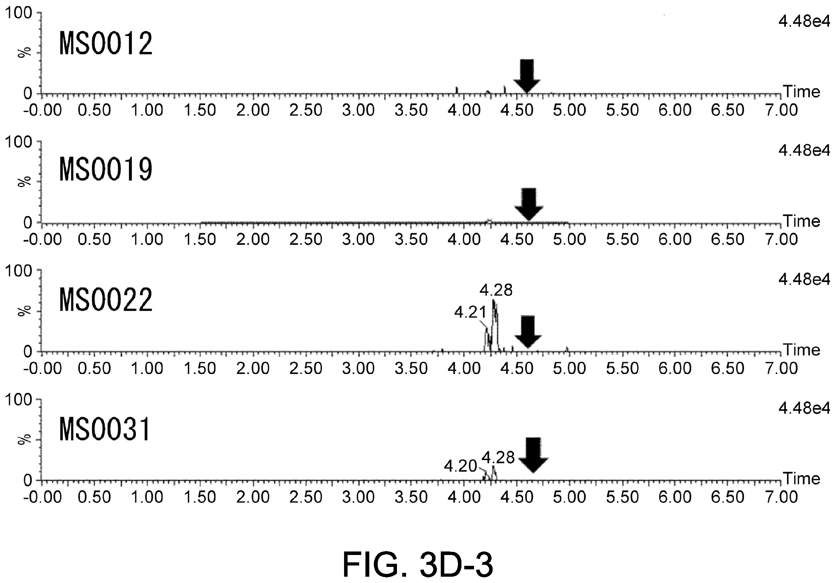

[Fig. 3D-1] Fig. 3D-1 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3D-1 shows the measurement results of a plasma sample to which a modified antibody corresponding to MS0003 alone was added. The peak intensity of MS0003, *i.e.*, 4.48e4, is set as 100% in the vertical axis of each chromatogram in Fig. 3D-1. The name of the measured target peptide is indicated in the upper left of each chromatogram. Each black arrow indicates the expected position of the peak for the measured target peptide.
[Fig. 3D-2] Fig. 3D-2 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3D-2 shows the measurement results of a plasma sample to which a modified antibody corresponding to MS0003 alone was added. The peak intensity of MS0003, *i.e.*, 4.48e4, is set as 100% in the vertical axis of each chromatogram in Fig. 3D-2. The name of the measured target peptide is indicated in the upper left of each chromatogram. Each black arrow indicates the expected position of the peak for the measured target peptide.
[Fig. 3D-3] Fig. 3D-3 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3D-3 shows the measurement results of a plasma sample to which a modified antibody corresponding to MS0003 alone was added. The peak intensity of MS0003, *i.e.*, 4.48e4, is set as 100% in the vertical axis of each chromatogram in Fig. 3D-3. The name of the measured target peptide is indicated in the upper left of each chromatogram. Each black arrow indicates the expected position of the peak for the measured target peptide.

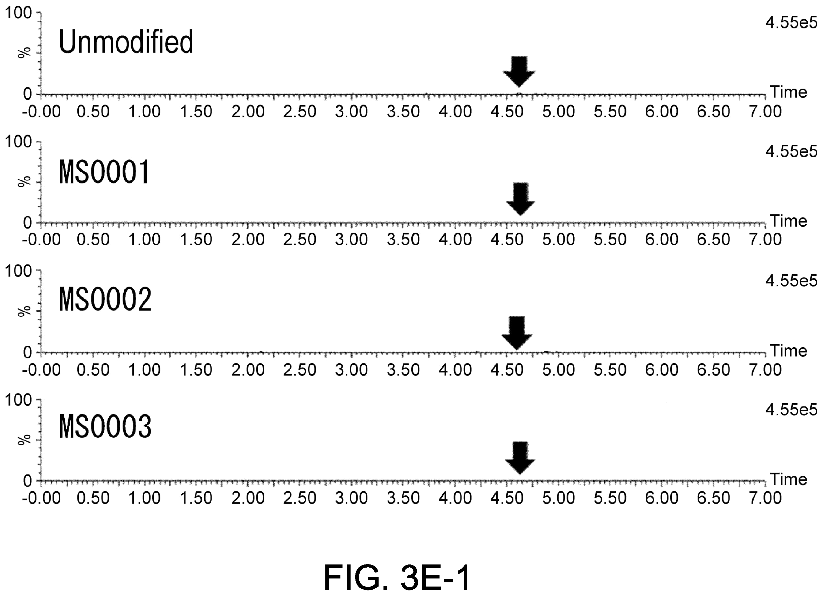

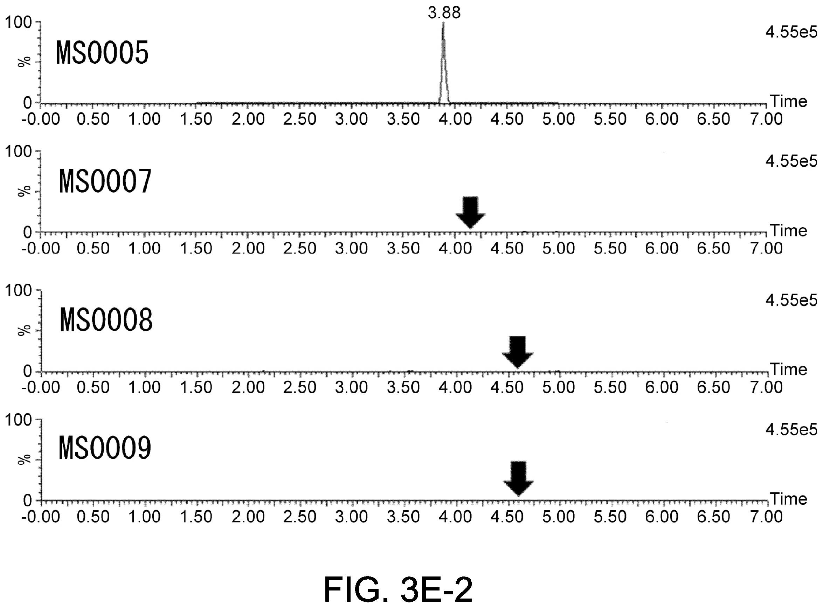

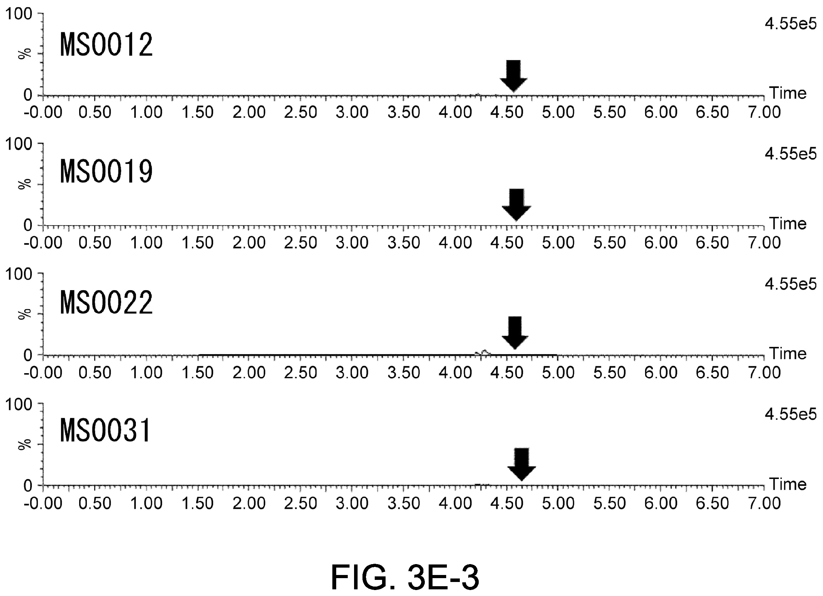

[Fig. 3E-1] Fig. 3E-1 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3E-1 shows the measurement results of a plasma sample to which a modified antibody corresponding to MS0005 alone was added. The peak intensity of MS0005, *i.e.*, 4.55e5, is set as 100% in the vertical axis of each chromatogram in Fig. 3E-1. The name of the measured target peptide is indicated in the upper left of each chromatogram. Each black arrow indicates the expected position of the peak for the measured target peptide.
[Fig. 3E-2] Fig. 3E-2 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3E-2 shows the measurement results of a plasma sample to which a modified antibody corresponding to MS0005 alone was added. The peak intensity of MS0005, *i.e.*, 4.55e5, is set as 100% in the vertical axis of each chromatogram in Fig. 3E-2. The name of the measured target peptide is indicated in the upper left of each chromatogram. Each black arrow indicates the expected position of the peak for the measured target peptide.
[Fig. 3E-3] Fig. 3E-3 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3E-3 shows the measurement results of a plasma sample to which a modified antibody corresponding to MS0005 alone was added. The peak intensity of MS0005, *i.e.*, 4.55e5, is set as 100% in the vertical axis of each chromatogram in Fig. 3E-3. The name of the measured target peptide is indicated in the upper left of each chromatogram. Each black arrow indicates the expected position of the peak for the measured target peptide.

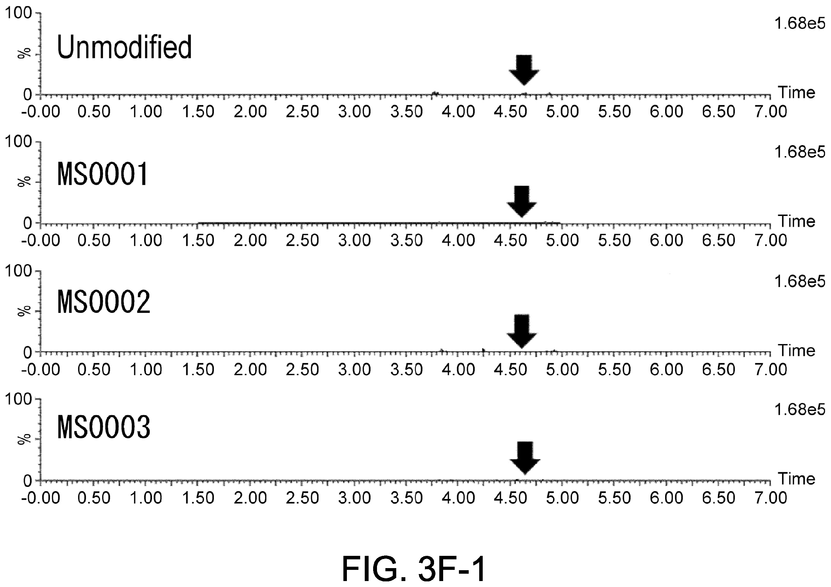

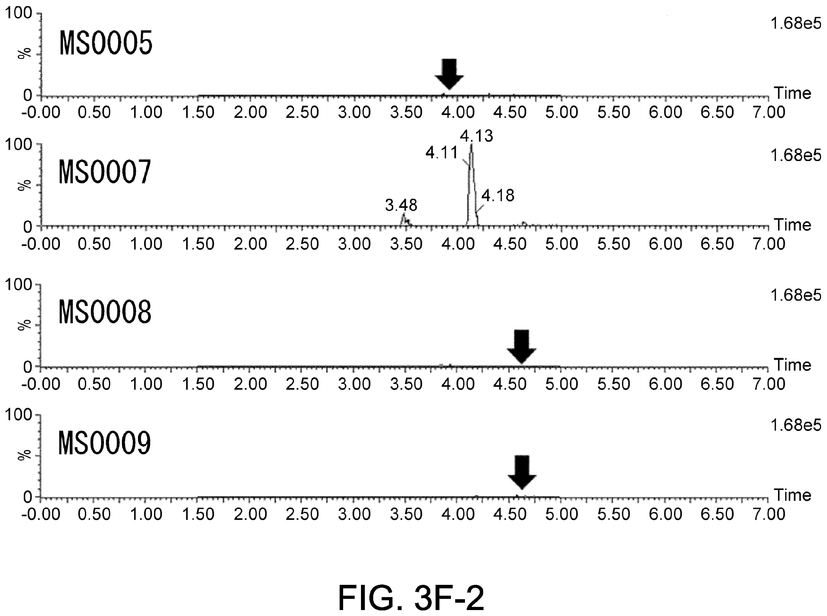

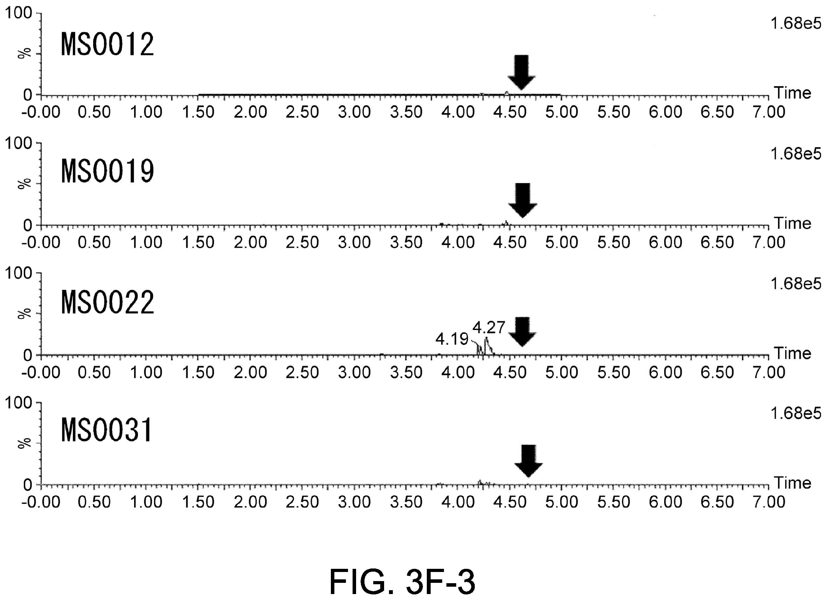

[Fig. 3F-1] Fig. 3F-1 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3F-1 shows the measurement results of a plasma sample to which a modified antibody corresponding to MS0007 alone was added. The peak intensity of MS0007, *i.e.*, 1.68e5, is set as 100% in the vertical axis of each chromatogram in Fig. 3F-1. The name of the measured target peptide is indicated in the upper left of each chromatogram. Each black arrow indicates the expected position of the peak for the measured target peptide.
[Fig. 3F-2] Fig. 3F-2 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3F-2 shows the measurement results of a plasma sample to which a modified antibody corresponding to MS0007 alone was added. The peak intensity of MS0007, *i*.*e*., 1.68e5, is set as 100% in the vertical axis of each chromatogram in Fig. 3F-2. The name of the measured target peptide is indicated in the upper left of each chromatogram. Each black arrow indicates the expected position of the peak for the measured target peptide.
[Fig. 3F-3] Fig. 3F-3 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3F-3 shows the measurement results of a plasma sample to which a modified antibody corresponding to MS0007 alone was added. The peak intensity of MS0007, *i.e.*, 1.68e5, is set as 100% in the vertical axis of each chromatogram in Fig. 3F-3. The name of the measured target peptide is indicated in the upper left of each chromatogram. Each black arrow indicates the expected position of the peak for the measured target peptide.

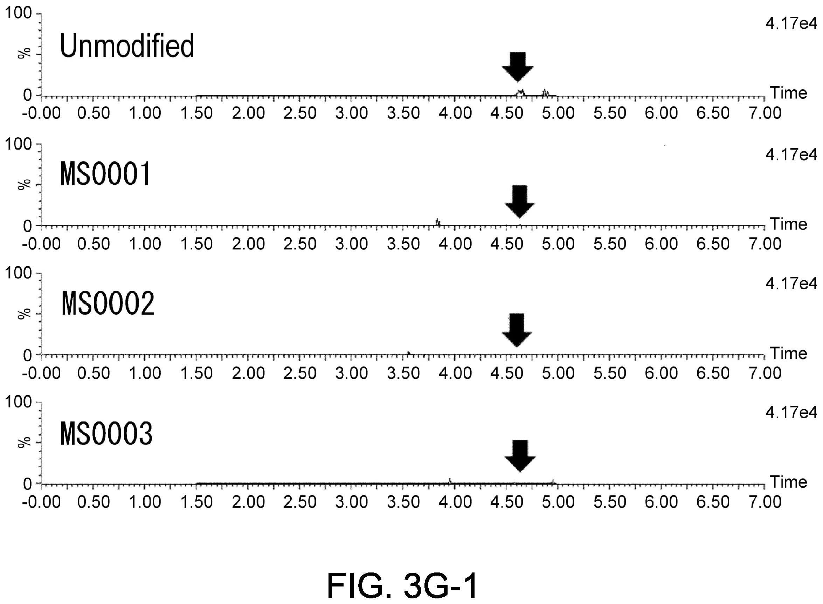

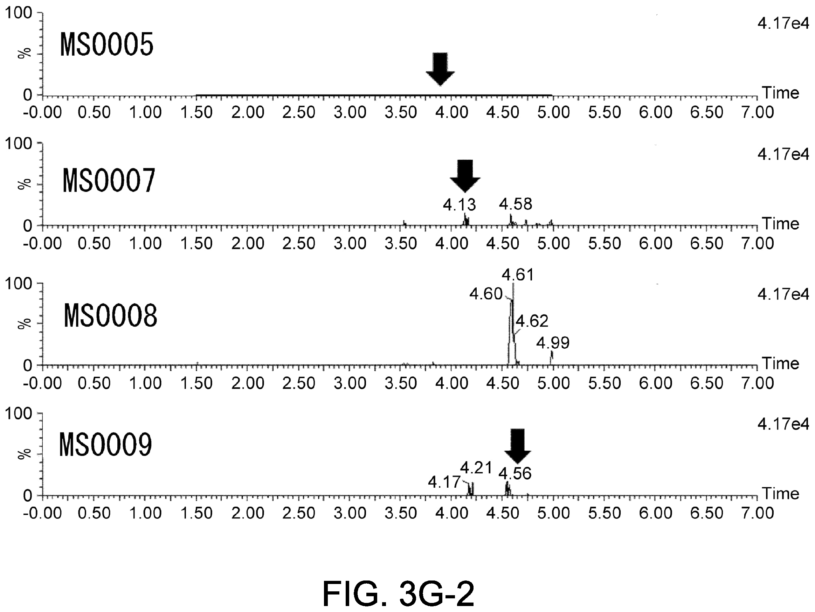

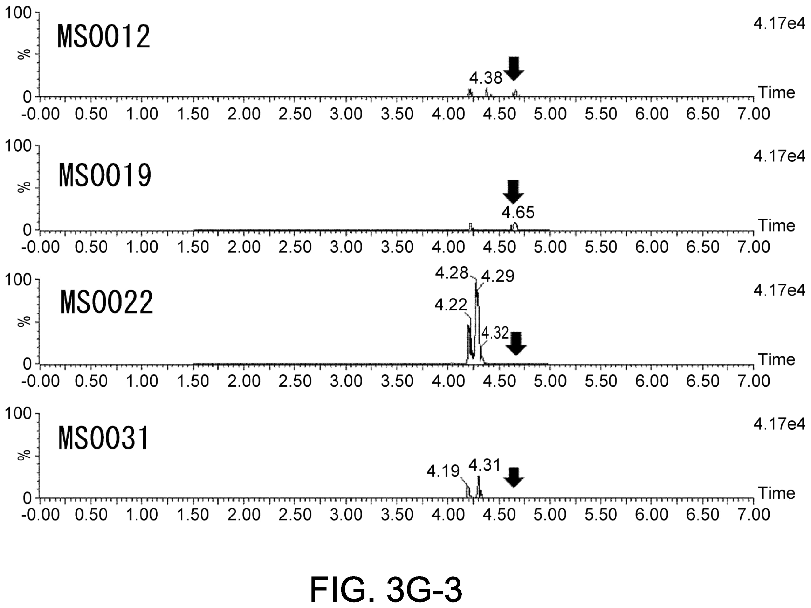

[Fig. 3G-1] Fig. 3G-1 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3G-1 shows the measurement results of a plasma sample to which a modified antibody corresponding to MS0008 alone was added. The peak intensity of MS0008, *i.e.*, 4.17e4, is set as 100% in the vertical axis of each chromatogram in Fig. 3G-1. The name of the measured target peptide is indicated in the upper left of each chromatogram. Each black arrow indicates the expected position of the peak for the measured target peptide.
[Fig. 3G-2] Fig. 3G-2 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3G-2 shows the measurement results of a plasma sample to which a modified antibody corresponding to MS0008 alone was added. The peak intensity of MS0008, *i.e.*, 4.17e4, is set as 100% in the vertical axis of each chromatogram in Fig. 3G-2. The name of the measured target peptide is indicated in the upper left of each chromatogram. Each black arrow indicates the expected position of the peak for the measured target peptide.
[Fig. 3G-3] Fig. 3G-3 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3G-3 shows the measurement results of a plasma sample to which a modified antibody corresponding to MS0008 alone was added. The peak intensity of MS0008, *i.e.*, 4.17e4, is set as 100% in the vertical axis of each chromatogram in Fig. 3G-3. The name of the measured target peptide is indicated in the upper left of each chromatogram. Each black arrow indicates the expected position of the peak for the measured target peptide.

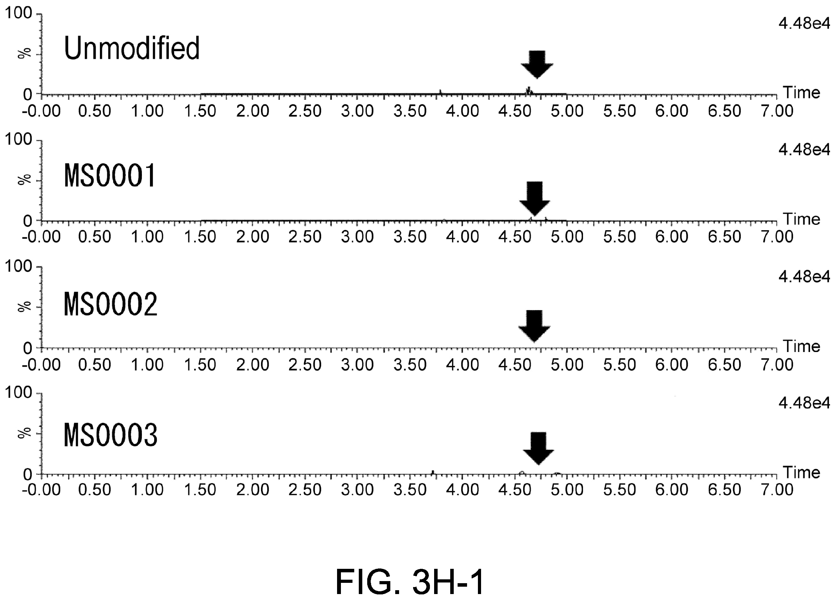

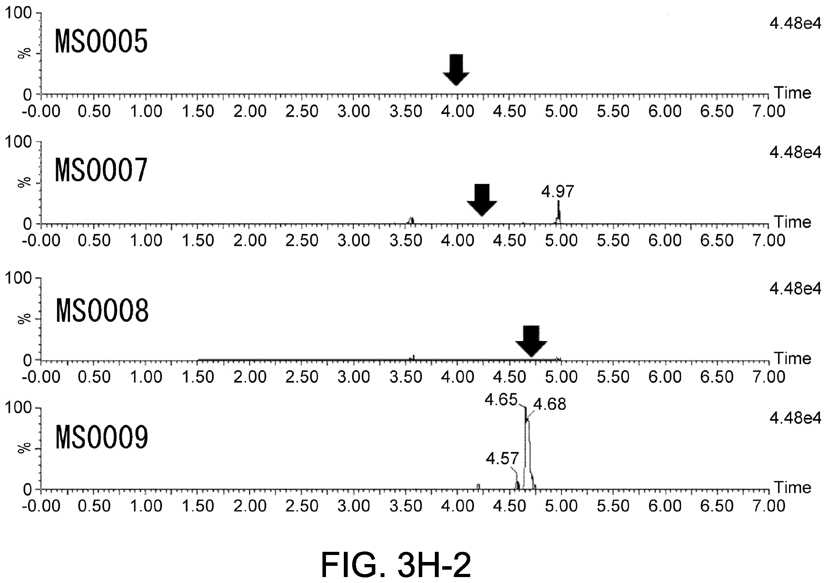

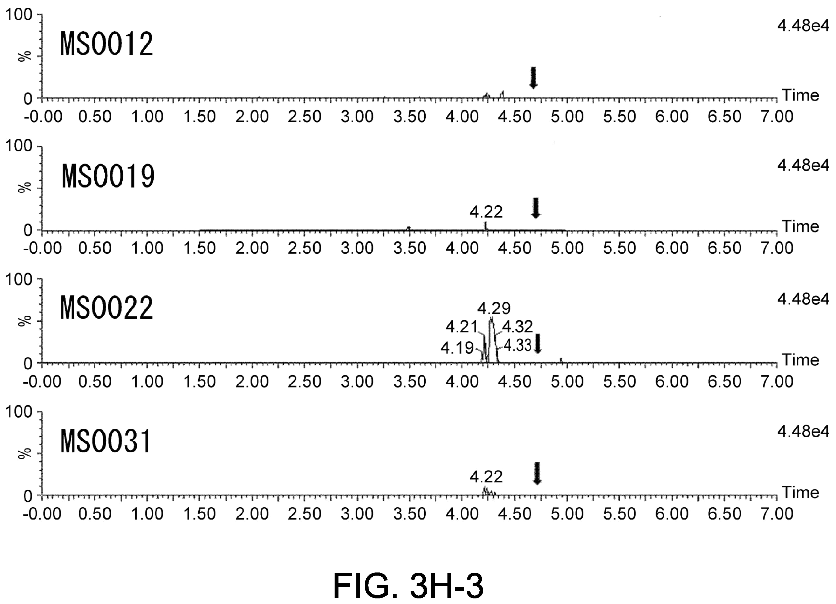

[Fig. 3H-1] Fig. 3H-1 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3H-1 shows the measurement results of a plasma sample to which a modified antibody corresponding to MS0009 alone was added. The peak intensity of MS0009, *i.e.*, 4.48e4, is set as 100% in the vertical axis of each chromatogram in Fig. 3H-1. The name of the measured target peptide is indicated in the upper left of each chromatogram. Each black arrow indicates the expected position of the peak for the measured target peptide.
[Fig. 3H-2] Fig. 3H-2 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3H-2 shows the measurement results of a plasma sample to which a modified antibody corresponding to MS0009 alone was added. The peak intensity of MS0009, *i.e.*, 4.48e4, is set as 100% in the vertical axis of each chromatogram in Fig. 3H-2. The name of the measured target peptide is indicated in the upper left of each chromatogram. Each black arrow indicates the expected position of the peak for the measured target peptide.
[Fig. 3H-3] Fig. 3H-3 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3H-3 shows the measurement results of a plasma sample to which a modified antibody corresponding to MS0009 alone was added. The peak intensity of MS0009, *i.e.*, 4.48e4, is set as 100% in the vertical axis of each chromatogram in Fig. 3H-3. The name of the measured target peptide is indicated in the upper left of each chromatogram. Each black arrow indicates the expected position of the peak for the measured target peptide.

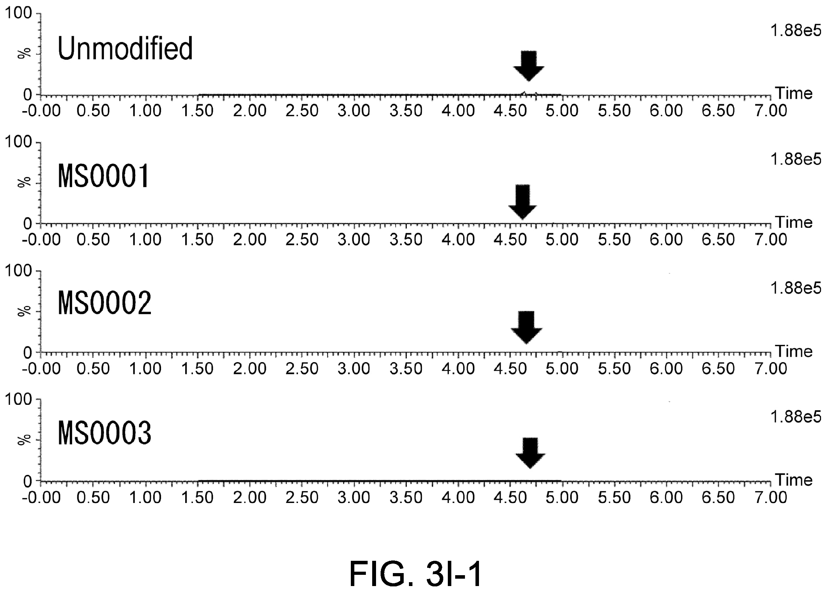

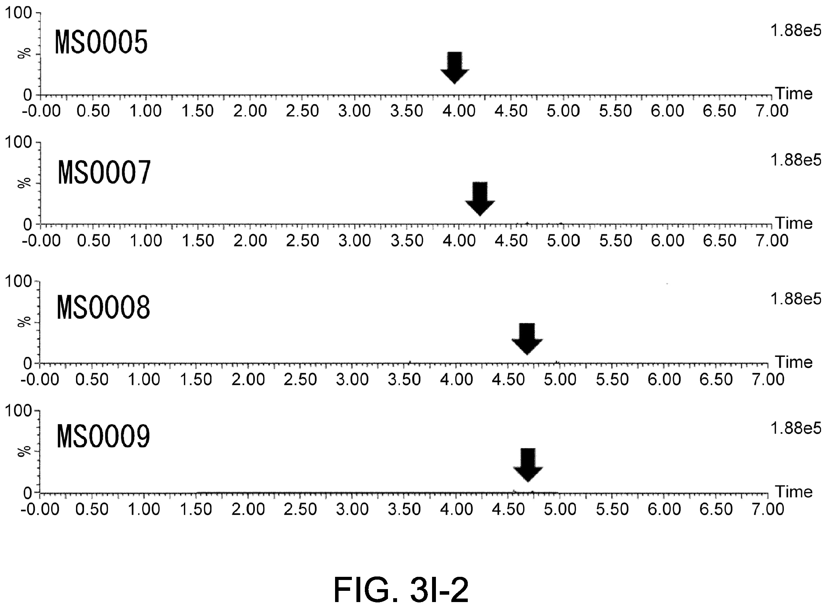

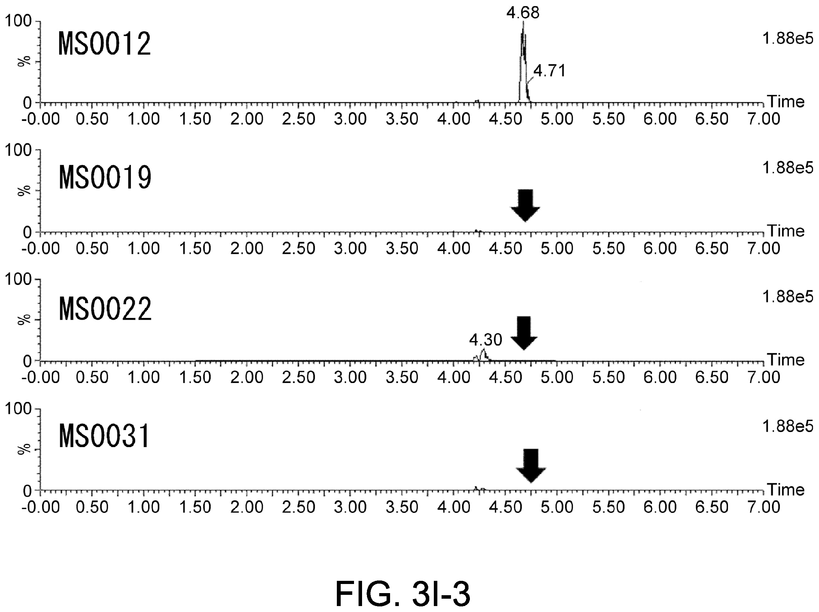

[Fig. 3I-1] Fig. 3I-1 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3I-1 shows the measurement results of a plasma sample to which a modified antibody corresponding to MS0012 alone was added. The peak intensity of MS0012, *i.e.*, 1.88e5, is set as 100% in the vertical axis of each chromatogram in Fig. 3I-1. The name of the measured target peptide is indicated in the upper left of each chromatogram. Each black arrow indicates the expected position of the peak for the measured target peptide.
[Fig. 3I-2] Fig. 3I-2 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3I-2 shows the measurement results of a plasma sample to which a modified antibody corresponding to MS0012 alone was added. The peak intensity of MS0012, *i.e.*, 1.88e5, is set as 100% in the vertical axis of each chromatogram in Fig. 3I-2. The name of the measured target peptide is indicated in the upper left of each chromatogram. Each black arrow indicates the expected position of the peak for the measured target peptide.
[Fig. 3I-3] Fig. 3I-3 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3I-3 shows the measurement results of a plasma sample to which a modified antibody corresponding to MS0012 alone was added. The peak intensity of MS0012, *i.e.*, 1.88e5, is set as 100% in the vertical axis of each chromatogram in Fig. 3I-3. The name of the measured target peptide is indicated in the upper left of each chromatogram. Each black arrow indicates the expected position of the peak for the measured target peptide.

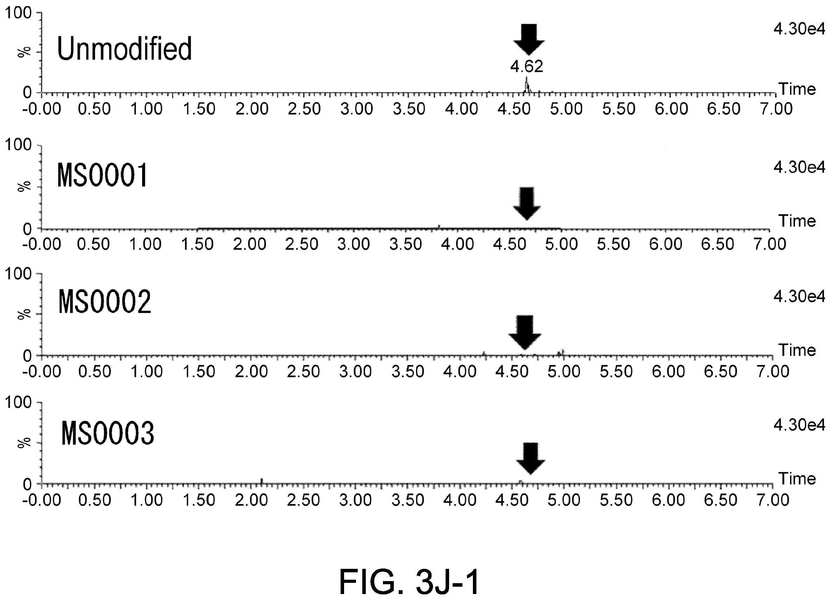

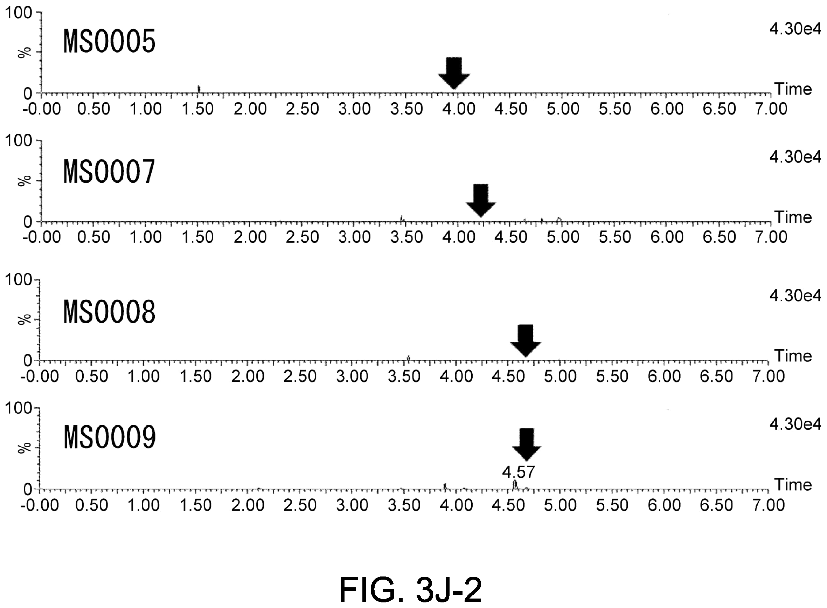

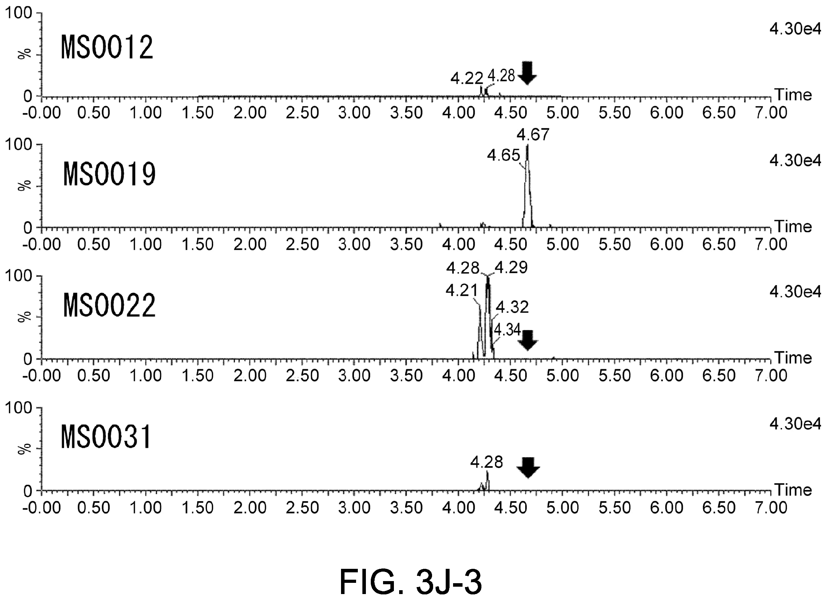

[Fig. 3J-1] Fig. 3J-1 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3J-1 shows the measurement results of a plasma sample to which a modified antibody corresponding to MS0019 alone was added. The peak intensity of MS0019, *i.e.*, 4.30e4, is set as 100% in the vertical axis of each chromatogram in Fig. 3J-1. The name of the measured target peptide is indicated in the upper left of each chromatogram. Each black arrow indicates the expected position of the peak for the measured target peptide.
[Fig. 3J-2] Fig. 3J-2 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3J-2 shows the measurement results of a plasma sample to which a modified antibody corresponding to MS0019 alone was added. The peak intensity of MS0019, *i.e.*, 4.30e4, is set as 100% in the vertical axis of each chromatogram in Fig. 3J-2. The name of the measured target peptide is indicated in the upper left of each chromatogram. Each black arrow indicates the expected position of the peak for the measured target peptide.
[Fig. 3J-3] Fig. 3J-3 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3J-3 shows the measurement results of a plasma sample to which a modified antibody corresponding to MS0019 alone was added. The peak intensity of MS0019, *i.e.*, 4.30e4, is set as 100% in the vertical axis of each chromatogram in Fig. 3J-3. The name of the measured target peptide is indicated in the upper left of each chromatogram. Each black arrow indicates the expected position of the peak for the measured target peptide.

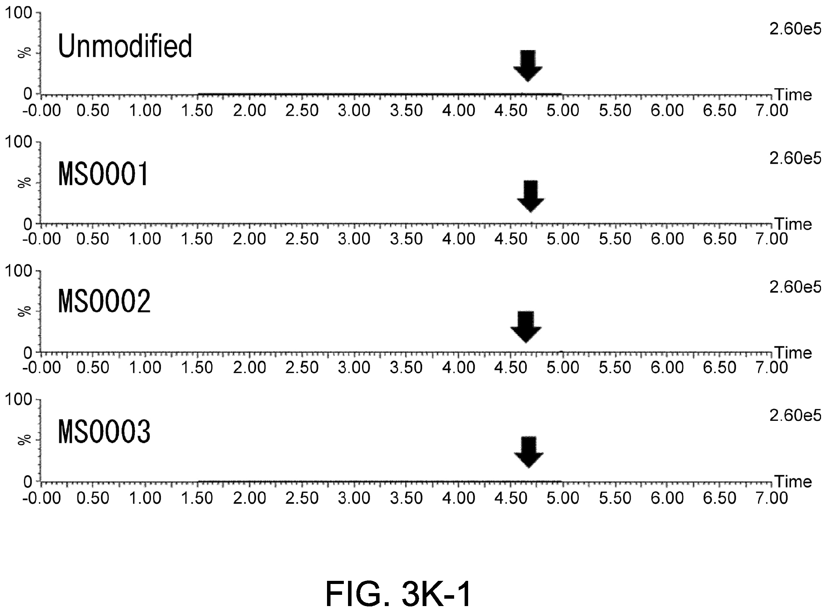

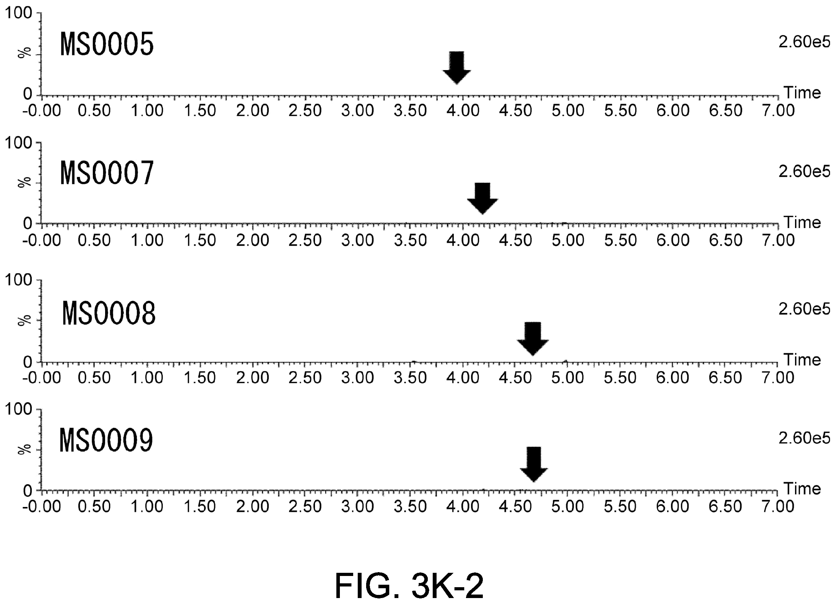

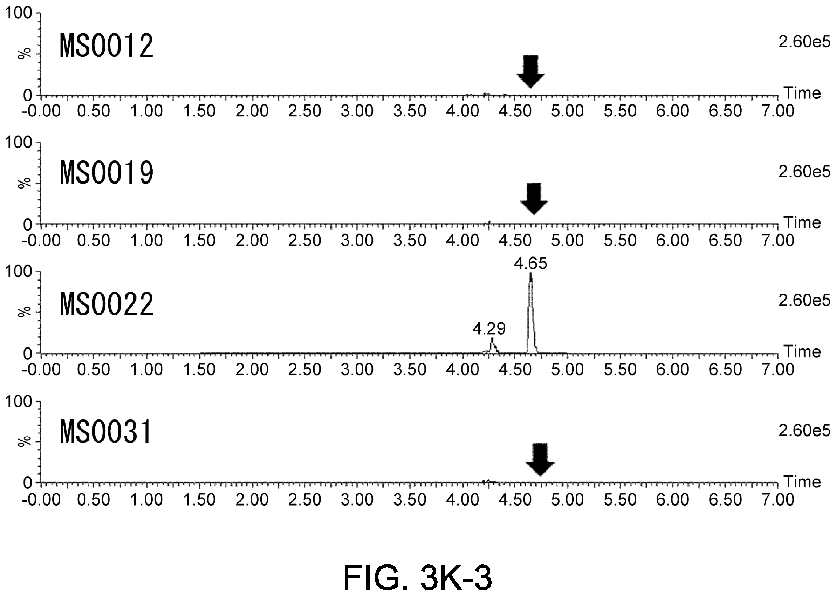

[Fig. 3K-1] Fig. 3K-1 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3K-1 shows the measurement results of a plasma sample to which a modified antibody corresponding to MS0022 alone was added. The peak intensity of MS0022, *i.e.*, 2.60e5, is set as 100% in the vertical axis of each chromatogram in Fig. 3K-1. The name of the measured target peptide is indicated in the upper left of each chromatogram. Each black arrow indicates the expected position of the peak for the measured target peptide.
[Fig. 3K-2] Fig. 3K-2 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3K-2 shows the measurement results of a plasma sample to which a modified antibody corresponding to MS0022 alone was added. The peak intensity of MS0022, *i.e.*, 2.60e5, is set as 100% in the vertical axis of each chromatogram in Fig. 3K-2. The name of the measured target peptide is indicated in the upper left of each chromatogram. Each black arrow indicates the expected position of the peak for the measured target peptide.
[Fig. 3K-3] Fig. 3K-3 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3K-3 shows the measurement results of a plasma sample to which a modified antibody corresponding to MS0022 alone was added. The peak intensity of MS0022, *i.e.*, 2.60e5, is set as 100% in the vertical axis of each chromatogram in Fig. 3K-3. The name of the measured target peptide is indicated in the upper left of each chromatogram. Each black arrow indicates the expected position of the peak for the measured target peptide.

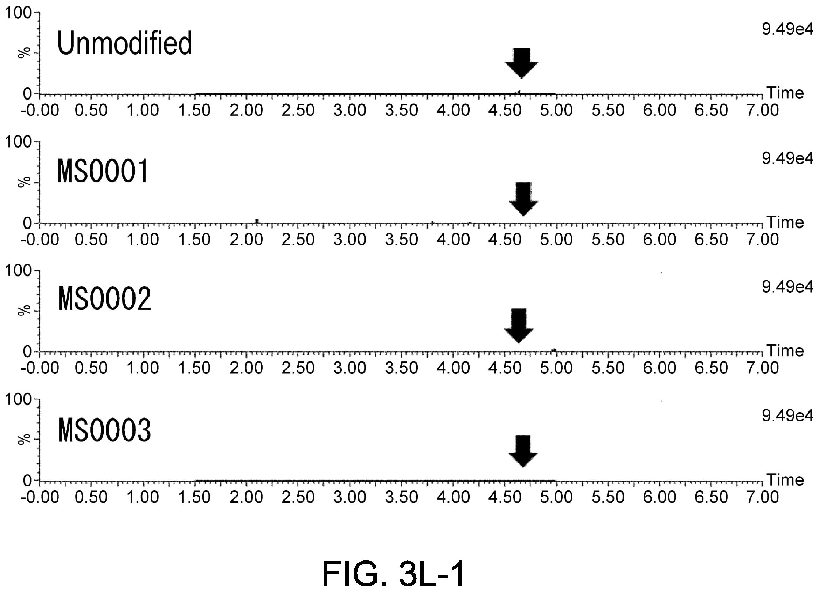

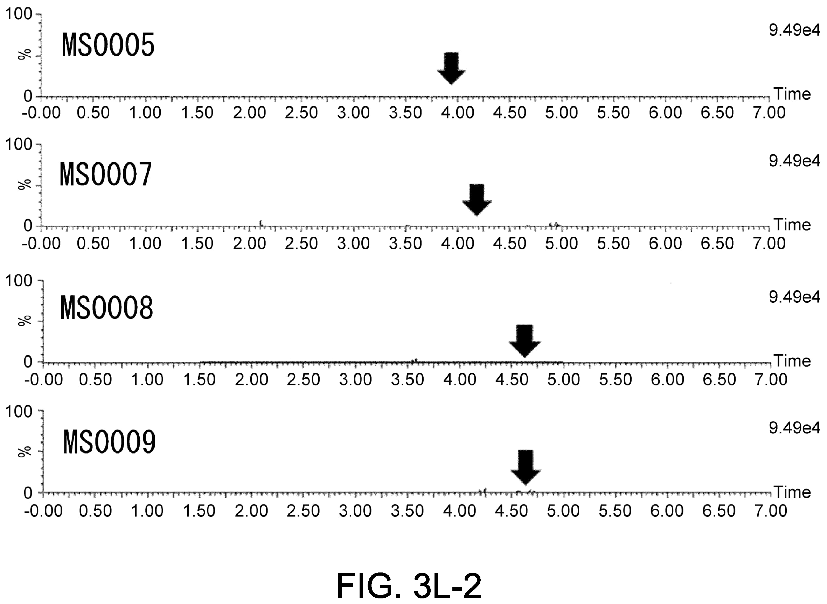

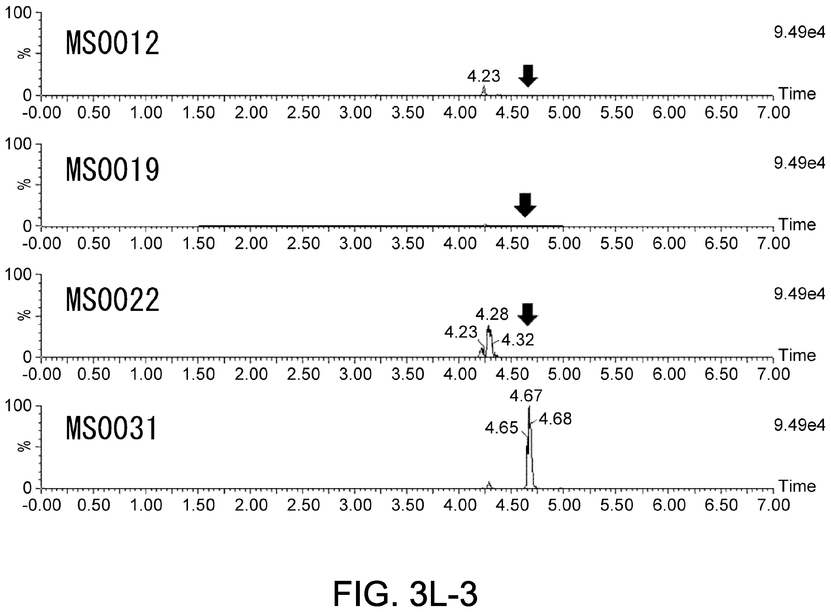

[Fig. 3L-1] Fig. 3L-1 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3L-1 shows the measurement results of a plasma sample to which a modified antibody corresponding to MS0031 alone was added. The peak intensity of MS0031, *i.e.*, 9.49e4, is set as 100% in the vertical axis of each chromatogram in Fig. 3L-1. The name of the measured target peptide is indicated in the upper left of each chromatogram. Each black arrow indicates the expected position of the peak for the measured target peptide.
[Fig. 3L-2] Fig. 3L-2 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3L-2 shows the measurement results of a plasma sample to which a modified antibody corresponding to MS0031 alone was added. The peak intensity of MS0031, *i.e.*, 9.49e4, is set as 100% in the vertical axis of each chromatogram in Fig. 3L-2. The name of the measured target peptide is indicated in the upper left of each chromatogram. Each black arrow indicates the expected position of the peak for the measured target peptide.
[Fig. 3L-3] Fig. 3L-3 shows SRM chromatograms of a plasma sample to which a single antibody standard solution was added (1.28 µg/mL mouse plasma concentration) to confirm specificity. The horizontal axis of each chromatogram shows the elution time (min) in LC. Fig. 3L-3 shows the measurement results of a plasma sample to which a modified antibody corresponding to MS0031 alone was added. The peak intensity of MS0031, *i.e.*, 9.49e4, is set as 100% in the vertical axis of each chromatogram in Fig. 3L-3. The name of the measured target peptide is indicated in the upper left of each chromatogram. Each black arrow indicates the expected position of the peak for the measured target peptide.

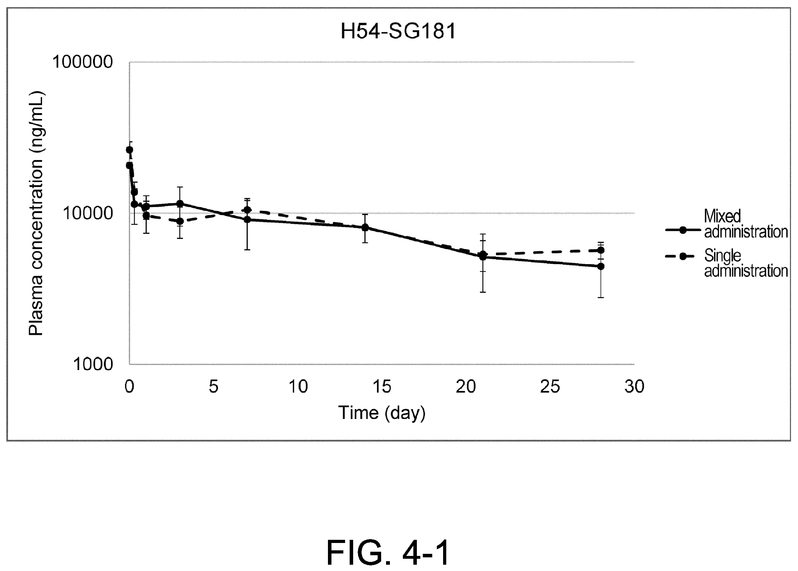

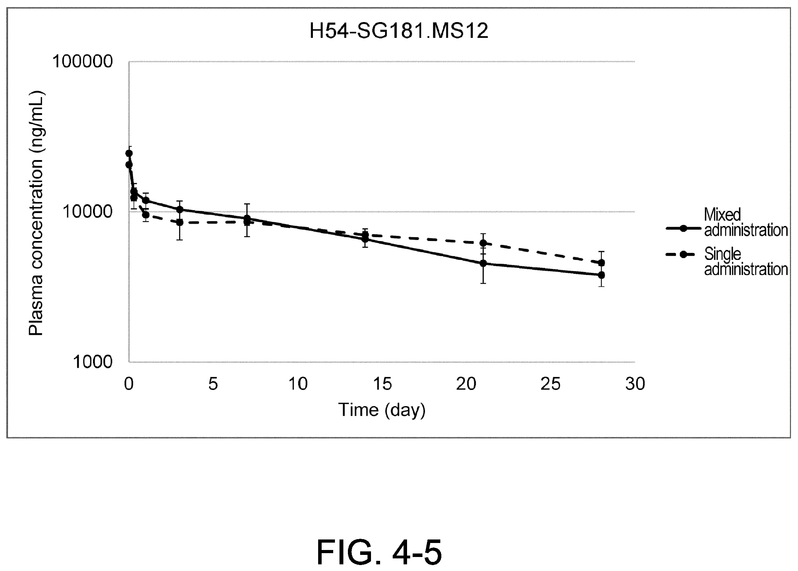

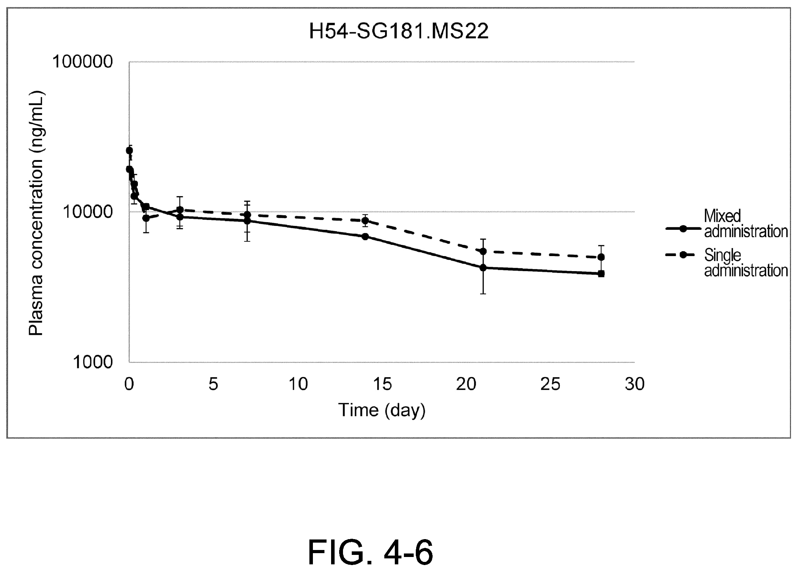

[Fig.4-1] Fig. 4-1 shows the plasma antibody concentration-time curves of H54-SG181 after mixed administration and single administration to mice. The title of the figure is the name of the measured target antibody, the horizontal axis is the elapsed time (days) after drug administration, and the vertical axis is the plasma concentration of the target antibody (ng/mL).

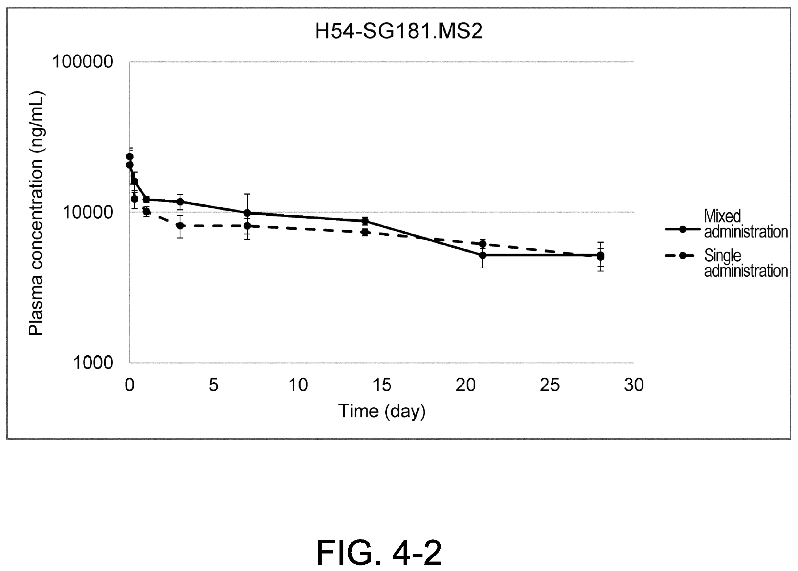

[Fig.4-2] Fig. 4-2 shows the plasma antibody concentration-time curves of H54-SG181.MS2 after mixed administration and single administration to mice. The title of the figure is the name of the measured target antibody, the horizontal axis is the elapsed time (days) after drug administration, and the vertical axis is the plasma concentration of the target antibody (ng/mL).

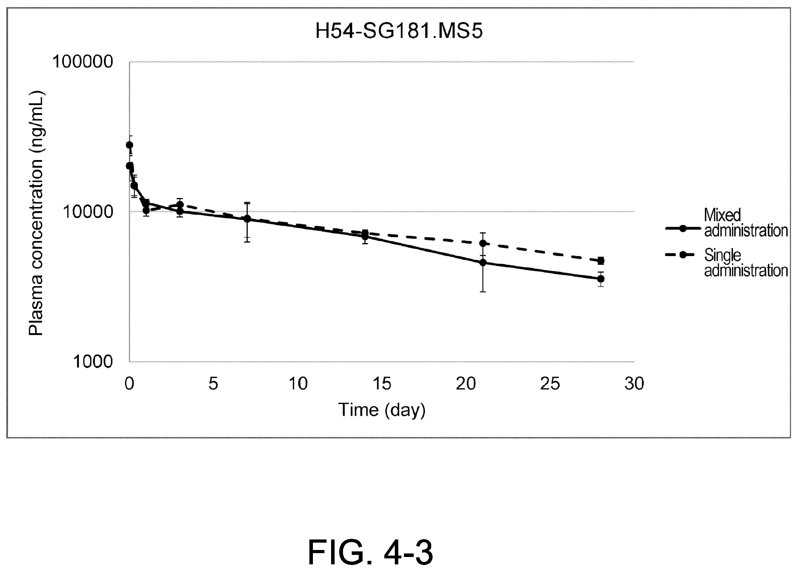

[Fig.4-3] Fig. 4-3 shows the plasma antibody concentration-time curves of H54-SG181.MS5 after mixed administration and single administration to mice. The title of the figure is the name of the measured target antibody, the horizontal axis is the elapsed time (days) after drug administration, and the vertical axis is the plasma concentration of the target antibody (ng/mL).

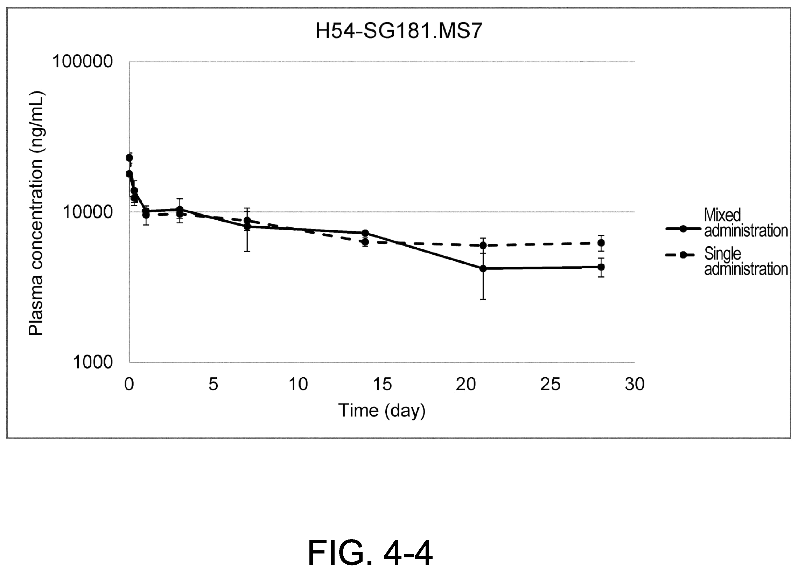

[Fig.4-4] Fig. 4-4 shows the plasma antibody concentration-time curves of H54-SG181.MS7 after mixed administration and single administration to mice. The title of the figure is the name of the measured target antibody, the horizontal axis is the elapsed time (days) after drug administration, and the vertical axis is the plasma concentration of the target antibody (ng/mL).
[Fig.4-5] Fig. 4-5 shows the plasma antibody concentration-time curves of H54-SG181.MS12 after mixed administration and single administration to mice. The title of the figure is the name of the measured target antibody, the horizontal axis is the elapsed time (days) after drug administration, and the vertical axis is the plasma concentration of the target antibody (ng/mL).
[Fig.4-6] Fig. 4-6 shows the plasma antibody concentration-time curves of H54-SG181.MS22 after mixed administration and single administration to mice. The title of the figure is the name of the measured target antibody, the horizontal axis is the elapsed time (days) after drug administration, and the vertical axis is the plasma concentration of the target antibody (ng/mL).

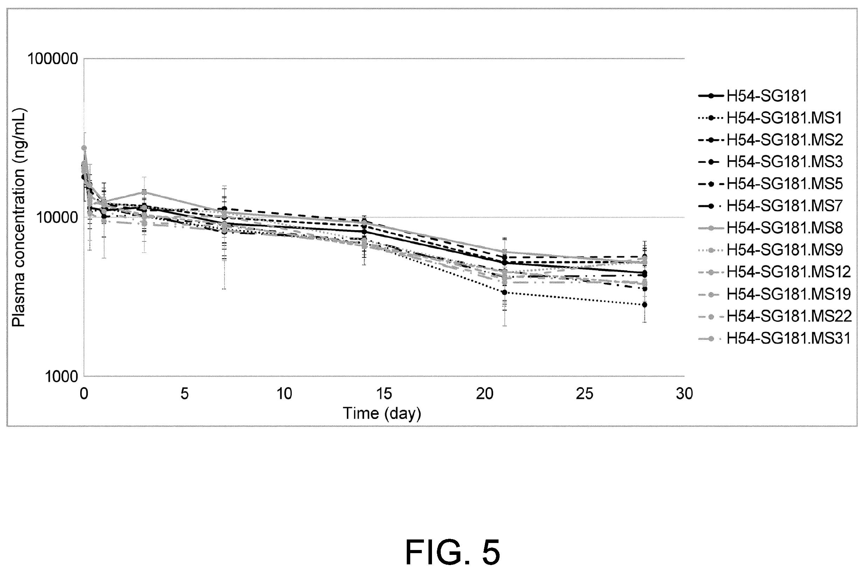

[Fig.5] Fig. 5 shows the plasma antibody concentration-time curves of the antibodies comprising unmodified sequence or alanine-modified sequences after mixed administration to mice. The horizontal axis of the figure is the elapsed time (days) after drug administration, and the vertical axis is the plasma concentration of the target antibody (ng/mL).

### [Description of Embodiments]

### I. DEFINITIONS

The term "and/or" herein is used to indicate any one of the subjects shown before and after "and/or", or any combination thereof. For example, "A, B, and/or C" includes the individual subjects "A", "B", and "C", and also combinations "A and B", "A and C", "B and C", and "A and B and C". The term "and/or" is used interchangeably with "both or either" or "all or any one of".

The term "antigen-binding molecule" herein, in its broadest sense, refers to a molecule that specifically binds to an antigen determinant (epitope). In one embodiment, an antigen binding molecule is an antibody, an antibody fragment, or an antibody derivative. In one embodiment, an antigen-binding molecule is a non-antibody protein, or a fragment thereof or a derivative thereof. In one embodiment, an antigen-binding molecule is an antibody-like molecule, or a fragment thereof or a derivative thereof. In one embodiment, an antigen-binding molecule is a multispecific antigen-binding molecule (e.g., bispecific antigen-binding molecule), or a fragment thereof or a derivative thereof. In one embodiment, an antigen-binding molecule is an antibody fragment comprising an antibody heavy chain variable region (VH) and an antibody light chain variable region (VL). In one embodiment, an antigen-binding molecule is an antibody fragment comprising a Fab. In one embodiment, an antigen-binding molecule is an IgG antibody.

The antigen-binding molecule of the present application is not limited in terms of its origin, and may be a human antibody, a mouse antibody, a rat antibody, or the like. It may also be a genetically modified antibody such as a chimeric antibody and a humanized antibody.

Herein, the term "multispecific antigen-binding molecule" is an antigen-binding molecule comprising two or more different antigen-binding domains specific to different antigens or epitopes. In one embodiment, the multispecific antigen-binding molecule in the present application is a multispecific antibody. The multispecific antibody is not particularly limited, but preferably is a monoclonal antibody.

Herein, an "antigen-binding domain" refers to a region that specifically binds and is complementary to a whole antigen or a portion thereof. Herein, an antigen-binding molecule comprises an antigen-binding domain. When the molecular weight of an antigen is large, an antigen-binding domain can only bind to a particular portion of the antigen. Such a particular portion is called an "epitope". In one embodiment, an antigen-binding domain comprises an antibody fragment which binds to a particular antigen. An antigen-binding domain can be provided from one or more antibody variable domains. In a non-limiting embodiment, an antigen-binding domain comprises an antibody light chain variable region (VL) and an antibody heavy chain variable region (VH). Examples of such antigen-binding domains include "single-chain Fv (scFv)", "single-chain antibody", "Fv", and "single-chain Fv2 (scFv2)".

Herein, "specifically bind" means that one of the specifically binding molecules binds to one or more binding partner molecules thereof without showing any significant binding to molecules other than the binding partner molecules. This is also used when an antigen-binding domain is specific to a particular epitope among multiple epitopes contained in an antigen. When an epitope bound by an antigen-binding domain is contained in multiple different antigens, antigen-binding molecules having the antigen-binding domain can bind to various antigens that have the epitope.

The term "antibody" herein is used in the broadest sense and encompasses various antibody structures, including but not limited to monoclonal antibodies, multispecific antibodies (e.g., bispecific antibodies), antibody fragments, and modified antibodies so long as they exhibit the desired antigen-binding activity.

The term "binding activity" refers to the strength of the total non-covalent interactions between one or more binding sites of a molecule (e.g., an antibody) and the molecule's binding partner (e.g., an antigen). Here, binding activity is not strictly limited to 1:1 interactions between the members of a binding pair (e.g., an antibody and an antigen). For example, when the members of a binding pair reflect a monovalent 1:1 interaction, the binding activity refers to intrinsic binding affinity ("affinity"). When members of a binding pair are capable of both monovalent and multivalent binding, the binding activity is the sum of these binding forces. The binding activity of a molecule X for its partner Y can generally be expressed by the dissociation constant (KD) or the "amount of analyte bound per unit amount of ligand". Binding activity can be measured by conventional methods known in the art, including those described herein.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g. scFv); and multispecific antibodies formed from antibody fragments.

The term "chimeric" antibody refers to an antibody in which a portion of the heavy and/or light chain is derived from a particular source or species, while the remainder of the heavy and/or light chain is derived from a different source or species.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of immunoglobulins are called alpha, delta, epsilon, gamma, and mu, respectively.

In one embodiment of the present application, constant regions are preferably antibody constant regions, more preferably IgG1, IgG2, IgG3, and IgG4-type antibody constant regions, and even more preferably human IgG1, IgG2, IgG3, and IgG4-type antibody constant regions. In another embodiment of the present application, constant regions are preferably heavy chain constant regions, more preferably IgG1, IgG2, IgG3, and IgG4-type heavy chain constant regions, and even more preferably human IgG1, IgG2, IgG3, and IgG4-type heavy chain constant regions. The amino acid sequences of the human IgG1 constant region, the human IgG2 constant region, the human IgG3 constant region, and the human IgG4 constant region are known. For the constant regions of human IgG1, human IgG2, human IgG3, and human IgG4 antibodies, a plurality of allotype sequences based on gene polymorphism are described in Sequences of proteins of immunological interest, NIH Publication No. 91-3242. Any of these sequences can be used in the present application. Amino acid-modified constant regions of the present application may contain other amino acid mutations or modifications, as long as they include an amino acid mutation of the present application.

The term "hinge region" means an antibody heavy chain polypeptide portion in a wild-type antibody heavy chain that joins the CH1 domain and the CH2 domain, e.g., from about position 216 to about position 230 according to the EU numbering system, or from about position 226 to about position 243 according to the Kabat numbering system. It is known that in a native IgG antibody, the cysteine residue at position 220 according to EU numbering in the hinge region forms a disulfide bond with the cysteine residue at position 214 in the antibody light chain. It is also known that disulfide bonds between the two antibody heavy chains are formed in the hinge region by the cysteine residues at position 226 according to EU numbering with each other and the cysteine residues at position 229 with each other. Herein, hinge regions include wild-type hinge regions and variants thereof resulting from substitution, addition, or deletion of amino acid residues in wild-type hinge regions.

The term "cytotoxic agent" as used herein refers to a substance that inhibits or prevents a cellular function and/or causes cell death or destruction. Cytotoxic agents include, but are not limited to, radioactive isotopes (e.g., ²¹¹At, ¹³¹I, ¹²⁵I, ⁹⁰Y, ¹⁸⁶Re, ¹⁸⁸Re, ¹⁵³Sm, ²¹²Bi, ³²P, ²¹²Pb and radioactive isotopes of Lu); chemotherapeutic agents or drugs (e.g., methotrexate, adriamycin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) or glycine-lysine (residues 446-447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full length antibody," "intact antibody," and "whole antibody" are used herein interchangeably to refer to an antibody having a structure substantially similar to a native antibody structure or having heavy chains that contain an Fc region as defined herein.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one embodiment, for the VL, the subgroup is subgroup kappa I as in Kabat et al., supra. In one embodiment, for the VH, the subgroup is subgroup III as in Kabat et al., supra.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (H1, H2, H3), and three in the VL (L1, L2, L3). Exemplary HVRs herein include:
(a) hypervariable loops occurring at amino acid residues 26-32 (L1), 50-52 (L2), 91-96 (L3), 26-32 (H1), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., supra.

An "immunoconjugate" is an antibody conjugated to one or more heterologous molecule(s), including but not limited to a cytotoxic agent.

An "individual" or "subject" is a mammal. Mammals include, but are not limited to, domesticated animals (e.g., cows, sheep, cats, dogs, horses, and pigs), primates (e.g., humans and non-human primates such as monkeys), rabbits, and rodents (e.g., mice, rats, and guinea pigs). In one embodiment, the individual or subject is a non-human animal. In a specific embodiment, the individual or subject is a human.

An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

The term "monoclonal antibody" as used herein refers to an antibody obtained from a population of substantially homogeneous antibodies, i.e., the individual antibodies composing the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present application may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked antibody" refers to an antibody that is not conjugated to a heterologous moiety (e.g., a cytotoxic moiety) or radiolabel. The naked antibody may be present in a pharmaceutical formulation.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, Megalign (DNASTAR) software, or GENETYX (registered trademark) (Genetyx Co., Ltd.). Those skilled in the art can determine appropriate parameters for aligning sequences, including any algorithms needed to achieve maximal alignment over the full length of the sequences being compared.

The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "variable region" or "variable domain" refers to a domain of an antibody heavy or light chain that is involved in binding the antibody to an antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

The term "vector," as used herein, refers to a nucleic acid molecule capable of propagating another nucleic acid to which it is linked. The term includes the vector as a self-replicating nucleic acid structure as well as the vector in the genome of a host cell into which it has been introduced. Certain vectors are capable of directing the expression of nucleic acids to which they are operatively linked. Such vectors are referred to herein as "expression vectors." The vector can be introduced into a host cell by a method using a virus, an electroporation method, or the like, but the introduction of a vector is not limited to in vitro introduction, and it is also possible to directly introduce a vector into a body.

In a further aspect, an antibody according to any of the above embodiments may incorporate any of the features, singly or in combination, as described in Sections 1-7 below:

### 1. Antibody Affinity

In certain embodiments, an antibody provided herein has a dissociation constant (Kd) of 1 µM or less, 100 nM or less, 10 nM or less, 1 nM or less, 0.1 nM or less, 0.01 nM or less, or 0.001 nM or less (e.g., 10⁻⁸ M or less, e.g., 10⁻⁸ M to 10⁻¹³ M, e.g., 10⁻⁹ M to 10⁻¹³ M).

In one embodiment, Kd is measured by a radiolabeled antigen binding assay (RIA). In one embodiment, an RIA is performed with the Fab version of an antibody of interest and its antigen. For example, solution binding affinity of Fabs for antigen is measured by equilibrating Fab with a minimal concentration of (¹²⁵I)-labeled antigen in the presence of a titration series of unlabeled antigen, then capturing bound antigen with an anti-Fab antibody-coated plate (see, e.g., Chen et al., J. Mol. Biol. 293:865-881(1999)). To establish conditions for the assay, MICROTITER (registered trademark) multi-well plates (Thermo Scientific) are coated overnight with 5 micro g/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23 degrees C). In a non-adsorbent plate (Nunc #269620), 100 pM or 26 pM [¹²⁵I]-antigen are mixed with serial dilutions of a Fab of interest (e.g., consistent with assessment of the anti-VEGF antibody, Fab-12, in Presta et al., Cancer Res. 57:4593-4599 (1997)). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN-20 (registered trademark)) in PBS. When the plates have dried, 150 micro l/well of scintillant (MICROSCINT-20 TM; Packard) is added, and the plates are counted on a TOPCOUNTTM gamma counter (Packard) for ten minutes. Concentrations of each Fab that give less than or equal to 20% of maximal binding are chosen for use in competitive binding assays.

According to another embodiment, Kd is measured using a BIACORE (registered trademark) surface plasmon resonance assay. For example, an assay using a BIACORE (registered trademark)-2000 or a BIACORE(registered trademark)-3000 (BIAcore, Inc., Piscataway, NJ) is performed at 25 degrees C with immobilized antigen CM5 chips at ~10 response units (RU). In one embodiment, carboxymethylated dextran biosensor chips (CM5, BIACORE, Inc.) are activated with N-ethyl-N'- (3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 micro g/ml (~0.2 micro M) before injection at a flow rate of 5 micro l/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20TM) surfactant (PBST) at 25 degrees C at a flow rate of approximately 25 micro l/min. Association rates (kₒₙ) and dissociation rates (k_{off}) are calculated using a simple one-to-one Langmuir binding model (BIACORE (registered trademark) Evaluation Software version 3.2) by simultaneously fitting the association and dissociation sensorgrams. The equilibrium dissociation constant (Kd) is calculated as the ratio k_{off}/kₒₙ. See, e.g., Chen et al., J. Mol. Biol. 293:865-881 (1999). If the on-rate exceeds 10⁶ M⁻¹ s⁻¹ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophotometer (Aviv Instruments) or a 8000-series SLM-AMINCOTM spectrophotometer (ThermoSpectronic) with a stirred cuvette.

### 2. Antibody Fragments

In certain embodiments, an antibody provided herein is an antibody fragment. Antibody fragments include, but are not limited to, Fab, Fab', Fab'-SH, F(ab')₂, Fv, and scFv fragments, and other fragments described below. For a review of certain antibody fragments, see Hudson et al. Nat. Med. 9:129-134 (2003). For a review of scFv fragments, see e.g. Pluckthun, in The Pharmacology of Monoclonal Antibodies, vol. 113, Rosenburg and Moore eds., Springer-Verlag, New York, pp. 269-315 (1994); see also WO 93/16185 ; and U.S. Patent Nos. 5,571,894 and 5,587,458. For discussion of Fab and F(ab')₂ fragments comprising salvage receptor binding epitope residues and having increased in vivo half-life, see U.S. Patent No. 5,869,046.

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see e.g. U.S. Patent No. 6,248,516 B1).

Antibody fragments can be made by various techniques, including but not limited to proteolytic digestion of an intact antibody as well as production by recombinant host cells (e.g. *E. coli* or phage), as described herein.

### 3. Chimeric and Humanized Antibodies

In certain embodiments, an antibody provided herein is a chimeric antibody. Certain chimeric antibodies are described, e.g., in U.S. Patent No. 4,816,567; and Morrison et al., Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

### 4. Human Antibodies

In certain embodiments, an antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSETM technology; U.S. Patent No. 5,770,429 describing HuMab (registered trademark) technology; U.S. Patent No. 7,041,870 describing K-M MOUSE (registered trademark) technology, and U.S. Patent Application Publication No. US 2007/0061900, describing VelociMouse (registered trademark) technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### 5. Library-Derived Antibodies

Antibodies of the present application may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement in vitro, as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### 6. Multispecific Antibodies

In certain embodiments, an antibody provided herein is a multispecific antibody, e.g. a bispecific antibody. Multispecific antibodies are monoclonal antibodies that have binding specificities for at least two different sites. In certain embodiments, one of the binding specificities is for any antigen and the other is for any other antigen. In certain embodiments, bispecific antibodies may bind to two different epitopes of an antigen. Bispecific antibodies may also be used to localize cytotoxic agents to cells which express an antigen. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004A1); cross-linking two or more antibodies or fragments (see, e.g., US Patent No. 4,676,980, and Brennan et al., Science, 229: 81 (1985)); using leucine zippers to produce bi-specific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (scFv) dimers (see, e.g. Gruber et al., J. Immunol., 152:5368 (1994)); and preparing trispecific antibodies as described, e.g., in Tutt et al. J. Immunol. 147: 60 (1991).

Engineered antibodies with three or more functional antigen binding sites, including "Octopus antibodies," are also included herein (see, e.g. US 2006/0025576A1).

The antibody or fragment herein also includes a "Dual Acting Fab" or "DAF" (see, US 2008/0069820, for example).

### 7. Antibody Variants

In certain embodiments, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

### a) Substitution, Insertion, and Deletion Variants

In certain embodiments, antibody variants having one or more amino acid substitutions are provided. Sites of interest for substitutional mutagenesis include the HVRs and FRs. Conservative substitutions are shown in Table 1 under the heading of "preferred substitutions." More substantial changes are provided in Table 1 under the heading of "exemplary substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

**(Table 1)**

| **Original Residue** | **Exemplary Substitutions** | **Preferred Substitution** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: norleucine, methionine (Met), alanine (Ala), valine (Val), leucine (Leu), isoleucine (Ile);
(2) neutral hydrophilic: cysteine (Cys), serine (Ser), threonine (Thr), asparagine (Asn), glutamine (Gln);
(3) acidic: aspartic acid (Asp), glutamic acid (Glu);
(4) basic: histidine (His), lysine (Lys), arginine (Arg);
(5) residues that influence chain orientation: glycine (Gly), proline (Pro);
(6) aromatic: tryptophan (Trp), tyrosine (Tyr), phenylalanine (Phe). Non-conservative substitutions will entail exchanging a member of one of these classes for another class.

One type of substitutional variant involves substituting one or more hypervariable region residues of a parent antibody (e.g. a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or residues that contact antigen, with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may, for example, be outside of antigen contacting residues in the HVRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex may be analyzed to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion of an enzyme (e.g. for ADEPT) or a polypeptide which increases the plasma half-life of the antibody to the N- or C-terminus of the antibody.

### b) Glycosylation Variants

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the present application may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e.g. complex, hybrid and high mannose structures) as measured by MALDI-TOF mass spectrometry, as described in WO 2008/077546, for example. Asn297 refers to the asparagine residue located at about position 297 in the Fc region (EU numbering of Fc region residues); however, Asn297 may also be located about +/- 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al., especially at Example 11), and knockout cell lines, such as alpha-1,6-fucosyltransferase gene, FUT8, knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Antibodies variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

### c) Fc Region Variants

In certain embodiments, one or more amino acid modifications may be introduced into the Fc region of an antibody provided herein, thereby generating an Fc region variant. The Fc region variant may comprise a human Fc region sequence (e.g., a human IgG1, IgG2, IgG3 or IgG4 Fc region) comprising an amino acid modification (e.g. a substitution) at one or more amino acid positions.

In certain embodiments, the present application contemplates an antibody variant that possesses some but not all effector functions, which make it a desirable candidate for applications in which the half-life of the antibody in vivo is important yet certain effector functions (such as complement and ADCC) are unnecessary or deleterious. In vitro and/or in vivo cytotoxicity assays can be conducted to confirm the reduction/depletion of CDC and/or ADCC activities. For example, Fc receptor (FcR) binding assays can be conducted to ensure that the antibody lacks Fc gamma R binding (hence likely lacking ADCC activity), but retains FcRn binding ability. The primary cells for mediating ADCC, NK cells, express Fc gamma RIII only, whereas monocytes express Fc gamma RI, Fc gamma RII and Fc gamma RIII. FcR expression on hematopoietic cells is summarized in Table 3 on page 464 of Ravetch and Kinet, Annu. Rev. Immunol. 9:457-492 (1991). Non-limiting examples of in vitro assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACT1TM non-radioactive cytotoxicity assay for flow cytometry (CellTechnology, Inc. Mountain View, CA; and CytoTox 96 (registered trademark) non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed in vivo, e.g., in an animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half-life determinations can also be performed using methods known in the art (see, e.g., Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with increased or decreased binding to FcRs are described. (See, e.g., U.S. Patent No. 6,737,056; WO 2004/056312, and Shields et al., J. Biol. Chem. 9(2): 6591-6604 (2001).)

In certain embodiments, an antibody variant comprises an Fc region with one or more amino acid substitutions which improve ADCC, e.g., substitutions at positions 298, 333, and/or 334 of the Fc region (EU numbering of residues).

In some embodiments, alterations are made in the Fc region that result in altered (i.e., either increased or decreased) C1q binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half-lives and increased binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which increase binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

### d) Cysteine Engineered Antibody Variants

In certain embodiments, it may be desirable to create cysteine engineered antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541.

### e) Antibody Derivatives

In certain embodiments, an antibody provided herein may be further modified to contain additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, polypropylene glycol homopolymers, polypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

Antibodies may be produced using recombinant methods and compositions, e.g., as described in U.S. Patent No. 4,816,567. In one embodiment, isolated nucleic acid encoding an antibody described herein is provided. Such nucleic acid may encode an amino acid sequence comprising the VL and/or an amino acid sequence comprising the VH of the antibody (e.g., the light and/or heavy chains of the antibody). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, e.g. a Chinese Hamster Ovary (CHO) cell or lymphoid cell (e.g., Y0, NS0, Sp2/0 cell). In one embodiment, a method of making an antibody is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an antibody, nucleic acid encoding an antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in E. coli.) After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. See, e.g., US Patent Nos. 5,959,177, 6,040,498, 6,420,548, 7,125,978, and 6,417,429 (describing PLANTIBODIESTM technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol.36:59 (1977)); baby hamster kidney cells (BHK); mouse sertoli cells (TM4 cells as described, e.g., in Mather, Biol. Reprod. 23:243-251 (1980)); monkey kidney cells (CV1); African green monkey kidney cells (VERO-76); human cervical carcinoma cells (HELA); canine kidney cells (MDCK); buffalo rat liver cells (BRL 3A); human lung cells (W138); human liver cells (Hep G2); mouse mammary tumor (MMT 060562); TRI cells, as described, e.g., in Mather et al., Annals N.Y. Acad. Sci. 383:44-68 (1982); MRC 5 cells; and FS4 cells. Other useful mammalian host cell lines include Chinese hamster ovary (CHO) cells, including DHFR- CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ), pp. 255-268 (2003).

In certain embodiments, the present application provides an antibody obtainable by a method set out above.

Antibodies provided herein may be identified, screened for, or characterized for their physical/chemical properties and/or biological activities by various assays known in the art.

In one aspect, an antibody of the present application is tested for its antigen binding activity, e.g., by known methods such as ELISA, Western blot, etc.

The present application also provides immunoconjugates comprising an antibody herein conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes.

In one embodiment, an immunoconjugate is an antibody-drug conjugate (ADC) in which an antibody is conjugated to one or more drugs, including but not limited to a maytansinoid (see U.S. Patent Nos. 5,208,020, 5,416,064 and European Patent EP 0 425 235 B1); an auristatin such as monomethylauristatin drug moieties DE and DF (MMAE and MMAF) (see U.S. Patent Nos. 5,635,483 and 5,780,588, and 7,498,298); a dolastatin; a calicheamicin or derivative thereof (see U.S. Patent Nos. 5,712,374, 5,714,586, 5,739,116, 5,767,285, 5,770,701, 5,770,710, 5,773,001, and 5,877,296; Hinman et al., Cancer Res. 53:3336-3342 (1993); and Lode et al., Cancer Res. 58:2925-2928 (1998)); an anthracycline such as daunomycin or doxorubicin (see Kratz et al., Current Med. Chem. 13:477-523 (2006); Jeffrey et al., Bioorganic & Med. Chem. Letters 16:358-362 (2006); Torgov et al., Bioconj. Chem. 16:717-721 (2005); Nagy et al., Proc. Natl. Acad. Sci. USA 97:829-834 (2000); Dubowchik et al., Bioorg. & Med. Chem. Letters 12:1529-1532 (2002); King et al., J. Med. Chem. 45:4336-4343 (2002); and U.S. Patent No. 6,630,579); methotrexate; vindesine; a taxane such as docetaxel, paclitaxel, larotaxel, tesetaxel, and ortataxel; a trichothecene; and CC1065.

In another embodiment, an immunoconjugate comprises an antibody as described herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolacca americana proteins (PAPI, PAPII, and PAP-S), momordica charantia inhibitor, curcin, crotin, saponaria officinalis inhibitor, gelonin, mitogellin, restrictocin, phenomycin, enomycin, and the tricothecenes.

In another embodiment, an immunoconjugate comprises an antibody as described herein conjugated to a radioactive atom to form a radioconjugate. A variety of radioactive isotopes are available for the production of radioconjugates. Examples include ²¹¹At, ¹³¹I, ¹²⁵I, ⁹⁰Y, ¹⁸⁶Re, ¹⁸⁸Re, ¹⁵³Sm, ²¹²Bi, ³²P, ²¹²Pb, and radioactive isotopes of Lu. When the radioconjugate is used for detection, it may comprise a radioactive atom for scintigraphic studies, for example Tc-99m or ¹²³I, or a spin label for nuclear magnetic resonance (NMR) imaging (also known as magnetic resonance imaging, MRI), such as iodine-123 again, iodine-131, indium-111, fluorine-19, carbon-13, nitrogen-15, oxygen-17, gadolinium, manganese or iron.

Conjugates of an antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionuclide to the antibody. See WO94/11026. The linker may be a "cleavable linker" facilitating release of the cytotoxic drug in the cell. For example, an acid-labile linker, peptidase-sensitive linker, photolabile linker, dimethyl linker or disulfide-containing linker (Chari et al., Cancer Res. 52:127-131 (1992); U.S. Patent No. 5,208,020) may be used.

The immunuoconjugates or ADCs herein expressly contemplate, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A).

The term "Fc region-comprising antibody" refers to an antibody that comprises an Fc region. The C-terminal lysine (residue 447 according to the EU numbering system) or C-terminal glycine-lysine (residues 446-447) of the Fc region may be removed, for example, during purification of the antibody or by recombinant engineering of the nucleic acid encoding the antibody. Accordingly, a composition comprising an antibody having an Fc region according to the present application can comprise an antibody with G446-K447, with G446 and without K447, with all G446-K447 removed, or a mixture of three types of antibodies described above.

A "functional Fc region" possesses an "effector function" of a native sequence Fc region. Exemplary "effector functions" include C1q binding; CDC; Fc receptor binding; ADCC; phagocytosis; down regulation of cell surface receptors (e.g. B cell receptor; BCR), etc. Such effector functions generally require the Fc region to be combined with a binding domain (e.g., an antibody variable domain) and can be assessed using various assays as disclosed, for example, in definitions herein.

A "native sequence Fc region" comprises an amino acid sequence identical to the amino acid sequence of an Fc region found in nature. Native sequence human Fc regions include a native sequence human IgG1 Fc region (non-A and A allotypes); native sequence human IgG2 Fc region; native sequence human IgG3 Fc region; and native sequence human IgG4 Fc region as well as naturally occurring variants thereof.

The antigen-binding molecule of the present application encompasses, for example, the antigen-binding molecules described in WO2020/027330. For example, the antigen-binding molecules of the present application include antigen-binding molecules comprising a first antigen-binding domain and a second antigen-binding domain in which the antigen-binding domains are linked to each other via one or more bonds.

In one embodiment, at least one of the one or more bonds linking the two antigen-binding domains is a covalent bond. In certain embodiments, a covalent bond is formed by direct crosslinking of an amino acid residue in the first antigen-binding domain with an amino acid residue in the second antigen-binding domain. The type of amino acid residue to be crosslinked is, for example, cysteine, and the covalent bond formed is, for example, a disulfide bond.

In another specific embodiment, the amino acid residue in the first antigen-binding domain and the amino acid residue in the second antigen-binding domain are crosslinked via a crosslinking agent to form a covalent bond. The crosslinking agent is, for example, an amine-reactive crosslinking agent, and the type of amino acid residue to be cross-linked is, for example, lysine.

In one embodiment, at least one of the one or more bonds linking the antigen binding domains is a non-covalent bond. In certain embodiments, the non-covalent bond is either an ionic bond, a hydrogen bond, or a hydrophobic bond. Ionic bonds are formed, for example, between acidic and basic amino acids. Acidic amino acids are, for example, aspartic acid (Asp) or glutamic acid (Glu). Basic amino acids are, for example, histidine (His), lysine (Lys), or arginine (Arg).

Amino acid residues from which the bonds between the antigen-binding domains (the bonds which link two antigen-binding domains) originate are respectively present in the first and second antigen-binding domains, and the bonds between the antigen-binding domains are formed by linking these amino acid residues. In one embodiment, at least one of the amino acid residues from which the bond between the antigen-binding domains originates is an artificially introduced mutated amino acid residue and, for example, it is an artificially introduced cysteine residue. Such a mutated amino acid residue can be introduced into a wild-type antigen-binding domain by, for example, a method of amino acid substitution. The present specification discloses the sites of amino acid residues from which the bond between the antigen-binding domains can originate for each of the CH1, CL, and hinge regions as constant regions and the VH, VL, and VHH regions as variable regions when the antigen-binding domains comprise for example an antibody fragment, and for example, cysteine residues can be introduced into such sites.

In one embodiment, at least one of the first and second antigen-binding domains has, by itself, activity of binding to an antigen (i.e., a single antigen-binding domain independently has antigen-binding activity). In certain embodiments, each of the first and second antigen-binding domains has, by itself, activity of binding to an antigen.

In one embodiment, the at least one bond which links the first antigen-binding domain and the second antigen-binding domain may be formed by linking amino acid residues present at the same position in the first antigen-binding domain and in the second antigen-binding domain with each other, or it may be formed by linking amino acid residues present at a respectively different position with each other.

In one embodiment, at least one of the amino acid residues from which the bonds between the antigen-binding domains originate is present within a constant region. In certain embodiments, the amino acid residue is present within the CH1 region, for example, at any of positions 119 to 123, 131 to 140, 148 to 150, 155 to 167, 174 to 178, 188 to 197, 201 to 214, and 218 to 219 according to EU numbering in the CH1 region. In certain embodiments, the amino acid residue is present at a position selected from the group consisting of positions 119, 122, 123, 131, 132, 133, 134, 135, 136, 137, 138, 139, 140, 148, 150, 155, 156, 157, 159, 160, 161, 162, 163, 164, 165, 167, 174, 176, 177, 178, 188, 189, 190, 191, 192, 193, 194, 195, 196, 197, 201, 203, 205, 206, 207, 208, 211, 212, 213, 214, 218, and 219 according to EU numbering in the CH1 region. In certain embodiments, the amino acid residue is present at position 134, 135, 136, 137, 191, 192, 193, 194, 195, or 196 according to EU numbering in the CH1 region. In certain embodiments, the amino acid residue is present at position 135, 136, or 191 according to EU numbering in the CH1 region.

In one embodiment, the constant region is derived from human. In certain embodiments, the subclass of the heavy chain constant region is any of IgG1, IgG2, IgG3, IgG4, IgA1, IgA2, IgM, IgD, and IgE. In certain embodiments, the subclass of the CH1 region is any of gamma 1, gamma 2, gamma 3, gamma 4, alpha 1, alpha 2, mu, delta, and epsilon.

In one embodiment, the at least one bond which links the first antigen-binding domain and the second antigen-binding domain is formed by linking an amino acid residue in the CH1 region of the first antigen-binding domain and an amino acid residue in the CH1 region of the second antigen-binding domain. In certain embodiments, the amino acid residues in the first antigen-binding domain and the second antigen-binding domain are each independently selected from the group consisting of positions 119, 120, 121, 122, and 123 according to EU numbering. In certain embodiments, the amino acid residues in the first antigen-binding domain and the second antigen-binding domain are each independently selected from the group consisting of positions 131, 132, 133, 134, 135, 136, 137, 138, 139, and 140 according to EU numbering. In certain embodiments, the amino acid residues in the first antigen-binding domain and the second antigen-binding domain are each independently selected from the group consisting of positions 148, 149, and 150 according to EU numbering. In certain embodiments, the amino acid residues in the first antigen-binding domain and the second antigen-binding domain are each independently selected from the group consisting of positions 155, 156, 157, 158, 159, 160, 161, 162, 163, 164, 165, 166, and 167 according to EU numbering. In certain embodiments, the amino acid residues in the first antigen-binding domain and the second antigen-binding domain are each independently selected from the group consisting of positions 174, 175, 176, 177, and 178 according to EU numbering. In certain embodiments, the amino acid residues in the first antigen-binding domain and the second antigen-binding domain are each independently selected from the group consisting of positions 188, 189, 190, 191, 192, 193, 194, 195, 196, and 197 according to EU numbering. In certain embodiments, the amino acid residues in the first antigen-binding domain and the second antigen-binding domain are each independently selected from the group consisting of positions 201, 202, 203, 204, 205, 206, 207, 208, 209, 210, 211, 212, 213, and 214 according to EU numbering. In certain embodiments, the amino acid residues in the first antigen-binding domain and the second antigen-binding domain are each independently selected from the group consisting of positions 218 and 219 according to EU numbering.

In one embodiment, the difference in the positions of the amino acid residues from which the bonds originate in each of the first antigen-binding domain and the second antigen-binding domain is three amino acids or less. In one embodiment, this means that when the position of the amino acid residue from which a bond originates in the CH1 region of the first antigen-binding domain and the position of the amino acid residue from which the bond originates in the CH1 region of the second antigen-binding domain are respectively compared according to EU numbering, the difference is three amino acids or less. In certain embodiments, the at least one bond which links the first antigen-binding domain and the second antigen-binding domain is formed by linking the amino acid residue at position 135 according to EU numbering in the CH1 region of the first antigen-binding domain and an amino acid residue at any of positions 132 to 138 according to EU numbering in the CH1 region of the second antigen-binding domain. In certain embodiments, the at least one bond which links the first antigen-binding domain and the second antigen-binding domain is formed by linking the amino acid residue at position 136 according to EU numbering in the CH1 region of the first antigen-binding domain and an amino acid residue at any of positions 133 to 139 according to EU numbering in the CH1 region of the second antigen-binding domain. In certain embodiments, the at least one bond which links the first antigen-binding domain and the second antigen-binding domain is formed by linking the amino acid residue at position 191 according to EU numbering in the CH1 region of the first antigen-binding domain and an amino acid residue at any of positions 188 to 194 according to EU numbering in the CH1 region of the second antigen-binding domain. In an exemplary embodiment, the at least one bond which links the first antigen-binding domain and the second antigen-binding domain is formed by linking the amino acid residues at position 135 according to EU numbering in the CH1 regions of the two antigen-binding domains with each other. In an exemplary embodiment, the at least one bond which links the first antigen-binding domain and the second antigen-binding domain is formed by linking the amino acid residues at position 136 according to EU numbering in the CH1 regions of the two antigen-binding domains with each other. In an exemplary embodiment, the at least one bond which links the first antigen-binding domain and the second antigen-binding domain is formed by linking the amino acid residues at position 191 according to EU numbering in the CH1 regions of the two antigen-binding domains with each other.

In one embodiment, at least one of amino acid residues from which the bonds between the antigen-binding domains originate is present within a CL region, for example, at any of positions 108 to 112, 121 to 128, 151 to 156, 184 to 190, 195 to 196, 200 to 203, and 208 to 213 according to Kabat numbering in the CL region. In certain embodiments, the amino acid residue is present at a position selected from the group consisting of positions 108, 109, 112, 121, 123, 126, 128, 151, 152, 153, 156, 184, 186, 188, 189, 190, 195, 196, 200, 201, 202, 203, 208, 210, 211, 212, and 213 according to Kabat numbering in the CL region. In certain embodiments, the amino acid residue is present at position 126 according to Kabat numbering in the CL region.

In one embodiment, the constant region is derived from human. In certain embodiments, the subclass of the CL region is κ or λ.

In one embodiment, the at least one bond which links the first antigen-binding domain and the second antigen-binding domain is formed by linking an amino acid residue in the CL region of the first antigen-binding domain and an amino acid residue in the CL region of the second antigen-binding domain. In certain embodiments, the amino acid residues in the first antigen-binding domain and the second antigen-binding domain are each independently selected from the group consisting of positions 108, 109, 110, 111, and 112 according to Kabat numbering. In certain embodiments, the amino acid residues in the first antigen-binding domain and the second antigen-binding domain are each independently selected from the group consisting of positions 121, 122, 123, 124, 125, 126, 127, and 128 according to Kabat numbering. In certain embodiments, the amino acid residues in the first antigen-binding domain and the second antigen-binding domain are each independently selected from the group consisting of positions 151, 152, 153, 154, 155, and 156 according to Kabat numbering. In certain embodiments, the amino acid residues in the first antigen-binding domain and the second antigen-binding domain are each independently selected from the group consisting of positions 184, 185, 186, 187, 188, 189, and 190 according to Kabat numbering. In certain embodiments, the amino acid residues in the first antigen-binding domain and the second antigen-binding domain are each independently selected from the group consisting of positions 195 and 196 according to Kabat numbering. In certain embodiments, the amino acid residues in the first antigen-binding domain and the second antigen-binding domain are each independently selected from the group consisting of positions 200, 201, 202, and 203 according to Kabat numbering. In certain embodiments, the amino acid residues in the first antigen-binding domain and the second antigen-binding domain are each independently selected from the group consisting of positions 208, 209, 210, 211, 212, and 213 according to Kabat numbering.

In one embodiment, the difference in the positions of the amino acid residues from which the bonds originate in each of the first antigen-binding domain and the second antigen-binding domain is three amino acids or less. In one embodiment, this means that when the position of the amino acid residue from which a bond originates in the CL region of the first antigen-binding domain and the position of the amino acid residue from which the bond originates in the CL region of the second antigen-binding domain are respectively compared according to EU numbering, the difference is three amino acids or less. In an exemplary embodiment, the at least one bond which links the first antigen-binding domain and the second antigen-binding domain is formed by linking the amino acid residues at position 126 according to Kabat numbering in the CL regions of the two antigen-binding domains with each other.

In one embodiment, the at least one bond which links the first antigen-binding domain and the second antigen-binding domain is formed by linking an amino acid residue in the CH1 region of the first antigen-binding domain and an amino acid residue in the CL region of the second antigen-binding domain. In certain embodiments, the amino acid residue in the CH1 region of the first antigen-binding domain is selected from the group consisting of positions 188, 189, 190, 191, 192, 193, 194, 195, 196, and 197 according to EU numbering, and the amino acid residue in the CL region of the second antigen-binding domain is selected from the group consisting of positions 121, 122, 123, 124, 125, 126, 127, and 128 according to Kabat numbering. In an exemplary embodiment, the at least one bond which links the first antigen-binding domain and the second antigen-binding domain is formed by linking the amino acid residue at position 191 according to EU numbering in the CH1 region of the first antigen-binding domain and an amino acid residue at position 126 according to Kabat numbering in the CL region of the second antigen-binding domain.

In one embodiment, at least one of the amino acid residues from which the bonds between the antigen-binding domains originate is present within a variable region. In certain embodiments, the amino acid residue is present within the VH region, for example, at any position selected from the group consisting of positions 6, 8, 16, 20, 25, 26, 28, 74, and 82b according to Kabat numbering in the VH region. In certain embodiments, the aforementioned amino acid residue is present within the VL region, for example, at any position selected from the group consisting of positions 21, 27, 58, 77, 100, 105, and 107 according to Kabat numbering in the VL region (subclass κ) and positions 6, 19, 33, and 34 according to Kabat numbering in the VL region (subclass λ). In certain embodiments, the amino acid residue is present within the VHH region, for example, at any position selected from the group consisting of positions 4, 6, 7, 8, 9, 10, 11, 12, 14, 15, 17, 20, 24, 27, 29, 38, 39, 40, 41, 43, 44, 45, 46, 47, 48, 49, 67, 69, 71, 78, 80, 82, 82c, 85, 88, 91, 93, 94, and 107 according to Kabat numbering in the VHH region.

In one embodiment, the first and/or second antigen-binding domains comprise a hinge region. In certain embodiments, at least one of the cysteine residues present within the wild-type hinge region has been substituted with another amino acid residue. Such cysteine residues are present, for example, at position 226 and/or position 229 according to EU numbering in the wild-type hinge region. In certain embodiments, at least one of amino acid residues from which the bonds between the antigen-binding domains originate is present within the hinge region, for example, at any position selected from the group consisting of positions 216, 218, and 219 according to EU numbering in the hinge region.

In one embodiment, the first antigen-binding domain and the second antigen-binding domain are linked with each other via two or more bonds.

In certain embodiments, at least one of the amino acid residues from which the bonds between the antigen-binding domains originate is an amino acid residue present in a wild-type sequence, for example, a cysteine residue in a wild-type hinge region. In certain embodiments, at least one of the bonds that link the first antigen-binding domain and the second antigen-binding domain is a disulfide bond formed by crosslinking between cysteine residues present within wild-type hinge regions. Such cysteine residues are present, for example, at position 226 and/or position 229 according to EU numbering in the wild-type hinge region.

In certain embodiments, at least one of the amino acid residues from which the bonds between the antigen-binding domains originate is present within an antibody fragment, and at least one is present within a hinge region. In an exemplary embodiment, the antigen-binding molecule of the present disclosure is F(ab')2 in which both the first and second antigen-binding domains comprise a Fab and a hinge region.

The antigen-binding molecule of the present application encompasses, for example, the antigen-binding molecules described in WO2018/097307.
For example, the antigen-binding molecule of the present application relates to an antigen-binding molecule as a polypeptide, wherein the polypeptide comprises an antigen-binding domain and a carrying moiety, wherein the carrying moiety comprises an inhibiting domain that inhibits the antigen-binding activity of the antigen-binding domain, and wherein the antigen-binding domain has a shorter half-life in blood than the carrying moiety.

The carrying moiety has an inhibiting domain that inhibits the antigen-binding activity of the antigen binding domain. Herein, the term "inhibiting domain" is limited only by the inhibition of the antigen binding activity of the antigen binding domain. The inhibiting domain can be a domain having any structure as long as the domain used can inhibit the antigen binding activity of the antigen binding domain. Examples of such an inhibiting domain include, but are not limited to, an antibody heavy chain variable region (VH), an antibody light chain variable region (VL), pre-B cell receptors, and single-domain antibodies. The inhibiting domain may constitute the whole of the carrying moiety or may constitute a portion of the carrying moiety.

In some embodiments, the antigen binding domain released from the polypeptide has higher antigen binding activity than that before the release. In other words, the antigen binding activity of the antigen binding domain is inhibited by the inhibiting domain in a state where the antigen binding domain is unreleased from the polypeptide. Whether the antigen binding activity of the antigen binding domain is inhibited by the inhibiting domain is confirmed by a method such as FACS (fluorescence activated cell sorting), ELISA (enzyme-linked immunosorbent assay), ECL (electrogenerated chemiluminescence), a SPR (surface plasmon resonance) method (Biacore), BLI (biolayer interferometry) (Octet). In some embodiments, the antigen binding activity of the antigen binding domain released from the polypeptide is a value equal to or larger than 2 times, 3 times, 4 times, 5 times, 6 times, 7 times, 8 times, 9 times, 10 times, 20 times, 30 times, 40 times, 50 times, 60 times, 70 times, 80 times, 90 times, 100 times, 200 times, 300 times, 400 times, 500 times, 600 times, 700 times, 800 times, 900 times, 1,000 times, 2000 times, or 3000 times the binding activity of the antigen binding domain unreleased from the polypeptide. In some more specific embodiments, the binding of the antigen binding domain before the release to the antigen is not seen when the antigen binding activity of the antigen binding domain is measured by one method selected from among the methods described above.

In some aspects, the cleavage site is cleaved so that the antigen binding domain becomes capable of being released from the polypeptide. In such aspects, therefore, the antigen binding activity can be compared between before and after the cleavage of the polypeptide. Specifically, the antigen binding activity measured using the cleaved polypeptide is a value equal to or larger than 2 times, 3 times, 4 times, 5 times, 6 times, 7 times, 8 times, 9 times, 10 times, 20 times, 30 times, 40 times, 50 times, 60 times, 70 times, 80 times, 90 times, 100 times, 200 times, 300 times, 400 times, 500 times, 600 times, 700 times, 800 times, 900 times, 1,000 times, 2000 times, or 3000 times the antigen binding activity measured using the uncleaved polypeptide. In some more specific embodiments, the binding of the antigen binding domain of the uncleaved polypeptide to the antigen is not seen when the antigen binding activity is measured by one method selected from among the methods described above.

In some aspects, the cleavage site is cleaved by protease. In such aspects, therefore, the antigen binding activity can be compared between before and after the protease treatment of the polypeptide. Specifically, the antigen binding activity measured using the polypeptide after the protease treatment is a value equal to or larger than 2 times, 3 times, 4 times, 5 times, 6 times, 7 times, 8 times, 9 times, 10 times, 20 times, 30 times, 40 times, 50 times, 60 times, 70 times, 80 times, 90 times, 100 times, 200 times, 300 times, 400 times, 500 times, 600 times, 700 times, 800 times, 900 times, 1,000 times, 2000 times, or 3000 times the antigen binding activity measured using the polypeptide without the protease treatment. In some more specific embodiments, the binding of the antigen binding domain of the protease-untreated polypeptide to the antigen is not seen when the antigen binding activity is measured by one method selected from among the methods described above.

In the present application, the polypeptide comprising an antigen binding domain and a carrying moiety has a longer half-life in blood than that of the antigen binding domain existing alone. In some embodiments, for the longer half-life of the polypeptide, the carrying moiety is designed so as to have a longer half-life in blood. In such embodiments, examples of the approach of extending the half-life in blood of the carrying moiety include, but are not limited to, a large molecular weight of the carrying moiety, FcRn binding activity possessed by the carrying moiety, albumin binding activity possessed by the carrying moiety, and the PEGylation of the carrying moiety. In some embodiments, the carrying moiety has a longer half-life in blood than that of the antigen binding domain (in other words, the antigen binding domain has a shorter half-life in blood than that of the carrying moiety).

In the present application, the half-lives of the antigen binding domain alone and the polypeptide, or the half-lives in blood of the antigen binding domain and the carrying moiety are preferably compared in terms of their half-lives in blood in humans. If the half-lives in blood are difficult to measure in humans, the half-lives in blood in humans can be predicted on the basis of their half-lives in blood in mice (e.g., normal mice, transgenic mice expressing a human antigen, and transgenic mice expressing human FcRn) or monkeys (e.g., cynomolgus monkeys).

In one embodiment, the approach of extending the half-life in blood of the carrying moiety includes a large molecular weight of the carrying moiety. In one embodiment, the approach of rendering the half-life in blood of the carrying moiety longer than that of the antigen binding domain includes a larger molecular weight of the carrying moiety than that of the antigen binding domain.

In one embodiment, the approach of extending the half-life in blood of the carrying moiety includes FcRn binding activity possessed by the carrying moiety. The carrying moiety can usually possess FcRn binding activity by a method of establishing a FcRn binding region in the carrying moiety. The FcRn binding region refers to a region having binding activity to FcRn and may have any structure as long as the region used has binding activity to FcRn.

The carrying moiety containing a FcRn binding region is capable of being taken up into cells and then brought back into plasma through the salvage pathway of FcRn. For example, an IgG molecule has a relatively long circulation time in plasma (slow disappearance) because FcRn known as a salvage receptor of the IgG molecule functions. An IgG molecule taken up into the endosome through pinocytosis binds to FcRn expressed in the endosome under intraendosomal acidic conditions. An IgG molecule that has failed to bind to FcRn is moved to the lysosome and degraded therein, whereas the IgG molecule bound with FcRn is transferred to cell surface, then dissociated from the FcRn under neutral conditions in plasma, and thereby brought back into plasma.

The FcRn binding region is preferably a region binding directly to FcRn. Preferred examples of the FcRn binding region can include antibody Fc regions. However, a region capable of binding to a polypeptide, such as albumin or IgG, which has FcRn binding capacity is capable of binding indirectly to FcRn via albumin, IgG, or the like. Therefore, the FcRn binding region may be a region binding to such a polypeptide having FcRn binding capacity.

The binding activity of the FcRn binding region to FcRn, particularly, human FcRn, may be measured by a method known to those skilled in the art, as mentioned in the above section about binding activity. The conditions therefor may be appropriately determined by those skilled in the art. The binding activity to human FcRn can be evaluated as KD (dissociation constant), apparent KD (apparent dissociation constant), kd (dissociation rate), or apparent kd (apparent dissociation rate), etc. These values can be measured by methods known to those skilled in the art. For example, Biacore (GE Healthcare), Scatchard plot, a flow cytometer, and the like can be used.

The conditions for measuring the binding activity of the FcRn binding region to FcRn are not particularly limited and may be appropriately selected by those skilled in the art. The binding activity can be measured under conditions involving, for example, a MES buffer and 37°C, as described in WO2009/125825. Also, the binding activity of the FcRn binding region to FcRn may be measured by a method known to those skilled in the art and can be measured using, for example, Biacore (GE Healthcare). In the measurement of the binding activity of the FcRn binding region to FcRn, FcRn and the FcRn binding region or the carrying moiety containing the FcRn binding region can be injected as analytes to chips on which the FcRn binding region or the carrying moiety containing the FcRn binding region and FcRn, respectively, are immobilized, followed by evaluation.

As for pH for use in the measurement conditions, the binding affinity of the FcRn binding region for FcRn may be evaluated at any pH of 4.0 to 6.5. Preferably, a pH of 5.8 to 6.0, which is close to pH in the early endosome *in vivo*, is used for determining the binding affinity of the FcRn binding region for human FcRn. As for temperature for use in the measurement conditions, the binding affinity of the FcRn binding region for FcRn may be evaluated at any temperature of 10°C to 50°C. Preferably, a temperature of 15°C to 40°C is used for determining the binding affinity of the FcRn binding region for human FcRn. More preferably, any temperature from 20°C to 35°C, for example, any one of 20, 21, 22, 23, 24, 25, 26, 27, 28, 29, 30, 31, 32, 33, 34, and 35°C, is also used for determining the binding affinity of the FcRn binding region for FcRn. The temperature of 25°C is one non-limiting example of the embodiment.

The FcRn binding activity possessed by the carrying moiety does not mean that the antigen binding domain has no FcRn binding activity. In the embodiments in which the carrying moiety has a longer half-life in blood than that of the antigen binding domain, the antigen binding domain may have no FcRn binding activity, as a matter of course, or the antigen binding domain may have FcRn binding activity as long as the FcRn binding activity is weaker than that of the carrying moiety.

In one embodiment, the method for extending the half-life in blood of the carrying moiety involves binding the carrying moiety to albumin. Since albumin does not undergo renal excretion and has FcRn binding activity, its half-life in blood is as long as 17 to 19 days (J Clin Invest. 1953 Aug; 32 (8): 746-768). Hence, it has been reported that a protein bound with albumin becomes bulky and capable of binding indirectly to FcRn and therefore has an increased half-life in blood (Antibodies 2015, 4 (3), 141-156).

In one embodiment, the alternative method for extending the half-life in blood of the carrying moiety involves PEGylating the carrying moiety. The PEGylation of a protein is considered to render the protein bulky and also suppress its degradation by protease in blood, thereby extending the half-life in blood of the protein (J Pharm Sci. 2008 Oct; 97 (10): 4167-83).

In some embodiments, the carrying moiety contains an antibody Fc region. In a specific embodiment, the carrying moiety contains a CH2 domain and a CH3 domain of a human IgG antibody. In a specific embodiment, the carrying moiety contains a moiety spanning from human IgG1 antibody heavy chain Cys226 or Pro230 to the carboxyl terminus of the heavy chain. However, the C-terminal lysine (Lys447) or glycine-lysine (Gly446-Lys447) of the Fc region may be present or absent.

In some embodiments, the carrying moiety contains an antibody constant region. In a more preferred embodiment, the carrying moiety contains an IgG antibody constant region. In a further preferred embodiment, the carrying moiety contains a human IgG antibody constant region.

In some further embodiments, the carrying moiety contains: a region substantially similar in structure to an antibody heavy chain constant region; and a region substantially similar in structure to an antibody light chain, connected to the region via a covalent bond such as a disulfide bond or a noncovalent bond such as a hydrogen bond or hydrophobic interaction.

Herein, the "polypeptide comprising an antigen binding domain and a carrying moiety" is usually a series of polypeptides connected through an amide bond, or a protein containing a plurality of polypeptides connected through an amide bond.

In some embodiments, the antigen binding domain is capable of being released from the polypeptide, and the antigen binding domain released from the polypeptide has higher antigen binding activity. Herein, the term "release" refers to the mutual separation of two moieties of the polypeptide. The release of the antigen binding domain from the polypeptide can be attributed to the cancelation of the interaction between the antigen binding domain and the carrying moiety. The antigen binding activity of the antigen binding domain incorporated in the polypeptide is inhibited. Hence, the antigen binding domain released from the polypeptide can be confirmed by measuring the antigen binding activity of a subject and comparing it with the antigen binding activity of the antigen binding domain incorporated in the polypeptide.

In some embodiments, the polypeptide comprises a cleavage site, and the cleavage site is cleaved so that the antigen binding domain is released from the polypeptide. The cleavage site can be cleaved by, for example, an enzyme, can be reduced with a reducing agent, or can be photodegraded. The cleavage site may be placed at any position in the polypeptide as long as the antigen binding domain can be released and does not lose its antigen binding activity after the release. The polypeptide may further contain an additional cleavage site other than the cleavage site for the release of the antigen binding domain. In one embodiment, the cleavage site comprises a protease cleavage sequence and can be cleaved by protease.

Herein, the term "cleaved" refers to a state where the antigen binding domain and the carrying moiety are separated from each other after alteration of the cleavage site by protease, reduction of a cysteine-cysteine disulfide bond at the cleavage site, and/or photoactivation. Herein, the term "uncleaved" refers to a state where the antigen binding domain is linked to the carrying moiety in the absence of the protease cleavage of the cleavage site, in the absence of the reduction of a cysteine-cysteine disulfide bond at the cleavage site, and/or in the absence of light.

The cleavage of the cleavage site can be detected by subjecting a solution containing the cleavage site-containing polypeptide to SDS-PAGE (polyacrylamide gel electrophoresis) and measuring the molecular weights of the fragments or detecting change in molecular weight between before and after the cleavage.

The cleavage site can be specifically modified (cleaved, reduced or photodegraded) by an agent (i.e., protease, a reducing agent, or light) at a rate of approximately 0.001 to 1500 x 10⁴ M⁻¹S⁻¹ or at least 0.001, 0.005, 0.01, 0.05, 0.1, 0.5, 1, 2.5, 5, 7.5, 10, 15, 20, 25, 50, 75, 100, 125, 150, 200, 250, 500, 750, 1000, 1250, or 1500 x 10⁴ M⁻¹S⁻¹.

The specific cleavage by protease is performed by the contact between the protease and the cleavage site or a molecule containing the cleavage site. The cleavage site can be cleaved in the presence of sufficient enzyme activity. The sufficient enzyme activity can refer to the ability of the enzyme to bring about cleavage upon contact with the cleavage site.

Herein, the term "protease" refers to an enzyme such as endopeptidase or exopeptidase which hydrolyzes a peptide bond, typically, endopeptidase. The protease used in the present application is limited only by being capable of cleaving the protease cleavage sequence and is not particularly limited by its type. In some embodiments, target tissue specific protease is used. The target tissue specific protease can refer to, for example, any of
(1) protease that is expressed at a higher level in the target tissue than in normal tissues,
(2) protease that has higher activity in the target tissue than in normal tissues,
(3) protease that is expressed at a higher level in the target cells than in normal cells, and
(4) protease that has higher activity in the target cells than in normal cells.

In a more specific embodiment, a cancer tissue specific protease or an inflammatory tissue specific protease is used.

Herein, the term "target tissue" means a tissue containing at least one target cell. In some embodiments, the target tissue is a cancer tissue. In some embodiments, the target tissue is an inflammatory tissue.

The term "cancer tissue" means a tissue containing at least one cancer cell. Thus, considering that, for example, the cancer tissue contains cancer cells and vascular vessels, the term refers to every cell type that contributes to the formation of tumor mass including cancer cells and endothelial cells. Herein, the tumor mass refers to a foci of tumor tissue. The term "tumor" is generally used to mean benign neoplasm or malignant neoplasm.

Herein, examples of the "inflammatory tissue" include the following:
- a joint tissue in rheumatoid arthritis or osteoarthritis,
- a lung (alveolus) tissue in bronchial asthma or COPD,
- a digestive organ tissue in inflammatory bowel disease, Crohn disease, or ulcerative colitis,
- a fibrotic tissue in fibrosis in the liver, the kidney, or the lung,
- a tissue under rejection of organ transplantation,
- a vascular vessel or heart (cardiac muscle) tissue in arteriosclerosis or heart failure,
- a visceral fat tissue in metabolic syndrome,
- a skin tissue in atopic dermatitis and other dermatitides, and
- a spinal nerve tissue in disk herniation or chronic lumbago.

Specifically expressed or specifically activated protease, or protease considered to be related to the disease condition of a target tissue (target tissue specific protease) is known for some types of target tissues. For example, International Publication Nos. WO2013/128194, WO2010/081173, and WO2009/025846 disclose protease specifically expressed in a cancer tissue. Also, J Inflamm (Lond). 2010; 7: 45, Nat Rev Immunol. 2006 Jul; 6 (7): 541-50, Nat Rev Drug Discov. 2014 Dec; 13 (12): 904-27, Respir Res. 2016 Mar 4; 17: 23, Dis Model Mech. 2014 Feb; 7 (2): 193-203, and Biochim Biophys Acta. 2012 Jan; 1824 (1): 133-45 disclose protease considered to be related to inflammation.

In addition to the protease specifically expressed in a target tissue, there also exists protease specifically activated in a target tissue. For example, protease may be expressed in an inactive form and then converted to an active form. Many tissues contain a substance inhibiting active protease and control the activity by the process of activation and the presence of the inhibitor (Nat Rev Cancer. 2003 Jul; 3 (7): 489-501). In a target tissue, the active protease may be specifically activated by escaping inhibition.

The active protease can be measured by use of a method using an antibody recognizing the active protease (PNAS 2013 Jan 2; 110 (1): 93-98) or a method of fluorescently labeling a peptide recognizable by protease so that the fluorescence is quenched before cleavage, but emitted after cleavage (Nat Rev Drug Discov. 2010 Sep; 9 (9): 690-701. doi: 10.1038/nrd3053).

From one viewpoint, the term "target tissue specific protease" can refer to any of
(i) protease that is expressed at a higher level in the target tissue than in normal tissues,
(ii) protease that has higher activity in the target tissue than in normal tissues,
(iii) protease that is expressed at a higher level in the target cells than in normal cells, and
(iv) protease that has higher activity in the target cells than in normal cells.

Specific examples of the protease include, but are not limited to, cysteine protease (including cathepsin families B, L, S, etc.), aspartyl protease (cathepsins D, E, K, O, etc.), serine protease (including matriptase (including MT-SP1), cathepsins A and G, thrombin, plasmin, urokinase (uPA), tissue plasminogen activator (tPA), elastase, proteinase 3, thrombin, kallikrein, tryptase, and chymase), metalloproteinase (metalloproteinase (MMP1-28) including both membrane-bound forms (MMP14-17 and MMP24-25) and secreted forms (MMP1-13, MMP18-23 and MMP26-28), A disintegrin and metalloproteinase (ADAM), A disintegrin and metalloproteinase with thrombospondin motifs (ADAMTS), meprin (meprin alpha and meprin beta), CD10 (CALLA), prostate-specific antigen (PSA), legumain, TMPRSS3, TMPRSS4, human neutrophil elastase (HNE), beta secretase (BACE), fibroblast activation protein alpha (FAP), granzyme B, guanidinobenzoatase (GB), hepsin, neprilysin, NS3/4A, HCV-NS3/4, calpain, ADAMDEC1, renin, cathepsin C, cathepsin V/L2, cathepsin X/Z/P, cruzipain, otubain 2, kallikrein-related peptidases (KLKs (KLK3, KLK4, KLK5, KLK6, KLK7, KLK8, KLK10, KLK11, KLK13, and KLK14)), bone morphogenetic protein 1 (BMP-1), activated protein C, blood coagulation-related protease (Factor VIIa, Factor IXa, Factor Xa, Factor XIa, and Factor XIIa), HtrA1, lactoferrin, marapsin, PACE4, DESC1, dipeptidyl peptidase 4 (DPP-4), TMPRSS2, cathepsin F, cathepsin H, cathepsin L2, cathepsin O, cathepsin S, granzyme A, Gepsin calpain 2, glutamate carboxypeptidase 2, AMSH-like proteases, AMSH, gamma secretase, antiplasmin cleaving enzyme (APCE), decysin 1, N-acetylated alpha-linked acidic dipeptidase-like 1 (NAALADL1), and furin.

From another viewpoint, the target tissue specific protease can refer to a cancer tissue specific protease or an inflammatory tissue specific protease.

Examples of cancer tissue specific protease include protease specifically expressed in a cancer tissue disclosed in International Publication Nos. WO2013/128194, WO2010/081173, and WO2009/025846.

As for the type of cancer tissue specific protease, the protease having higher expression specificity in the cancer tissue to be treated is more effective for reducing adverse reactions. Preferable cancer tissue specific protease has a concentration in the cancer tissue at least 5 times, more preferably at least 10 times, further preferably at least 100 times, particularly preferably at least 500 times, most preferably at least 1000 times higher than its concentration in normal tissues. Also, preferable cancer tissue specific protease has activity in the cancer tissue at least 2 times, more preferably at least 3 times, at least 4 times, at least 5 times, or at least 10 times, further preferably at least 100 times, particularly preferably at least 500 times, most preferably at least 1000 times higher than its activity in normal tissues.

The cancer tissue specific protease may be in a form bound with a cancer cell membrane or may be in a form secreted extracellularly without being bound with a cell membrane. When the cancer tissue specific protease is not bound with a cancer cell membrane, it is preferred for immunocyte-mediated cytotoxicity specific for cancer cells that the cancer tissue specific protease should exist within or in the vicinity of the cancer tissue. Herein, the "vicinity of the cancer tissue" means to fall within the scope of location where the protease cleavage sequence specific for the cancer tissue is cleaved so that the antigen binding domain exerts antigen binding activity. However, it is preferred that damage on normal cells should be minimized in this scope of location.

From an alternative viewpoint, cancer tissue specific protease is any of
(i) protease that is expressed at a higher level in the cancer tissue than in normal tissues,
(ii) protease that has higher activity in the cancer tissue than in normal tissues,
(iii) protease that is expressed at a higher level in the cancer cells than in normal cells, and
(iv) protease that has higher activity in the cancer cells than in normal cells.

One type of cancer tissue specific protease may be used alone, or two or more types of cancer tissue specific proteases may be combined. The number of types of cancer tissue specific protease can be appropriately set by those skilled in the art in consideration of the cancer type to be treated.

From these viewpoints, cancer tissue specific protease is preferably serine protease or metalloproteinase, more preferably matriptase (including MT-SP1), urokinase (uPA), or metalloproteinase, further preferably MT-SP1, uPA, MMP2, or MMP9, among the proteases listed above.

As for the type of inflammatory tissue specific protease, the protease having higher expression specificity in the inflammatory tissue to be treated is more effective for reducing adverse reactions. Preferable inflammatory tissue specific protease has a concentration in the inflammatory tissue at least 5 times, more preferably at least 10 times, further preferably at least 100 times, particularly preferably at least 500 times, most preferably at least 1000 times higher than its concentration in normal tissues. Also, preferable inflammatory tissue specific protease has activity in the inflammatory tissues at least 2 times, more preferably at least 3 times, at least 4 times, at least 5 times, or at least 10 times, further preferably at least 100 times, particularly preferably at least 500 times, most preferably at least 1000 times higher than its activity in normal tissues.

The inflammatory tissue specific protease may be in a form bound with an inflammatory cell membrane or may be in a form secreted extracellularly without being bound with a cell membrane. When the inflammatory tissue specific protease is not bound with an inflammatory cell membrane, it is preferred for immunocyte-mediated cytotoxicity specific for inflammatory cells that the inflammatory tissue specific protease should exist within or in the vicinity of the inflammatory tissue. Herein, the "vicinity of the inflammatory tissue" means to fall within the scope of location where the protease cleavage sequence specific for the inflammatory tissue is cleaved so that the antigen binding domain exerts antigen binding activity. However, it is preferred that damage on normal cells should be minimized in this scope of location.

From an alternative viewpoint, inflammatory tissue specific protease is any of
(i) protease that is expressed at a higher level in the inflammatory tissue than in normal tissues,
(ii) protease that has higher activity in the inflammatory tissue than in normal tissues,
(iii) protease that is expressed at a higher level in the inflammatory cells than in normal cells, and
(iv) protease that has higher activity in the inflammatory cells than in normal cells.

One type of inflammatory tissue specific protease may be used alone, or two or more types of inflammatory tissue specific proteases may be combined. The number of types of inflammatory tissue specific protease can be appropriately set by those skilled in the art in consideration of the pathological condition to be treated.

From these viewpoints, t inflammatory tissue specific protease is preferably metalloproteinase among the proteases listed above. The metalloproteinase is more preferably ADAMTS5, MMP2, MMP7, MMP9, or MMP13.

The protease cleavage sequence is a particular amino acid sequence that is specifically recognized by target tissue specific protease when the polypeptide is hydrolyzed by the target tissue specific protease in an aqueous solution.

The protease cleavage sequence is preferably an amino acid sequence that is hydrolyzed with high specificity by target tissue specific protease more specifically expressed in the target tissue or cells to be treated or more specifically activated in the target tissue/ cells to be treated, from the viewpoint of reduction in adverse reactions.

Specific examples of the protease cleavage sequence include target sequences that are specifically hydrolyzed by the above-listed protease specifically expressed in a cancer tissue disclosed in International Publication Nos. WO2013/128194, WO2010/081173, and WO2009/025846, the protease specific for an inflammatory tissue, and the like. A sequence artificially altered by, for example, introducing an appropriate amino acid mutation to a target sequence that is specifically hydrolyzed by known protease can also be used. Alternatively, a protease cleavage sequence identified by a method known to those skilled in the art as described in Nature Biotechnology 19, 661-667 (2001) may be used.

Furthermore, a naturally occurring protease cleavage sequence may be used. For example, TGFβ is converted to a latent form by protease cleavage. Likewise, a protease cleavage sequence in a protein that changes its molecular form by protease cleavage can also be used.

Examples of the protease cleavage sequence that can be used include, but are not limited to, sequences disclosed in International Publication No. WO2015/116933, International Publication No. WO2015/048329, International Publication No. WO2016/118629, International Publication No. WO2016/179257, International Publication No. WO2016/179285, International Publication No. WO2016/179335, International Publication No. WO2016/179003, International Publication No. WO2016/046778, International Publication No. WO2016/014974, U.S. Patent Publication No. US2016/0289324, U.S. Patent Publication No. US2016/0311903, PNAS (2000) 97: 7754-7759, Biochemical Journal (2010) 426: 219-228, and Beilstein J Nanotechnol. (2016) 7: 364-373.

The protease cleavage sequence is more preferably an amino acid sequence that is specifically hydrolyzed by suitable target tissue specific protease as mentioned above.

In one embodiment, a flexible linker is further attached to either one end or both ends of the protease cleavage sequence. The flexible linker at one end of the protease cleavage sequence can be referred to as a first flexible linker, and the flexible linker at the other end can be referred to as a second flexible linker. In a particular embodiment, the protease cleavage sequence and the flexible linker have any of the following formulas:
(protease cleavage sequence),
(first flexible linker)-(protease cleavage sequence),
(protease cleavage sequence)-(second flexible linker), and
(first flexible linker)-(protease cleavage sequence)-(second flexible linker).

The flexible linker according to the present embodiment is preferably a peptide linker. The first flexible linker and the second flexible linker each independently and arbitrarily exist and are identical or different flexible linkers each containing at least one flexible amino acid (Gly, etc.). The flexible linker contains, for example, a sufficient number of residues (amino acids arbitrarily selected from Arg, Ile, Gln, Glu, Cys, Tyr, Trp, Thr, Val, His, Phe, Pro, Met, Lys, Gly, Ser, Asp, Asn, Ala, etc., particularly Gly, Ser, Asp, Asn, and Ala, in particular, Gly and Ser, especially Gly, etc.) for the protease cleavage sequence to obtain the desired protease accessibility.

The flexible linker suitable for use at both ends of the protease cleavage sequence is usually a flexible linker that improves the access of protease to the protease cleavage sequence and elevates the cleavage efficiency of the protease. A suitable flexible linker may be readily selected and can be preferably selected from among different lengths such as 1 amino acid (Gly, etc.) to 20 amino acids, 2 amino acids to 15 amino acids, or 3 amino acids to 12 amino acids including 4 amino acids to 10 amino acids, 5 amino acids to 9 amino acids, 6 amino acids to 8 amino acids, or 7 amino acids to 8 amino acids. In some embodiments, the flexible linker is a peptide linker of 1 to 7 amino acids.

Examples of the flexible linker include, but are not limited to, glycine polymers (G)n, glycine-serine polymers (including e.g., (GS)n, (GSGGS: SEQ ID NO: 1834)n and (GGGS: SEQ ID NO: 1835)n, wherein n is an integer of at least 1), glycine-alanine polymers, alanine-serine polymers, and other flexible linkers well known in conventional techniques.

Among them, glycine and glycine-serine polymers are receiving attention because these amino acids are relatively unstructured and easily function as neutral tethers between components.

Herein, the "association" can refer to, for example, a state where two or more polypeptide regions interact with each other. In general, a hydrophobic bond, a hydrogen bond, an ionic bond, or the like is formed between the intended polypeptide regions to form an associate. As one example of common association, an antibody typified by a natural antibody is known to retain a paired structure of a heavy chain variable region (VH) and a light chain variable region (VL) through a noncovalent bond or the like therebetween.

In some embodiments, the inhibiting domain of the carrying moiety associates with the antigen binding domain. The inhibiting domain may constitute a portion of the carrying moiety or may constitute the whole of the carrying moiety. From another viewpoint, the inhibiting domain can also be defined as a moiety associating with the antigen binding domain, in the carrying moiety.

In a more specific embodiment, the antigen binding domain which is a single-domain antibody and the inhibiting domain which is VL, VH or VHH form association as found between antibody VH and antibody VL. In a further specific embodiment, the antigen binding domain which is a single-domain antibody and the inhibiting domain which is VL, VH or VHH form association as found between antibody VH and antibody VL, and in a state of the association thus formed, the inhibiting domain conformationally inhibits the binding of the antigen binding domain to the antigen or conformationally changes the antigen binding site of the antigen binding domain so that the antigen binding activity of the single-domain antibody is inhibited by the VL, the VH or the VHH. In an embodiment using VHH as the single-domain antibody, it is considered that the binding of the VHH to the antigen is conformationally inhibited by the inhibiting domain when CDR3, a main antigen binding site of the VHH, or its neighboring site exists at the interface of association with the inhibiting domain.

The association of the antigen binding domain with the inhibiting domain may be canceled, for example, by cleaving the cleavage site. The cancelation of the association can be used interchangeably with, for example, the cancelation of the state where two or more polypeptide regions interact with each other. The interaction between the two or more polypeptide regions may be wholly canceled, or the interaction between the two or more polypeptide regions may be partially canceled.

Herein, the "interface" usually refers to a face at which two regions associate or interact with each other. Amino acid residues forming the interface are usually one or more amino acid residues contained in each polypeptide region subjected to the association and more preferably refer to amino acid residues that approach each other upon association and participate in interaction. Specifically, the interaction includes a noncovalent bond such as a hydrogen bond, electrostatic interaction, or salt bridge formation between the amino acid residues approaching each other upon association.

Herein, the "amino acid residues forming the interface" specifically refers to amino acid residues contained in polypeptide regions constituting the interface. As one example, the polypeptide regions constituting the interface refer to polypeptide regions responsible for intramolecular or intermolecular selective binding in antibodies, ligands, receptors, substrates, etc. Specific examples of such polypeptide regions in antibodies can include a heavy chain variable region and a light chain variable region. In some embodiments, examples of such polypeptide regions can include an antigen binding domain and an inhibiting domain.

Examples of the amino acid residues forming the interface include, but are not limited to, amino acid residues approaching each other upon association. The amino acid residues approaching each other upon association can be found, for example, by analyzing the conformations of polypeptides and examining the amino acid sequences of polypeptide regions forming the interface upon association of the polypeptides.

In some embodiments, an amino acid residue involved in association in the antigen binding domain, or an amino acid residue involved in association in the inhibiting domain can be altered in order to promote the association of the antigen binding domain with the inhibiting domain. In a further specific embodiment, an amino acid residue forming the interface with the inhibiting domain, in the antigen binding domain, or an amino acid residue forming the interface with the antigen binding domain, in the inhibiting domain can be altered. In a preferred embodiment, the amino acid residue forming the interface can be altered by a method of introducing a mutation to the interface amino acid residue such that two or more amino acid residues forming the interface have different charges. The alteration of the amino acid residue to result in different charges includes the alteration of a positively charged amino acid residue to a negatively charged amino acid residue or an uncharged amino acid residue, the alteration of a negatively charged amino acid residue to a positively charged amino acid residue or an uncharged amino acid residue, and the alteration of an uncharged amino acid residue to a positively or negatively charged amino acid residue. Such an amino acid alteration is performed for the purpose of promoting the association and is not limited by the position of the amino acid alteration or the type of the amino acid as long as the purpose of promoting the association can be achieved. Examples of the alteration include, but are not limited to, substitution.

In some embodiments, VHH serving as the antigen binding domain associates with VL serving as the inhibiting domain. The amino acid residue involved in association with VL, in VHH can refer to, for example, an amino acid residue forming the interface between the VHH and the VL. Examples of the amino acid residue involved in association with VL, in VHH include, but are not limited to, amino acid residues at positions 37, 44, 45, and 47 (J. Mol. Biol. (2005) 350, 112-125). The activity of the VHH is inhibited by promoting the association between the VHH and the VL. Likewise, the amino acid residue involved in association with VHH, in VL can refer to, for example, an amino acid residue forming the interface between the VHH and the VL.

An amino acid residue involved in association with VL, in VHH can be altered in order to promote the association between the VHH and the VL. Examples of such an amino acid substitution include, but are not limited to, F37V, Y37V, E44G, Q44G, R45L, H45L, G47W, F47W, L47W, T47W, or/and S47W. Instead of altering each residue in VHH, VHH originally having an amino acid residue 37V, 44G, 45L, or/and 47W may be used.

Instead of the VHH amino acid, an amino acid residue involved in association with VHH, in VL may be altered, and amino acid alterations may also be introduced to both VHH and VL, as long as the purpose of promoting the association between the VHH and the VL can be achieved.

In some alternative embodiments, the antigen binding domain and the inhibiting domain can be associated with each other by using VHH as the antigen binding domain and using VH or VHH as the inhibiting domain. An amino acid residue involved in association with VH or VHH serving as the inhibiting domain, in VHH serving as the antigen binding domain can be identified and altered in order to promote the association of the antigen binding domain VHH with the inhibiting domain VH or VHH. Also, an amino acid residue involved in association with VHH serving as the antigen binding domain, in VH or VHH serving as the inhibiting domain, can be identified and altered.

In the case of using a single-domain antibody other than VHH as the antigen binding domain, an amino acid residue involved in association, in the antigen binding domain or the inhibiting domain can also be identified and altered similarly to above.

In some embodiments, the carrying moiety and the antigen binding domain are fused via a linker. In a more specific embodiment, the carrying moiety and the antigen binding domain are fused via a linker containing a cleavage site. In an alternative specific embodiment, the carrying moiety and the antigen binding domain are fused via a linker, and the fusion protein thus formed contains a cleavage site.

In another embodiment, the carrying moiety and the antigen binding domain are fused without a linker. In a more specific embodiment, an amino bond is formed between the N-terminal amino acid of the carrying moiety and the C-terminal amino acid of the antigen binding domain to form a fusion protein. The formed fusion protein contains a cleavage site. In a particular embodiment, one to several N-terminal amino acids of the carrying moiety or/and one to several C-terminal amino acids of the antigen binding domain are altered, and the N terminus of the carrying moiety and the C terminus of the antigen binding domain are fused to form a cleavage site near the fusion position. More specifically, the cleavage site can be formed, for example, by converting four C-terminal amino acids of the antigen binding domain to a LSGR (SEQ ID NO: 1836) sequence and converting four N-terminal amino acids of the carrying moiety to a SDNH (SEQ ID NO: 1837) sequence.

In some embodiments, the cleavage site of the polypeptide comprising a carrying moiety and an antigen binding domain comprises a protease cleavage sequence. The protease cleavage sequence may be placed at any position in the polypeptide as long as the antigen binding domain is released by protease cleavage and does not lose its antigen binding activity after the release.

In some embodiments, the carrying moiety comprises an antibody constant region, and the N terminus of the antibody constant region and the C terminus of the antigen binding domain are fused via a linker or without a linker.

In a particular embodiment, the protease cleavage sequence is located within the antibody constant region contained in the carrying moiety. In this case, the protease cleavage sequence can be located within the antibody constant region such that the antigen binding domain is released by protease cleavage. In a specific embodiment, the protease cleavage sequence is located within an antibody heavy chain constant region contained in the carrying moiety, and more specifically located on the antigen binding domain side with respect to amino acid position 140 (EU numbering) in the antibody heavy chain constant region, preferably on the antigen binding domain side with respect to amino acid position 122 (EU numbering) in the antibody heavy chain constant region. In an alternative specific embodiment, the protease cleavage sequence is located within an antibody light chain constant region contained in the carrying moiety, and more specifically located on the antigen binding domain side with respect to amino acid position 130 (EU numbering) (Kabat numbering position 130) in the antibody light chain constant region, preferably on the antigen binding domain side with respect to amino acid position 113 (EU numbering) (Kabat numbering position 113) in the antibody light chain constant region.

In some embodiments, the antigen binding domain is a single-domain antibody, and the C terminus of the single-domain antibody and the N terminus of the carrying moiety are fused via a linker or without a linker.

In a particular embodiment, the protease cleavage sequence is located within the single-domain antibody. In a more specific embodiment, the single-domain antibody is a single-domain antibody prepared from VH, or VHH, and the protease cleavage sequence is located on the carrying moiety side with respect to amino acid position 35b (Kabat numbering) of the single-domain antibody, preferably on the carrying moiety side with respect to amino acid position 95 (Kabat numbering) of the single-domain antibody, more preferably on the carrying moiety side with respect to amino acid position 109 (Kabat numbering) of the single-domain antibody. In an alternative specific embodiment, the single-domain antibody is a single-domain antibody prepared from VL, and the protease cleavage sequence is located on the carrying moiety side with respect to amino acid position 32 (Kabat numbering) of the single-domain antibody, preferably on the carrying moiety side with respect to amino acid position 91 (Kabat numbering) of the single-domain antibody, more preferably on the carrying moiety side with respect to amino acid position 104 (Kabat numbering) of the single-domain antibody.

In some embodiments, the carrying moiety comprises an antibody constant region, the antigen binding domain is a single-domain antibody, and the antibody constant region and the single-domain antibody are fused via a linker or without a linker. In a more specific embodiment, the N terminus of the antibody constant region and the C terminus of the single-domain antibody are fused via a linker or without a linker. In an alternative specific embodiment, the C terminus of the antibody constant region and the N terminus of the single-domain antibody are fused via a linker or without a linker.

In a particular embodiment, the protease cleavage sequence is located within the antibody constant region contained in the carrying moiety. In a more specific embodiment, the protease cleavage sequence is located on the single-domain antibody side with respect to amino acid position 140 (EU numbering) in an antibody heavy chain constant region, preferably on the single-domain antibody side with respect to amino acid position 122 (EU numbering) in an antibody heavy chain constant region. In an alternative specific embodiment, the protease cleavage sequence is located on the antigen binding domain side with respect to amino acid position 130 (EU numbering) (Kabat numbering position 130) in an antibody light chain constant region, preferably on the antigen binding domain side with respect to amino acid position 113 (EU numbering) (Kabat numbering position 113) in an antibody light chain constant region.

In a particular embodiment, the protease cleavage sequence is located within the single-domain antibody. In a more specific embodiment, the single-domain antibody is a single-domain antibody prepared from VH, or VHH, and the protease cleavage sequence is located on the antibody constant region side with respect to amino acid position 35b (Kabat numbering) of the single-domain antibody, preferably on the antibody constant region side with respect to amino acid position 95 (Kabat numbering) of the single-domain antibody, more preferably on the antibody constant region side with respect to amino acid position 109 (Kabat numbering) of the single-domain antibody. In an alternative specific embodiment, the single-domain antibody is a single-domain antibody prepared from VL, and the protease cleavage sequence is located on the antibody constant region side with respect to amino acid position 32 (Kabat numbering) of the single-domain antibody, preferably on the antibody constant region side with respect to amino acid position 91 (Kabat numbering) of the single-domain antibody, more preferably on the antibody constant region side with respect to amino acid position 104 (Kabat numbering) of the single-domain antibody.

In a particular embodiment, the protease cleavage sequence is located near the boundary between the antigen binding domain and the carrying moiety. The phrase "near the boundary between the antigen binding domain and the carrying moiety" refers to a moiety that resides upstream or downstream of the linking site between the antigen binding domain and the carrying moiety and does not largely influence the secondary structure of the antigen binding domain.

In a more specific embodiment, the antigen binding domain is linked to the antibody constant region contained in the carrying moiety, and the protease cleavage sequence is located near the boundary between the antigen binding domain and the antibody constant region. The phrase "near the boundary between the antigen binding domain and the antibody constant region" can refer to near the boundary between the antigen binding domain and an antibody heavy chain constant region, or near the boundary between the antigen binding domain and an antibody light chain constant region. When the antigen binding domain is a single-domain antibody prepared from VH, or VHH and is connected to an antibody heavy chain constant region, the phrase "near the boundary between the antigen binding domain and the antibody constant region" can refer to between amino acid position 101 (Kabat numbering) of the single-domain antibody and amino acid position 140 (EU numbering) of the antibody heavy chain constant region and can preferably refer to between amino acid position 109 (Kabat numbering) of the single-domain antibody and amino acid position 122 (EU numbering) of the antibody heavy chain constant region. When the antigen binding domain is a single-domain antibody prepared from VH, or VHH and is connected to an antibody light chain constant region, the phrase "near the boundary between the antigen binding domain and the antibody light chain constant region" can refer to between amino acid position 101 (Kabat numbering) of the single-domain antibody and amino acid position 130 (EU numbering) (Kabat numbering position 130) of the antibody light chain constant region and can preferably refer to between amino acid position 109 (Kabat numbering) of the single-domain antibody and amino acid position 113 (EU numbering) (Kabat numbering position 113) of the antibody light chain constant region. When the antigen binding domain is a single-domain antibody prepared from VL, the phrase "near the boundary between the antigen binding domain and the antibody constant region" refers to between amino acid position 96 (Kabat numbering) of the single-domain antibody and the prescribed position of the antibody constant region, preferably between amino acid position 104 (Kabat numbering) of the single-domain antibody and the prescribed position of the antibody constant region.

In some embodiments, the polypeptide is an IgG antibody-like molecule. Examples of such embodiments include, but are not limited to: an embodiment in which the carrying moiety comprises an IgG antibody constant region, a single-domain antibody serving as the antigen binding domain takes the place of VH of an IgG antibody, and the antigen binding activity is inhibited by VL; an embodiment in which the carrying moiety comprises an IgG antibody constant region, a single-domain antibody serving as the antigen binding domain takes the place of VL of an IgG antibody, and the antigen binding activity is inhibited by VH; and an embodiment in which the carrying moiety comprises an IgG antibody constant region, a single-domain antibody serving as the antigen binding domain takes the place of one of VH and VL of an IgG antibody, and an additional single-domain antibody inhibits the antigen binding activity of the antigen binding domain takes the place of the other domain of the IgG antibody.

The term "IgG antibody-like molecule" used herein is used to define a molecule having moieties substantially similar in structure to constant domains or constant regions as in an IgG antibody, and moieties substantially similar in structure to variable domains or variable regions as in the IgG antibody, and having conformation substantially similar to that of the IgG antibody. However, herein, the "IgG antibody-like molecule" may or may not exert antigen binding activity while retaining the structures similar to those of the IgG antibody.

The polypeptide may comprise one or more antigen binding domains. One or more inhibiting domains may inhibit the antigen binding activity of a plurality of antigen binding domains. A plurality of antigen binding domains may each be associated with the inhibiting domain. A plurality of antigen binding domains may each be fused with the carrying moiety. A plurality of antigen binding domains may each be capable of released from the polypeptide. The cleavage site(s) for release a plurality of antigen binding domains may be a plurality of cleavage sites corresponding to the number of antigen binding domains.

In some embodiments, the antigen binding domain is further linked to a second antigen binding domain. Examples of the second antigen binding domain include, but are not limited to, single-domain antibodies, antibody fragments, a module called A domain of approximately 35 amino acids contained in an in vivo cell membrane protein avimer (International Publication Nos. WO2004/044011 and WO2005/040229), adnectin containing a 10Fn3 domain serving as a protein binding domain derived from a glycoprotein fibronectin expressed on cell membranes (International Publication No. WO2002/032925), Affibody containing an IgG binding domain scaffold constituting a three-helix bundle composed of 58 amino acids of protein A (International Publication No. WO1995/001937), DARPins (designed ankyrin repeat proteins) which are molecular surface-exposed regions of ankyrin repeats (AR) each having a 33-amino acid residue structure folded into a subunit of a turn, two antiparallel helices, and a loop (International Publication No. WO2002/020565), anticalin having four loop regions connecting eight antiparallel strands bent toward the central axis in one end of a barrel structure highly conserved in lipocalin molecules such as neutrophil gelatinase-associated lipocalin (NGAL) (International Publication No. WO2003/029462), and a depressed region in the internal parallel sheet structure of a horseshoe-shaped fold composed of repeated leucine-rich-repeat (LRR) modules of an immunoglobulin structure-free variable lymphocyte receptor (VLR) as seen in the acquired immune systems of jawless vertebrates such as lamprey or hagfish (International Publication No. WO2008/016854). In a preferred embodiment, the second antigen binding domain has antigen binding specificity different from that of the antigen binding domain. In a preferred embodiment, the molecular weight of the antigen binding domain and the second antigen binding domain linked is 60 kDa or smaller.

In some more specific embodiments, the antigen binding domain and the second antigen binding domain are single-domain antibodies differing in antigen binding specificity, the antigen binding domain and the second antigen binding domain linked are capable of being released from the polypeptide, and the antigen binding domain and the second antigen binding domain form a bispecific antigen binding molecule after release. Examples of such a bispecific antigen binding molecule include, but are not limited to, a bispecific antigen binding molecule having an antigen binding domain specifically binding to the target cell surface antigen and a second antigen binding domain specifically binding to an immunocyte surface antigen, a bispecific antigen binding molecule having an antigen binding domain and a second antigen binding domain binding to different subunits of the same antigen, and a bispecific antigen binding molecule having an antigen binding domain and a second antigen binding domain binding to different epitopes in the same antigen. Such a bispecific antigen binding molecule can recruit immunocytes to the vicinity of target cells and is thus considered useful in the treatment of a disease caused by the target cells.

The antigen binding activity of the second antigen binding domain may or may not be inhibited by the carrying moiety. The second antigen binding domain may or may not be associated with a partial structure of the carrying moiety. Particularly, when the antigen binding domain and the second antigen binding domain differ in antigen binding specificity, the antigen binding domain in an unreleased state cannot exert antigen binding activity, even if the antigen binding activity of the second antigen binding domain is not inhibited and even if the second antigen binding domain is not associated with a partial structure of the carrying moiety. This bispecific antigen binding molecule comprising the antigen binding domain linked to the second antigen binding domain cannot exert a function of bispecifically binding to two types of antigens.

The antigen-binding molecule of the present application encompasses, for example, the antigen-binding molecules described in WO2018/097308.
In one aspect, the antigen-binding molecule of the present application relates to an antigen-binding molecule as a ligand binding molecule capable of binding to a ligand, wherein the molecule is a polypeptide having at least one cleavage site, and wherein the ligand binding of the molecule is attenuated when the molecule is in a state of being cleaved at the at least one cleavage site.

The ligand binding molecule is a molecule capable of binding to a ligand, particularly, a molecule capable of binding to a ligand when uncleaved. In this context, the "binding" usually refers to binding through interaction based mainly on a noncovalent bond such as electrostatic force, van der Waals' force, or a hydrogen bond. Preferred examples of the ligand binding form of the ligand binding molecule include, but are not limited to, antigen-antibody reaction through which an antigen binding region, an antigen binding molecule, an antibody, an antibody fragment, or the like binds to the antigen.

The phrase "capable of binding to a ligand" means that the ligand binding molecule is capable of binding to the ligand even if the ligand binding molecule and the ligand are different molecules and does not mean that the ligand binding molecule and the ligand are connected through a covalent bond. For example, the phrase "capable of binding to a ligand" does not mean that the ligand and the ligand binding molecule are connected through a covalent bond via a linker. Also, the phrase "ligand binding is attenuated" means that the capability of binding (binding capacity) described above is attenuated. For example, when the ligand and the ligand binding molecule are connected through a covalent bond via a linker, the cleavage of the linker does not mean that the ligand binding is attenuated. Meanwhile, the ligand binding molecule may be connected with the ligand via a linker or the like as long as the ligand binding molecule is capable of binding to the ligand.

The ligand binding molecule of the present invention is limited only by binding to the ligand when uncleaved, and can be a molecule having any structure as long as the molecule used can bind to the ligand of interest when uncleaved. Examples of the ligand binding molecule include, but are not limited to, an antibody heavy chain variable region (VH), an antibody light chain variable region (VL), a single-domain antibody (sdAb), a module called A domain of approximately 35 amino acids contained in an in vivo cell membrane protein avimer (International Publication Nos. WO2004/044011 and WO2005/040229), adnectin containing a 10Fn3 domain serving as a protein binding domain derived from a glycoprotein fibronectin expressed on cell membranes (International Publication No. WO2002/032925), Affibody containing an IgG binding domain scaffold constituting a three-helix bundle composed of 58 amino acids of protein A (International Publication No. WO1995/001937), DARPins (designed ankyrin repeat proteins) which are molecular surface-exposed regions of ankyrin repeats (AR) each having a 33-amino acid residue structure folded into a subunit of a turn, two antiparallel helices, and a loop (International Publication No. WO2002/020565), anticalin having four loop regions connecting eight antiparallel strands bent toward the central axis in one end of a barrel structure highly conserved in lipocalin molecules such as neutrophil gelatinase-associated lipocalin (NGAL) (International Publication No. WO2003/029462), and a depressed region in the internal parallel sheet structure of a horseshoe-shaped fold composed of repeated leucine-rich-repeat (LRR) modules of an immunoglobulin structure-free variable lymphocyte receptor (VLR) as seen in the acquired immune systems of jawless vertebrates such as lamprey or hagfish (International Publication No. WO2008/016854).

The ligand binding molecule is a polypeptide comprising a cleavage site. The cleavage site can be cleaved by, for example, an enzyme, can be reduced with a reducing agent, or can be photodegraded. The cleavage site may be placed at any position in the polypeptide as long as the ligand binding of the ligand binding molecule can be attenuated by the cleavage of the cleavage site. The polypeptide may contain one or more cleavage sites.

The ligand binding molecule of the present invention binds to the ligand more weakly (i.e., ligand binding is attenuated) in a cleaved state compared with an uncleaved state. In an embodiment in which the ligand binding of the ligand binding molecule is based on antigen-antibody reaction, the attenuation of the ligand binding can be evaluated on the basis of the ligand binding activity of the ligand binding molecule.

The ligand binding activity of the ligand binding molecule can be confirmed by a well-known method such as FACS, an ELISA format, AlphaScreen (Amplified Luminescent Proximity Homogeneous Assay), or a BIACORE method using surface plasmon resonance (SPR) phenomena, or BLI (bio-layer interferometry) (Octet) (Proc. Natl. Acad. Sci. USA (2006) 103 (11), 4005-4010).

AlphaScreen is carried out on the basis of the following principle according to ALPHA Technology using two beads, a donor and an acceptor. Luminescence signals are detected only when these two beads are located in proximity through the interaction between a molecule bound with the donor bead and a molecule bound with the acceptor bead. A laser-excited photosensitizer in the donor bead converts ambient oxygen to singlet oxygen in an excited state. The singlet oxygen diffuses around the donor bead and reaches the acceptor bead located in proximity thereto to thereby cause chemiluminescent reaction in the bead, which finally emits light. When the molecule bound with the donor bead does not interact with the molecule bound with the acceptor bead, no chemiluminescent reaction occurs because singlet oxygen produced by the donor bead does not reach the acceptor bead.

For example, a biotin-labeled ligand binding molecule is bound to the donor bead, while a glutathione S transferase (GST)-tagged ligand is bound to the acceptor bead. In the absence of an untagged competitor ligand binding molecule, the ligand binding molecule interacts with the ligand to generate signals of 520 to 620 nm. The untagged ligand binding molecule competes with the tagged ligand binding molecule for the interaction with the ligand. Decrease in fluorescence resulting from the competition can be quantified to determine relative binding affinity. The biotinylation of the ligand binding molecule such as an antibody using sulfo-NHS-biotin or the like is known in the art. A method which involves, for example: fusing a polynucleotide encoding the ligand in flame with a polynucleotide encoding GST; expressing a GST-fused ligand from cells or the like carrying a vector that permits expression of the resulting fusion gene; and purifying the GST-fused ligand using a glutathione column can be appropriately adopted as a method for tagging the ligand with GST. The obtained signals are preferably analyzed using, for example, software GRAPHPAD PRISM (GraphPad Software, Inc., San Diego) adapted to a one-site competition model based on nonlinear regression analysis.

One (ligand) of the substances between which the interaction is to be observed is immobilized onto a thin gold film of a sensor chip. The sensor chip is irradiated with light from the back such that total reflection occurs at the interface between the thin gold film and glass. As a result, a site having a drop in reflection intensity (SPR signal) is formed in a portion of reflected light. The other (analyte) of the substances between which the interaction is to be observed is flowed on the surface of the sensor chip and bound to the ligand so that the mass of the immobilized ligand molecule is increased to change the refractive index of the solvent on the sensor chip surface. This change in the refractive index shifts the position of the SPR signal (on the contrary, the dissociation of the bound molecules gets the signal back to the original position). The Biacore system plots on the ordinate the amount of the shift, i.e., change in mass on the sensor chip surface, and displays time-dependent change in mass as assay data (sensorgram). Kinetics: an association rate constant (ka) and a dissociation rate constant (kd) are determined from the curve of the sensorgram, and a dissociation constant (KD) is determined from the ratio between these constants. Inhibition assay or equilibrium analysis is also preferably used in the BIACORE method. Examples of the inhibition assay are described in Proc. Natl. Acad. Sci. USA (2006) 103 (11), 4005-4010, and examples of the equilibrium analysis are described in Methods Enzymol. 2000; 323: 325-40.

The phrase "ligand binding function of the ligand binding molecule is attenuated" means that the amount of a test ligand binding molecule bound with the ligand is, for example, 50% or less, preferably 45% or less, 40% or less, 35% or less, 30% or less, 20% or less, or 15% or less, particularly preferably 10% or less, 9% or less, 8% or less, 7% or less, 6% or less, 5% or less, 4% or less, 3% or less, 2% or less, or 1% or less, of the amount of a control ligand binding molecule bound with the ligand on the basis of the measurement method described above. The desired index may be appropriately used as an index for binding activity. For example, a dissociation constant (KD) may be used. In the case of using a dissociation constant (KD) as an index for evaluating binding activity, a larger dissociation constant (KD) of the test ligand binding molecule for the ligand than that of a control ligand binding molecule for the ligand means that the test ligand binding molecule has weaker binding activity against the ligand than that of the control ligand binding molecule. The phrase "ligand binding function is attenuated" means that the dissociation constant (KD) of the test ligand binding molecule for the ligand is, for example, at least 2 times, preferably at least 5 times or at least 10 times, particularly preferably at least 100 times the dissociation constant (KD) of the control ligand binding molecule for the ligand.

Examples of the control ligand binding molecule include an uncleaved form of the ligand binding molecule.

In one embodiment, the ligand is released from the ligand binding molecule by the cleavage of the cleavage site in the ligand-binding molecule. In this context, when the ligand is bound with a portion of the ligand binding molecule via a linker having no cleavage site, the ligand is released while connected with this portion of the ligand binding molecule via the linker. Even when the ligand is released together with a portion of the ligand binding molecule as mentioned above, it can be concluded that the ligand is released from the ligand binding molecule as long as the ligand is released from the most part of the ligand binding molecule.

A method for detecting the release of the ligand from the ligand binding molecule by the cleavage of the cleavage site includes a method of detecting the ligand using, for example, an antibody for ligand detection that recognizes the ligand. When the ligand binding molecule is an antibody fragment, the antibody for ligand detection preferably binds to the same epitope as that for the ligand binding molecule. The ligand detected using the antibody for ligand detection can be confirmed by a well-known method such as FACS, an ELISA format, AlphaScreen (amplified luminescent proximity homogeneous assay), a BIACORE method using surface plasmon resonance (SPR) phenomena, or BLI (bio-layer interferometry) (Octet) (Proc. Natl. Acad. Sci. USA (2006) 103 (11), 4005-4010).

In the case of detecting the release of the ligand using, for example, Octet, the antibody for ligand detection that recognizes the ligand is biotinylated and contacted with a biosensor. Then, binding to the ligand in a sample can be measured to detect the release of the ligand. Specifically, the amount of the ligand is measured in a sample containing the ligand binding molecule before protease treatment or after protease treatment and the ligand, using the antibody for ligand detection. The amount of the ligand detected in the sample can be compared between before and after protease treatment to detect the release of the ligand. Alternatively, the amount of the ligand is measured in a sample containing protease, the ligand binding molecule, and the ligand and a sample containing the ligand binding molecule and the ligand without containing protease, using the antibody for ligand detection. The amount of the ligand detected in the sample can be compared between the presence and absence of protease to detect the release of the ligand. More specifically, the release of the ligand can be detected by a method described in Examples of the present application. When the ligand binding molecule is fused with the ligand to form a fusion protein, the amount of the ligand is measured in a sample containing the fusion protein before protease treatment or after protease treatment, using the antibody for ligand detection. The amount of the ligand detected in the sample can be compared between before and after protease treatment to detect the release of the ligand. Alternatively, the amount of the ligand is measured in a sample containing protease and the fusion protein and a sample containing the fusion protein without containing protease, using the antibody for ligand detection. The amount of the ligand detected in the sample can be compared between the presence and absence of protease to detect the release of the ligand. More specifically, the release of the ligand can be detected by a method described in Examples of the present application.

In an embodiment in which the physiological activity of the ligand is inhibited upon binding to the ligand binding molecule, the release from the ligand binding molecule can be detected by a method of measuring the physiological activity of the ligand in a sample. Specifically, the physiological activity of the ligand can be measured in a sample containing the ligand binding molecule before protease treatment or after protease treatment and the ligand and compared between before and after protease treatment to detect the release of the ligand. Alternatively, the physiological activity of the ligand can be measured in a sample containing protease, the ligand binding molecule, and the ligand and a sample containing the ligand binding molecule and the ligand without containing protease and compared between these samples to detect the release of the ligand. When the ligand binding molecule is fused with the ligand to form a fusion protein, the physiological activity of the ligand can be measured in a sample containing the fusion protein before protease treatment or after protease treatment and compared between before and after protease treatment to detect the release of the ligand. Alternatively, the physiological activity of the ligand can be measured in a sample containing protease and the fusion protein and a sample containing the fusion protein without containing protease and compared between these samples to detect the release of the ligand.

In one embodiment, the cleavage site comprises a protease cleavage sequence and is cleaved by protease.

In some embodiments, a domain having ligand binding activity is separated from the ligand binding molecule by the cleavage of the cleavage site or the protease cleavage sequence in the ligand binding molecule so that the binding to the ligand is attenuated. In an embodiment using an IgG antibody as the ligand binding molecule, for example, the cleavage site or the protease cleavage sequence is established in an antibody variable region, and a complete antibody variable region cannot be formed in a cleaved state so that the binding to the ligand is attenuated.

In some embodiments, VH and VL contained in the ligand binding molecule associate with each other. The association between the antibody VH and the antibody VL may be canceled, for example, by the cleavage of the cleavage site or the protease cleavage sequence. The cancelation of the association can be used interchangeably with, for example, the whole or partial cancelation of the state where two or more polypeptide regions interact with each other. For the cancelation of the association between the VH and the VL, the interaction between the VH and the VL may be wholly canceled, or the interaction between the VH and the VL may be partially canceled.

The ligand binding molecule encompasses a ligand binding molecule in which the association between antibody VL or a portion thereof and antibody VH or a portion thereof is canceled by the cleavage of the cleavage site or canceled by the protease cleavage of the protease cleavage sequence.

In some embodiments, the ligand binding molecule comprises antibody VH and antibody VL, and the antibody VH and the antibody VL in the ligand binding molecule are associated with each other in a state where the cleavage site or the protease cleavage sequence of the ligand binding molecule is uncleaved, whereas the association between the antibody VH and the antibody VL in the ligand binding molecule is canceled by the cleavage of the cleavage site or the protease cleavage sequence. The cleavage site or the protease cleavage sequence in the ligand binding molecule may be placed at any position in the ligand binding molecule as long as the ligand binding of the ligand binding molecule can be attenuated by the cleavage of the cleavage site or the protease cleavage sequence.

In some embodiments, the ligand binding molecule comprises antibody VH, antibody VL, and an antibody constant region.

As mentioned by Rothlisberger et al. (J Mol Biol. 2005 Apr 8; 347 (4): 773-89), it is known that the VH and VL domains or the CH and CL domains of an antibody interact with each other via many amino acid side chains. VH-CH1 and VL-CL are known to be capable of forming a stable structure as a Fab domain. As previously reported, amino acid side chains generally interact between VH and VL with a dissociation constant in the range of 10⁻⁵ M to 10⁻⁸ M. When only VH and VL domains exist, only a small proportion may form an associated state.

In some embodiments, the ligand binding molecule is designed such that the cleavage site or the protease cleavage sequence is established in the ligand binding molecule comprising antibody VH and antibody VL, and the entire heavy chain-light chain interaction is present between two peptides in the Fab structure before cleavage, whereas the interaction between the peptide containing the VH (or a portion of the VH) and the peptide containing the VL (or a portion of the VL) is attenuated by the cleavage of the cleavage site or the protease cleavage sequence so that the association between the VH and the VL is canceled.

In one embodiment, the cleavage site or the protease cleavage sequence is located within the antibody constant region. In a more specific embodiment, the cleavage site or the protease cleavage sequence is located on the variable region side with respect to amino acid position 140 (EU numbering) in an antibody heavy chain constant region, preferably on the variable region side with respect to amino acid position 122 (EU numbering) in an antibody heavy chain constant region. In some specific embodiments, the cleavage site or the protease cleavage sequence is inserted at any position in a sequence from antibody heavy chain constant region amino acid position 118 (EU numbering) to antibody heavy chain constant region amino acid position 140 (EU numbering). In another more specific embodiment, the cleavage site or the protease cleavage sequence is located on the variable region side with respect to amino acid position 130 (EU numbering) (Kabat numbering position 130) in an antibody light chain constant region, preferably on the variable region side with respect to amino acid position 113 (EU numbering) (Kabat numbering position 113) in an antibody light chain constant region or on the variable region side with respect to amino acid position 112 (EU numbering) (Kabat numbering position 112) in an antibody light chain constant region. In some specific embodiments, the cleavage site or the protease cleavage sequence is inserted at any position in a sequence from antibody light chain constant region amino acid position 108 (EU numbering) (Kabat numbering position 108) to antibody light chain constant region amino acid position 131 (EU numbering) (Kabat numbering position 131).

In one embodiment, the cleavage site or the protease cleavage sequence is located within the antibody VH or within the antibody VL. In a more specific embodiment, the cleavage site or the protease cleavage sequence is located on the antibody constant region side with respect to amino acid position 7 (Kabat numbering) of the antibody VH, preferably on the antibody constant region side with respect to amino acid position 40 (Kabat numbering) of the antibody VH, more preferably on the antibody constant region side with respect to amino acid position 101 (Kabat numbering) of the antibody VH, further preferably on the antibody constant region side with respect to amino acid position 109 (Kabat numbering) of the antibody VH or on the antibody constant region side with respect to amino acid position 111 (Kabat numbering) of the antibody VH. In a more specific embodiment, the cleavage site or the protease cleavage sequence is located on the antibody constant region side with respect to amino acid position 7 (Kabat numbering) of the antibody VL, preferably on the antibody constant region side with respect to amino acid position 39 (Kabat numbering) of the antibody VL, more preferably on the antibody constant region side with respect to amino acid position 96 (Kabat numbering) of the antibody VL, further preferably on the antibody constant region side with respect to amino acid position 104 (Kabat numbering) of the antibody VL or on the antibody constant region side with respect to amino acid position 105 (Kabat numbering) of the antibody VL.

In some more specific embodiments, the cleavage site or the protease cleavage sequence is inserted at a position of residues constituting a loop structure in the antibody VH or the antibody VL, and residues close to the loop structure. The loop structure in the antibody VH or the antibody VL refers to a moiety that does not form a secondary structure such as α-helix or β-sheet, in the antibody VH or the antibody VL. Specifically, the position of the residues constituting the loop structure and the residues close to the loop structure can refer to the range of amino acid position 7 (Kabat numbering) to amino acid position 16 (Kabat numbering), amino acid position 40 (Kabat numbering) to amino acid position 47 (Kabat numbering), amino acid position 55 (Kabat numbering) to amino acid position 69 (Kabat numbering), amino acid position 73 (Kabat numbering) to amino acid position 79 (Kabat numbering), amino acid position 83 (Kabat numbering) to amino acid position 89 (Kabat numbering), amino acid position 95 (Kabat numbering) to amino acid position 99 (Kabat numbering), or amino acid position 101 (Kabat numbering) to amino acid position 113 (Kabat numbering) of the antibody VH, or amino acid position 7 (Kabat numbering) to amino acid position 19 (Kabat numbering), amino acid position 39 (Kabat numbering) to amino acid position 46 (Kabat numbering), amino acid position 49 (Kabat numbering) to amino acid position 62 (Kabat numbering), or amino acid position 96 (Kabat numbering) to amino acid position 107 (Kabat numbering) of the antibody VL.

In some more specific embodiments, the cleavage site or the protease cleavage sequence is inserted at any position in a sequence from amino acid position 7 (Kabat numbering) to amino acid position 16 (Kabat numbering), from amino acid position 40 (Kabat numbering) to amino acid position 47 (Kabat numbering), from amino acid position 55 (Kabat numbering) to amino acid position 69 (Kabat numbering), from amino acid position 73 (Kabat numbering) to amino acid position 79 (Kabat numbering), from amino acid position 83 (Kabat numbering) to amino acid position 89 (Kabat numbering), from amino acid position 95 (Kabat numbering) to amino acid position 99 (Kabat numbering), or from amino acid position 101 (Kabat numbering) to amino acid position 113 (Kabat numbering) of the antibody VH.

In some more specific embodiments, the cleavage site or the protease cleavage sequence is inserted at any position in a sequence from amino acid position 7 (Kabat numbering) to amino acid position 19 (Kabat numbering), from amino acid position 39 (Kabat numbering) to amino acid position 46 (Kabat numbering), from amino acid position 49 (Kabat numbering) to amino acid position 62 (Kabat numbering), or from amino acid position 96 (Kabat numbering) to amino acid position 107 (Kabat numbering) of the antibody VL.

In one embodiment, the cleavage site or the protease cleavage sequence is located near the boundary between the antibody VH and the antibody constant region. The phrase "near the boundary between the antibody VH and the antibody heavy chain constant region" can refer to between amino acid position 101 (Kabat numbering) of the antibody VH and amino acid position 140 (EU numbering) of the antibody heavy chain constant region and can preferably refer to between amino acid position 109 (Kabat numbering) of the antibody VH and amino acid position 122 (EU numbering) of the antibody heavy chain constant region, or between amino acid position 111 (Kabat numbering) of the antibody VH and amino acid position 122 (EU numbering) of the antibody heavy chain constant region. When antibody VH is fused with an antibody light chain constant region, the phrase "near the boundary between the antibody VH and the antibody light chain constant region" can refer to between amino acid position 101 (Kabat numbering) of the antibody VH and amino acid position 130 (EU numbering) (Kabat numbering position 130) of the antibody light chain constant region and can preferably refer to between amino acid position 109 (Kabat numbering) of the antibody VH and amino acid position 113 (EU numbering) (Kabat numbering position 113) of the antibody light chain constant region, or between amino acid position 111 (Kabat numbering) of the antibody VH and amino acid position 112 (EU numbering) (Kabat numbering position 112) of the antibody light chain constant region.

In one embodiment, the cleavage site or the protease cleavage sequence is located near the boundary between the antibody VL and the antibody constant region. The phrase "near the boundary between the antibody VL and the antibody light chain constant region" can refer to between amino acid position 96 (Kabat numbering) of the antibody VL and amino acid position 130 (EU numbering) (Kabat numbering position 130) of the antibody light chain constant region and can preferably refer to between amino acid position 104 (Kabat numbering) of the antibody VL and amino acid position 113 (EU numbering) (Kabat numbering position 113) of the antibody light chain constant region, or between amino acid position 105 (Kabat numbering) of the antibody VL and amino acid position 112 (EU numbering) (Kabat numbering position 112) of the antibody light chain constant region. When antibody VL is fused with an antibody heavy chain constant region, the phrase "near the boundary between the antibody VL and the antibody heavy chain constant region" can refer to between amino acid position 96 (Kabat numbering) of the antibody VL and amino acid position 140 (EU numbering) of the antibody heavy chain constant region and can preferably refer to between amino acid position 104 (Kabat numbering) of the antibody VL and amino acid position 122 (EU numbering) of the antibody heavy chain constant region, or between amino acid position 105 (Kabat numbering) of the antibody VL and amino acid position 122 (EU numbering) of the antibody heavy chain constant region.

The cleavage site or the protease cleavage sequence can be established at a plurality of positions in the ligand binding molecule and can be established at a plurality of positions selected from, for example, within the antibody constant region, within the antibody VH, within the antibody VL, near the boundary between the antibody VH and the antibody constant region, and near the boundary between antibody VL and the antibody constant region. Those skilled in the art with reference to the present application can change the form of a molecule comprising antibody VH, antibody VL, and an antibody constant region, for example, by swapping the antibody VH with the antibody VL. Such a molecular form is included in the scope of the present application.

The antigen-binding molecule of the present application encompasses, for example, the antigen-binding molecules described in WO2019/230868.
In one embodiment, the antigen-binding molecule of the present application relates to an antigen-binding molecule as a ligand binding molecule, wherein the ligand binding molecule comprises a single-domain antibody, wherein the single-domain antibody is capable of binding to a ligand, and at least one cleavage site is introduced into the single-domain antibody, and wherein the binding of the ligand binding molecule to the ligand in a state of being cleaved at the cleavage site is attenuated as compared to the binding of the ligand binding molecule to the ligand in a state of being uncleaved at the cleavage site.

In one embodiment, the term "single-domain antibody" is an antibody capable of exhibiting an antigen-binding activity by that domain alone, and is used interchangeably with "single-domain antibody". The structure of a single-domain antibody is not limited as long as it can exert an antigen-binding activity by that domain alone. Ordinary antibodies, exemplified by IgG antibodies and such, show antigen binding activity in a state where a variable region is formed by VH and VL pairing, whereas a single-domain antibody does not pair with other domains, and it is known that the domain structure of the single-domain antibody itself can exert antigen-binding activity. Single-domain antibodies usually have a relatively low molecular weight and exist in the form of monomers. In several embodiments, the form of the single-domain antibody is similar to those of an antibody heavy chain variable region or an antibody light chain variable region.

Examples of single-domain antibodies include, but are not limited to, the variable region of the heavy chain antibody from animals of the family Camelidae (VHH), an antigen-binding molecule congenitally lacking a light chain such as shark VNAR, or antibody fragments that include all or part of an antibody VH domain or all or part of an antibody VL domain. Examples of single-domain antibodies that are antibody fragments that include all or part of an antibody VH/VL domain include, but are not limited to, an artificially-produced single-domain antibody starting from the human antibody VH or human antibody VL (hereinafter, single-domain VH antibody, single-domain VL antibody) as described in US Patent No. 6,248,516 B1, and such.

The single-domain antibody can be obtained from an animal capable of producing the single-domain antibody or by the immunization of the animal capable of producing the single-domain antibody. Examples of the animal capable of producing the single-domain antibody include, but are not limited to, animals of the family Camelidae, and transgenic animals harboring a gene capable of raising the single-domain antibody. The animals of the family Camelidae include camels, lamas, alpacas, one-hump camels and guanacos, etc. Examples of the transgenic animals harboring a gene capable of raising the single-domain antibody include, but are not limited to, transgenic animals described in WO2015/143414 and U.S. Patent Publication No. US2011/0123527 A1. The framework sequences of the single-domain antibody obtained from the animal may be converted to human germline sequences or sequences similar thereto to obtain a humanized single-domain antibody. The humanized single-domain antibody (e.g., humanized VHH) is also one embodiment of the single-domain antibody.

The single-domain antibody can be obtained from an animal capable of producing the single-domain antibody or by the immunization of the animal capable of producing the single-domain antibody. Examples of the animal capable of producing the single-domain antibody include, but are not limited to, animals of the family Camelidae, and transgenic animals harboring a gene capable of raising the single-domain antibody. The animals of the family Camelidae include camels, lamas, alpacas, one-hump camels and guanacos, etc. Examples of the transgenic animals harboring a gene capable of raising the single-domain antibody include, but are not limited to, transgenic animals described in WO2015/143414 and U.S. Patent Publication No. US2011/0123527 A1. The framework sequences of the single-domain antibody obtained from the animal may be converted to human germline sequences or sequences similar thereto to obtain a humanized single-domain antibody. The humanized single-domain antibody (e.g., humanized VHH) is also one embodiment of the single-domain antibody.

Alternatively, the single-domain antibody can be obtained by ELISA, panning, or the like from a polypeptide library containing single-domain antibodies. Examples of the polypeptide library containing single-domain antibodies include, but are not limited to, naive antibody libraries obtained from various animals or humans (e.g., Methods in Molecular Biology 2012 911 (65-78); and Biochimica et Biophysica Acta - Proteins and Proteomics 2006 1764: 8 (1307-1319)), antibody libraries obtained by the immunization of various animals (e.g., Journal of Applied Microbiology 2014 117: 2 (528-536)), and synthetic antibody libraries prepared from antibody genes of various animals or humans (e.g., Journal of Biomolecular Screening 2016 21: 1 (35-43); Journal of Biological Chemistry 2016 291:24 (12641-12657); and AIDS 2016 30: 11 (1691-1701)).

In the present application, there is an embodiment in which a cleavage site/protease cleavage sequence is introduced into a single-domain antibody, but the expression "single-domain antibody" can be used regardless of whether or not a cleavage site/protease cleavage sequence is introduced.

In several embodiments, the single-domain antibody can generally be defined as a polypeptide comprising:
a) an amino acid sequence consisting of four framework regions/sequences having three complementarity determining regions/sequences inserted therebetween (the amino acid residue at position 11 according to Kabat numbering is selected from the group consisting of L, M, S, V, and W, and is preferably L), and/or
b) an amino acid sequence consisting of four framework regions/sequences having three complementarity determining regions/sequences inserted therebetween (the amino acid residue at position 37 according to Kabat numbering is selected from the group consisting of F, Y, H, I, L, and V, and is preferably F or Y), and/or
c) an amino acid sequence consisting of four framework regions/sequences having three complementarity determining regions/sequences inserted therebetween (the amino acid residue at position 44 according to Kabat numbering is selected from the group consisting of G, E, A, D, Q, R, S, and L, and is preferably G, E or Q, and is more preferably G or E), and/or
d) an amino acid sequence consisting of four framework regions/sequences having three complementarity determining regions/sequences inserted therebetween (the amino acid residue at position 45 according to Kabat numbering is selected from the group consisting of L, R, C, I, L, P, Q, and V, and is preferably L or R), and/or
e) an amino acid sequence consisting of four framework regions/sequences having three complementarity determining regions/sequences inserted therebetween (the amino acid residue at position 47 according to Kabat numbering is selected from the group consisting of W, L, F, A, G, I, M, R, S, V, and Y, and is preferably W, L, F or R), and/or
f) an amino acid sequence consisting of four framework regions/sequences having three complementarity determining regions/sequences inserted therebetween (the amino acid residue at position 83 according to Kabat numbering is selected from the group consisting of R, K, N, E, G, I, M, Q, and T, and is preferably K or R, and is more preferably K), and/or
g) an amino acid sequence consisting of four framework regions/sequences having three complementarity determining regions/sequences inserted therebetween (the amino acid residue at position 84 according to Kabat numbering is selected from the group consisting of P, A, L, R, S, T, D, and V, and is preferably P), and/or
h) an amino acid sequence consisting of four framework regions/sequences having three complementarity determining regions/sequences inserted therebetween (the amino acid residue at position 103 according to Kabat numbering is selected from the group consisting of W, P, R and S, and is preferably W), and/or
i) an amino acid sequence consisting of four framework regions/sequences having three complementarity determining regions/sequences inserted therebetween (the amino acid residue at position 104 according to Kabat numbering is G or D, and is preferably G), and/or
j) an amino acid sequence consisting of four framework regions/sequences having three complementarity determining regions/sequences inserted therebetween (the amino acid residue at position 108 according to Kabat numbering is selected from the group consisting of Q, L and R, and is preferably Q or L).

More specifically, but not exclusively, a single-domain antibody can be defined as a polypeptide comprising any one of the amino acid sequences consisting of four framework regions/sequences having the following three complementarity determining regions/sequences inserted therebetween:
k) an amino acid sequence in which the amino acid residues at positions 43 to 46 according to Kabat numbering are KERE (SEQ ID NO: 1838) or KQRE (SEQ ID NO: 1839);
l) an amino acid sequence in which the amino acid residues at positions 44 to 47 according to Kabat numbering are GLEW (SEQ ID NO: 1840); and,
m) an amino acid sequence in which the amino acid residues at positions 83 to 84 according to Kabat numbering are KP or EP.

In some embodiments, the cleavage site/protease cleavage sequence in the ligand-binding molecule is cleaved, thus fragmenting the single-domain antibody in the ligand-binding molecule, and the binding to the ligand is attenuated.

In one embodiment, the cleavage site/protease cleavage sequence is introduced into the single-domain antibody within the ligand-binding molecule. In some more specific embodiments, the cleavage site/protease cleavage sequence is introduced at the position of residues forming a loop structure, and at the position of residues close to a loop structure, within the single-domain antibody. The loop structure within a single-domain antibody refers to a part that does not form a secondary structure such as an α-helix and β-sheet within the single-domain antibody.

In an embodiment in which the single-domain antibody is VHH or a single-domain VH antibody, the position of residues forming a loop structure and residues close to a loop structure may specifically refer to the range of: amino acid 7 (Kabat numbering) to amino acid 17 (Kabat numbering); amino acid 12 (Kabat numbering) to amino acid 17 (Kabat numbering); amino acid 31 (Kabat numbering) to amino acid 35b (Kabat numbering); amino acid 40 (Kabat numbering) to amino acid 47 (Kabat numbering); amino acid 50 (Kabat numbering) to amino acid 65 (Kabat numbering); amino acid 55 (Kabat numbering) to amino acid 69 (Kabat numbering); amino acid 73 (Kabat numbering) to amino acid 79 (Kabat numbering); amino acid 83 (Kabat numbering) to amino acid 89 (Kabat numbering); amino acid 95 (Kabat numbering) to amino acid 99 (Kabat numbering); amino acid 95 (Kabat numbering) to amino acid 102 (Kabat numbering); and amino acid 101 (Kabat numbering) to amino acid 113 (Kabat numbering) of the single-domain antibody.

In an embodiment where the single-domain antibody is VHH or a single-domain VH antibody, the cleavage site/protease cleavage sequence is introduced into one or more positions contained in one or more sequences selected from the following sequences of the single-domain antibody:
the sequence from amino acid 7 (Kabat numbering) to amino acid 17 (Kabat numbering) of the single-domain antibody; the sequence from amino acid 12 (Kabat numbering) to amino acid 17 (Kabat numbering) of the single-domain antibody; the sequence from amino acid 31 (Kabat numbering) to amino acid 35b (Kabat numbering) of the single-domain antibody; the sequence from amino acid 40 (Kabat numbering) to amino acid 47 (Kabat numbering) of the single-domain antibody; the sequence from amino acid 50 (Kabat numbering) to amino acid 65 (Kabat numbering) of the single-domain antibody; the sequence from amino acid 55 (Kabat numbering) to amino acid 69 (Kabat numbering) of the single-domain antibody; the sequence from amino acid 73 (Kabat numbering) to amino acid 79 (Kabat numbering) of the single-domain antibody; the sequence from amino acid 83 (Kabat numbering) to amino acid 89 (Kabat numbering) of the single-domain antibody; the sequence from amino acid 95 (Kabat numbering) to amino acid 99 (Kabat numbering) of the single-domain antibody; the sequence from amino acid 95 (Kabat numbering) to amino acid 102 (Kabat numbering) of the single-domain antibody; and the sequence from amino acid 101 (Kabat numbering) to amino acid 113 (Kabat numbering) of the single-domain antibody.

In an embodiment where the single-domain antibody is a single-domain VL antibody, the position of residues forming a loop structure and residues close to a loop structure may specifically refer to the range of: amino acid 7 (Kabat numbering) to amino acid 19 (Kabat numbering); amino acid 24 (Kabat numbering) to amino acid 34 (Kabat numbering); amino acid 39 (Kabat numbering) to amino acid 46 (Kabat numbering); amino acid 49 (Kabat numbering) to amino acid 62 (Kabat numbering); amino acid 50 (Kabat numbering) to amino acid 56 (Kabat numbering); amino acid 89 (Kabat numbering) to amino acid 97 (Kabat numbering); and amino acid 96 (Kabat numbering) to amino acid 107 (Kabat numbering) of the single-domain antibody.

In an embodiment where the single-domain antibody is a single-domain VL antibody, the cleavage site/protease cleavage sequence is introduced into one or more positions contained in one or more sequences selected from the following sequences of the single-domain antibody:
the sequence from amino acid 7 (Kabat numbering) to amino acid 19 (Kabat numbering) of the single-domain antibody; the sequence from amino acid 24 (Kabat numbering) to amino acid 34 (Kabat numbering) of the single-domain antibody; the sequence from amino acid 39 (Kabat numbering) to amino acid 46 (Kabat numbering) of the single-domain antibody; the sequence from amino acid 49 (Kabat numbering) to amino acid 62 (Kabat numbering) of the single-domain antibody; the sequence from amino acid 50 (Kabat numbering) to amino acid 56 (Kabat numbering) of the single-domain antibody; the sequence from amino acid 89 (Kabat numbering) to amino acid 97 (Kabat numbering) of the single-domain antibody; and the sequence from amino acid 96 (Kabat numbering) to amino acid 107 (Kabat numbering) of the single-domain antibody.

Multiple cleavage sites/protease cleavage sequences can be provided within a ligand-binding molecule, for example, in multiple positions within the single-domain antibody in the ligand-binding molecule.

The antigen-binding molecule of the present application encompasses, for example, the antigen-binding molecules described in WO2022/034920. In one aspect, the antigen-binding molecule of the present application relates to a "one-armed (or one-arm) antigen-binding molecule". In one aspect, the "one-armed antigen-binding molecule" refers to an antigen-binding molecule that specifically binds to an antigen through an antigen-binding site, an antigen-binding moiety, or any molecules having binding activity to the antigen fused to a first Fc region variant comprised in an Fc polypeptide of the "one-armed antigen-binding molecule", the Fc polypeptide comprising the first Fc region variant and second Fc region variant. In a certain aspect, the second Fc region variant comprised in the Fc polypeptide of the "one-armed antigen-binding molecule" is not fused to any other antigen-binding sites, antigen-binding moieties etc., which can bind to the same antigen to which the first antigen binding site, the first antigen binding moiety, etc. binds and/or to any other antigen-binding sites, antigen-binding moieties etc., which can bind to different antigen (or epitope) from the one to which the first antigen binding site, the first antigen binding moiety, etc. binds.

In contrast, a "two-armed (or two-arm) antigen-binding molecule" refers to an antigen-binding molecule that specifically binds to an antigen through two antigen-binding sites, two antigen-binding moieties, or any two molecules having binding activity to the antigen. One and the other of the two antigen-binding sites, two antigen-binding moieties, etc. are fused to a first Fc region and a second Fc region, respectively, comprised in an Fc polypeptide of the "two-armed antigen-binding molecule", unless otherwise indicated. In one non-limiting embodiment, IgG-type conventional bivalent antibodies can be examples of the "two-armed antigen-binding molecules".

The antigen-binding molecule of the present application encompasses, for example, the antigen-binding molecules described in WO2018/155611 and combinations thereof. In one embodiment, the antigen-binding molecule of the present application is an antibody-half molecule. Herein, "antibody-half molecule" means a single molecule when the binding between H chains in an antibody is dissociated, and is sometimes generally called a monovalent antibody. Examples of an antibody-half molecule in the case where the antibody is IgG include a complex composed of one H chain and one L chain. Antibody-half molecules include molecules consisting of one H chain which are produced by dissociating the inter-H chain bonds of so-called heavy chain antibodies (also called VHHs (VH originating from heavy-chain antibody)), which are antibodies consisting of two H chains found in camelid antibodies and such.

In one embodiment, the antibody-half molecules include those derived from chimeric antibodies or humanized antibodies.

In one embodiment, the antibody-half molecules include those derived from various isotypes such as IgG, IgM, IgA, IgD, and IgE. The antibody-half molecules are preferably those derived from IgG. There are IgG1, IgG2, IgG3, and IgG4 in IgG. The antibody-half molecules may be derived from any of these subtypes. The antibody-half molecules are preferably derived from IgG1 or IgG2 from the viewpoint of easily exerting effector function.

The antigen-binding molecule of the present application encompasses, for example, the antigen-binding molecules described in WO2019/235426. In one embodiment, the antigen-binding molecule of the present application relates to a cytosol-penetrating antigen-binding molecule, specifically, an antigen-binding molecule comprising a tripartite motif 21 (TRIM21)-binding domain or an antigen-binding molecule comprising an altered TRIM21-binding domain.

The term "cytosol-penetrating domain" refers to a domain that can penetrate into the cytosol of a living cell. The mode for penetration into the cytosol is not particularly limited, and may be through incorporation via the endocytosis process followed by endosome escape, or through other processes. Examples other than endosome escape include penetration of the antigen-binding molecule directly through the cell membrane. One single antigen-binding molecule may penetrate into the cytosol both by endosome escape and by direct penetration through the cell membrane. The cytosol-penetrating domain may be of any type as long as it has a cytosol-penetrating ability, but in one preferred embodiment, the cytosol-penetrating domain is an antibody variable region (heavy chain variable region and/or light chain variable region), an antibody constant region, a peptide moiety, or a nucleic acid moiety. When the cytosol-penetrating domain is an antibody variable region or constant region, the cytosol-penetrating domain may be part or all of the variable region or constant region. Examples of the peptide moiety as a cytosol-penetrating domain include cell-penetrating peptides (CPP), protein transduction domains (PTD), etc. (see, for example, WO 2017156630). Examples of the nucleic acid moiety as a cytosol-penetrating domain include oligo-nucleic acids having a phosphorothioate backbone (see, for example, WO 2015031837). In one embodiment, a cytosol-penetrating antigen-binding molecule is a fusion or a complex of an antigen-binding molecule having no cytosol-penetrating ability with a cytosol-penetrating domain.

### A. Antigen-binding molecules comprising an altered TRIM21-binding domain

In one aspect, the present disclosure relates to an antigen-binding molecule comprising an altered TRIM21-binding domain wherein the antigen-binding molecule has an increased or decreased cytosolic half-life compared to a parent antigen-binding molecule. In one embodiment, the altered TRIM21-binding domain comprises one or more amino acid alterations that result in a decrease or an increase in binding affinity for TRIM21 compared to a wildtype TRIM21-binding domain. In one embodiment, the altered TRIM21-binding domain comprises an amino acid substitution in the TRIM21-binding domain at one or more positions selected from the group consisting of EU253, EU309, EU311, EU312, EU314, EU315, EU345, EU428, EU432, EU433, EU434, EU435, EU436, EU437, EU438, EU439, and EU440. Antigen-binding molecules of the present disclosure may contain a substitution at additional positions.

The altered TRIM21-binding domain of the present disclosure may contain substitutions at two or more positions In the present disclosure, this is referred to as a "combination" of substitutions. For example, a TRIM21-binding domain specified by the combination "EU424/EU434/EU436" is a TRIM21-binding domain comprising substitutions at positions EU424, EU434, and EU436.

The term "TRIM21-binding domain" used herein means a protein domain that binds directly or indirectly to TRIM21. In one embodiment, TRIM21 is mammalian TRIM21, and preferably human TRIM21. In a more preferred embodiment, a TRIM21-binding domain binds to both human TRIM21 and mouse TRIM21. An example of a TRIM21-binding domain that directly binds to TRIM21 is an antibody Fc region. On the other hand, a region that may bind to a polypeptide having human TRIM21-binding activity such as IgG can bind indirectly to human TRIM21 through IgG or such. Therefore, such a human TRIM21-binding domain may be a region that binds to a polypeptide having human TRIM21-binding activity.

A TRIM21-binding domain that directly binds to TRIM21 in one embodiment, mammalian TRIM21 in a preferred embodiment, or a human TRIM21 in a more preferred embodiment, is an Fc region or an Fc region of an antigen-binding molecule. Specifically, the TRIM21-binding domain is an antibody Fc region. In a preferred embodiment, the TRIM21-binding domain is a mammalian Fc region, and in a more preferred embodiment, it is a human Fc region. Specifically, the TRIM21-binding domain of the present disclosure is an Fc region that contains the second and third constant domains (CH2 and CH3), and in a more preferred embodiment, contains the hinge, CH2, and CH3, of a human immunoglobulin. In a preferred embodiment, the immunoglobulin is IgG. In a preferred embodiment, the TRIM21-binding domain is a human IgG1 Fc region.

"An increase in binding affinity for TRIM21" refers to an increase compared to the binding affinity of a parent antigen-binding molecule for TRIM21. "A decrease in binding affinity for TRIM21" refers to a decrease compared to the binding affinity of a parent antigen-binding molecule for TRIM21.

The term "parent antigen-binding molecule" as used herein refers to an antigen-binding molecule which does not contain the amino acid alterations of the present disclosure, that is, one or more amino acid alterations that result in a decrease or an increase in binding affinity for TRIM21, but is identical in the rest of the molecule to an antigen-binding molecule containing an altered TRIM21-binding domain. A parent antigen-binding molecule may be an antigen-binding molecule comprising a wildtype Fc region, or an antigen-binding molecule comprising an altered Fc region. In a preferred embodiment, the antigen-binding molecule is an antibody or an antibody derivative. The term "wildtype" refers to a naturally-occurring sequence.

When binding affinity is represented by dissociation constant (KD), "an increase in binding affinity for TRIM21" may be shown by the fact that the KD value of an antigen-binding molecule comprising an altered TRIM21-binding domain for binding to TRIM21 is smaller than the KD value of a parent antigen-binding molecule for binding to TRIM21. Furthermore, "a decrease in binding affinity for TRIM21" may be shown by the fact that the KD value of an antigen-binding molecule comprising an altered TRIM21-binding domain for binding to TRIM21 is larger than the KD value of a parent antigen-binding molecule for binding to TRIM21.

In measuring binding affinity for TRIM21, the measurement conditions can be appropriately selected by those skilled in the art, and are not particularly limited. For example, as described in the Examples of the present disclosure, measurement can be carried out in HBS-P buffer at 25°C, pH7.4. Furthermore, the binding affinity (KD) of an antigen-binding molecule or a TRIM21-binding domain for TRIM21 can be measured by a method known to those skilled in the art, for example, by using a BIACORE^{®} surface plasmon resonance assay. The binding affinity of an antigen-binding molecule (or TRIM21-binding domain) for TRIM21 can be assayed, for example, by immobilizing the antigen-binding molecule (or TRIM21-binding domain) onto a chip and allowing TRIM21 to flow over the chip as an analyte.

TRIM21 binds to the Fc region of an antibody incorporated into the cytosol, and activates the innate immune response and inflammation via NFκB and IFN signals. It has been reported that the NF-κB signaling activated by an anti-human adenovirus type 5 antibody containing an Fc with decreased binding affinity for TRIM21 is decreased compared to the NF-κB signaling activated by an anti-human adenovirus type 5 antibody containing a wildtype Fc (Nat Immunol. 2013 Apr;14(4):327-36). Therefore, in one embodiment, an antigen-binding molecule comprising an altered TRIM21-binding domain which comprises an amino acid alteration that results in a decrease in binding affinity for TRIM21, has a decreased ability to activate NF-κB signaling compared to the parent antigen-binding molecule. When an antigen-binding molecule comprising an altered TRIM21-binding domain provided by the present disclosure is used in a therapeutic composition, the antigen-binding molecule with a decreased ability to activate NFκB signaling can be expected to cause no or reduced inflammatory responses as a side effect.

### B. Antigen-binding molecules comprising an altered TRIM21-binding domain and having an increased cytosolic half-life

In one aspect, the present disclosure relates to an antigen-binding molecule comprising an altered TRIM21-binding domain, which has an increased cytosolic half-life compared to a parent antigen-binding molecule. When the antigen-binding molecule comprising an altered TRIM21-binding domain comprises a cytosolic antigen-binding domain, the antigen-binding molecule can be expected to have a decreased ability to remove cytosolic antigens compared to the parent antigen-binding molecule. Alternatively, when the antigen-binding molecule comprising an altered TRIM21-binding domain is an agonistic antigen-binding molecule or an antagonistic antigen-binding molecule against a cytosolic antigen, the antigen-binding molecule can be expected to exhibit prolonged or enhanced agonistic activity or antagonistic activity compared to the parent antigen-binding molecule. In one embodiment, the altered TRIM21-binding domain comprises one or more amino acid alterations that result in an increase or a decrease in binding affinity for TRIM21 compared to the wildtype TRIM21-binding domain. In a preferred embodiment, the altered TRIM21-binding domain comprises one or more amino acid alterations that result in a decrease in binding affinity for human TRIM21 or mouse TRIM21 compared to the wildtype TRIM21-binding domain. In a more preferred embodiment, the altered TRIM21-binding domain comprises one or more amino acid alterations that result in a decrease in binding affinity for human TRIM21 and mouse TRIM21 compared to the wildtype TRIM21-binding domain.

An amino acid after substitution (an amino acid to substitute for an amino acid in a parent TRIM21-binding domain) may be any amino acid including, but not limited to, the group consisting of alanine (Ala, A), arginine (Arg, R), asparagine (Asn, N), aspartic acid (Asp, D), cysteine (Cys, C), glutamic acid (Glu, E), glutamine (Gln, Q), glycine (Gly, G), histidine (His, H), isoleucine (Ile, I), leucine (Leu, L), lysine (Lys, K), methionine (Met, M), phenylalanine (Phe, F), proline (Pro, P), serine (Ser, S), threonine (thr, T), tryptophan (trp, W), tyrosine (tyr, Y), and valine (Val, V), unless specifically mentioned herein.

In one embodiment, an antigen-binding molecule comprising an altered TRIM21-binding domain and having an increased cytosolic half-life contains an amino acid substitution in the TRIM21-binding domain at one or more positions selected from the group consisting of EU253, EU309, EU311, EU312, EU314, EU315, EU345, EU428, EU432, EU433, EU434, EU435, EU436, EU437, EU438, EU439, and EU440. In a preferred embodiment, an antigen-binding molecule of the present disclosure contains an amino acid substitution in the TRIM21-binding domain at one or more positions selected from the group consisting of EU253, EU314, EU315, EU428, EU432, EU433, EU434, EU435, EU436, and EU437. The amino acid after substitution may be any amino acid unless specifically mentioned herein.

In one embodiment, an antigen-binding molecule comprising an altered TRIM21-binding domain and having an increased cytosolic half-life contains one or more amino acid alterations that result in a decrease in binding affinity for TRIM21 compared to the wildtype TRIM21-binding domain.

The antigen-binding molecule of the present application encompasses, for example, the antigen-binding molecules described in WO2020/022262. In one embodiment, the antigen-binding molecule of the present application relates to a cytosol-penetrating antigen-binding molecule, that is, an antigen-binding molecule comprising a cell surface antigen-binding domain, a cytosol antigen-binding domain, and a cytosol-penetrating domain.

The term "cytosol-penetrating antigen-binding molecule" refers to an antigen-binding molecule that can penetrate into the cytosol of a living cell. The mode for the penetration into the cytosol is not particularly limited, and the antigen-binding molecule may be uptaken via the endocytosis process and then go through the endosome escape, or may undergo other processes. Examples other than the endosome escape include the case where an antigen-binding molecule directly penetrates through the cell membrane. The same antigen-binding molecule may penetrate into cytosol by both modes, i.e. the endosome escape and the direct penetration of the cell membrane. Antigen-binding molecules capable of penetrating into cytosol via methods other than endosome escape are reported (e.g., WO 2013102659). In one embodiment, the cytosol-penetrating antigen-binding molecules are antibodies or antibody derivatives. In another embodiment, the cytosol-penetrating antigen-binding molecules comprise a cytosol-penetrating domain.

The terms "cytosol-penetrating domain" and "region having cytosol-penetrating ability" are used interchangeably to refer to a domain that allows for penetration into the cytosol of a living cell. The mode for the penetration into the cytosol is not particularly limited, and may be through the uptake via the endocytosis process and following endosome escape, or may through other processes. The mode for the escape from endosomes is not particularly limited. For example, transfer into cytosol is achieved by the cytosol-penetrating domain in an endosome (acidic pH) interacting with the endosome membrane to create a pore on the endosome membrane. Examples other than the endosome escape include the case where an antigen-binding molecule directly penetrates through the cell membrane. The same antigen-binding molecule may penetrate into cytosol by both modes, i.e. the endosome escape and the direct penetration of the cell membrane. In one embodiment, the cytosol-penetrating domain has one or both of the endosome-escaping ability and cell membrane-permeating ability. In one embodiment, the cytosol-penetrating domain does not have an ability to be uptaken by endocytosis. In another embodiment, the cytosol-penetrating domain has an ability to be uptaken by endocytosis. The cytosol-penetrating domain may be of any type as long as it has cytosol-penetrating ability, but in one preferred embodiment, the cytosol-penetrating domain is an antibody or a fragment thereof, a peptide moiety, or a nucleic acid moiety. In the case that the cytosol-penetrating domain is an antibody or a fragment thereof, the cytosol-penetrating domain includes the whole or a portion of a combination of an antibody light chain variable region (VL) and an antibody heavy chain variable region (VH); or a single-domain antibody variable region, such as a heavy-chain antibody variable region (Variable region of Heavy chain of Heavy chain antibody: VHH), an antibody heavy chain variable region (VH), an antibody light chain variable region (VL), and a variable region of an immunoglobulin new antigen receptor (IgNAR) (VNAR). Examples of an antibody or a fragment thereof having cytosol-penetrating ability include, for example, Cytotransmab comprising a light chain variable region which exhibits cytosol-penetrating ability (Nat Commun. 2017 May 10;8:15090). Other examples of cytosol-penetrating domains are "scFv (single chain Fv)", "single chain antibody", "Fv", "scFv2 (single chain Fv 2)", "Fab" or "F(ab')2", etc. Examples of peptide moieties as cytosol-penetrating domain include cell-penetrating peptides (CPP), protein transduction domains (PTD), etc. (see, for example, WO 2017156630). Examples of nucleic acid moieties as cytosol-penetrating domain include oligo-nucleic acids having a phosophorothioate backbone (see, for example, WO 2015031837). In one embodiment, the cytosol-penetrating antigen-binding molecule is a fusion or a complex of an antigen-binding molecule having no cytosol-penetrating ability and a cytosol-penetrating domain.

Whether a domain of interest is a cytosol-penetrating domain or not (whether the domain of interest has cytosol-penetrating ability or not) can be checked by various methods known to those skilled in the art. For example, assessment can be made by fusing the domain of interest with a molecule having no cytosol-penetrating ability (e.g., an antigen-binding molecule), contacting the fused molecule with a cell, and then seeing if the fused molecule is detected in the cytosol of the cell. Various methods are known for detecting the presence of a molecule of interest in cytosol, and, for example, BirA assay and imaging analysis using fluorescence microscopy described in the Examples below are known.

In one embodiment, the cytosol-penetrating domain may interact with a cell surface antigen and/or a cytosolic antigen.

"Cytosol-trafficking" ability of an antigen-binding molecule means the ability of the antigen-binding molecule to traffic to the cytosol, and the term "penetrate", "traffic" or "traffick" are used interchangeably.

"Fuse/fusing/fusion" means that components (e.g., a Fab region and an Fc domain subunit) are linked together directly or by peptide bond via one or more peptide linkers.

The "cell surface antigen-binding domain" in the antigen-binding molecule of the present disclosure can bind to an epitope that is present in an antigen expressed on the surface of a cell. The "cell surface antigen" represents an antigen structure that is expressed by a cell and is present on the surface of the cell such that an antigen-binding domain can access it. In one embodiment, the cytosol-penetrating antigen-binding molecules of the present disclosure comprise a cell surface antigen-binding domain, and therefore, are specifically incorporated into cells that express the cell surface antigen (target cells). This enables specific delivery of the cytosol-penetrating antigen-binding molecules to target cells. In one embodiment, the cell surface antigen-binding domain can be provided with one or more antibody variable domains.

The "cytosolic antigen-binding domain" in the antigen-binding molecules of the present disclosure can bind to an epitope that is present in an antigen expressed in the cytosol. The "cytosolic antigen" represents an antigen structure that is expressed by a cell and is present in the cytosol. Cytosolic antigen may be a protein, a nucleic acid such as DNA or RNA, or a molecule expressed in organelle such as cell nucleus and mitochondria. The cytosolic antigen-binding molecule of the present disclosure comprises a cytosolic antigen-binding domain, and therefore, can bind to the cytosolic antigen in the cytosol of a target cell and thus can neutralize, inhibit, or activate, etc. the function of the antigen. In one embodiment, the cytosolic antigen-binding domain can be provided with one or more antibody variable domains. In one further embodiment, the cytosolic antigen-binding domain may be enzymes or shRNAs, etc.

The antigen-binding molecule of the present application encompasses, for example, molecules which have one additional Fab attached to the N-terminus of one of the Fabs in an IgG antibody (which normally has two Fabs), or molecules which lack one of the Fabs in an IgG antibody and have one additional Fab attached to the N-terminus of the other Fab.

The antigen-binding molecule of the present application also encompasses, for example, not only Fabs in which VH is linked with CH1 and VL is linked with CL, but also Fabs in which VH is linked with CL and VL is linked with CH1. The latter Fabs are termed "cross Mabs".

The antigen-binding molecule of the present application encompasses, for example, molecules comprising VH, VL, CH1, and CL that are all present within one polypeptide. Such molecules are termed "single chain Fabs (scFabs)".

The antigen-binding molecule of the present application encompasses, for example, molecules in which XTEN is fused to an antibody. XTEN is an unstructured, biodegradable protein polymer. Therapeutic peptides and proteins can be fused or chemically bound to XTEN to prolong their half-life. XTEN polymers are considered as nonimmunogenic polypeptides, and composed of six hydrophilic, chemically stable amino acids: A, E, G, P, S, and T. XTEN has a wide variety of sequence variations, and exemplarily includes XTEN864, which is described in Podust et al. (Journal of Controlled Release, Volume 240, 28 October 2016, Pages 52-66)

The antigen-binding molecule of the present application encompasses, for example, molecules in which a peptide is fused to an antibody (provided that the peptide is not a partial domain of an antibody).

The antigen-binding molecule of the present application encompasses, for example, molecules in which a non-antibody protein is fused to an antibody.

The antigen-binding molecule of the present application encompasses, for example, molecules in which a partial domain of an antibody is fused to an antibody.

The term "variant Fc region" comprises an amino acid sequence which differs from that of a native sequence Fc region by virtue of at least one amino acid modification, preferably one or more amino acid substitution(s). Preferably, the variant Fc region has at least one amino acid substitution compared to a native sequence Fc region or to the Fc region of a parent polypeptide, e.g. from about one to about ten amino acid substitutions, and preferably from about one to about five amino acid substitutions in a native sequence Fc region or in the Fc region of the parent polypeptide. The variant Fc region herein will preferably possess at least about 80% homology with a native sequence Fc region and/or with an Fc region of a parent polypeptide, and most preferably at least about 90% homology therewith, more preferably at least about 95% homology therewith.

The location of the binding target of an antibody of the present application may be taken into consideration in preparation and administration of the antibody. When the binding target is an intracellular molecule, certain embodiments provide for the antibody or antigen-binding fragment thereof to be introduced into the cell where the binding target is located. In one embodiment, an antibody of the present application can be expressed intracellularly as an intrabody. The term "intrabody," as used herein, refers to an antibody or antigen-binding portion thereof that is expressed intracellularly and that is capable of selectively binding to a target molecule, as described, e.g., in Marasco, Gene Therapy 4: 11-15 (1997); Kontermann, Methods 34: 163-170 (2004); U.S. Patent Nos. 6,004,940 and 6,329,173; U.S. Patent Application Publication No. 2003/0104402, and PCT Publication No. WO2003/077945. See also, for example, WO96/007321 published March 14, 1996, concerning the use of gene therapy to generate intracellular antibodies.

Intracellular expression of an intrabody may be effected by introducing a nucleic acid encoding the desired antibody or antigen-binding portion thereof (lacking the wild-type leader sequence and secretory signals normally associated with the gene encoding that antibody or antigen-binding fragment) into a target cell. One or more nucleic acids encoding all or a portion of an antibody of the present application can be delivered to a target cell, such that one or more intrabodies are expressed which are capable of binding to an intracellular target polypeptide and modulating the activity of the target polypeptide. Any standard method of introducing nucleic acids into a cell may be used, including, but not limited to, microinjection, ballistic injection, electroporation, calcium phosphate precipitation, liposomes, and transfection with retroviral, adenoviral, adeno-associated viral and vaccinia vectors carrying the nucleic acid of interest.

In certain embodiments, nucleic acid (optionally contained in a vector) may be introduced into a patient's cells by *in vivo* and *ex vivo* methods. In one example of in vivo delivery, nucleic acid is injected directly into the patient, e.g., at the site where therapeutic intervention is required. In a further example of in vivo delivery, nucleic acid is introduced into a cell using transfection with viral vectors (such as adenovirus, Herpes simplex I virus, or adeno-associated virus) and lipid-based systems (useful lipids for lipid-mediated transfer of the gene are DOTMA, DOPE and DC-Chol, for example). For review of certain gene marking and gene therapy protocols, see Anderson et al., Science 256:808-813 (1992), and WO 93/25673 and the references cited therein. In an example of *ex vivo* treatment, a patient's cells are removed, nucleic acid is introduced into those isolated cells, and the modified cells are administered to the patient either directly or, for example, encapsulated within porous membranes which are implanted into the patient (see, e.g., U.S. Patent Nos. 4,892,538 and 5,283,187). A commonly used vector for *ex vivo* delivery of a nucleic acid is a retroviral vector.

In another embodiment, internalizing antibodies are provided. Antibodies can possess certain characteristics that enhance delivery of antibodies into cells, or can be modified to possess such characteristics. Techniques for achieving this are known in the art. For example, cationization of an antibody is known to facilitate its uptake into cells (see, e.g., U.S. Patent No. 6,703,019). Lipofections or liposomes can also be used to deliver the antibody into cells. Where antibody fragments are used, the smallest inhibitory fragment that specifically binds to the target protein may be used. For example, based upon the variable region sequences of an antibody, peptide molecules can be designed that retain the ability to bind the target protein sequence. Such peptides can be synthesized chemically and/or produced by recombinant DNA technology. See, e.g., Marasco et al., Proc. Natl. Acad. Sci. USA 90: 7889-7893 (1993).

Entry of antibodies into target cells can be enhanced by other methods known in the art. For example, certain sequences, such as those derived from HIV Tat or the Antennapedia homeodomain protein are able to direct efficient uptake of heterologous proteins across cell membranes. See, e.g., Chen et al., Proc. Natl. Acad. Sci. USA 96:4325-4329 (1999).

When the binding target of an antibody is located in the brain, certain embodiments provide for the antibody to traverse the blood-brain barrier. Several art-known approaches exist for transporting molecules across the blood-brain barrier, including, but not limited to, physical methods, lipid-based methods, stem cell-based methods, and receptor and channel-based methods.

Physical methods of transporting an antibody across the blood-brain barrier include, but are not limited to, circumventing the blood-brain barrier entirely, or by creating openings in the blood-brain barrier. Circumvention methods include, but are not limited to, direct injection into the brain (see, e.g., Papanastassiou et al., Gene Therapy 9: 398-406 (2002)), interstitial infusion/convection-enhanced delivery (see, e.g., Bobo et al., Proc. Natl. Acad. Sci. USA 91: 2076-2080 (1994)), and implanting a delivery device in the brain (see, e.g., Gill et al., Nature Med. 9: 589-595 (2003); and Gliadel WafersTM, Guildford Pharmaceutical). Methods of creating openings in the barrier include, but are not limited to, ultrasound (see, e.g., U.S. Patent Publication No. 2002/0038086), osmotic pressure (e.g., by administration of hypertonic mannitol (Neuwelt, E. A., Implication of the Blood-Brain Barrier and its Manipulation, Vols 1 & 2, Plenum Press, N.Y. (1989)), permeabilization by, e.g., bradykinin or permeabilizer A-7 (see, e.g., U.S. Patent Nos. 5,112,596, 5,268,164, 5,506,206, and 5,686,416), and transfection of neurons that straddle the blood-brain barrier with vectors containing genes encoding the antibody (see, e.g., U.S. Patent Publication No. 2003/0083299).

Lipid-based methods of transporting an antibody across the blood-brain barrier include, but are not limited to, encapsulating the antibody in liposomes that are coupled to antibody binding fragments that bind to receptors on the vascular endothelium of the blood-brain barrier (see, e.g., U.S. Patent Application Publication No. 2002/0025313), and coating the antibody in low-density lipoprotein particles (see, e.g., U.S. Patent Application Publication No. 2004/0204354) or apolipoprotein E (see, e.g., U.S. Patent Application Publication No. 2004/0131692).

Stem-cell based methods of transporting an antibody across the blood-brain barrier entail genetically engineering neural progenitor cells (NPCs) to express the antibody of interest and then implanting the stem cells into the brain of the individual to be treated. See Behrstock et al. Gene Ther. 15 Dec. 2005 advanced online publication (reporting that NPCs genetically engineered to express the neurotrophic factor GDNF reduced symptoms of Parkinson disease when implanted into the brains of rodent and primate models).

Receptor and channel-based methods of transporting an antibody across the blood-brain barrier include, but are not limited to, using glucocorticoid blockers to increase permeability of the blood-brain barrier (see, e.g., U.S. Patent Application Publication Nos. 2002/0065259, 2003/0162695, and 2005/0124533); activating potassium channels (see, e.g., U.S. Patent Application Publication No. 2005/0089473), inhibiting ABC drug transporters (see, e.g., U.S. Patent Application Publication No. 2003/0073713); coating antibodies with a transferrin and modulating activity of the one or more transferrin receptors (see, e.g., U.S. Patent Application Publication No. 2003/0129186), and cationizing the antibodies (see, e.g., U.S. Patent No. 5,004,697).

### II.Methods for designing, selecting, or producing modified antigen-binding molecules, antigen-binding molecules designed, selected, or produced by the methods, or peptides designed by the methods

The present application provides a method for designing, selecting, or producing a modified antigen-binding molecule comprising an artificially modified peptide portion detectable by mass spectrometry. The method of the present application comprises generating a modified peptide portion by introducing an artificial modification to a peptide portion in an antigen-binding molecule, which is excised by treatment with a digestive enzyme (unmodified peptide portion), producing a modified antigen-binding molecule comprising the modified peptide portion instead of the peptide portion of the antigen-binding molecule before modification, and then selecting the modified peptide portion detectable by mass spectrometry and the antigen-binding molecule comprising the modified peptide portion, according to the properties of the modified antigen-binding molecule.

The peptide portion in an antigen-binding molecule, which is excised by treatment with a digestive enzyme (unmodified peptide portion), can have any sequence as long as it is detectable by mass spectrometry. The peptide portion detectable by mass spectrometry refers to a peptide portion that can be excised from the antigen-binding molecule by a digestive enzyme and ionized. When the antigen-binding molecule comprises all or part of an antibody constant region, the unmodified peptide portion is preferably a peptide portion comprised in all or part of the antibody constant region. When the antigen-binding molecule comprises antibody VH and/or VL, the unmodified peptide portion may be a peptide portion comprised in the frameworks of the VH and/or VL. Examples of the unmodified peptide portions comprised in human antibodies include, but are not limited to, the following.

**(Table 2-1) Peptide portions detectable by mass spectrometry in native human antibody**

| SEQ ID NO: | Digestive enzyme | Location in native human antibody |
|---|---|---|
| 5 and 875-887 | Trypsin | IgG1 constant region |
| 5 and 888-896 | Lys-C | IgG1 constant region |
| 897-918 | Arg-C | IgG1 constant region |
| 899-912 | Chymotrypsin | IgG1 constant region |
| 913-926 | Glu-C (phosphate buffer) | IgG1 constant region |
| 927-933 | Glu-C (bicarbonate buffer) | IgG1 constant region |
| 934-939 | Asp-N | IgG1 constant region |
| 940-950 | Trypsin | IgG2 constant region |
| 951-958 | Lys-C | IgG2 constant region |
| 959-961 | Arg-C | IgG2 constant region |
| 962-975 | Chymotrypsin | IgG2 constant region |
| 976-992 | Glu-C (phosphate buffer) | IgG2 constant region |
| 993-1000 | Glu-C (bicarbonate buffer) | IgG2 constant region |
| 1001-1005 | Asp-N | IgG2 constant region |
| 1006-1020 | Trypsin | IgG3 constant region |
| 1021-1028 | Lys-C | IgG3 constant region |
| 1029-1035 | Arg-C | IgG3 constant region |
| 1036-1049 | Chymotrypsin | IgG3 constant region |
| 1050-1066 | Glu-C (phosphate buffer) | IgG3 constant region |
| 1067-1072 | Glu-C (bicarbonate buffer) | IgG3 constant region |
| 1073-1079 | Asp-N | IgG3 constant region |
| 1080-1091 | Trypsin | IgG4 constant region |
| 1092-1099 | Lys-C | IgG4 constant region |
| 1100-1102 | Arg-C | IgG4 constant region |
| 1103-1118 | Chymotrypsin | IgG4 constant region |
| 1119-1136 | Glu-C (phosphate buffer) | IgG4 constant region |
| 1137-1145 | Glu-C (bicarbonate buffer) | IgG4 constant region |
| 1146-1150 | Asp-N | IgG4 constant region |
| 1151-1163 | Trypsin | IgA1 constant region |
| 1164-1170 | Lys-C | IgA1 constant region |
| 1171-1176 | Arg-C | IgA1 constant region |
| 1177-1196 | Chymotrypsin | IgA1 constant region |
| 1197-1208 | Glu-C (phosphate buffer) | IgA1 constant region |
| 1209-1212 | Glu-C (bicarbonate buffer) | IgA1 constant region |

**(Table 2-2) Peptide portions detectable by mass spectrometry in native human antibody**

| SEQ ID NO: | Digestive enzyme | Location in native human antibody |
|---|---|---|
| 1213-1217 | Asp-N | IgA1 constant region |
| 1218-1227 | Trypsin | IgA2 constant region |
| 1228-1233 | Lys-C | IgA2 constant region |
| 1234-1239 | Arg-C | IgA2 constant region |
| 1240-1258 | Chymotrypsin | IgA2 constant region |
| 1259-1272 | Glu-C (phosphate buffer) | IgA2 constant region |
| 1273-1278 | Glu-C (bicarbonate buffer) | IgA2 constant region |
| 1279-1284 | Asp-N | IgA2 constant region |
| 1285-1309 | Trypsin | IgM constant region |
| 1310-1321 | Lys-C | IgM constant region |
| 1322-1328 | Arg-C | IgM constant region |
| 1329-1352 | Chymotrypsin | IgM constant region |
| 1353-1374 | Glu-C (phosphate buffer) | IgM constant region |
| 1375-1385 | Glu-C (bicarbonate buffer) | IgM constant region |
| 1386-1393 | Asp-N | IgM constant region |
| 1394-1410 | Trypsin | IgD constant region |
| 1411-1418 | Lys-C | IgD constant region |
| 1419-1427 | Arg-C | IgD constant region |
| 1428-1444 | Chymotrypsin | IgD constant region |
| 1445-1457 | Glu-C (phosphate buffer) | IgD constant region |
| 1458-1465 | Glu-C (bicarbonate buffer) | IgD constant region |
| 1466-1468 | Asp-N | IgD constant region |
| 1469-1489 | Trypsin | IgE constant region |
| 1490-1496 | Lys-C | IgE constant region |
| 1497-1510 | Arg-C | IgE constant region |
| 1511-1531 | Chymotrypsin | IgE constant region |
| 1532-1548 | Glu-C (phosphate buffer) | IgE constant region |
| 1549-1556 | Glu-C (bicarbonate buffer) | IgE constant region |
| 1557-1564 | Asp-N | IgE constant region |

The modified peptide portion detectable by mass spectrometry can be designed by introducing an artificial modification to the unmodified peptide portion in the antigen-binding molecule, *e.g.*, the peptide portions detectable by mass spectrometry in a native human antibody shown in Tables 2-1 and 2-2.

In one embodiment, the artificial modification can be performed by one or more methods selected from: a) substituting one or more amino acid residues in the unmodified peptide portion with other types of amino acid residues, or deleting it; b) inserting one or more amino acid residues into the unmodified peptide portion; and c) adding amino acid residues to the N terminal or C terminal of the unmodified peptide portion.

In one embodiment, the artificial modification can be performed by: substituting one or more amino acid residues other than Ala in the unmodified peptide portion with Ala; substituting one or more amino acid residues other than Ile in the unmodified peptide portion with Ile; substituting one or more amino acid residues other than Val in the unmodified peptide portion with Val; substituting one or more amino acid residues other than Leu in the unmodified peptide portion with Leu; or substituting one or more amino acid residues other than Phe in the unmodified peptide portion with Phe.

In one embodiment, the artificial modification can be performed by inserting one or more types of amino acid residues selected from one or more of Ala, Ile, Val, Leu, and Phe into the unmodified peptide portion. The amino acid residues can be inserted at one, two, or three positions in the unmodified peptide portion. In one embodiment, the artificial modification can be performed by adding one or more types of amino acid residues selected from Ala, Ile, Val, Leu, and Phe to the N terminal or C terminal of the unmodified peptide portion. The amino acid residues to be inserted or added may be one, two, or three residues. The amino acid residues to be inserted or added may be Ala, Ile, Val, Leu, Phe, Ala-Ala, Ile-Ile, Val-Val, Leu-Leu, Phe-Phe, Ala-Ile, Ala-Val, Ala-Leu, Ala-Phe, Ile-Val, Ile-Leu, Ile-Phe, Val-Leu, Val-Phe, Leu-Phe, or Ala-Ala-Ala.

The modified peptide portion preferably does not have a site that is cleaved by a digestive enzyme that excises the unmodified peptide portion from the unmodified antigen-binding molecule.

The modified peptide portion is preferably detectable by mass spectrometry after a sample comprising the modified antigen-binding molecule, which comprises the modified peptide portion instead of the unmodified peptide portion of the unmodified antigen-binding molecule, is treated with the same type of digestive enzyme that excises the unmodified peptide portion.

The peak of the modified peptide portion is preferably detected by mass spectrometry after a sample comprising the modified antigen-binding molecule, which comprises the modified peptide portion instead of the unmodified peptide portion of the unmodified antigen-binding molecule, is treated with the same type of digestive enzyme that excises the unmodified peptide portion.

The modified peptide portion is preferably not comprised in a protein derived from an animal to which the modified antigen-binding molecule comprising the modified peptide portion instead of the unmodified peptide portion of the unmodified antigen-binding molecule is administered. The absence in the protein derived from the animal to which the modified peptide portion is administered can be confirmed by prediction by *in silico* methods known to those skilled in the art.

The modified peptide portion is preferably separable by liquid-phase chromatography from an animal-derived peptide that is excised when a biological sample (blank matrix) derived from the animal to which the modified antigen-binding molecule comprising the modified peptide portion instead of the unmodified peptide portion of the unmodified antigen-binding molecule is administered is treated with the same type of digestive enzyme.

The mass spectrometry peak of the modified peptide portion preferably does not interfere with the mass spectrometry peak of an animal-derived peptide that is excised when a biological sample (blank matrix) from the animal to which the modified antigen-binding molecule comprising the modified peptide portion instead of the unmodified peptide portion of the unmodified antigen-binding molecule is administered is treated with the same type of digestive enzyme.

The modified antigen-binding molecule comprising the modified peptide portion instead of the unmodified peptide portion of the unmodified antigen-binding molecule preferably has sufficient thermal stability. The modified antigen-binding molecule having sufficient thermal stability refers to a modified antigen-binding molecule with a Tm value of 40°C or higher, 45°C or higher, 50°C or higher, 55°C or higher, 60°C or higher, 65°C or higher, or 70°C or higher.

The modified antigen-binding molecule comprising the modified peptide portion instead of the unmodified peptide portion of the unmodified antigen-binding molecule preferably has thermal stability similar to that of the unmodified antigen-binding molecule. Having similar thermal stability means that the difference in Tm value between two molecules is within 20°C, within 15°C, within 10°C, within 9°C, within 8°C, within 7°C, within 6°C, within 5°C, within 4°C, within 3°C, within 2°C, or within 1°C.

The antigen-binding activity of the modified antigen-binding molecule comprising the modified peptide portion instead of the unmodified peptide portion of the unmodified antigen-binding molecule is preferably 10% or more, 20% or more, 30% or more, 40% or more, 50% or more, 60% or more, 70% or more, 80% or more, or 90% or more of the antigen-binding activity of the unmodified antigen-binding molecule, that is, the antigen-binding activity of the modified antigen-binding molecule is not significantly decreased compared to that of the unmodified antigen-binding molecule. The antigen-binding activity of the modified antigen-binding molecule comprising the modified peptide portion instead of the unmodified peptide portion of the unmodified antigen-binding molecule is preferably 200% or less, 190% or less, 180% or less, 170% or less, 160% or less, 150% or less, 140% or less, 130% or less, 120% or less, or 110% or less of the antigen-binding activity of the unmodified antigen-binding molecule, that is, the antigen-binding activity of the modified antigen-binding molecule is not significantly increased compared to that of the unmodified antigen-binding molecule.

The modified antigen-binding molecule comprising the modified peptide portion instead of the unmodified peptide portion of the unmodified antigen-binding molecule preferably has antigen-binding activity similar to that of the unmodified antigen-binding molecule. Having antigen-binding activity similar to that of the unmodified antigen-binding molecule means that the antigen-binding activity of the modified antigen-binding molecule comprising the modified peptide portion instead of the unmodified peptide portion of the unmodified antigen-binding molecule is 10% or more and 100% or less, 20% or more and 100% or less, 30% or more and 100% or less, 40% or more and 100% or less, 50% or more and 100% or less, 60% or more and 100% or less, 70% or more and 100% or less, 80% or more and 100% or less, 90% or more and 100% or less, 100% or more and 200% or less, 100% or more and 190% or less, 100% or more and 180% or less, 100% or more and 170% or less, 100% or more and 160% or less, 100% or more and 150% or less, 100% or more and 140% or less, 100% or more and 130% or less, 100% or more and 120% or less, or 100% or more and 110% or less of the antigen-binding activity of the unmodified antigen-binding molecule.

If a modified antigen-binding molecule produced by including a modified peptide portion instead of an unmodified peptide portion in the antigen-binding molecule satisfies one or more of: having sufficient thermal stability; having thermal stability similar to that of the unmodified antigen-binding molecule; having antigen-binding activity that is not significantly decreased compared to that of the unmodified antigen-binding molecule; having antigen-binding activity that is not significantly increased compared to that of the unmodified antigen-binding molecule; and having antigen-binding activity similar to that of the unmodified antigen-binding molecule, then the modified antigen-binding molecule can be selected as the antigen-binding molecule to be administered in combination with the unmodified antigen-binding molecule in a cassette dosing study.

The present application provides a method for designing, selecting, or producing a modified antigen-binding molecule comprising an artificially modified peptide portion that is detectable by mass spectrometry, wherein the method comprises:
(I) identifying a peptide which is obtained after treating one antigen-binding molecule with a digestive enzyme and detectable by mass spectrometry;
(II) designing an artificially modified peptide by any of the following:
   (II-1) deleting or substituting one or more amino acid residues in the peptide identified in (I) with other amino acid residues;
   (II-2) inserting one or more amino acid residues into the peptide identified in (I);
   (II-3) adding an amino acid residue to the N-terminus or C-terminus of the peptide identified in (I);
   (II-4) a combination of (II-1) and (II-2);
   (II-5) a combination of (II-1) and (II-3);
   (II-6) a combination of (II-2) and (II-3); and
   (II-7) a combination of (II-1), (II-2), and (II-3);
(III) performing the modification of (II) on the peptide portion identified in (I) in the antigen-binding molecule of (I) to produce a modified antigen-binding molecule;
(IV) selecting the modified antigen-binding molecule produced in (III) if the modified antigen-binding molecule satisfies any of the following:
   (IV-1) the Tm value of the modified antigen-binding molecule is 50°C or higher;
   (IV-2) the antigen-binding activity of the modified antigen-binding molecule is 10% or more of the antigen-binding activity of the antigen-binding molecule of (I); and
   (IV-3) both (IV-1) and (IV-2) above; and
(V) selecting the modified antigen-binding molecule selected in (IV) if a portion contained in the modified antigen-binding molecule that corresponds to the peptide designed in (II) is detectable by mass spectrometry after treating a biological sample containing the modified antigen-binding molecule with the digestive enzyme of (I).

The method for designing, selecting, or producing a modified antigen-binding molecule can further comprise the following steps:
(VI) performing a pharmacokinetic test using mass spectrometry after treating a biological sample comprising the modified antigen-binding molecule of (I) with the digestive enzyme of (I), to obtain a pharmacokinetic profile or a pharmacokinetic parameter;
(VII) performing a pharmacokinetic test using mass spectrometry after treating a biological sample comprising the modified antigen-binding molecule selected in (V) with the digestive enzyme of (I), to obtain a pharmacokinetic profile or a pharmacokinetic parameter; and
(VIII) selecting the modified antigen-binding molecule if the pharmacokinetic profile or pharmacokinetic parameter obtained in (VI) is equivalent to the pharmacokinetic profile or pharmacokinetic parameter obtained in (VII).

The antigen-binding molecule in step (I) above can be, for example, an antigen-binding molecule comprising an antigen-binding domain, a CL domain, and a human IgG CH1 domain. Explanation of the antigen-binding molecule is provided above.

The peptide detectable by mass spectrometry in step (I) above includes a peptide excised after treating the antigen-binding molecule with a digestive enzyme. The peptide detectable by mass spectrometry in step (I) above is preferably a peptide comprised in an antibody constant region. The peptide detectable by mass spectrometry in step (I) above is preferably a peptide not comprised in an animal-derived protein present in a biological sample of the animal subjected to administration in the pharmacokinetics test. The peptide detectable by mass spectrometry in step (I) above preferably includes, but is not limited to, a peptide derived from the human IgG CH1 domain, a peptide consisting of the amino acid sequence of position 120 to position 133 of the human IgG CH1 domain according to the EU index, a peptide consisting of the amino acid sequence between position 121 to position 133 of the human IgG CH1 domain according to the EU index, and peptides shown in Table 2-1 and Table 2-2.

In the present application, detectable by mass spectrometry means that, when the mass spectrometry is performed in selected reaction monitoring (SRM) measurement mode, the peak can be detected at the SRM transition in which ionized peptide is set as the target. Quantifiable by mass spectrometry means that, when the mass spectrometry is performed in selected reaction monitoring (SRM) measurement mode, the peak area at the SRM transition in which ionized peptide is set as the target through mass spectrometry can be compared with a calibration curve to calculate the concentration of the target peptide.

The other type of amino acid residue in step (II-1) above can be Ala, Ile, Val, Leu, or Phe. In step (II-2) above, the amino acid residues inserted can be 1, 2, or 3 residues, and the examples include Ala, Ile, Val, Leu, Phe, Ala-Ala, Ile-Ile, Val-Val, Leu-Leu, Phe-Phe, Ala-Ile, Ala-Val, Ala-Leu, Ala-Phe, Ile-Val, Ile-Leu, Ile-Phe, Val-Leu, Val-Phe, Leu-Phe, and Ala-Ala-Ala. In step (II-2) above, the amino acid residues can be inserted into 1, 2, or 3 positions in the peptide identified in (I). In step (II-3) above, the amino acid residues added can be 1, 2, or 3 residues. The peptide designed in step (II) above, for example, does not have a site cleaved by the digestive enzyme in (I). Explanation of the digestive enzyme is provided below.

In the present application, step (I) above can be performed by utilizing *in silico* predictions or existing knowledge. Step (I) above can be performed, for example, by using skyline software to digest a protein *in silico* and predict an excised peptide, or by utilizing existing knowledge, *i.e.*, cleavage specificity of various digestive enzymes, to identify an excised sequence in the target protein. Alternatively, step (I) above can also be performed by treating a biological sample comprising an antigen-binding molecule by a digestive enzyme to produce a peptide from the antigen-binding molecule and verifying whether the peptide is detectable by mass spectrometry or not.

In the present application, steps (II) to (VIII) above can be performed by utilizing methods known to those skilled in the art and the explanation herein. With regard to step (IV) above, the Tm value of the modified antigen-binding molecule can be, for example, 40°C or higher, 45°C or higher, 50°C or higher, 55°C or higher, 60°C or higher, 65°C or higher, or 70°C or higher, and 100°C or lower. In addition, with regard to step (IV) above, the difference in Tm value of the modified antigen-binding molecule and the antigen-binding molecule of step (I) is, for example, within 20°C, within 15°C, within 10°C, within 9°C, within 8°C, within 7°C, within 6°C, within 5°C, within 4°C, within 3°C, within 2°C, or within 1°C. Moreover, the antigen-binding activity of the modified antigen-binding molecule can be, for example, 10% or more, 10% or more, 20% or more, 30% or more, 40% or more, 50% or more, 60% or more, 70% or more, 80% or more, or 90% or more of the antigen-binding activity of the antigen-binding molecule in step (I), and can also be, for example, 200% or less, 190% or less, 180% or less, 170% or less, 160% or less, 150% or less, 140% or less, 130% or less, 120% or less, or 110% or less of the antigen-binding activity of the antigen-binding molecule in step (I).

The present application also provides a modified antigen-binding molecule designed, selected, or produced by the above method, and a peptide designed in step (II) of the above method.

### III. Peptides, antigen-binding molecules, or methods for detecting and/or quantifying peptides or antigen-binding molecules

The present application provides a method for detecting a peptide derived from an antigen-binding molecule in a biological sample, comprising the following steps:
(a) treating the biological sample comprising the antigen-binding molecule with a digestive enzyme to generate a peptide from the antigen-binding molecule,
   wherein the peptide is produced by excising an antibody constant region comprised in the antigen-binding molecule with the digestive enzyme treatment, and has an amino acid sequence different from that of the corresponding site in the constant region of a native antibody; and
(b) subjecting the biological sample treated in (a) to mass spectrometry to detect the peptide.

Examples of the peptide include the peptides designed by the method provided in the present application.

The present application provides a method for detecting a peptide, comprising the following steps:
(a) preparing a sample comprising a peptide consisting of the amino acid sequence between position 121 and position 133 according to the EU index in a non-native human IgG CH1 domain,
   wherein the amino acid sequence between position 121 and position 133 according to the EU index in the non-native human IgG CH1 domain is different from the corresponding sequence in a native human IgG of the same isotype as the non-native human IgG; and
(b) subjecting the sample prepared in (a) to mass spectrometry to detect the peptide.

The present application also provides a method for quantifying a peptide, comprising the following steps:
(a) preparing a sample comprising a peptide consisting of the amino acid sequence between position 121 and position 133 according to the EU index in a non-native human IgG CH1 domain,
   wherein the amino acid sequence between position 121 and position 133 according to the EU index in the non-native human IgG CH1 domain is different from the corresponding sequence in a native human IgG of the same isotype as the non-native human IgG;
(b) subjecting the sample prepared in (a) to mass spectrometry to detect the peptide; and
(c) quantifying the peptide based on the analysis result in (b).

The present application provides a method for detecting each of multiple types of peptides in a sample, comprising the following steps:
(a) preparing a sample comprising multiple types of peptides,
   wherein the multiple types of peptides consist of the amino acid sequence between position 121 and position 133 according to the EU index in a native or non-native human IgG CH1 domain, and wherein the amino acid sequence differs between the multiple types of peptides; and
(b) subjecting the sample prepared in (a) to mass spectrometry to detect each of the multiple types of peptides.

The present application also provides a method for quantifying each of multiple types of peptides in a sample, comprising the following steps:
(a) preparing a sample comprising multiple types of peptides,
   wherein the multiple types of peptides consist of the amino acid sequence between position 121 and position 133 according to the EU index in a native or non-native human IgG CH1 domain, and wherein the amino acid sequence differs between the multiple types of peptides;
(b) subjecting the sample prepared in (a) to mass spectrometry to detect each of the multiple types of peptides; and
(c) quantifying each of the multiple types of peptides based on the analysis result in (b).

In the present application, examples of the sample include, but are not limited to, a solution comprising a peptide and a biological sample.

Furthermore, the present application provides a method for detecting and/or quantifying an antigen-binding molecule in a biological sample, comprising the following steps:
(a) treating the biological sample comprising the antigen-binding molecule with a digestive enzyme to generate a peptide from the antigen-binding molecule,
   wherein the antigen-binding molecule comprises an antigen-binding domain, a CL domain, and a non-native human IgG CH1 domain,
   wherein the amino acid sequence between position 121 and position 133 according to the EU index in the non-native human IgG CH1 domain is different from the corresponding sequence in a native human IgG of the same isotype as the non-native human IgG,
   wherein the peptide consists of the amino acid sequence between position 121 and position 133 according to the EU index in the non-native human IgG CH1 domain;
(b) subjecting the biological sample treated in (a) to mass spectrometry to detect the peptide; and
(c) detecting and/or quantifying the antigen-binding molecule based on the analysis result in (b).

Furthermore, the present application provides a method for detecting and/or quantifying each of multiple types of antigen-binding molecules in a biological sample, comprising the following steps:
(a) treating the biological sample comprising the multiple types of antigen-binding molecules with a digestive enzyme to generate multiple types of peptides from the multiple types of antigen-binding molecules,
   wherein the multiple types of antigen-binding molecules comprise antigen-binding molecules comprising an antigen-binding domain, a CL domain, and a native or non-native human IgG CH1 domain,
   wherein the amino acid sequence between position 121 and position 133 according to the EU index in the human IgG CH1 domain of the multiple types of antigen-binding molecules is different among the multiple types of antigen-binding molecules,
   wherein the multiple types of peptides consist of the amino acid sequence between position 121 and position 133 according to the EU index in the native or non-native human IgG CH1 domain, and the amino acid sequence is different among the multiple types of peptides;
(b) subjecting the biological sample treated in (b) to mass spectrometry to detect each of the multiple types of peptides; and
(c) detecting and/or quantifying each of the multiple types of antigen-binding molecules based on the analysis result in (b).

Prior to treating the biological sample with a digestive enzyme, the method of the present application may comprise the following steps:
(i) treating an individual or subject with one or more types of antigen-binding molecules; and
(ii) collecting a biological sample comprising the antigen-binding molecules from the individual or subject.

In the present application, examples of the treatment include administration, but are not limited thereto.

In the present application, the biological sample can be any component collected from the individual or subject, and the examples include blood, plasma, serum, cell, urine, ascites, pleural effusion, sweat, papillary secretion, seminal fluid, synovial fluid, feces, bone marrow fluid, bile, cerebrospinal fluid (CSF), milk, bronchial lavage fluid, amniotic fluid, saliva, vitreous, tear, tissue, or tissue extract. The tissue includes, but is not limited to, tissue derived from brain, liver, lung, and kidney. In the present application, the antigen-binding molecule comprised in the biological sample can be an intact antigen-binding molecule.

In the method of the present application, step (b), *i.e.*, a mass spectrometry step, can include: contacting the biological sample treated in step (a) or the sample prepared in step (a) with an affinity capture medium or a chromatographic adsorbent and eluting a peptide-enriched biological sample or a peptide-enriched sample; and subjecting the enriched biological sample or the enriched sample to mass spectrometry. Furthermore, in the method of the present application, step (a), *i.e.*, an enzymatic digestion step, can include: contacting the biological sample with an affinity capture medium or a chromatographic adsorbent and eluting the antigen-binding molecule-enriched biological sample; and treating the enriched biological sample with a digestive enzyme.

The affinity capture medium of the present application includes, but is not limited to, protein A, protein G, and anti-Fc antibody. The affinity capture medium of the present application may be bound to beads (*e.g.*, magnetic beads). That is, the affinity capture medium of the present application includes, for example, bead-loaded protein A, bead-loaded protein G, or bead-loaded anti-Fc antibody. Furthermore, the chromatographic adsorbent of the present application includes, but is not limited to, a solid-phase extraction (SPE) adsorbent. The anti-Fc antibody is an antibody that specifically recognizes the Fc region of a natural or modified antibody, and examples include, but are not limited to, the antibodies described in WO2019/112027 and WO2020/213706.

Furthermore, in the method of the present application, step (b), *i.e.*, a mass spectrometry step, can include subjecting the biological sample treated in step (a) or the sample prepared in step (a) to mass spectrometry in combination with liquid chromatography. Furthermore, in the method of the present application, step (b) can include separating a fraction comprising peptides from the biological sample treated in step (a) or the sample prepared in step (a) by liquid chromatography, and subjecting the separated fraction to mass spectrometry. Furthermore, in the method of the present application, step (a) can include separating a fraction comprising the antigen-binding molecules from the biological sample by liquid chromatography, and treating the separated fraction with a digestive enzyme.

In the method of the present application, prior to the mass spectrometry step, denaturation, reduction, or alkylation of the antigen-binding molecules can also be performed in addition to digestion of the antigen-binding molecules. Furthermore, in the method of the present application, prior to the mass spectrometry step, denaturation, reduction, or alkylation of the antigen-binding molecules, or removal of contaminants (purification of the antigen-binding molecules or peptides), etc. can also be performed in addition to digestion of the antigen-binding molecules.

Digestion of the antigen-binding molecules can be performed with, for example, a digestive enzyme. A "digestive enzyme" is an enzyme that can cleave or hydrolyze peptides or proteins into fragments in a specific, general, or random manner. The digestive enzyme of the present application is not particularly limited as long as it is capable of cleaving the antigen-binding molecule, and examples include proteases. Proteases include, but are not limited to, trypsin, papain, endoprotease LysC, endoprotease ArgC, *Staphylococcus aureus* V8, chymotrypsin, Asp-N, Asn-C, pepsin, endoprotease GluC, proalanase, mutants thereof, and the proteases listed in Tables 2-1 and 2-2.

Denaturation of the antigen-binding molecule can be performed with, for example, a denaturing agent. The denaturing agent of the present application is not particularly limited as long as it is capable of denaturing the antigen-binding molecule, and examples include urea and guanidine hydrochloride.

Reduction of the antigen-binding molecule can be performed with, for example, a reducing agent. The reducing agent of the present application is not particularly limited as long as it can reduce the antigen-binding molecule, and examples include dithiothreitol and tris(2-carboxyethyl)phosphine.

Alkylation of the antigen-binding molecule can be performed with, for example, an alkylating agent. The alkylating agent of the present application is not particularly limited as long as it can alkylate the antigen-binding molecule, and examples include iodoacetamide.

Removal of contaminants can be performed with, for example, an affinity capture medium or chromatographic adsorbent.

In the present application, mass spectrometry can be performed by MS, LC-MS, MS/MS, or LC-MS/MS, but is not limited thereto. Mass spectrometry is well known to those skilled in the art and is now largely automated and performed in a high-throughput manner. Those skilled in the art can select the equipment and software to be used as appropriate. High-resolution MS that can distinguish between multiple peptides that differ in m/z by only 1 is also well known to those skilled in the art. Those skilled in the art can also select such high-resolution MS as appropriate.

In the present application, examples of mass spectrometer include, but are not limited to, quadrupole mass spectrometer, triple quadrupole mass spectrometer, ion trap mass spectrometer, time-of-flight mass spectrometer, MALDI-TOF mass spectrometer, and electric field Fourier transform mass spectrometer.

In the method of the present application, mass spectrometry can also be performed by tandem mass spectrometry or multistage mass spectrometry. Furthermore, in the method of the present application, mass spectrometry can also be performed by tandem mass spectrometry or multistage mass spectrometry in selected reaction monitoring (SRM) measurement mode.

In the present application, tandem mass spectrometry refers to a technique in which a precursor ion is selected and dissociated in the first mass spectrometry step, followed by performing m/z separation and detection of the resulting product ion in the second mass spectrometry step. Tandem mass spectrometry is used interchangeably with MS/MS. In the present application, multistage mass spectrometry refers to an analytical method in which ion selection, dissociation, and m/z separation are repeated two or more times to obtain a mass spectrum of n^{th} product ions. Multistage mass spectrometry is used interchangeably with MSⁿ .

In the present application, selective reaction monitoring refers to a technique in tandem mass spectrometry or multistage mass spectrometry in which, instead of acquiring a product ion spectrum by scanning the mass analyzer, a mass spectrometer is operated to continuously detect only the signal level of product ions of a specific mass-to-charge ratio (m/z) value produced by dissociating precursor ions of a specific m/z value. For example, in selective reaction monitoring in tandem mass spectrometry using a triple quadrupole mass spectrometer, the first quadrupole analyzer Q1 (corresponding to the first mass spectrometry step) and the third quadrupole analyzer Q3 (corresponding to the second mass spectrometry step) are both set to continuously transmit ions of a specific m/z value, thereby operating the mass spectrometer to continuously detect only the signal of product ions of a specific m/z value produced by dissociation of a precursor ion of a specific m/z value. Furthermore, in selective reaction monitoring, the combination of the m/z value of the selected precursor ion and the m/z value of the selected product ion is not limited to one set. Selective reaction monitoring which selects multiple combinations of precursor ion m/z values and product ion m/z values may be referred to as multiple reaction monitoring (MRM). When multistage mass spectrometry is used, selective reaction monitoring may be referred to as consecutive reaction monitoring (CRM). While multiple reaction monitoring monitors multiple parallel reactions at the same level, such as isolation and selection of precursor ion, followed by isolation and selection of product ion, consecutive reaction monitoring monitors multiple consecutive reactions, such as isolation and selection of precursor ion, followed by isolation and selection of primary product ion, followed by isolation and selection of secondary product ion.

In the present application, the above-mentioned step (b), *i.e.*, a mass spectrometry step, can include the following steps:
(1) separating components in the biological sample treated in step (a) or components in the sample prepared in step (a) by liquid chromatography to obtain an eluate comprising separated peptides;
(2) a first mass spectrometry step of ionizing the peptides in the eluate to generate precursor ions and selecting a precursor ion derived from the target peptide from the generated precursor ions; and
(3) a second mass spectrometry step of generating product ions from the precursor ion selected in the first mass spectrometry step by collision-induced dissociation, and selecting and detecting one or more product ions derived from the target peptide.

The above method can include, prior to step (1), a step of deproteinizing the biological sample treated in step (a) or the sample prepared in step (a) in the presence of a strong acid with deproteinizing ability. In such a step, unwanted proteins or unwanted peptides present in the biological sample treated in step (a) or the sample prepared in step (a) are removed by the strong acid with deproteinizing ability.

Deproteinization can be performed extremely easily using a strong acid with deproteinizing ability. For example, a strong acid with deproteinizing ability can be added to the biological sample treated in step (a) or the sample prepared in step (a) and stirred, and then centrifuged to precipitate concomitants. The resulting supernatant can be used as the sample for analysis. Any strong acid commonly used for deproteinization can be used, such as trichloroacetic acid, hydrochloric acid, phosphoric acid, etc.

In "(1) separating components in the biological sample treated in step (a) or components in the sample prepared in step (a) by liquid chromatography to obtain an eluate comprising separated peptides", the components contained in the biological sample treated in step (a) or the components contained in the sample prepared in step (a) are separated by liquid chromatography, prior to performing mass spectrometry. The fractions comprising peptides eluted from the liquid chromatograph serve as samples for mass spectrometry.

In the present application, the type of liquid chromatography can be arbitrarily selected from various known liquid chromatography methods as long as it can efficiently separate a mixture of components in the sample for quantification. For example, it can be selected from reverse-phase column chromatography, cation exchange chromatography, anion exchange chromatography, hydrophobic interaction chromatography, isoelectric focusing chromatography, size exclusion chromatography, and affinity chromatography, and is preferably reverse-phase column chromatography.

The column used can be appropriately selected depending on the liquid chromatography method to be performed. For reverse-phase column chromatography, an ODS-based column is preferably selected. Examples of commercially available ODS-based columns include YMC Pack ODS-A (YMC Co. Ltd.) and PLRP-S (Polymer Laboratories Inc.). The mobile phase is preferably selected from solvents that do not adversely affect the column or liquid chromatography device. If the fraction eluted from the liquid chromatograph is directly used as a sample for mass spectrometry, it is preferable to select a solvent suitable for the ionization adopted in mass spectrometry. For example, when electrospray ionization (ESI) or atmospheric pressure chemical ionization (APCI) is used for ionization in mass spectrometry, the mobile phase is preferably volatile and preferably a polar solvent. Examples of such solvents include water, aqueous salt solutions, alcohol, and acetonitrile. Acetonitrile is an example of a solvent that can be suitably used as a mobile phase in the method of the present application.

In the method of the present application, liquid chromatography may be performed only once, or may be repeated several times, as necessary. The sample fractionated in the first liquid chromatography run can be separated using a different type of column to increase the degree of purification of the target substance.

In "(2) a first mass spectrometry step of ionizing the peptides in the eluate to generate precursor ions and selecting a precursor ion derived from the target peptide from the generated precursor ions", at first, the eluted fraction obtained in step (1) is introduced into a mass spectrometer, where the components in the sample are ionized to generate precursor ions. In the present application, precursor ions refer to monovalent or multivalent cations or anions generated by ionization in the first mass spectrometry step, in particular, hydrogenated monovalent or multivalent ions. The ionization method used in the method of the present application can be selected from among ionization methods capable of generating hydrogenated monovalent or multivalent ions. Examples of such ionization methods include electrospray ionization (ESI), atmospheric pressure chemical ionization (APCI), thermospray ionization (TSP), and matrix-assisted laser desorption ionization (MALDI), and ESI is preferred.

Among the precursor ions generated by ionization in the first mass spectrometry step, a precursor ion to be transferred to the second mass spectrometry step is selected. The selection of the precursor ion to be transferred to the second mass spectrometry step can be performed with reference to the Examples. First, prior to performing the method of the present application, a mass spectrum of the target peptide is created, and the generation of the precursor ion is confirmed from the molecular weight of the peptide. If multiple types of precursor ions with different valences are generated depending on the number of hydrogen atoms added, the precursor ion with the strongest ionic strength is selected. When performing the method of the present application, the precursor ion selected above can be selected as the precursor ion to be transferred to the second mass spectrometry step.

In "(3) a second mass spectrometry step of generating product ions from the precursor ion selected in the first mass spectrometry step by collision-induced dissociation, and selecting and detecting one or more product ions derived from the target peptide", the target peptide-derived precursor ion selected in step (2) is bombarded with an inert gas such as nitrogen gas or argon gas to generate product ions. In the method of the present application, one product ion may be detected, or alternatively, several product ions may be detected.

A mass spectrometry method having a first mass spectrometry step and a second mass spectrometry step is a so-called MS/MS method. Selected reaction monitoring (SRM) and selected ion monitoring (SIM) are known as commonly used methods for detecting ions in the MS/MS method, however, SRM is preferred as the method for detecting ions in this application. In SRM, an SRM transition (a combination of the m/z value of the precursor ion and the m/z value of the product ion) is determined. As an SRM transition, a product ion having an m/z value greater than the m/z value of the precursor ion can be selected, but is not limited thereto.

If product ions derived from the target peptide are detected as described above, it is determined that the target peptide is detected. Furthermore, since the target peptide has a sequence unique to the antigen-binding molecule, if the target peptide is detected, it is determined that the antigen-binding molecule is detected.

The target peptide or target antigen-binding molecule is quantified from the product ions detected as described above. Quantification of the target peptide or target antigen-binding molecule can be performed by known quantification methods, such as calibration curve method (external standard method, standard addition method, internal standard method) and isotope dilution method, and is not particularly limited. For example, first, a fixed amount (known amount) of the target peptide or target antigen-binding molecule is added to a biological sample or sample to prepare a standard solution, and then the method of the present application is performed on the standard solution to create a calibration curve from the obtained signal intensity and the known amount of the standard solution. Using a biological sample or sample to prepare a standard solution is an important step in ensuring the accuracy of quantification. The method of the present application is performed on the peptide or antigen-binding molecule in a sample to be measured, and the target peptide or target antigen-binding molecule can be quantified using the obtained signal intensity and the calibration curve. For example, a biological sample or sample comprising a known concentration of the target peptide is subjected to mass spectrometry, and a calibration curve is created with the concentration of the target peptide on the horizontal axis and the peak area of an ion derived from the peptide on the vertical axis, then the peak area of the ion derived from the target peptide is applied to calculate the amount of the target peptide. Alternatively, for example, a sample comprising both a known concentration of the target antigen-binding molecule and a known concentration of an internal standard antibody (*e.g.*, an antibody with a target peptide labeled with a stable isotope) is treated with a digestive enzyme and then analyzed with LC-MS, and a calibration curve is created with the concentration of the target antigen-binding molecule on the horizontal axis and the ratio of the peak area of an ion derived from the target peptide to the peak area of an ion derived from the corresponding peptide comprised in the internal standard antibody on the vertical axis, then the ratio of the peak area of the ion derived from the target peptide to the peak area of the ion derived from the corresponding peptide comprised in the internal standard antibody is applied to calculate the amount of the target antigen-binding molecule. For specific examples, see the methods described in the Examples.

In the method of the present application, step (1) may be performed using a liquid chromatographic apparatus, and the obtained fractions may be introduced into an MS/MS apparatus to perform steps (2) and (3). Alternatively, the sample for quantification obtained by the pretreatment of step (a) may be introduced into an apparatus capable of performing LC-MS/MS. Many apparatus capable of performing MS/MS and LC-MS/MS are already commercially available. The method of the present application may be performed using any apparatus as long as the method can be performed, but a liquid chromatography/tandem mass spectrometer capable of performing SRM is preferable. A specific example of a liquid chromatography/tandem mass spectrometer for performing the method of the present application is a high-performance liquid chromatography-coupled electrospray ionization mass spectrometer (LC/ESI-MS/MS), such as an LC/ESI-MS/MS using a Xevo TQ-XS triple quadrupole instrument (Waters) coupled to ACQUITY UPLC I-class (Waters). Examples of conditions for performing LC/ESI-MS/MS using a Xevo TQ-XS triple quadrupole instrument (Waters) coupled to ACQUITY UPLC I-class (Waters) include, but are not limited to, the conditions described in the Examples.

Furthermore, by performing step (1) with a liquid chromatograph and then performing steps (2) and (3) simultaneously on each of the separated components , it is possible to detect and/or quantify multiple types of target antigen-binding molecules comprised in the biological sample at the same time.

The peptides in the method of the present application include a peptide consisting of an amino acid sequence between positions 121 and 133 according to the EU index in a non-native human IgG CH1 domain, where the amino acid sequence differs from the corresponding sequence in a native human IgG of the same isotype as the non-native human IgG. The present application also provides such peptides. The present application also provides nucleic acids encoding the peptides.

In the present application, "the amino acid sequence between positions 121 and 133 according to the EU index in a non-native human IgG CH1 domain" is used interchangeably with "an amino acid sequence in which one or more amino acids have been modified in the amino acid sequence between positions 121 and 133 according to the EU index of a native human IgG of the same isotype as the non-native human IgG".

In the present application, "a peptide consisting of the amino acid sequence between positions 121 and 133 according to the EU index in a non-native human IgG CH1 domain, where the amino acid sequence differs from the corresponding sequence in a native human IgG of the same isotype as the non-native human IgG" is used interchangeably with "a peptide consisting of an amino acid sequence in which one or more amino acids have been modified in the amino acid sequence between positions 121 and 133 according to the EU index of a native human IgG of the same isotype as the non-native human IgG".

In the present application, "a peptide consisting of" is used interchangeably with "a peptide consisting only of".

In the method of the present application, the multiple types of peptides include a set including two or more types of peptides consisting of amino acid sequences between positions 121 and 133 according to the EU index in a native or non-native human IgG CH1 domain, where the amino acid sequences differ between said two or more types of peptides. The present application also provides such a set of peptides. The present application also provides nucleic acids encoding the set of peptides.

In the present application, multiple types of peptides (*i.e.*, a set of peptides) can be the following peptides:
(I) multiple types of peptides including two or more types of peptides consisting of an amino acid sequence between positions 121 and 133 according to the EU index in a non-native human IgG CH1 domain, wherein the amino acid sequence differs from the corresponding sequence in a native human IgG of the same isotype as the non-native human IgG; or
(II) multiple types of peptides including a peptide consisting of an amino acid sequence between positions 121 and 133 according to the EU index in a native human IgG CH1 domain and one or more types of peptides consisting of an amino acid sequence between positions 121 and 133 according to the EU index in a non-native human IgG CH1 domain, wherein the amino acid sequence differs from the corresponding sequence in a native human IgG of the same isotype as the non-native human IgG.

The antigen-binding molecule in the method of the present application includes an antigen-binding molecule comprising an antigen-binding domain, a CL domain, and a non-native human IgG CH1 domain, wherein the amino acid sequence between positions 121 and 133 according to the EU index in the non-native human IgG CH1 domain differs from the corresponding sequence in a native human IgG of the same isotype as the non-native human IgG. The present application also provides such antigen-binding molecules. The present application also provides nucleic acids encoding the antigen-binding molecules.

In the present application, examples of the antigen-binding domain include a VH domain and/or a VL domain. In the present application, the VH domain and the VL domain may be, for example, humanized domains. Furthermore, in the present application, for example, the C-terminus of the VH domain may be linked to the N-terminus of the non-native human IgG CH1 domain, and the C-terminus of the VL domain may be linked to the N-terminus of the CL domain, or the C-terminus of the VL domain may be linked to the N-terminus of the non-native human IgG CH1 domain, and the C-terminus of the VH domain may be linked to the N-terminus of the CL domain.

The antigen-binding molecule of the present application can further comprise an antibody Fc region. Such an antigen-binding molecule can also be referred to as an Fc region-containing antigen-binding molecule. Examples of the Fc region-containing antigen-binding molecules include, but are not limited to, Fc region-containing antibodies.

The antigen-binding molecule of the present application can be, for example, a non-native IgG antibody. Examples of the non-native IgG antibodies include a non-native IgG1 antibody, a non-native IgG2 antibody, a non-native IgG3 antibody, and a non-native IgG4 antibody. In the present application, examples of the native IgG antibodies include a native IgG1 antibody, a native IgG2 antibody, a native IgG3 antibody, and a native IgG4 antibody.

The antigen-binding molecule of the present application may be conjugated to a drug moiety. Such substances are referred to as antigen-binding molecule-drug conjugates, and encompass antibody-drug conjugates (*i.e.*, immunoconjugates). In the present application, the drug moiety includes any compounds, portions, or groups that have cellular toxic, cytotoxic, or cytostatic effects. The drug moiety may exert cellular toxic, cytotoxic, or cytostatic effects through mechanisms including tubulin binding, DNA binding or intercalation, and inhibition of RNA polymerase, protein synthesis, and topoisomerase, although these mechanisms should be understood as examples. Certain cytotoxic agents tend to be inactive or have reduced activity when conjugated to a large antibody or protein receptor ligand. Examples of the drug moiety include, but are not limited to, maytansinoids, dolastatins, auristatins, calicheamicins, pyrrolobenzodiazepines (PBDs), PNU-159682, anthracyclines, duocarmycins, vinca alkaloids, taxanes, trichothecenes, CC1065, duocarmycins, camptothecins, elinafides, and stereoisomers, isosteres, analogs, or derivatives thereof.

In the present application, "antigen-binding molecule comprising an antigen-binding domain, a CL domain, and a non-native human IgG CH1 domain, wherein the amino acid sequence between positions 121 and 133 according to the EU index in the non-native human IgG CH1 domain differs from the corresponding sequence in a native human IgG of the same isotype as the non-native human IgG" is used interchangeably with "antigen-binding molecule comprising an antigen-binding domain, a CL domain, and a non-native human IgG CH1 domain, which comprises an amino acid sequence in which one or more amino acids have been modified in the amino acid sequence between positions 121 and 133 according to the EU index in a native human IgG of the same isotype as the non-native human IgG".

In the present application, "more" in "the amino acid sequence in which one or more amino acids have been modified" includes, but is not limited to, 10 or less, 9 or less, 8 or less, 7 or less, 6 or less, 5 or less, 4 or less, 3 or less, 2 or less, or 1 or less, or 1 to 10, 1 to 9, 1 to 8, 1 to 7, 1 to 6, 1 to 5, 1 to 4, 1 to 3, or 1 to 2. In addition, in the present application, "amino acid" is used interchangeably with "amino acid residue".

In the present application, "modification" in "the amino acid sequence in which one or more amino acids have been modified" includes substitution, deletion, insertion, or addition, without limitation as long as the modified amino acid sequence is specific to the antigen-binding molecule and the peptide consisting of the amino acid sequence is detectable by mass spectrometry. "[S]equence specific to an antigen-binding molecule" is used interchangeably with "sequence peculiar to an antigen-binding molecule" or "sequence unique to an antigen-binding molecule".

In one embodiment, "modification" in "amino acid sequence in which one or more amino acids have been modified" can include a modification after which the properties, functions, and/or pharmacokinetics (PK) of the antigen-binding molecule are maintained to a certain extent, but is not limited thereto.

Examples of the properties include, but are not limited to, physicochemical properties such as Tm values, and pharmacokinetics. In the present application, a Tm value means a thermal denaturation midpoint temperature. Tm values can be measured using, for example, a differential scanning fluorimetry (DSF) method (J Pharm Sci. 2013 Aug;102(8):2471-83). Examples of the Tm value of the modified antigen-binding molecule include 40°C or higher, 45°C or higher, 50°C or higher, 55°C or higher, 60°C or higher, 65°C or higher, and 70°C or higher, preferably 50°C or higher. Examples of the Tm value of the modified antigen-binding molecule include Tm values that are different from the Tm value of the antigen-binding molecule before modification by 20°C or less, 15°C or less, 10°C or less, 9°C or less, 8°C or less, 7°C or less, 6°C or less, 5°C or less, 4°C or less, 3°C or less, 2°C or less, and 1°C or less. Examples of the Tm value of the antigen-binding molecule after modification include, but are not limited to, the Tm values listed in Tables 5-1 to 5-9.

Examples of the functions include, but are not limited to, antigen-binding activity. The antigen-binding activity of the modified antigen-binding molecule can be 10% or more, 20% or more, 30% or more, 40% or more, 50% or more, 60% or more, 70% or more, 80% or more, or 90% or more, preferably 50% or more of the antigen-binding activity of the antigen-binding molecule before modification, and can be 200% or less, 190% or less, 180% or less, 170% or less, 160% or less, 150% or less, 140% or less, 130% or less, 120% or less, or 110% or less of the antigen-binding activity of the antigen-binding molecule before modification. Antigen-binding activity can be measured using, for example, Biacore^{™} T200 (Cytiva).

In the present application, "pharmacokinetics" (also referred to as PK) refers to changes in the concentration (amount) of a drug *in vivo* through a series of *in vivo* processes including absorption, distribution, metabolism, and excretion after administration of the drug.

After a drug is administered, the processes of absorption, distribution, metabolism, and excretion proceed in parallel *in vivo*. The following basic pharmacokinetic parameters have been established to describe these processes separately: (1) bioavailability (F), (2) volume of distribution (Vd or V), (3) fraction unbound in blood (fuB), (4) clearance (CL), and (5) cumulative amount of drug excreted in urine (Ae) (Biometrics Vol. 36, Special Issue, S 3-S 18 (2015)). The area under the blood concentration-time curve (AUC), maximum blood concentration (Cmax), time to maximum blood concentration (Tmax), and the like are known as indices of bioavailability. The steady-state volume of distribution (V ss) and the like are known as indices of the volume of distribution. Other known pharmacokinetic parameters include blood concentration half-life (t 1/2) and mean residence time (MRT).

The pharmacokinetic profile and pharmacokinetic parameters of the modified antigen-binding molecule are preferably equivalent to those of the antigen-binding molecule before modification.

"Equivalent" pharmacokinetic parameter means that at least one pharmacokinetic parameter of the modified antigen-binding molecule is within the range of 50% to 100%, within the range of 60% to 100%, within the range of 70% to 100%, within the range of 80% to 100%, within the range of 90% to 100%, within the range of 100% to 110%, within the range of 100% to 120%, within the range of 100% to 130%, within the range of 100% to 140%, or within the range of 100% to 150% of that pharmacokinetic parameter of the antigen-binding molecule before modification.

"Equivalent" pharmacokinetic profile means that at least two pharmacokinetic parameters of the modified antigen-binding molecule are within the range of 50% to 100%, within the range of 60% to 100%, within the range of 70% to 100%, within the range of 80% to 100%, within the range of 90% to 100%, within the range of 100% to 110%, within the range of 100% to 120%, within the range of 100% to 130%, within the range of 100% to 140%, or within the range of 100% to 150% of the same pharmacokinetic parameters of the antigen-binding molecule before modification, respectively.

The method of the present application can be used in evaluation of pharmacokinetics. Evaluation of pharmacokinetics can be appropriately performed according to known methods such as "Guidelines for Non-Clinical Pharmacokinetic Studies" (Iyakushin No. 496, June 26, 1998), or can be performed according to the Examples of the present application.

In the present application, "the amino acid sequence between positions X and Y according to the EU index" does not include the amino acids at positions X and Y. Therefore, "the amino acid sequence between positions X and Y according to the EU index" is used interchangeably with "the amino acid sequence between positions X and Y according to the EU index (not including positions X and Y)" or "the amino acid sequence from the site adjacent to the C-terminus side of position X to the site adjacent to the N-terminus side of position Y according to the EU index". The amino acid sequence from the site adjacent to the C-terminus side of position X to the site adjacent to the N-terminus side of position Y according to the EU index includes the amino acid at the site adjacent to the C-terminus side of position X and the amino acid at the site adjacent to the N-terminus side of position Y. Furthermore, the site adjacent to the C-terminus side of position X according to the EU index does not necessarily mean position X+1 (X plus 1) according to the EU index. In addition, the site adjacent to the N-terminus side of position Y according to the EU index does not necessarily mean position Y-1 (Y minus 1) according to the EU index.

For example, "the amino acid sequence between positions 121 and 133 according to the EU index" is used interchangeably with "the amino acid sequence between positions 121 and 133 according to the EU index (not including positions 121 and 133)" or "the amino acid sequence from the site adjacent to the C-terminus side of position 121 according to the EU index to the site adjacent to the N-terminus side of position 133 according to the EU index". The site adjacent to the C-terminus side of position 121 according to the EU index means position 122 according to the EU index if there is no amino acid insertion between positions 121 and 122 according to the EU index, whereas if there is an amino acid insertion, it does not mean position 122 according to the EU index but rather means the site adjacent to the C-terminus side of position 121 according to the EU index where the amino acid has been inserted. Furthermore, the site adjacent to the N-terminus side of position 133 according to the EU index means position 132 according to the EU index if there is no amino acid insertion between positions 132 and 133, whereas if there is an amino acid insertion, it does not mean position 132 according to the EU index but rather means the site adjacent to the N-terminus side of position 133 according to the EU index where the amino acid has been inserted.

In the present application, the amino acid sequence between positions 121 and 133 according to the EU index in a non-native human IgG CH1 domain has, for example, any characteristic selected from the following characteristics:
(1) an amino acid at any one or more sites of positions 122 to 132 is different from an amino acid present at corresponding sites in a native human IgG of the same isotype, and the amino acid at said sites is an amino acid other than Lys, Arg, and Met (provided that the amino acid at position 122 is not Pro);
(2) an amino acid other than Lys, Arg, and Met is inserted at one or more sites selected from a site between positions 121 and 122, a site between positions 122 and 123, a site between positions 123 and 124, a site between positions 124 and 125, a site between positions 125 and 126, a site between positions 126 and 127, a site between positions 127 and 128, a site between positions 128 and 129, a site between positions 129 and 130, a site between positions 130 and 131, a site between positions 131 and 132, and a site between positions 132 and 133 (provided that the amino acid at the site between positions 121 and 122 is not Pro).

In the present application, 1 to 10 (*e.g.*, 1, 2, 3, 4, 5, 6, 7, 8, 9, or 10) amino acids can be inserted per site.

In the present application, "an amino acid at any one or more sites of positions 122 to 132 is different from an amino acid present at corresponding sites in a native human IgG of the same isotype, and the amino acid at said sites is an amino acid other than Lys, Arg, and Met (provided that the amino acid at position 122 is not Pro)" is used interchangeably with "for an amino acid at any one or more sites of positions 122 to 132, an amino acid present at corresponding sites in a native human IgG of the same isotype is substituted with an amino acid other than Lys, Arg, and Met (provided that the amino acid at position 122 is not substituted with Pro)" or "an amino acid at any one or more sites of positions 122 to 132 is substituted with an amino acid other than Lys, Arg, and Met (provided that the amino acid at position 122 is not substituted with Pro)".

In the present application, the amino acid sequence between positions 121 and 133 according to the EU index in the non-native human IgG CH1 domain has, for example, any characteristic selected from the following characteristics:
(1) an amino acid at any one or more sites of positions 122 to 128 and positions 130 to 132 is Ala;
(2) an amino acid at any one or more sites of positions 122 to 132 is Ile;
(3) an amino acid at any one or more sites of positions 122 to 124 and positions 126 to 132 is Val;
(4) an amino acid at any one or more sites of positions 122 to 127 and positions 129 to 132 is Leu;
(5) an amino acid at any one or more sites of positions 122 to 125 and positions 127 to 132 is Phe;
(6) Ala, Ile, Val, Leu, Phe, Ala-Ala, Ile-Ile, Val-Val, Leu-Leu, Phe-Phe, Ala-Ile, Ala-Val, Ala-Leu, Ala-Phe, Ile-Val, Ile-Leu, Ile-Phe, Val-Leu, Val-Phe, Leu-Phe, or Ala-Ala-Ala is inserted at one or more sites selected from a site between positions 121 and 122, a site between positions 122 and 123, a site between positions 123 and 124, a site between positions 124 and 125, a site between positions 125 and 126, a site between positions 126 and 127, a site between positions 127 and 128, a site between positions 128 and 129, a site between positions 129 and 130, a site between positions 130 and 131, a site between positions 131 and 132, and a site between positions 132 and 133.

In the present application, "an amino acid at one or more sites is X" is used interchangeably with "an amino acid at one or more sites is substituted with X". For example, "an amino acid at any one or more sites of positions 122 to 128 and positions 130 to 132 is Ala" is used interchangeably with "an amino acid at any one or more sites of positions 122 to 128 and positions 130 to 132 is substituted with Ala".

In the present application, "X-Y is inserted" means that X and Y are inserted in order from the N-terminus side. For example, "Ala-Ala is inserted" means that Ala and Ala are inserted in order from the N-terminus side. In addition, in the present application, "X-Y-Z is inserted" means that X, Y, and Z are inserted in order from the N-terminus side (the same applies when a larger number of amino acids are inserted). For example, "Ala-Ala-Ala is inserted" means that Ala, Ala, and Ala are inserted in order from the N-terminus side. In the present application, "in order from the N-terminus side" is used interchangeably with "in the N-terminal to C-terminal direction", "in order from the N-terminal to C-terminal direction", or "linked in the N-terminal to C-terminal direction".

In the present application, the non-native human IgG CH1 domain includes a non-native human IgG1 CH1 domain, a non-native human IgG2 CH1 domain, a non-native human IgG3 CH1 domain, and a non-native human IgG4 CH1 domain.

In the present application, the amino acid sequence between positions 121 and 133 according to the EU index in a non-native human IgG1 CH1 domain includes, but is not limited to, the amino acid sequence of any one of SEQ ID NOs: 6 to 294. Furthermore, in the present application, the amino acid sequence between positions 121 and 133 according to the EU index in a non-native human IgG2 CH1 domain, a non-native human IgG3 CH1 domain, or a non-native human IgG4 CH1 domain includes, but is not limited to, the amino acid sequence of any one of SEQ ID NOs: 1565 to 1833.

In the present application, the amino acid sequence between positions 121 and 133 according to the EU index in a native human IgG1 CH1 domain includes, but is not limited to, the amino acid sequence of SEQ ID NO: 5. Furthermore, in the present application, the amino acid sequence between positions 121 and 133 according to the EU index in a native human IgG2 CH1 domain, a native human IgG3 CH1 domain, or a native human IgG4 CH1 domain includes, but is not limited to, the amino acid sequence of SEQ ID NO: 945.

Examples of the set of peptides of the present application include, but are not limited to, the following sets:
(1) a set comprising two or more types of peptides consisting of an amino acid sequence between positions 121 and 133 according to the EU index in a non-native human IgG CH1 domain, wherein the amino acid sequence between positions 121 and 133 according to the EU index in the non-native human IgG CH1 domain is selected from the group consisting of SEQ ID NOs: 6 to 294 and 1565 to 1833;
(2) a set comprising two or more types of peptides consisting of an amino acid sequence between positions 121 and 133 according to the EU index in a non-native human IgG CH1 domain, wherein the amino acid sequence between positions 121 and 133 according to the EU index in the non-native human IgG CH1 domain is selected from the group consisting of SEQ ID NOs: 6 to 294;
(3) a set comprising two or more types of peptides consisting of an amino acid sequence between positions 121 and 133 according to the EU index in a non-native human IgG CH1 domain, wherein the amino acid sequences between positions 121 and 133 according to the EU index in the non-native human IgG CH1 domain is selected from the group consisting of SEQ ID NOs: 1565 to 1833;
(4) a set comprising a peptide consisting of an amino acid sequence between positions 121 and 133 according to the EU index in a native human IgG CH1 domain and one or more types of peptides consisting of an amino acid sequence between positions 121 and 133 according to the EU index in a non-native human IgG CH1 domain, wherein the amino acid sequence between positions 121 and 133 according to the EU index in the native human IgG CH1 domain is SEQ ID NO: 5, and the amino acid sequence between positions 121 and 133 according to the EU index in the non-native human IgG CH1 domain is selected from the group consisting of SEQ ID NOs: 6 to 294;
(5) a set comprising a peptide consisting of an amino acid sequence between positions 121 and 133 according to the EU index in a native human IgG CH1 domain and one or more types of peptides consisting of an amino acid sequence between positions 121 and 133 according to the EU index in a non-native human IgG CH1 domain, wherein the amino acid sequence between positions 121 and 133 according to the EU index in the native human IgG CH1 domain is SEQ ID NO: 945, and the amino acid sequence between positions 121 and 133 according to the EU index in the non-native human IgG CH1 domain is selected from the group consisting of SEQ ID NOs: 1565 to 1833.

In the present application, "a method for detecting each of multiple types of peptides in a sample", "a method for quantifying each of multiple types of peptides in a sample", and "a method for detecting and/or quantifying each of multiple types of antigen-binding molecules in a biological sample" include a method for simultaneously detecting and/or quantifying each of multiple types of peptides or each of multiple types of antigen-binding molecules.

A method for simultaneously detecting each of multiple types of peptides from a sample comprising these peptides can be performed using tandem mass spectrometry in selected reaction monitoring (SRM) measurement mode. SRM transitions need to be different for each peptide to be detected. By simultaneously monitoring multiple SRM transitions, multiple types of target peptides can be analyzed concurrently.

In the present application, examples of a set of peptides with different SRM transitions that can be used when detecting each of multiple types of peptides from one sample include, but are not limited to, a set of two or more types (*e.g.*, 2, 3, 4, 5, 6, 7, 8, 9, 10, 11, 12, or 13 types) of peptides selected from the group consisting of (1) to (13) below, a set of 12 types of peptides of (1) to (4) and (6) to (13) below, a set of 12 types of peptides of (1), (2), and (4) to (13) below, or a set of 13 types of peptides of (1) to (13) below:
(1) a peptide consisting of the amino acid sequence of SEQ ID NO:5;
(2) a peptide consisting of the amino acid sequence of SEQ ID NO:6;
(3) a peptide consisting of the amino acid sequence of SEQ ID NO:7;
(4) a peptide consisting of the amino acid sequence of SEQ ID NO:8;
(5) a peptide consisting of the amino acid sequence of SEQ ID NO:9;
(6) a peptide consisting of the amino acid sequence of SEQ ID NO:10;
(7) a peptide consisting of the amino acid sequence of SEQ ID NO:12;
(8) a peptide consisting of the amino acid sequence of SEQ ID NO:13;
(9) a peptide consisting of the amino acid sequence of SEQ ID NO:14;
(10) a peptide consisting of the amino acid sequence of SEQ ID NO:17;
(11) a peptide consisting of the amino acid sequence of SEQ ID NO:24;
(12) a peptide consisting of the amino acid sequence of SEQ ID NO:27; and
(13) a peptide consisting of the amino acid sequence of SEQ ID NO:36.

These peptides have precursor ion m/z values different from each other, or have different product ion m/z values when they have the same precursor ion m/z value, and therefore, can be detected separately from the same sample by mass spectrometry, especially mass spectrometry in SRM measurement mode.

Furthermore, these peptides have a property of being excised in sufficient amount upon trypsin treatment of antigen-binding molecules comprising IgG antibody CH1, and being ionized sufficiently for mass spectrometry. That is, they have sufficient sensitivity in mass spectrometry.

These peptides are not comprised in proteins naturally occurring in non-human animals (*e.g.*, mice, rats, rabbits, and monkeys) used in pharmacokinetic studies. In other words, the mass spectrometry peaks of these peptides do not interfere with the mass spectrometry peak of a peptide derived from a non-human animal used in pharmacokinetic studies that is excised upon trypsin treatment of a biological sample (blank matrix) derived from the animal. In other words, these peptides are specifically derived from the antigen-binding molecules, and thus, suitable for detecting the antigen-binding molecules in pharmacokinetic studies, particularly in cassette dosing studies.

Furthermore, among these peptides, antigen-binding molecules comprising peptides consisting of the amino acid sequences of SEQ ID NOs: 5, 7, 12, 10, 17, 27, 13, 14, 24, 36, 6, and 8 have a lower limit of quantitation (LLOQ) in mass spectrometry that is lower than antigen-binding molecules comprising other peptides, and are suitable for cassette dosing studies. Antigen-binding molecules comprising peptides consisting of the amino acid sequences of SEQ ID NOs: 5, 7, 12, 10, 17, 27, 13, 14, 24, and 36 have a lower limit of quantitation (LLOQ) in mass spectrometry that is even lower, and are suitable for cassette dosing studies. Antigen-binding molecules comprising peptides consisting of the amino acid sequences of SEQ ID NOs: 7, 12, 10, 17, and 27 have a lower limit of quantitation (LLOQ) in mass spectrometry that is the lowest, and are suitable for cassette dosing studies. The peptide consisting of the amino acid sequence of SEQ ID NO: 5 is present in the constant region of native IgG1 and can be used without modification from the native sequence, and thus, the antigen-binding molecule comprising this peptide is suitable for cassette dosing studies.

Preferred embodiments of a set of two types of peptides selected from the group consisting of (1) to (13) above include, but are not limited to, the following:
- a set of a peptide consisting of the amino acid sequence of SEQ ID NO: 5 and a peptide consisting of the amino acid sequence of SEQ ID NO: 7;
- a set of a peptide consisting of the amino acid sequence of SEQ ID NO: 5 and a peptide consisting of the amino acid sequence of SEQ ID NO: 12;
- a set of a peptide consisting of the amino acid sequence of SEQ ID NO: 5 and a peptide consisting of the amino acid sequence of SEQ ID NO: 10;
- a set of a peptide consisting of the amino acid sequence of SEQ ID NO: 5 and a peptide consisting of the amino acid sequence of SEQ ID NO: 17;
- a set of a peptide consisting of the amino acid sequence of SEQ ID NO: 7 and a peptide consisting of the amino acid sequence of SEQ ID NO: 12;
- a set of a peptide consisting of the amino acid sequence of SEQ ID NO: 7 and a peptide consisting of the amino acid sequence of SEQ ID NO: 10;
- a set of a peptide consisting of the amino acid sequence of SEQ ID NO: 7 and a peptide consisting of the amino acid sequence of SEQ ID NO: 17;
- a set of a peptide consisting of the amino acid sequence of SEQ ID NO: 12 and a peptide consisting of the amino acid sequence of SEQ ID NO: 10;
- a set of a peptide consisting of the amino acid sequence of SEQ ID NO: 12 and a peptide consisting of the amino acid sequence of SEQ ID NO: 17;
- a set of a peptide consisting of the amino acid sequence of SEQ ID NO: 10 and a peptide consisting of the amino acid sequence of SEQ ID NO: 17;
- a set of a peptide consisting of the amino acid sequence of SEQ ID NO: 5 and a peptide consisting of the amino acid sequence of SEQ ID NO: 27;
- a set of a peptide consisting of the amino acid sequence of SEQ ID NO: 7 and a peptide consisting of the amino acid sequence of SEQ ID NO: 27;
- a set of a peptide consisting of the amino acid sequence of SEQ ID NO: 10 and a peptide consisting of the amino acid sequence of SEQ ID NO: 27;
- a set of a peptide consisting of the amino acid sequence of SEQ ID NO: 12 and a peptide consisting of the amino acid sequence of SEQ ID NO: 27; or
- a set of a peptide consisting of the amino acid sequence of SEQ ID NO: 17 and a peptide consisting of the amino acid sequence of SEQ ID NO: 27.

Furthermore, preferred embodiments of a set of three types of peptides selected from the group consisting of (1) to (13) above include, but are not limited to, the following:
- a set of a peptide consisting of the amino acid sequence of SEQ ID NO: 5, a peptide consisting of the amino acid sequence of SEQ ID NO: 7, and a peptide consisting of the amino acid sequence of SEQ ID NO: 12;
- a set of a peptide consisting of the amino acid sequence of SEQ ID NO: 5, a peptide consisting of the amino acid sequence of SEQ ID NO: 7, and a peptide consisting of the amino acid sequence of SEQ ID NO: 10;
- a set of a peptide consisting of the amino acid sequence of SEQ ID NO: 5, a peptide consisting of the amino acid sequence of SEQ ID NO: 7, and a peptide consisting of the amino acid sequence of SEQ ID NO: 17;
- a set of a peptide consisting of the amino acid sequence of SEQ ID NO: 5, a peptide consisting of the amino acid sequence of SEQ ID NO: 12, and a peptide consisting of the amino acid sequence of SEQ ID NO: 10;
- a set of a peptide consisting of the amino acid sequence of SEQ ID NO: 5, a peptide consisting of the amino acid sequence of SEQ ID NO: 12, and a peptide consisting of the amino acid sequence of SEQ ID NO: 17;
- a set of a peptide consisting of the amino acid sequence of SEQ ID NO: 5, a peptide consisting of the amino acid sequence of SEQ ID NO: 10, and a peptide consisting of the amino acid sequence of SEQ ID NO: 17;
- a set of a peptide consisting of the amino acid sequence of SEQ ID NO: 7, a peptide consisting of the amino acid sequence of SEQ ID NO: 12, and a peptide consisting of the amino acid sequence of SEQ ID NO: 10;
- a set of a peptide consisting of the amino acid sequence of SEQ ID NO: 7, a peptide consisting of the amino acid sequence of SEQ ID NO: 12, and a peptide consisting of the amino acid sequence of SEQ ID NO: 17;
- a set of a peptide consisting of the amino acid sequence of SEQ ID NO: 12, a peptide consisting of the amino acid sequence of SEQ ID NO: 10, and a peptide consisting of the amino acid sequence of SEQ ID NO: 17;
- a set of a peptide consisting of the amino acid sequence of SEQ ID NO: 7, a peptide consisting of the amino acid sequence of SEQ ID NO: 10, and a peptide consisting of the amino acid sequence of SEQ ID NO: 17;
- a set of a peptide consisting of the amino acid sequence of SEQ ID NO: 5, a peptide consisting of the amino acid sequence of SEQ ID NO: 7, and a peptide consisting of the amino acid sequence of SEQ ID NO: 27;
- a set of a peptide consisting of the amino acid sequence of SEQ ID NO: 5, a peptide consisting of the amino acid sequence of SEQ ID NO: 12, and a peptide consisting of the amino acid sequence of SEQ ID NO: 27;
- a set of a peptide consisting of the amino acid sequence of SEQ ID NO: 5, a peptide consisting of the amino acid sequence of SEQ ID NO:10, and a peptide consisting of the amino acid sequence of SEQ ID NO: 27;
- a set of a peptide consisting of the amino acid sequence of SEQ ID NO: 5, a peptide consisting of the amino acid sequence of SEQ ID NO: 17, and a peptide consisting of the amino acid sequence of SEQ ID NO: 27;
- a set of a peptide consisting of the amino acid sequence of SEQ ID NO: 7, a peptide consisting of the amino acid sequence of SEQ ID NO: 10, and a peptide consisting of the amino acid sequence of SEQ ID NO: 27;
- a set of a peptide consisting of the amino acid sequence of SEQ ID NO: 7, a peptide consisting of the amino acid sequence of SEQ ID NO: 12, and a peptide consisting of the amino acid sequence of SEQ ID NO: 27;
- a set of a peptide consisting of the amino acid sequence of SEQ ID NO: 7, a peptide consisting of the amino acid sequence of SEQ ID NO: 17, and a peptide consisting of the amino acid sequence of SEQ ID NO: 27;
- a set of a peptide consisting of the amino acid sequence of SEQ ID NO: 12, a peptide consisting of the amino acid sequence of SEQ ID NO: 10, and a peptide consisting of the amino acid sequence of SEQ ID NO: 27;
- a set of a peptide consisting of the amino acid sequence of SEQ ID NO: 10, a peptide consisting of the amino acid sequence of SEQ ID NO: 17, and a peptide consisting of the amino acid sequence of SEQ ID NO: 27; or
- a set of a peptide consisting of the amino acid sequence of SEQ ID NO: 12, a peptide consisting of the amino acid sequence of SEQ ID NO: 17, and a peptide consisting of the amino acid sequence of SEQ ID NO: 27.

Furthermore, preferred embodiments of a set of four types of peptides selected from the group consisting of (1) to (13) above include, but are not limited to, the following:
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, and a peptide consisting of the amino acid sequence of SEQ ID NO: 10;
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, and a peptide consisting of the amino acid sequence of SEQ ID NO: 17;
- a set of a peptide consisting of the amino acid sequence of SEQ ID NO: 5, a peptide consisting of the amino acid sequence of SEQ ID NO: 12, a peptide consisting of the amino acid sequence of SEQ ID NO: 10, and a peptide consisting of the amino acid sequence of SEQ ID NO: 17;
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 10, and a peptide consisting of the amino acid sequence of SEQ ID NO: 17;
- a set of a peptide consisting of the amino acid sequence of SEQ ID NO: 7, a peptide consisting of the amino acid sequence of SEQ ID NO: 12, a peptide consisting of the amino acid sequence of SEQ ID NO: 10, and a peptide consisting of the amino acid sequence of SEQ ID NO: 17;
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 10, and a peptide consisting of the amino acid sequence of SEQ ID NO: 27;
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, and a peptide consisting of the amino acid sequence of SEQ ID NO: 27;
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 17, and a peptide consisting of the amino acid sequence of SEQ ID NO: 27;
- a set of a peptide consisting of the amino acid sequence of SEQ ID NO: 5, a peptide consisting of the amino acid sequence of SEQ ID NO: 10, a peptide consisting of the amino acid sequence of SEQ ID NO: 12, and a peptide consisting of the amino acid sequence of SEQ ID NO: 27;
- a set of a peptide consisting of the amino acid sequence of SEQ ID NO: 5, a peptide consisting of the amino acid sequence of SEQ ID NO: 10, a peptide consisting of the amino acid sequence of SEQ ID NO: 17, and a peptide consisting of the amino acid sequence of SEQ ID NO: 27;
- a set of a peptide consisting of the amino acid sequence of SEQ ID NO: 5, a peptide consisting of the amino acid sequence of SEQ ID NO: 12, a peptide consisting of the amino acid sequence of SEQ ID NO: 17, and a peptide consisting of the amino acid sequence of SEQ ID NO: 27;
- a set of a peptide consisting of the amino acid sequence of SEQ ID NO: 7, a peptide consisting of the amino acid sequence of SEQ ID NO: 10, a peptide consisting of the amino acid sequence of SEQ ID NO: 12, and a peptide consisting of the amino acid sequence of SEQ ID NO: 27;
- a set of a peptide consisting of the amino acid sequence of SEQ ID NO: 7, a peptide consisting of the amino acid sequence of SEQ ID NO: 10, a peptide consisting of the amino acid sequence of SEQ ID NO: 17, and a peptide consisting of the amino acid sequence of SEQ ID NO: 27;
- a set of a peptide consisting of the amino acid sequence of SEQ ID NO: 7, a peptide consisting of the amino acid sequence of SEQ ID NO: 12, a peptide consisting of the amino acid sequence of SEQ ID NO: 17, and a peptide consisting of the amino acid sequence of SEQ ID NO: 27; or
- a set of a peptide consisting of the amino acid sequence of SEQ ID NO: 10, a peptide consisting of the amino acid sequence of SEQ ID NO: 12, a peptide consisting of the amino acid sequence of SEQ ID NO: 17, and a peptide consisting of the amino acid sequence of SEQ ID NO: 27.

Furthermore, preferred embodiments of a set of five types of peptides selected from the group consisting of (1) to (13) above include, but are not limited to, the following:
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, and a peptide consisting of the amino acid sequence of SEQ ID NO: 17;
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 10, a peptide consisting of the amino acid sequence of SEQ ID NO: 12, and a peptide consisting of the amino acid sequence of SEQ ID NO: 27;
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 10, a peptide consisting of the amino acid sequence of SEQ ID NO: 17, and a peptide consisting of the amino acid sequence of SEQ ID NO: 27;
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 17, and a peptide consisting of the amino acid sequence of SEQ ID NO: 27;
- a set of a peptide consisting of the amino acid sequence of SEQ ID NO: 5, a peptide consisting of the amino acid sequence of SEQ ID NO: 10, a peptide consisting of the amino acid sequence of SEQ ID NO: 12, a peptide consisting of the amino acid sequence of SEQ ID NO: 17, and a peptide consisting of the amino acid sequence of SEQ ID NO: 27; or
- a set of a peptide consisting of the amino acid sequence of SEQ ID NO: 7, a peptide consisting of the amino acid sequence of SEQ ID NO: 10, a peptide consisting of the amino acid sequence of SEQ ID NO: 12, a peptide consisting of the amino acid sequence of SEQ ID NO: 17, and a peptide consisting of the amino acid sequence of SEQ ID NO: 27.

Furthermore, preferred embodiments of a set of six types of peptides selected from the group consisting of (1) to (13) above include, but are not limited to, a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, and a peptide consisting of the amino acid sequence of SEQ ID NO: 27.

Furthermore, preferred embodiments of a set of seven types of peptides selected from the group consisting of (1) to (13) above include, but are not limited to, the following:
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, and a peptide consisting of the amino acid sequence of SEQ ID NO: 13;
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, and a peptide consisting of the amino acid sequence of SEQ ID NO: 14;
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, and a peptide consisting of the amino acid sequence of SEQ ID NO: 24; or
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, and a peptide consisting of the amino acid sequence of SEQ ID NO: 36.

Furthermore, preferred embodiments of a set of eight types of peptides selected from the group consisting of (1) to (13) above include, but are not limited to, the following:
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, and a peptide consisting of the amino acid sequence of SEQ ID NO: 14;
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, and a peptide consisting of the amino acid sequence of SEQ ID NO: 24;
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, and a peptide consisting of the amino acid sequence of SEQ ID NO: 36;
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 14, and a peptide consisting of the amino acid sequence of SEQ ID NO: 24;
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 14, and a peptide consisting of the amino acid sequence of SEQ ID NO: 36; or
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 24, and a peptide consisting of the amino acid sequence of SEQ ID NO: 36.

Furthermore, preferred embodiments of a set of nine types of peptides selected from the group consisting of (1) to (13) above include, but are not limited to, the following:
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, a peptide consisting of the amino acid sequence of SEQ ID NO: 14, and a peptide consisting of the amino acid sequence of SEQ ID NO: 24;
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, a peptide consisting of the amino acid sequence of SEQ ID NO: 14, and a peptide consisting of the amino acid sequence of SEQ ID NO: 36;
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, a peptide consisting of the amino acid sequence of SEQ ID NO: 24, and a peptide consisting of the amino acid sequence of SEQ ID NO: 36; or
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 14, a peptide consisting of the amino acid sequence of SEQ ID NO: 24, and a peptide consisting of the amino acid sequence of SEQ ID NO: 36.

Furthermore, preferred embodiments of a set of ten types of peptides selected from the group consisting of (1) to (13) above include, but are not limited to, a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, a peptide consisting of the amino acid sequence of SEQ ID NO: 14, a peptide consisting of the amino acid sequence of SEQ ID NO: 24, and a peptide consisting of the amino acid sequence of SEQ ID NO: 36.

Furthermore, preferred embodiments of a set of eleven types of peptides selected from the group consisting of (1) to (13) above include, but are not limited to, the following:
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, a peptide consisting of the amino acid sequence of SEQ ID NO: 14, a peptide consisting of the amino acid sequence of SEQ ID NO: 24, and a peptide consisting of the amino acid sequence of SEQ ID NO: 36, and a peptide consisting of the amino acid sequence of SEQ ID NO: 6; or
- a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, a peptide consisting of the amino acid sequence of SEQ ID NO: 14, a peptide consisting of the amino acid sequence of SEQ ID NO: 24, and a peptide consisting of the amino acid sequence of SEQ ID NO: 36, and a peptide consisting of the amino acid sequence of SEQ ID NO: 8.

Furthermore, preferred embodiments of a set of twelve types of peptides selected from the group consisting of (1) to (13) above include, but are not limited to, a set of a peptide consisting of the amino acid sequence of SEQ ID NO: 5, a peptide consisting of the amino acid sequence of SEQ ID NO: 7, a peptide consisting of the amino acid sequence of SEQ ID NO: 12, a peptide consisting of the amino acid sequence of SEQ ID NO: 10, a peptide consisting of the amino acid sequence of SEQ ID NO: 17, a peptide consisting of the amino acid sequence of SEQ ID NO: 27, a peptide consisting of the amino acid sequence of SEQ ID NO: 13, a peptide consisting of the amino acid sequence of SEQ ID NO: 14, a peptide consisting of the amino acid sequence of SEQ ID NO: 24, and a peptide consisting of the amino acid sequence of SEQ ID NO: 36, a peptide consisting of the amino acid sequence of SEQ ID NO: 6, and a peptide consisting of the amino acid sequence of SEQ ID NO: 8.

In the present application, examples of a set of peptides with different SRM transitions that can be used when detecting multiple types of peptides from one sample include, but are not limited to, a set of two or more types (*e.g.*, 2, 3, 4, 5, 6, 7, 8, 9, 10, 11, 12, 13, 14, 15, 16, 17, 18, 19, 20, 21, 22, 23, 24, 25, and 26 types) of peptides selected from the group consisting of (1) to (26) below:
(1) a peptide consisting of the amino acid sequence of SEQ ID NO: 5;
(2) a peptide consisting of the amino acid sequence of SEQ ID NO: 6;
(3) a peptide consisting of the amino acid sequence of SEQ ID NO: 7;
(4) a peptide consisting of the amino acid sequence of SEQ ID NO: 8;
(5) a peptide consisting of the amino acid sequence of SEQ ID NO: 9;
(6) a peptide consisting of the amino acid sequence of SEQ ID NO: 10;
(7) a peptide consisting of the amino acid sequence of SEQ ID NO: 12;
(8) a peptide consisting of the amino acid sequence of SEQ ID NO: 13;
(9) a peptide consisting of the amino acid sequence of SEQ ID NO: 14;
(10) a peptide consisting of the amino acid sequence of SEQ ID NO: 17;
(11) a peptide consisting of the amino acid sequence of SEQ ID NO: 24;
(12) a peptide consisting of the amino acid sequence of SEQ ID NO: 27;
(13) a peptide consisting of the amino acid sequence of SEQ ID NO: 36;
(14) a peptide consisting of the amino acid sequence of SEQ ID NO: 38;
(15) a peptide consisting of the amino acid sequence of SEQ ID NO: 40;
(16) a peptide consisting of the amino acid sequence of SEQ ID NO: 42;
(17) a peptide consisting of the amino acid sequence of SEQ ID NO: 45;
(18) a peptide consisting of the amino acid sequence of SEQ ID NO: 46;
(19) a peptide consisting of the amino acid sequence of SEQ ID NO: 47;
(20) a peptide consisting of the amino acid sequence of SEQ ID NO: 73;
(21) a peptide consisting of the amino acid sequence of SEQ ID NO: 75;
(22) a peptide consisting of the amino acid sequence of SEQ ID NO: 76;
(23) a peptide consisting of the amino acid sequence of SEQ ID NO: 78;
(24) a peptide consisting of the amino acid sequence of SEQ ID NO: 79;
(25) a peptide consisting of the amino acid sequence of SEQ ID NO: 80; and
(26) a peptide consisting of the amino acid sequence of SEQ ID NO: 82.

These peptides have precursor ion m/z values different from each other, or have different product ion m/z values when they have the same precursor ion m/z value, and therefore, can be detected separately from the same sample by mass spectrometry, especially mass spectrometry in SRM measurement mode.

Furthermore, these peptides have a property of being excised in sufficient amount upon trypsin treatment of antigen-binding molecules comprising IgG antibody CH1, and being ionized sufficiently for mass spectrometry. That is, they have sufficient sensitivity in mass spectrometry.

These peptides are not comprised in proteins naturally occurring in non-human animals (*e.g.*, mice, rats, rabbits, and monkeys) used in pharmacokinetic studies. In other words, the mass spectrometry peaks of these peptides do not interfere with the mass spectrometry peak of a peptide derived from a non-human animal used in pharmacokinetic studies that is excised upon trypsin treatment of a biological sample (blank matrix) derived from the animal. In other words, these peptides are specifically derived from the antigen-binding molecules administered to the animal, and thus, they are suitable for detecting the antigen-binding molecules in pharmacokinetic studies, particularly in cassette dosing studies.

The set of peptides of the present application include a set used for a specific purpose. Examples of the set used for a specific purpose include, but are not limited to, a set for use in the method of the present application, such as the method described in any one of [C1] to [C47] above, a set for use in a method for distinguishing between multiple types of antigen-binding molecules, and a set for use as labels for distinguishing between multiple types of antigen-binding molecules.

The present application also provides a method for administering a mixture of multiple types of antigen-binding molecules, each comprising a peptide from the set of the present application, to an individual or subject. The individual or subject can be a human or non-human animal.

The present application provides a method for selecting an antigen-binding molecule, comprising the following steps:
(a) detecting and/or quantifying each of multiple types of antigen-binding molecules in a biological sample derived from an individual or subject to whom a mixture of the multiple types of antigen-binding molecules comprising each of the peptides in the set of the present application has been administered; and
(b) selecting the antigen-binding molecule based on the detection and/or quantification results of (a).

The result of the detection and/or quantification of antigen-binding molecules in a biological sample can be used to determine whether the antigen-binding molecules have the desired pharmacokinetics or not, and whether the antigen-binding molecules have the desired tissue distribution or not, and these determinations can be used to select desired antigen-binding molecules.

The present application also provides an antigen-binding molecule selected by the method for selecting. The present application further provides an antigen-binding molecule in which the amino acid sequence between positions 121 and 133 according to the EU index in the IgG CH1 domain of the antigen-binding molecule selected by the method for selecting has been changed to the native sequence between positions 121 and 133 according to the EU index in the CH1 domain of an IgG antibody of the same isotype.

All prior art documents cited in this specification are hereby incorporated by reference.

The present disclosure is further illustrated by the following examples, but is not limited thereto. It will be understood that various other embodiments can be practiced in light of the general description above.

### [EXAMPLES]

### Example 1. Design of modified peptides

One technique for detecting, quantifying, or investigating *in vivo* kinetics and distribution of antigen-binding molecules such as antibodies is to conduct mass spectrometry on peptides having specific sequences comprised in the antigen-binding molecules (WO2012/155019). An example of measurement using a unique sequence contained in each antibody is described in Clin Vaccine Immunol. 2017 May; 24(5): e00545-16. Since sensitivity is known to vary depending on a sequence, generalization is difficult when using a unique sequence contained in each antibody. There is also a method for identifying antibodies or antigen-binding molecules by adding amino acid sequences that serve as barcodes to the antigen-binding molecules such as antibodies, and measuring the barcode portions. However, with this method, the barcode portion is exposed on the surface, and the properties of the amino acids of the barcode portion may alter the blood kinetics and distribution. Examples in which negative charge improved blood kinetics are shown in WO2007114319 and WO2009041643. Given these circumstances, the inventors recognized the need to design a sequence that does not alter antigen binding and physicochemical properties, while using a fixed sequence.

To date, the following four types of cassette dosing studies (cocktail administration studies) have been performed on antibodies:
(1) a method of mixing three types of antibodies each recognizing different antigens, and measuring them by an ECL method using three different antigens (Pharmaceutical Research volume 38, pages 583-592 (2021));
(2) a method of labeling antibodies with different radioactive metals (Methods Mol Biol. 2014; 1141: 147-157);
(3) a method of measuring sequences comprising antibody-specific sequences present in the variable regions of the antibodies by using a mass spectrometer (Clin Vaccine Immunol. 2017 May; 24(5): e00545-16); and
(4) a method of adding amino acid sequences that serve as barcodes to the end of antibodies and measuring the barcode sites.

However, method (1) above cannot measure antibodies that recognize the same antigen. In method (2) above, the amino acid sequence on the surface of the antibody, which is the target of labeling, is affected when metal labeling is performed, and this might change antigen binding or affect physicochemical properties. In method (3) above, the measurement system must be adjusted depending on each variable region, making it difficult to achieve a consistent level of sensitivity. In method (4) above, it is possible to increase the types of barcodes; however, since the barcode is located on the outer side of the test substance, it might change physicochemical properties and affect kinetics. For example, an increase in positively charged amino acids such as arginine and lysine is generally thought to worsen kinetics. In addition, the barcode sequence might exhibit unexpected target binding (non-specific binding).

For example, the following technologies are known:
(A) FLYCODE (registered trademark) (https://linkstertx.com/flycode/) (a technology that simultaneously detects PK, etc. of multiple antibodies by attaching peptide barcodes to the antibodies);
(B) Multiplexed *in vivo* Drug Discovery Using a Novel Protein Barcoding Technology (Pierce Ogden, at PEGS Boston, 2023) (a technology that simultaneously detects PK, etc. of multiple antibodies by attaching 10-20aa peptide barcodes called mCode to the antibodies).

Since these prior art techniques add peptide barcodes that are not originally present in the antibodies, the charges of the entire antibody molecules may change, and this may result in differences in PK between antibodies with and without barcodes.

Therefore, the inventors recognized the need for a technique that is capable of simultaneously evaluating multiple samples without affecting antigen binding and physicochemical properties, and that is useful even if the antigen is the same.

As a method of evaluating samples without affecting antigen binding and physicochemical properties, the present inventors invented a technique of designing unique sequences by introducing modifications or insertions to a portion of the sequences of antibodies and analyzing and/or evaluating the sequences by a mass spectrometer.

The Fab domain of an antibody is composed of VH, CH1, VL, and CL. Due to the interaction between CH1 and CL, parts of sequences are located in the interior of the CH1/CL-associated structure. Modifications and insertions of amino acids in the interior of the associated structure are unlikely to alter the physicochemical properties, since they are not exposed on the outer surface of the structure (a surface in contact with a solvent). Modifications at such sites are not considered to result in non-specific or unexpected target binding, since they are not exposed on the exterior. Therefore, sites in the interior of the associated structure are suitable for introducing modifications and insertions to design new sequences for application. An example of such sites in the interior of the associated structure is a site between 120 and 133 according to the EU numbering system. Using the N-terminal peptide at positions 121 to 133 according to EU numbering in human IgG1 CH1 as an example, Tables 3-1 to 3-12 show the sequences of modified peptides. Labels a, b, and c are used in Tables 3-1 to 3-12 to represent the insertion sites between positions 121 and 133 according to EU numbering.

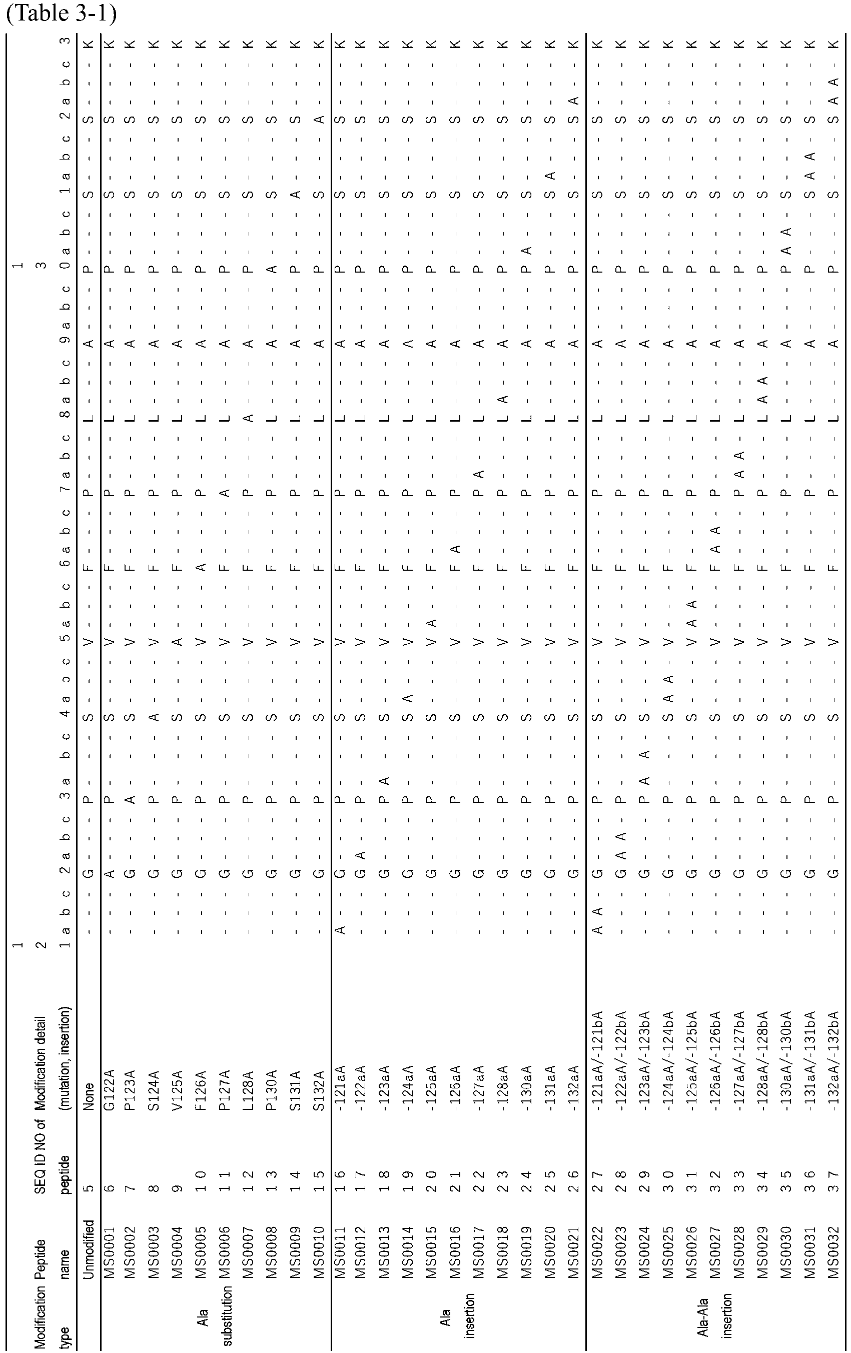

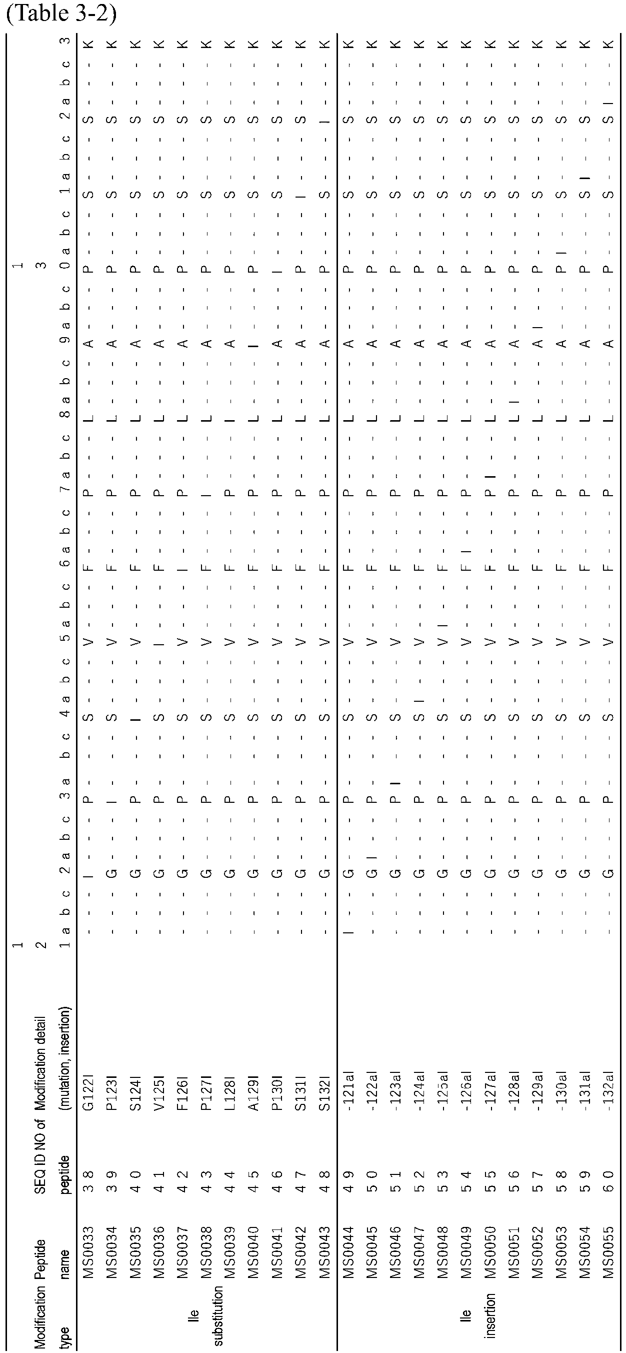

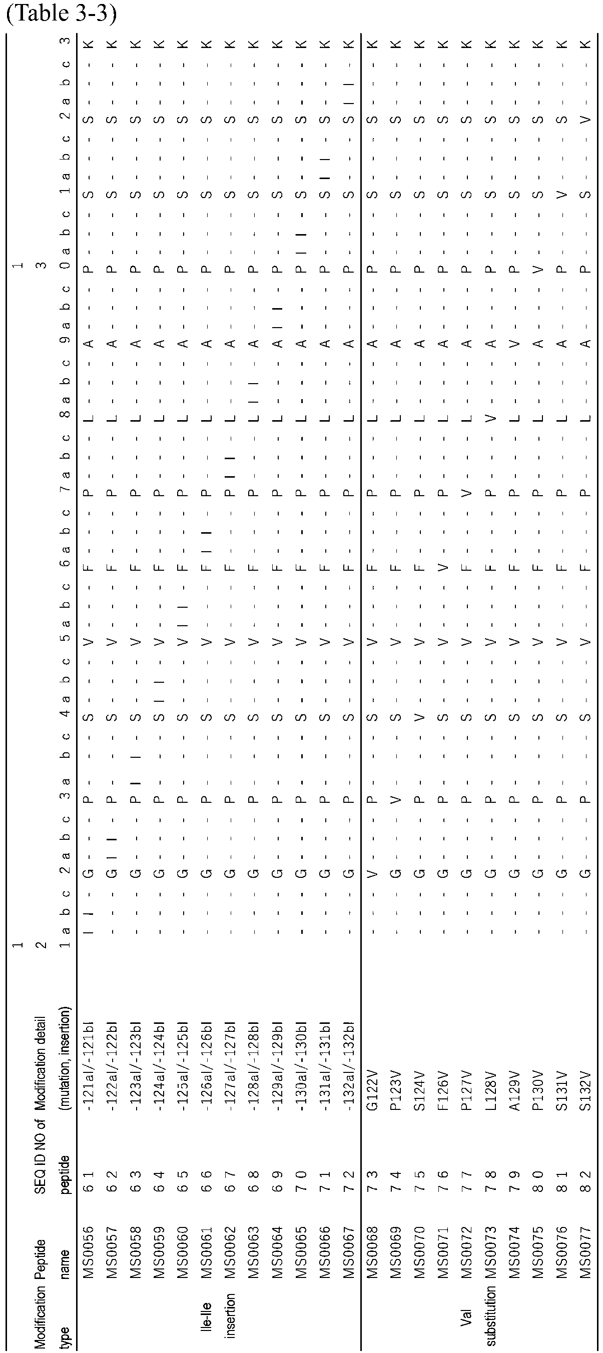

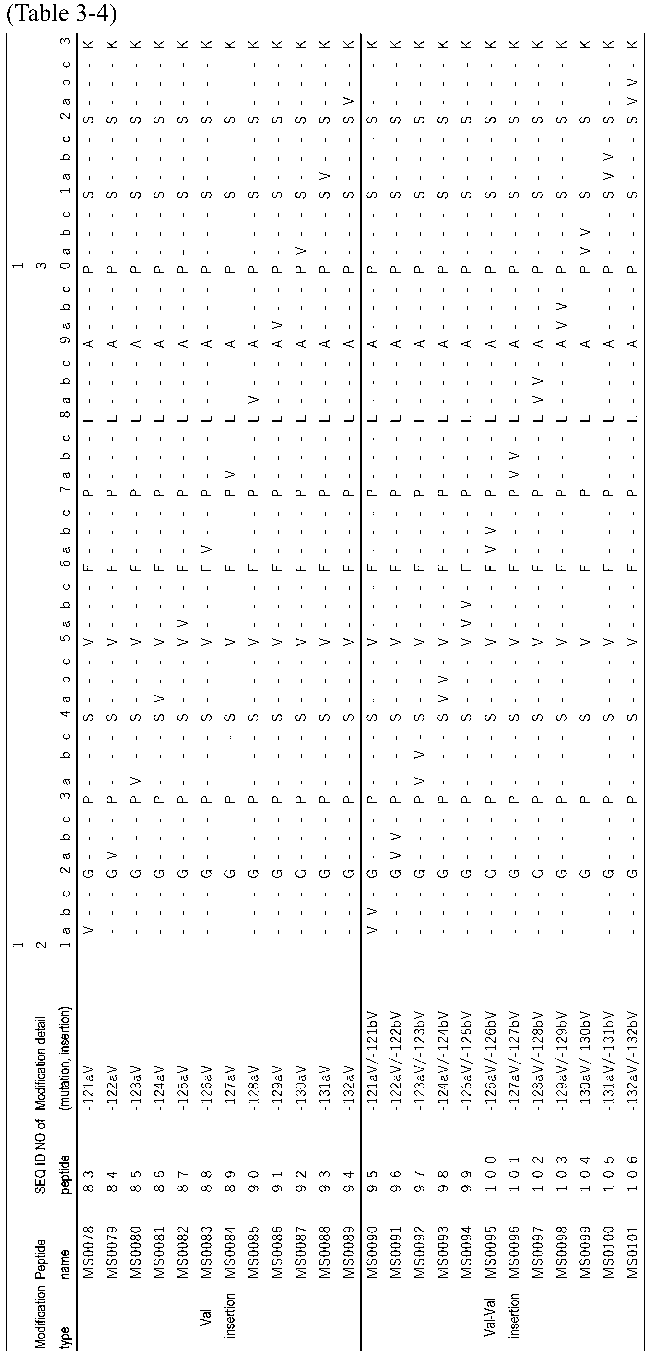

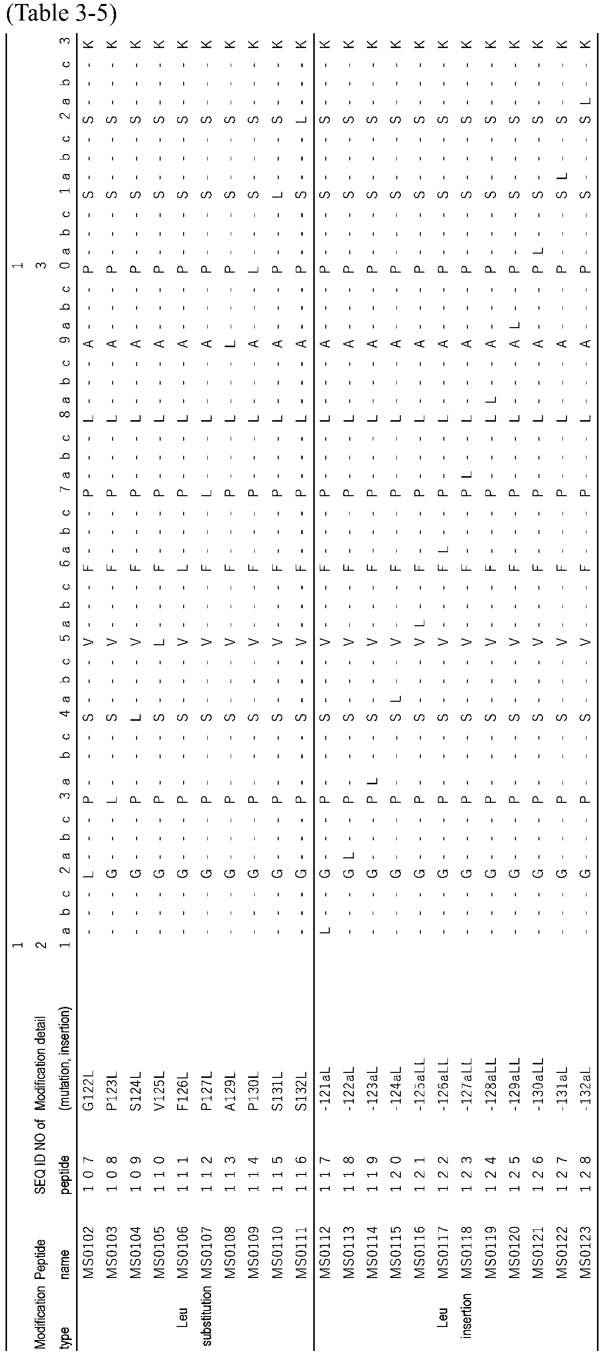

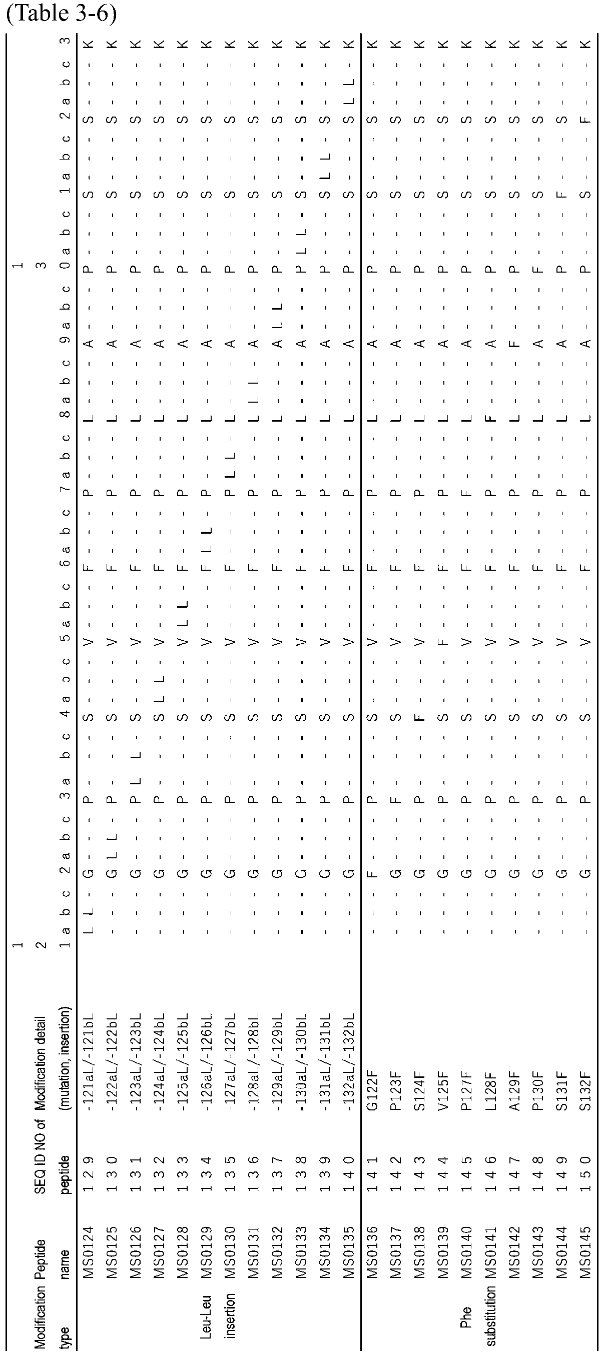

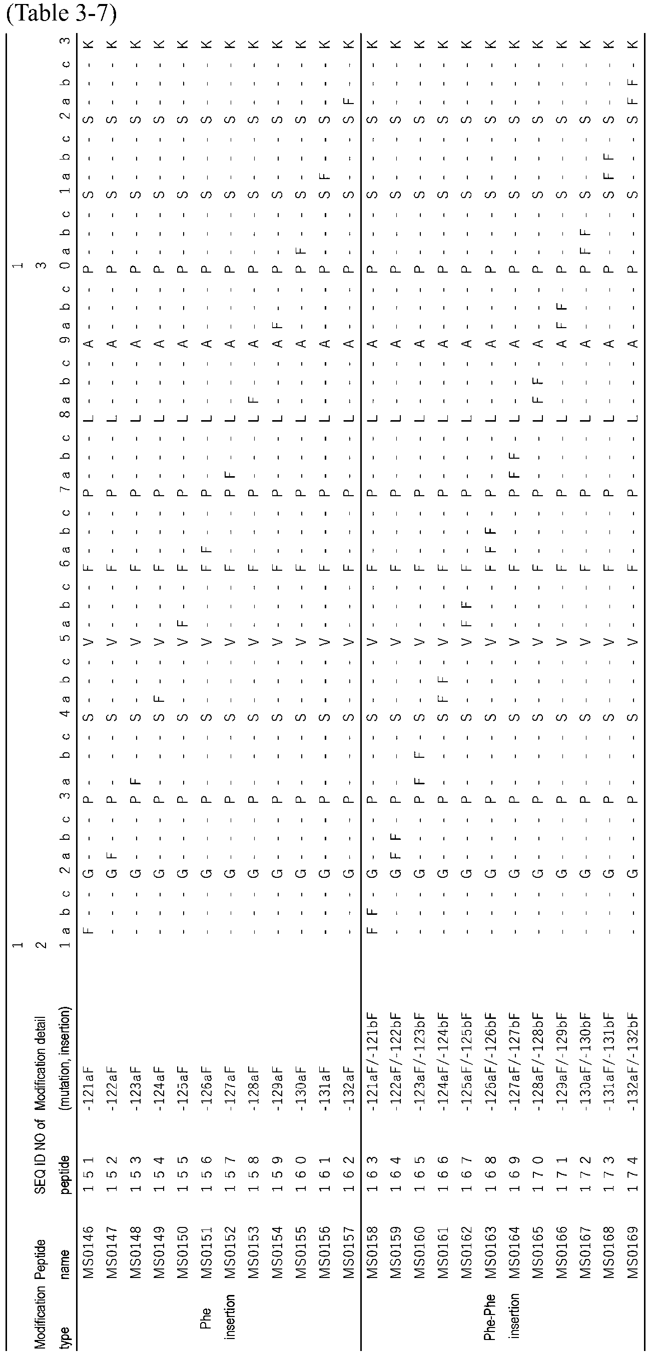

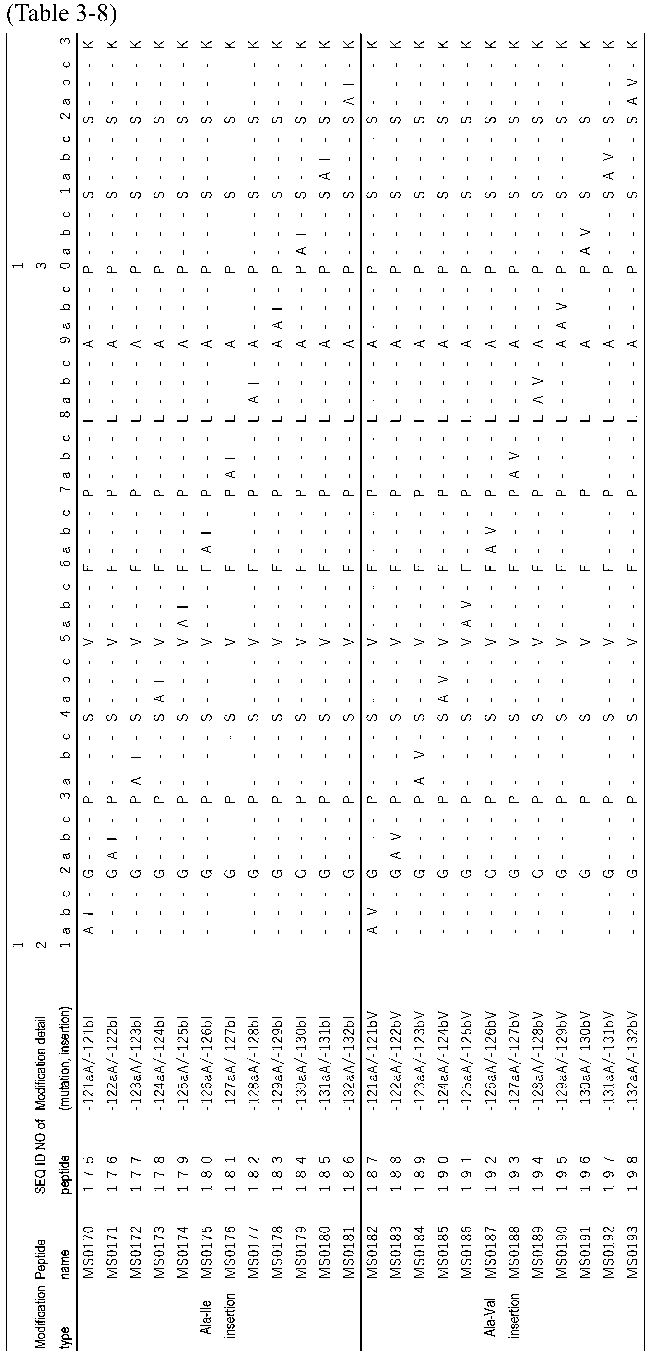

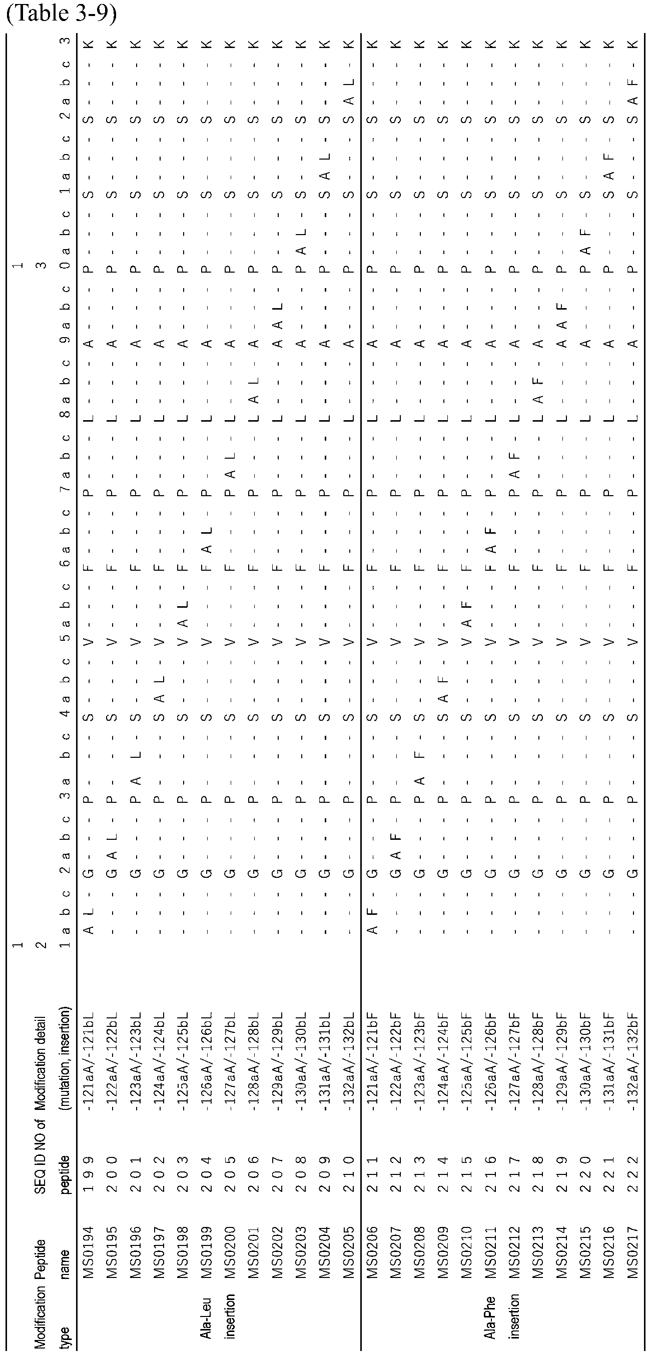

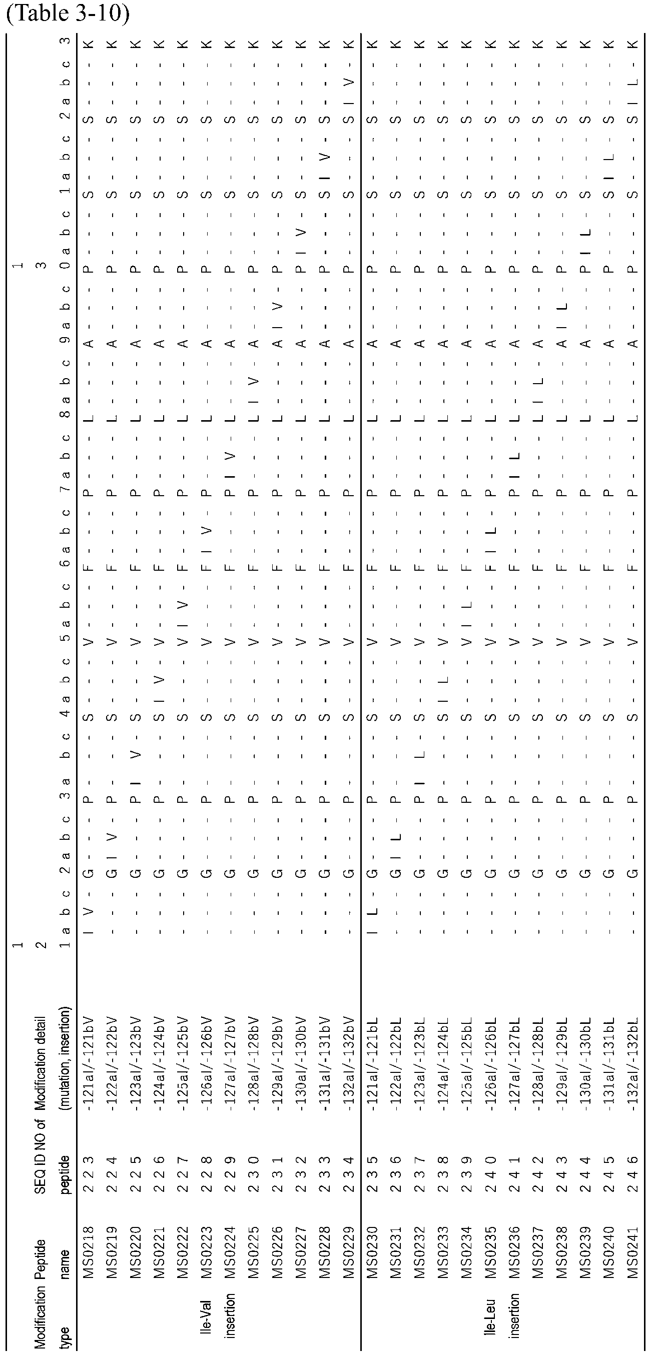

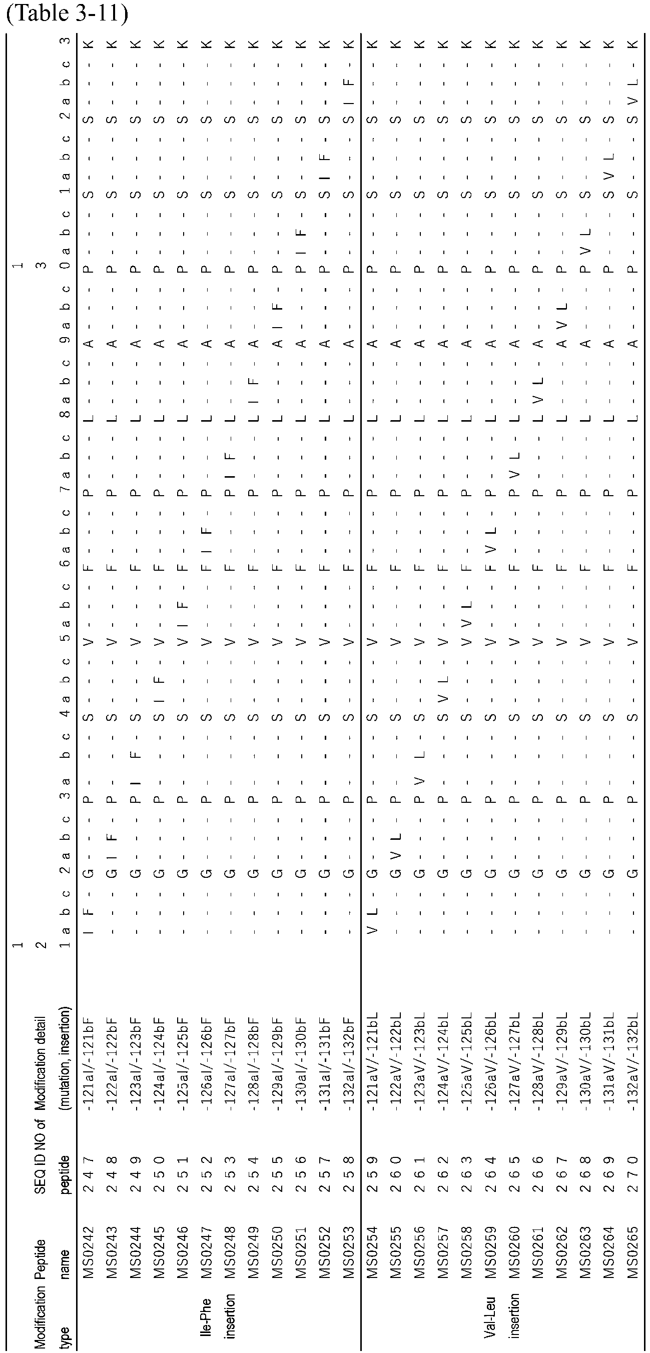

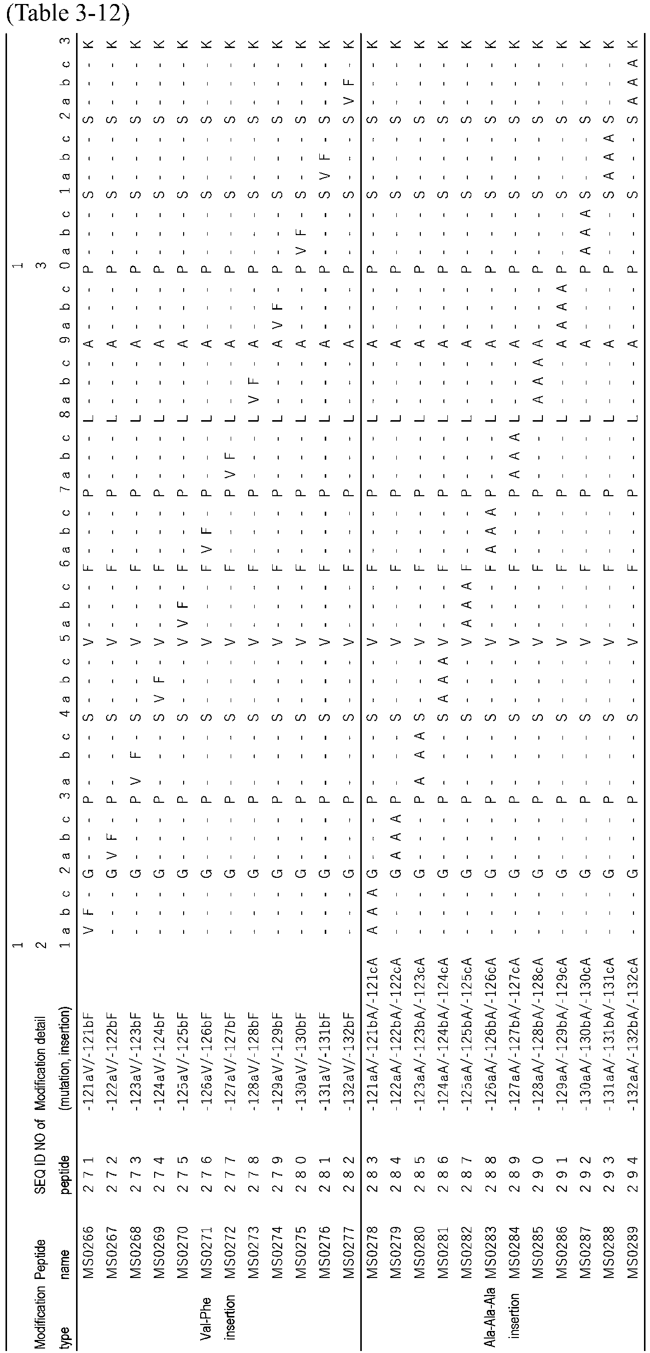

### Example 2. Evaluation of binding and physical properties of modified antibodies comprising modified peptides

### Example 2-1. Preparation of modified antibodies comprising modified peptides

First, the heavy chain variable region (VH) (SEQ ID NO: 1) and the light chain variable region (VL) (SEQ ID NO: 2) of an anti-human IL-6R antibody (H54) were designed. Genes encoding the VH and VL thus designed were synthesized and linked to the genes encoding the heavy chain constant region of SEQ ID NO: 3 (SG181) (a human IgG1-derived sequence lacking the C-terminal glycine and lysine) and the light chain constant region of SEQ ID NO: 4 (SK1), respectively. Each of the linked genes was cloned into an expression vector.

Antibodies comprising modified peptides were prepared as follows: modifications (amino acid substitutions or insertions) were designed in the heavy chain constant region of SEQ ID NO: 3 (SG181) (a human IgG1-derived sequence lacking the C-terminal glycine and lysine), and the heavy chain constant region was linked to the gene encoding the VH as set forth in SEQ ID NO: 1.

Using the genes prepared above, the antibodies listed in Tables 4-1 to 4-9 were transiently expressed in mammalian cells by methods known to those skilled in the art, and purified as needed by methods known to those skilled in the art. The concentrations after purification are shown in Tables 4-1 to 4-9.

**(Table 4-1) Modified antibodies comprising modified peptides**

| Modification type | Peptide name | Antibody name | Antibody name (another name) | Heavy chain full length SEQ ID NO | Heavy chain constant region SEQ ID NO | Light chain full length SEQ ID NO |
|---|---|---|---|---|---|---|
| Original sequence | Before modification | H54-SG181/L28-SK1 | H54-SG181 | 2 9 5 | 3 | |
| | MS0001 | H54-SG181.MS0001/L28-SK1 | H54-SG181.MS1 | 2 9 6 | 5 8 5 | 8 7 4 |
| | MS0002 | H54-SG181.MS0002/L28-SK1 | H54-SG181.MS2 | 2 9 7 | 5 8 6 | 8 7 4 |
| | MS0003 | H54-SG181.MS0003/L28-SK1 | H54-SG181.MS3 | 2 9 8 | 5 8 7 | 8 7 4 |
| | MS0004 | H54-SG181.MS0004/L28-SK1 | H54-SG181.MS4 | 2 9 9 | 5 8 8 | 8 7 4 |
| Ala substitution | MS0005 | H54-SG181.MS0005/L28-SK1 | H54-SG181.MS5 | 3 0 0 | 5 8 9 | 8 7 4 |
| | MS0006 | H54-SG181.MS0006/L28-SK1 | H54-SG181.MS6 | 3 0 1 | 5 9 0 | 8 7 4 |
| | MS0007 | H54-SG181.MS0007/L28-SK1 | H54-SG181.MS7 | 3 0 2 | 5 9 1 | 8 7 4 |
| | MS0008 | H54-SG181.MS0008/L28-SK1 | H54-SG181.MS8 | 3 0 3 | 5 9 2 | 8 7 4 |
| | MS0009 | H54-SG181.MS0009/L28-SK1 | H54-SG181.MS9 | 3 0 4 | 5 9 3 | 8 7 4 |
| | MS0010 | H54-SG181.MS0010/L28-SK1 | H54-SG181.MS10 | 3 0 5 | 5 9 4 | 8 7 4 |
| | MS0011 | H54-SG181.MS0011/L28-SK1 | H54-SG181.MS11 | 3 0 6 | 5 9 5 | 8 7 4 |
| | MS0012 | H54-SG181.MS0012/L28-SK1 | H54-SG181.MS12 | 3 0 7 | 5 9 6 | 8 7 4 |
| | MS0013 | H54-SG181.MS0013/L28-SK1 | H54-SG181.MS13 | 3 0 8 | 5 9 7 | 8 7 4 |
| | MS0014 | H54-SG181.MS0014/L28-SK1 | H54-SG181.MS14 | 3 0 9 | 5 9 8 | 8 7 4 |
| | MS0015 | H54-SG181.MS0015/L28-SK1 | H54-SG181.MS15 | 3 1 0 | 5 9 9 | 8 7 4 |
| Ala insertion | MS0016 | H54-SG181.MS0016/L28-SK1 | H54-SG181.MS16 | 3 1 1 | 6 0 0 | 8 7 4 |
| | MS0017 | H54-SG181.MS0017/L28-SK1 | H54-SG181.MS17 | 3 1 2 | 6 0 1 | 8 7 4 |
| | MS0018 | H54-SG181.MS0018/L28-SK1 | H54-SG181.MS18 | 3 1 3 | 6 0 2 | 8 7 4 |
| | MS0019 | H54-SG181.MS0019/L28-SK1 | H54-SG181.MS19 | 3 1 4 | 6 0 3 | 8 7 4 |
| | MS0020 | H54-SG181.MS0020/L28-SK1 | H54-SG181.MS20 | 3 1 5 | 6 0 4 | 8 7 4 |
| | MS0021 | H54-SG181.MS0021/L28-SK1 | H54-SG181.MS21 | 3 1 6 | 6 0 5 | 8 7 4 |
| | MS0022 | H54-SG181.MS0022/L28-SK1 | H54-SG181.MS22 | 3 1 7 | 6 0 6 | 8 7 4 |
| | MS0023 | H54-SG181.MS0023/L28-SK1 | H54-SG181.MS23 | 3 1 8 | 6 0 7 | 8 7 4 |
| | MS0024 | H54-SG181.MS0024/L28-SK1 | H54-SG181.MS24 | 3 1 9 | 6 0 8 | 8 7 4 |
| | MS0025 | H54-SG181.MS0025/L28-SK1 | H54-SG181.MS25 | 3 2 0 | 6 0 9 | 8 7 4 |
| | MS0026 | H54-SG181.MS0026/L28-SK1 | H54-SG181.MS26 | 3 2 1 | 6 1 0 | 8 7 4 |
| Ala-Ala insertion | MS0027 | H54-SG181.MS0027/L28-SK1 | H54-SG181.MS27 | 3 2 2 | 6 1 1 | 8 7 4 |
| | MS0028 | H54-SG181.MS0028/L28-SK1 | H54-SG181.MS28 | 3 2 3 | 6 1 2 | 8 7 4 |
| | MS0029 | H54-SG181.MS0029/L28-SK1 | H54-SG181.MS29 | 3 2 4 | 6 1 3 | 8 7 4 |
| | MS0030 | H54-SG181.MS0030/L28-SK1 | H54-SG181.MS30 | 3 2 5 | 6 1 4 | 8 7 4 |
| | MS0031 | H54-SG181.MS0031/L28-SK1 | H54-SG181.MS31 | 3 2 6 | 6 1 5 | 8 7 4 |
| | MS0032 | H54-SG181.MS0032/L28-SK1 | H54-SG181.MS32 | 3 2 7 | 6 1 6 | 8 7 4 |

**(Table 4-2) Modified antibodies comprising modified peptides**

| Modification type | Peptide name | Antibody name | Antibody name (another name) | Heavy chain full length SEQ ID NO | Heavy chain constant region SEQ ID NO | Light chain full length SEQ ID NO |
|---|---|---|---|---|---|---|
| | MS0033 | H54-SG181.MS0033/L28-SK1 | H54-SG181.MS33 | 3 2 8 | 6 1 7 | 8 7 4 |
| | MS0034 | H54-SG181.MS0034/L28-SK1 | H54-SG181.MS34 | 3 2 9 | 6 1 8 | 8 7 4 |
| | MS0035 | H54-SG181.MS0035/L28-SK1 | H54-SG181.MS35 | 3 3 0 | 6 1 9 | 8 7 4 |
| | MS0036 | H54-SG181.MS0036/L28-SK1 | H54-SG181.MS36 | 3 3 1 | 6 2 0 | 8 7 4 |
| IIe **substitution** | MS0037 | H54-SG181.MS0037/L28-SK1 | H54-SG181.MS37 | 3 3 2 | 6 2 1 | 8 7 4 |
| | MS0038 | H54-SG181.MS0038/L28-SK1 | H54-SG181.MS38 | 3 3 3 | 6 2 2 | 8 7 4 |
| | MS0039 | H54-SG181.MS0039/L28-SK1 | H54-SG181.MS39 | 3 3 4 | 6 2 3 | 8 7 4 |
| | MS0040 | H54-SG181.MS0040/L28-SK1 | H54-SG181.MS40 | 3 3 5 | 6 2 4 | 8 7 4 |
| | MS0041 | H54-SG181.MS0041/L28-SK1 | H54-SG181.MS41 | 3 3 6 | 6 2 5 | 8 7 4 |
| | MS0042 | H54-SG181.MS0042/L28-SK1 | H54-SG181.MS42 | 3 3 7 | 6 2 6 | 8 7 4 |
| | MS0043 | H54-SG181.MS0043/L28-SK1 | H54-SG181.MS43 | 3 3 8 | 6 2 7 | 8 7 4 |
| | MS0044 | H54-SG181.MS0044/L28-SK1 | H54-SG181.MS44 | 3 3 9 | 6 2 8 | 8 7 4 |
| | MS0045 | H54-SG181.MS0045/L28-SK1 | H54-SG181.MS45 | 3 4 0 | 6 2 9 | 8 7 4 |
| | MS0046 | H54-SG181.MS0046/L28-SK1 | H54-SG181.MS46 | 3 4 1 | 6 3 0 | 8 7 4 |
| | MS0047 | H54-SG181.MS0047/L28-SK1 | H54-SG181.MS47 | 3 4 2 | 6 3 1 | 8 7 4 |
| IIe **insertion** | MS0048 | H54-SG181.MS0048/L28-SK1 | H54-SG181.MS48 | 3 4 3 | 6 3 2 | 8 7 4 |
| | MS0049 | H54-SG181.MS0049/L28-SK1 | H54-SG181.MS49 | 3 4 4 | 6 3 3 | 8 7 4 |
| | MS0050 | H54-SG181.MS0050/L28-SK1 | H54-SG181.MS50 | 3 4 5 | 6 3 4 | 8 7 4 |
| | MS0051 | H54-SG181.MS0051/L28-SK1 | H54-SG181.MS51 | 3 4 6 | 6 3 5 | 8 7 4 |
| | MS0052 | H54-SG181.MS0052/L28-SK1 | H54-SG181.MS52 | 3 4 7 | 6 3 6 | 8 7 4 |
| | MS0053 | H54-SG181.MS0053/L28-SK1 | H54-SG181.MS53 | 3 4 8 | 6 3 7 | 8 7 4 |
| | MS0054 | H54-SG181.MS0054/L28-SK1 | H54-SG181.MS54 | 3 4 9 | 6 3 8 | 8 7 4 |
| | MS0055 | H54-SG181.MS0055/L28-SK1 | H54-SG181.MS55 | 3 5 0 | 6 3 9 | 8 7 4 |
| | MS0056 | H54-SG181.MS0056/L28-SK1 | H54-SG181.MS56 | 3 5 1 | 6 4 0 | 8 7 4 |
| | MS0057 | H54-SG181.MS0057/L28-SK1 | H54-SG181.MS57 | 3 5 2 | 6 4 1 | 8 7 4 |
| | MS0058 | H54-SG181.MS0058/L28-SK1 | H54-SG181.MS58 | 3 5 3 | 6 4 2 | 8 7 4 |
| | MS0059 | H54-SG181.MS0059/L28-SK1 | H54-SG181.MS59 | 3 5 4 | 6 4 3 | 8 7 4 |
| | MS0060 | H54-SG181.MS0060/L28-SK1 | H54-SG181.MS60 | 3 5 5 | 6 4 4 | 8 7 4 |
| IIe-IIe insertion | MS0061 | H54-SG181.MS0061/L28-SK1 | H54-SG181.MS61 | 3 5 6 | 6 4 5 | 8 7 4 |
| | MS0062 | H54-SG181.MS0062/L28-SK1 | H54-SG181.MS62 | 3 5 7 | 6 4 6 | 8 7 4 |
| | MS0063 | H54-SG181.MS0063/L28-SK1 | H54-SG181.MS63 | 3 5 8 | 6 4 7 | 8 7 4 |
| | MS0064 | H54-SG181.MS0064/1.28-5K1 | H54-SG181.MS64 | 3 5 9 | 6 4 8 | 8 7 4 |
| | MS0065 | H54-SG181.MS0065/L28-SK1 | H54-SG181.MS65 | 3 6 0 | 6 4 9 | 8 7 4 |
| | MS0066 | H54-SG181.MS0066/L28-SK1 | H54-SG181.MS66 | 3 6 1 | 6 5 0 | 8 7 4 |
| | MS0067 | H54-SG181.MS0067/L28-SK1 | H54-SG181.MS67 | 3 6 2 | 6 5 1 | 8 7 4 |

**(Table 4-3) Modified antibodies comprising modified peptides**

| Modification type | Peptide name | Antibody name | Antibody name (another name) | Heavy chain full length SEQ ID NO | Heavy chain constant region SEQ ID NO | Light chain full length SEQ ID NO |
|---|---|---|---|---|---|---|
| | MS0068 | H54-SG181.MS0068/L28-SK1 | H54-SG181.MS68 | 3 6 3 | 6 5 2 | 8 7 4 |
| | MS0069 | H54-SG181.MS0069/L28-SK1 | H54-SG181.MS69 | 3 6 4 | 6 5 3 | 8 7 4 |
| | MS0070 | H54-SG181.MS0070/L28-SK1 | H54-SG181.MS70 | 3 6 5 | 6 5 4 | 8 7 4 |
| | MS0071 | H54-SG181.MS0071/L28-SK1 | H54-SG181.MS71 | 3 6 6 | 6 5 5 | 8 7 4 |
| Val substitution | MS0072 | H54-SG181.MS0072/L28-SK1 | H54-SG181.MS72 | 3 6 7 | 6 5 6 | 8 7 4 |
| | MS0073 | H54-SG181.MS0073/L28-SK1 | H54-SG181.MS73 | 3 6 8 | 6 5 7 | 8 7 4 |
| | MS0074 | H54-SG181.MS0074/L28-SK1 | H54-SG181.MS74 | 3 6 9 | 6 5 8 | 8 7 4 |
| | MS0075 | H54-SG181.MS0075/L28-SK1 | H54-SG181.MS75 | 3 7 0 | 6 5 9 | 8 7 4 |
| | MS0076 | H54-SG181.MS0076/L28-SK1 | H54-SG181.MS76 | 3 7 1 | 6 6 0 | 8 7 4 |
| | MS0077 | H54-SG181.MS0077/L28-SK1 | H54-SG181.MS77 | 3 7 2 | 6 6 1 | 8 7 4 |
| | MS0078 | H54-SG181.MS0078/L28-SK1 | H54-SG181.MS78 | 3 7 3 | 6 6 2 | 8 7 4 |
| | MS0079 | H54-SG181.MS0079/L28-SK1 | H54-SG181.MS79 | 3 7 4 | 6 6 3 | 8 7 4 |
| | MS0080 | H54-SG181.MS0080/L28-SK1 | H54-SG181.MS80 | 3 7 5 | 6 6 4 | 8 7 4 |
| | MS0081 | H54-SG181.MS0081/L28-SK1 | H54-SG181.MS81 | 3 7 6 | 6 6 5 | 8 7 4 |
| | MS0082 | H54-SG181.MS0082/L28-SK1 | H54-SG181.MS82 | 3 7 7 | 6 6 6 | 8 7 4 |
| | MS0083 | H54-SG181.MS0083/L28-SK1 | H54-SG181.MS83 | 3 7 8 | 6 6 7 | 8 7 4 |
| Val insertion | MS0084 | H54-SG181.MS0084/L28-SK1 | H54-SG181.MS84 | 3 7 9 | 6 6 8 | 8 7 4 |
| | MS0085 | H54-SG181.MS0085/L28-SK1 | H54-SG181.MS85 | 3 8 0 | 6 6 9 | 8 7 4 |
| | MS0086 | H54-SG181.MS0086/L28-SK1 | H54-SG181.MS86 | 3 8 1 | 6 7 0 | 8 7 4 |
| | MS0087 | H54-SG181.MS0087/L28-SK1 | H54-SG181.MS87 | 3 8 2 | 6 7 1 | 8 7 4 |
| | MS0088 | H54-SG181.MS0088/L28-SK1 | H54-SG181.MS88 | 3 8 3 | 6 7 2 | 8 7 4 |
| | MS0089 | H54-SG181.MS0089/L28-SK1 | H54-SG181.MS89 | 3 8 4 | 6 7 3 | 8 7 4 |
| | MS0090 | H54-SG181.MS0090/L28-SK1 | H54-SG181.MS90 | 3 8 5 | 6 7 4 | 8 7 4 |
| | MS0091 | H54-SG181.MS0091/L28-SK1 | H54-SG181.MS91 | 3 8 6 | 6 7 5 | 8 7 4 |
| | MS0092 | H54-SG181.MS0092/L28-SK1 | H54-SG181.MS92 | 3 8 7 | 6 7 6 | 8 7 4 |
| | MS0093 | H54-SG181.MS0093/L28-SK1 | H54-SG181.MS93 | 3 8 8 | 6 7 7 | 8 7 4 |
| | MS0094 | H54-SG181.MS0094/L28-SK1 | H54-SG181.MS94 | 3 8 9 | 6 7 8 | 8 7 4 |
| Val-Val insertion | MS0095 | H54-SG181.MS0095/L28-SK1 | H54-SG181.MS95 | 3 9 0 | 6 7 9 | 8 7 4 |
| | MS0096 | H54-SG181.MS0096/L28-SK1 | H54-SG181.MS96 | 3 9 1 | 6 8 0 | 8 7 4 |
| | MS0097 | H54-SG181.MS0097/L28-SK1 | H54-SG181.MS97 | 3 9 2 | 6 8 1 | 8 7 4 |
| | MS0098 | H54-SG181.MS0098/L28-SK1 | H54-SG181.MS98 | 3 9 3 | 6 8 2 | 8 7 4 |
| | MS0099 | H54-SG181.MS0099/L28-SK1 | H54-SG181.MS99 | 3 9 4 | 6 8 3 | 8 7 4 |
| | MS0100 | H54-SG181.MS0100/L28-SK1 | H54-SG181.MS100 | 3 9 5 | 6 8 4 | 8 7 4 |
| | MS0101 | H54-SG181.MS0101/L28-SK1 | H54-SG181.MS101 | 3 9 6 | 6 8 5 | 8 7 4 |

**(Table 4-4) Modified antibodies comprising modified peptides**

| Modification type | Peptide name | Antibody name | Antibody name (another name) | Heavy chain full length SEQ ID NO | Heavy chain constant region SEQ ID NO | Light chain full length SEQ ID NO |
|---|---|---|---|---|---|---|
| | MS0102 | H54-SG181.MS0102/L28-SK1 | H54-SG181.MS102 | 3 9 7 | 6 8 6 | 8 7 4 |
| | MS0103 | H54-SG181.MS0103/L28-SK1 | H54-SG181.MS103 | 3 9 8 | 6 8 7 | 8 7 4 |
| | MS0104 | H54-SG181.MS0104/L28-SK1 | H54-SG181.MS104 | 3 9 9 | 6 8 8 | 8 7 4 |
| | MS0105 | H54-SG181.MS0105/L28-SK1 | H54-SG181.MS105 | 4 0 0 | 6 8 9 | 8 7 4 |
| Leu substitution | MS0106 | H54-SG181.MS0106/L28-SK1 | H54-SG181.MS106 | 4 0 1 | 6 9 0 | 8 7 4 |
| | MS0107 | H54-SG181.MS0107/L28-SK1 | H54-SG181.MS107 | 4 0 2 | 6 9 1 | 8 7 4 |
| | MS0108 | H54-SG181.MS0108/L28-SK1 | H54-SG181.MS108 | 4 0 3 | 6 9 2 | 8 7 4 |
| | MS0109 | H54-SG181.MS0109/L28-SK1 | H54-SG181.MS109 | 4 0 4 | 6 9 3 | 8 7 4 |
| | MS0110 | H54-SG181.MS0110/L28-SK1 | H54-SG181.MS110 | 4 0 5 | 6 9 4 | 8 7 4 |
| | MS0111 | H54-SG181.MS0111/L28-SK1 | H54-SG181.MS111 | 4 0 6 | 6 9 5 | 8 7 4 |
| | MS0112 | H54-SG181.MS0112/L28-SK1 | H54-SG181.MS112 | 4 0 7 | 6 9 6 | 8 7 4 |
| | MS0113 | H54-SG181.MS0113/L28-SK1 | H54-SG181.MS113 | 4 0 8 | 6 9 7 | 8 7 4 |
| | MS0114 | H54-SG181.MS0114/L28-SK1 | H54-SG181.MS114 | 4 0 9 | 6 9 8 | 8 7 4 |
| | MS0115 | H54-SG181.MS0115/L28-SK1 | H54-SG181.MS115 | 4 1 0 | 6 9 9 | 8 7 4 |
| | MS0116 | H54-SG181.MS0116/L28-SK1 | H54-SG181.MS116 | 4 1 1 | 7 0 0 | 8 7 4 |
| Leu insertion | MS0117 | H54-SG181.MS0117/L28-SK1 | H54-SG181.MS117 | 4 1 2 | 7 0 1 | 8 7 4 |
| | MS0118 | H54-SG181.MS0118/L28-SK1 | H54-SG181.MS118 | 4 1 3 | 7 0 2 | 8 7 4 |
| | MS0119 | H54-SG181.MS0119/L28-SK1 | H54-SG181.MS119 | 4 1 4 | 7 0 3 | 8 7 4 |
| | MS0120 | H54-SG181.MS0120/L28-SK1 | H54-SG181.MS120 | 4 1 5 | 7 0 4 | 8 7 4 |
| | MS0121 | H54-SG181.MS0121/L28-SK1 | H54-SG181.MS121 | 4 1 6 | 7 0 5 | 8 7 4 |
| | MS0122 | H54-SG181.MS0122/L28-SK1 | H54-SG181.MS122 | 4 1 7 | 7 0 6 | 8 7 4 |
| | MS0123 | H54-SG181.MS0123/L28-SK1 | H54-SG181.MS123 | 4 1 8 | 7 0 7 | 8 7 4 |
| | MS0124 | H54-SG181.MS0124/L28-SK1 | H54-SG181.MS124 | 4 1 9 | 7 0 8 | 8 7 4 |
| | MS0125 | H54-SG181.MS0125/L28-SK1 | H54-SG181.MS125 | 4 2 0 | 7 0 9 | 8 7 4 |
| | MS0126 | H54-SG181.MS0126/L28-SK1 | H54-SG181.MS126 | 4 2 1 | 7 1 0 | 8 7 4 |
| | MS0127 | H54-SG181.MS0127/L28-SK1 | H54-SG181.MS127 | 4 2 2 | 7 1 1 | 8 7 4 |
| | MS0128 | H54-SG181.MS0128/L28-SK1 | H54-SG181.MS128 | 4 2 3 | 7 1 2 | 8 7 4 |
| | MS0129 | H54-SG181.MS0129/L28-SK1 | H54-SG181.MS129 | 4 2 4 | 7 1 3 | 8 7 4 |
| Leu-Leu **insertion** | MS0130 | H54-SG181.MS0130/L28-SK1 | H54-SG181.MS130 | 4 2 5 | 7 1 4 | 8 7 4 |
| | MS0131 | H54-SG181.MS0131/L28-SK1 | H54-SG181.MS131 | 4 2 6 | 7 1 5 | 8 7 4 |
| | MS0132 | H54-SG181.MS0132/L28-SK1 | H54-SG181.MS132 | 4 2 7 | 7 1 6 | 8 7 4 |
| | MS0133 | H54-SG181.MS0133/L28-SK1 | H54-SG181.MS133 | 4 2 8 | 7 1 7 | 8 7 4 |
| | MS0134 | H54-SG181.MS0134/L28-SK1 | H54-SG181.MS134 | 4 2 9 | 7 1 8 | 8 7 4 |
| | MS0135 | H54-SG181.MS0135/L28-SK1 | H54-SG181.MS135 | 4 3 0 | 7 1 9 | 8 7 4 |

**(Table 4-5) Modified antibodies comprising modified peptides**

| Modification type | Peptide name | Antibody name | Antibody name (another name) | Heavy chain full length SEQ ID NO | Heavy chain constant region SEQ ID NO | Light chain full length SEQ ID NO |
|---|---|---|---|---|---|---|
| | MS0136 | H54-SG181.MS0136/L28-SK1 | H54-SG181.MS136 | 4 3 1 | 7 2 0 | 8 7 4 |
| | MS0137 | H54-SG181.MS0137/L28-SK1 | H54-SG181.MS137 | 4 3 2 | 7 2 1 | 8 7 4 |
| | MS0138 | H54-SG181.MS0138/L28-SK1 | H54-SG181.MS138 | 4 3 3 | 7 2 2 | 8 7 4 |
| Phe substitution | MS0139 | H54-SG181.MS0139/L28-SK1 | H54-SG181.MS139 | 4 3 4 | 7 2 3 | 8 7 4 |
| | MS0140 | H54-SG181.MS0140/L28-SK1 | H54-SG181.MS140 | 4 3 5 | 7 2 4 | 8 7 4 |
| | MS0141 | H54-SG181.MS0141/L28-SK1 | H54-SG181.MS141 | 4 3 6 | 7 2 5 | 8 7 4 |
| | MS0142 | H54-SG181.MS0142/L28-SK1 | H54-SG181.MS142 | 4 3 7 | 7 2 6 | 8 7 4 |
| | MS0143 | H54-SG181.MS0143/L28-SK1 | H54-SG181.MS143 | 4 3 8 | 7 2 7 | 8 7 4 |
| | MS0144 | H54-SG181.MS0144/L28-SK1 | H54-SG181.MS144 | 4 3 9 | 7 2 8 | 8 7 4 |
| | MS0145 | H54-SG181.MS0145/L28-SK1 | H54-SG181.MS145 | 4 4 0 | 7 2 9 | 8 7 4 |
| | MS0146 | H54-SG181.MS0146/L28-SK1 | H54-SG181.MS146 | 4 4 1 | 7 3 0 | 8 7 4 |
| | MS0147 | H54-SG181.MS0147/L28-SK1 | H54-SG181.MS147 | 4 4 2 | 7 3 1 | 8 7 4 |
| | MS0148 | H54-SG181.MS0148/L28-SK1 | H54-SG181.MS148 | 4 4 3 | 7 3 2 | 8 7 4 |
| | MS0149 | H54-SG181.MS0149/L28-SK1 | H54-SG181.MS149 | 4 4 4 | 7 3 3 | 8 7 4 |
| | MS0150 | H54-SG181.MS0150/L28-SK1 | H54-SG181.MS150 | 4 4 5 | 7 3 4 | 8 7 4 |
| Phe **insertion** | MS0151 | H54-SG181.MS0151/L28-SK1 | H54-SG181.MS151 | 4 4 6 | 7 3 5 | 8 7 4 |
| | MS0152 | H54-SG181.MS0152/L28-SK1 | H54-SG181.MS152 | 4 4 7 | 7 3 6 | 8 7 4 |
| | MS0153 | H54-SG181.MS0153/L28-SK1 | H54-SG181.MS153 | 4 4 8 | 7 3 7 | 8 7 4 |
| | MS0154 | H54-SG181.MS0154/L28-SK1 | H54-SG181.MS154 | 4 4 9 | 7 3 8 | 8 7 4 |
| | MS0155 | H54-SG181.MS0155/L28-SK1 | H54-SG181.MS155 | 4 5 0 | 7 3 9 | 8 7 4 |
| | MS0156 | H54-SG181.MS0156/L28-SK1 | H54-SG181.MS156 | 4 5 1 | 7 4 0 | 8 7 4 |
| | MS0157 | H54-SG181.MS0157/L28-SK1 | H54-SG181.MS157 | 4 5 2 | 7 4 1 | 8 7 4 |
| | MS0158 | H54-SG181.MS0158/L28-SK1 | H54-SG181.MS158 | 4 5 3 | 7 4 2 | 8 7 4 |
| | MS0159 | H54-SG181.MS0159/L28-SK1 | H54-SG181.MS159 | 4 5 4 | 7 4 3 | 8 7 4 |
| | MS0160 | H54-SG181.MS0160/L28-SK1 | H54-SG181.MS160 | 4 5 5 | 7 4 4 | 8 7 4 |
| | MS0161 | H54-SG181.MS0161/L28-SK1 | H54-SG181.MS161 | 4 5 6 | 7 4 5 | 8 7 4 |
| | MS0162 | H54-SG181.MS0162/L28-SK1 | H54-SG181.MS162 | 4 5 7 | 7 4 6 | 8 7 4 |
| Phe-Phe **insertion** | MS0163 | H54-SG181.MS0163/L28-SK1 | H54-SG181.MS163 | 4 5 8 | 7 4 7 | 8 7 4 |
| | MS0164 | H54-SG181.MS0164/L28-SK1 | H54-SG181.MS164 | 4 5 9 | 7 4 8 | 8 7 4 |
| | MS0165 | H54-SG181.MS0165/L28-SK1 | H54-SG181.MS165 | 4 6 0 | 7 4 9 | 8 7 4 |
| | MS0166 | H54-SG181.MS0166/L28-SK1 | H54-SG181.MS166 | 4 6 1 | 7 5 0 | 8 7 4 |
| | MS0167 | H54-SG181.MS0167/L28-SK1 | H54-SG181.MS167 | 4 6 2 | 7 5 1 | 8 7 4 |
| | MS0168 | H54-SG181.MS0168/L28-SK1 | H54-SG181.MS168 | 4 6 3 | 7 5 2 | 8 7 4 |
| | MS0169 | H54-SG181.MS0169/L28-SK1 | H54-SG181.MS169 | 4 6 4 | 7 5 3 | 8 7 4 |

**(Table 4-6) Modified antibodies comprising modified peptides**

| Modification type | Peptide name | Antibody name | Antibody name (another name) | Heavy chain full length SEQ ID NO | Heavy chain constant region SEQ ID NO | Light chain full length SEQ ID NO |
|---|---|---|---|---|---|---|
| | MS0170 | H54-SG181.MS0170/L28-SK1 | H54-SG181.MS170 | 4 6 5 | 7 5 4 | 8 7 4 |
| | MS0171 | H54-SG181.MS0171/L28-SK1 | H54-SG181.MS171 | 4 6 6 | 7 5 5 | 8 7 4 |
| | MS0172 | H54-SG181.MS0172/L28-SK1 | H54-SG181.MS172 | 4 6 7 | 7 5 6 | 8 7 4 |
| | MS0173 | H54-SG181.MS0173/L28-SK1 | H54-SG181.MS173 | 4 6 8 | 7 5 7 | 8 7 4 |
| | MS0174 | H54-SG181.MS0174/L28-SK1 | H54-SG181.MS174 | 4 6 9 | 7 5 8 | 8 7 4 |
| Ala-lle insertion | MS0175 | H54-SG181.MS0175/L28-SK1 | H54-SG181.MS175 | 4 7 0 | 7 5 9 | 8 7 4 |
| | MS0176 | H54-SG181.MS0176/L28-SK1 | H54-SG181.MS176 | 4 7 1 | 7 6 0 | 8 7 4 |
| | MS0177 | H54-SG181.MS0177/L28-SK1 | H54-SG181.MS177 | 4 7 2 | 7 6 1 | 8 7 4 |
| | MS0178 | H54-SG181.MS0178/L28-SK1 | H54-SG181.MS178 | 4 7 3 | 7 6 2 | 8 7 4 |
| | MS0179 | H54-SG181.MS0179/L28-SK1 | H54-SG181.MS179 | 4 7 4 | 7 6 3 | 8 7 4 |
| | MS0180 | H54-SG181.MS0180/L28-SK1 | H54-SG181.MS180 | 4 7 5 | 7 6 4 | 8 7 4 |
| | MS0181 | H54-SG181.MS0181/L28-SK1 | H54-SG181.MS181 | 4 7 6 | 7 6 5 | 8 7 4 |
| | MS0182 | H54-SG181.MS0182/L28-SK1 | H54-SG181.MS182 | 4 7 7 | 7 6 6 | 8 7 4 |
| | MS0183 | H54-SG181.MS0183/L28-SK1 | H54-SG181.MS183 | 4 7 8 | 7 6 7 | 8 7 4 |
| | MS0184 | H54-SG181.MS0184/L28-SK1 | H54-SG181.MS184 | 4 7 9 | 7 6 8 | 8 7 4 |
| | MS0185 | H54-SG181.MS0185/L28-SK1 | H54-SG181.MS185 | 4 8 0 | 7 6 9 | 8 7 4 |
| Ala-Val insertion | MS0186 | H54-SG181.MS0186/L28-SK1 | H54-SG181.MS186 | 4 8 1 | 7 7 0 | 8 7 4 |
| | MS0187 | H54-SG181.MS0187/L28-SK1 | H54-SG181.MS187 | 4 8 2 | 7 7 1 | 8 7 4 |
| | MS0188 | H54-SG181.MS0188/L28-SK1 | H54-SG181.MS188 | 4 8 3 | 7 7 2 | 8 7 4 |
| | MS0189 | H54-SG181.MS0189/L28-SK1 | H54-SG181.MS189 | 4 8 4 | 7 7 3 | 8 7 4 |
| | MS0190 | H54-SG181.MS0190/L28-SK1 | H54-SG181.MS190 | 4 8 5 | 7 7 4 | 8 7 4 |
| | MS0191 | H54-SG181.MS0191/L28-SK1 | H54-SG181.MS191 | 4 8 6 | 7 7 5 | 8 7 4 |
| | MS0192 | H54-SG181.MS0192/L28-SK1 | H54-SG181.MS192 | 4 8 7 | 7 7 6 | 8 7 4 |
| | MS0193 | H54-SG181.MS0193/L28-SK1 | H54-SG181.MS193 | 4 8 8 | 7 7 7 | 8 7 4 |
| | MS0194 | H54-SG181.MS0194/L28-SK1 | H54-SG181.MS194 | 4 8 9 | 7 7 8 | 8 7 4 |
| | MS0195 | H54-SG181.MS0195/L28-SK1 | H54-SG181.MS195 | 4 9 0 | 7 7 9 | 8 7 4 |
| | MS0196 | H54-SG181.MS0196/L28-SK1 | H54-SG181.MS196 | 4 9 1 | 7 8 0 | 8 7 4 |
| | MS0197 | H54-SG181.MS0197/L28-SK1 | H54-SG181.MS197 | 4 9 2 | 7 8 1 | 8 7 4 |
| | MS0198 | H54-SG181.MS0198/L28-SK1 | H54-SG181.MS198 | 4 9 3 | 7 8 2 | 8 7 4 |
| Ala-Leu insertion | MS0199 | H54-SG181.MS0199/L28-SK1 | H54-SG181.MS199 | 4 9 4 | 7 8 3 | 8 7 4 |
| | MS0200 | H54-SG181.MS0200/L28-SK1 | H54-SG181.MS200 | 4 9 5 | 7 8 4 | 8 7 4 |
| | MS0201 | H54-SG181.MS0201/L28-SK1 | H54-SG181.MS201 | 4 9 6 | 7 8 5 | 8 7 4 |
| | MS0202 | H54-SG181.MS0202/L28-SK1 | H54-SG181.MS202 | 4 9 7 | 7 8 6 | 8 7 4 |
| | MS0203 | H54-SG181.MS0203/L28-SK1 | H54-SG181.MS203 | 4 9 8 | 7 8 7 | 8 7 4 |
| | MS0204 | H54-SG181.MS0204/L28-SK1 | H54-SG181.MS204 | 4 9 9 | 7 8 8 | 8 7 4 |
| | MS0205 | H54-SG181.MS0205/L28-SK1 | H54-SG181.MS205 | 5 0 0 | 7 8 9 | 8 7 4 |

**(Table 4-7) Modified antibodies comprising modified peptides**

| Modification type | Peptide name | Antibody name | Antibody name (another name) | Heavy chain full length SEQ ID NO | Heavy chain constant region SEQ ID NO | Light chain full length SEQ ID NO |
|---|---|---|---|---|---|---|
| | MS0206 | H54-SG181.MS0206/L28-SK1 | H54-SG181.MS206 | 5 0 1 | 7 9 0 | 8 7 4 |
| | MS0207 | H54-SG181.MS0207/L28-SK1 | H54-SG181.MS207 | 5 0 2 | 7 9 1 | 8 7 4 |
| | MS0208 | H54-SG181.MS0208/L28-SK1 | H54-SG181.MS208 | 5 0 3 | 7 9 2 | 8 7 4 |
| | MS0209 | H54-SG181.MS0209/L28-SK1 | H54-SG181.MS209 | 5 0 4 | 7 9 3 | 8 7 4 |
| Ala-Phe insertion | MS0210 | H54-SG181.MS0210/L28-SK1 | H54-SG181.MS210 | 5 0 5 | 7 9 4 | 8 7 4 |
| | MS0211 | H54-SG181.MS0211/L28-SK1 | H54-SG181.MS211 | 5 0 6 | 7 9 5 | 8 7 4 |
| | MS0212 | H54-SG181.MS0212/L28-SK1 | H54-SG181.MS212 | 5 0 7 | 7 9 6 | 8 7 4 |
| | MS0213 | H54-SG181.MS0213/L28-SK1 | H54-SG181.MS213 | 5 0 8 | 7 9 7 | 8 7 4 |
| | MS0214 | H54-SG181.MS0214/L28-SK1 | H54-SG181.MS214 | 5 0 9 | 7 9 8 | 8 7 4 |
| | MS0215 | H54-SG181.MS0215/L28-SK1 | H54-SG181.MS215 | 5 1 0 | 7 9 9 | 8 7 4 |
| | MS0216 | H54-SG181.MS0216/L28-SK1 | H54-SG181.MS216 | 5 1 1 | 8 0 0 | 8 7 4 |
| | MS0217 | H54-SG181.MS0217/L28-SK1 | H54-SG181.MS217 | 5 1 2 | 8 0 1 | 8 7 4 |
| | MS0218 | H54-SG181.MS0218/L28-SK1 | H54-SG181.MS218 | 5 1 3 | 802 | 8 7 4 |
| | MS0219 | H54-SG181.MS0219/L28-SK1 | H54-SG181.MS219 | 5 1 4 | 803 | 8 7 4 |
| | MS0220 | H54-SG181.MS0220/L28-SK1 | H54-SG181.MS220 | 5 1 5 | 804 | 8 7 4 |
| | MS0221 | H54-SG181.MS0221/L28-SK1 | H54-SG181.MS221 | 5 1 6 | 805 | 8 7 4 |
| | MS0222 | H54-SG181.MS0222/L28-SK1 | H54-SG181.MS222 | 5 1 7 | 806 | 8 7 4 |
| Ile-Val insertion | MS0223 | H54-SG181.MS0223/L28-SK1 | H54-SG181.MS223 | 5 1 8 | 807 | 8 7 4 |
| | MS0224 | H54-SG181.MS0224/L28-SK1 | H54-SG181.MS224 | 5 1 9 | 808 | 8 7 4 |
| | MS0225 | H54-SG181.MS0225/L28-SK1 | H54-SG181.MS225 | 5 2 0 | 809 | 8 7 4 |
| | MS0226 | H54-SG181.MS0226/L28-SK1 | H54-SG181.MS226 | 5 2 1 | 810 | 8 7 4 |
| | MS0227 | H54-SG181.MS0227/L28-SK1 | H54-SG181.MS227 | 5 2 2 | 811 | 8 7 4 |
| | MS0228 | H54-SG181.MS0228/L28-SK1 | H54-SG181.MS228 | 5 2 3 | 812 | 8 7 4 |
| | MS0229 | H54-SG181.MS0229/L28-SK1 | H54-SG181.MS229 | 5 2 4 | 813 | 8 7 4 |
| | MS0230 | H54-SG181.MS0230/L28-SK1 | H54-SG181.MS230 | 5 2 5 | 8 1 4 | 8 7 4 |
| | MS0231 | H54-SG181.MS0231/L28-SK1 | H54-SG181.MS231 | 5 2 6 | 8 1 5 | 8 7 4 |
| | MS0232 | H54-SG181.MS0232/L28-SK1 | H54-SG181.MS232 | 5 2 7 | 8 1 6 | 8 7 4 |
| | MS0233 | H54-SG181.MS0233/L28-SK1 | H54-SG181.MS233 | 5 2 8 | 8 1 7 | 8 7 4 |
| | MS0234 | H54-SG181.MS0234/L28-SK1 | H54-SG181.MS234 | 5 2 9 | 8 1 8 | 8 7 4 |
| Ile-Leu insertion | MS0235 | H54-SG181.MS0235/L28-SK1 | H54-SG181.MS235 | 5 3 0 | 8 1 9 | 8 7 4 |
| | MS0236 | H54-SG181.MS0236/L28-SK1 | H54-SG181.MS236 | 5 3 1 | 8 2 0 | 8 7 4 |
| | MS0237 | H54-SG181.MS0237/L28-SK1 | H54-SG181.MS237 | 5 3 2 | 8 2 1 | 8 7 4 |
| | MS0238 | H54-SG181.MS0238/L28-SK1 | H54-SG181.MS238 | 5 3 3 | 8 2 2 | 8 7 4 |
| | MS0239 | H54-SG181.MS0239/L28-SK1 | H54-SG181.MS239 | 5 3 4 | 8 2 3 | 8 7 4 |
| | MS0240 | H54-SG181.MS0240/L28-SK1 | H54-SG181.MS240 | 5 3 5 | 8 2 4 | 8 7 4 |
| | MS0241 | H54-SG181.MS0241/L28-SK1 | H54-SG181.MS241 | 5 3 6 | 8 2 5 | 8 7 4 |

**(Table 4-8) Modified antibodies comprising modified peptides**

| Modification type | Peptide name | Antibody name | Antibody name (another name) | Heavy chain full length SEQ ID NO | Heavy chain constant region SEQ ID NO | Light chain full length SEQ ID NO |
|---|---|---|---|---|---|---|
| | MS0242 | H54-SG181.MS0242/L28-SK1 | H54-SG181.MS242 | 5 3 7 | 8 2 6 | 8 7 4 |
| | MS0243 | H54-SG181.MS0243/L28-SK1 | H54-SG181.MS243 | 5 3 8 | 8 2 7 | 8 7 4 |
| | MS0244 | H54-SG181.MS0244/L28-SK1 | H54-SG181.MS244 | 5 3 9 | 8 2 8 | 8 7 4 |
| | MS0245 | H54-SG181.MS0245/L28-SK1 | H54-SG181.MS245 | 5 4 0 | 8 2 9 | 8 7 4 |
| Ile-Phe insertion | MS0246 | H54-SG181.MS0246/L28-SK1 | H54-SG181.MS246 | 5 4 1 | 8 3 0 | 8 7 4 |
| | MS0247 | H54-SG181.MS0247/L28-SK1 | H54-SG181.MS247 | 5 4 2 | 8 3 1 | 8 7 4 |
| | MS0248 | H54-SG181.MS0248/L28-SK1 | H54-SG181.MS248 | 5 4 3 | 8 3 2 | 8 7 4 |
| | MS0249 | H54-SG181.MS0249/L28-SK1 | H54-SG181.MS249 | 5 4 4 | 8 3 3 | 8 7 4 |
| | MS0250 | H54-SG181.MS0250/L28-SK1 | H54-SG181.MS250 | 5 4 5 | 8 3 4 | 8 7 4 |
| | MS0251 | H54-SG181.MS0251/L28-SK1 | H54-SG181.MS251 | 5 4 6 | 8 3 5 | 8 7 4 |
| | MS0252 | H54-SG181.MS0252/L28-SK1 | H54-SG181.MS252 | 5 4 7 | 8 3 6 | 8 7 4 |
| | MS0253 | H54-SG181.MS0253/L28-SK1 | H54-SG181.MS253 | 5 4 8 | 8 3 7 | 8 7 4 |
| | MS0254 | H54-SG181.MS0254/L28-SK1 | H54-SG181.MS254 | 5 4 9 | 8 3 8 | 8 7 4 |
| | MS0255 | H54-SG181.MS0255/L28-SK1 | H54-SG181.MS255 | 5 5 0 | 8 3 9 | 8 7 4 |
| | MS0256 | H54-SG181.MS0256/L28-SK1 | H54-SG181.MS256 | 5 5 1 | 8 4 0 | 8 7 4 |
| | MS0257 | H54-SG181.MS0257/L28-SK1 | H54-SG181.MS257 | 5 5 2 | 8 4 1 | 8 7 4 |
| | MS0258 | H54-SG181.MS0258/L28-SK1 | H54-SG181.MS258 | 5 5 3 | 8 4 2 | 8 7 4 |
| Val-Leu insertion | MS0259 | H54-SG181.MS0259/L28-SK1 | H54-SG181.MS259 | 5 5 4 | 8 4 3 | 8 7 4 |
| | MS0260 | H54-SG181.MS0260/L28-SK1 | H54-SG181.MS260 | 5 5 5 | 8 4 4 | 8 7 4 |
| | MS0261 | H54-SG181.MS0261/L28-SK1 | H54-SG181.MS261 | 5 5 6 | 8 4 5 | 8 7 4 |
| | MS0262 | H54-SG181.MS0262/L28-SK1 | H54-SG181.MS262 | 5 5 7 | 8 4 6 | 8 7 4 |
| | MS0263 | H54-SG181.MS0263/L28-SK1 | H54-SG181.MS263 | 5 5 8 | 8 4 7 | 8 7 4 |
| | MS0264 | H54-SG181.MS0264/L28-SK1 | H54-SG181.MS264 | 5 5 9 | 8 4 8 | 8 7 4 |
| | MS0265 | H54-SG181.MS0265/L28-SK1 | H54-SG181.MS265 | 5 6 0 | 8 4 9 | 8 7 4 |
| | MS0266 | H54-SG181.MS0266/L28-SK1 | H54-SG181.MS266 | 5 6 1 | 8 5 0 | 8 7 4 |
| | MS0267 | H54-SG181.MS0267/L28-SK1 | H54-SG181.MS267 | 5 6 2 | 8 5 1 | 8 7 4 |
| | MS0268 | H54-SG181.MS0268/L28-SK1 | H54-SG181.MS268 | 5 6 3 | 8 5 2 | 8 7 4 |
| | MS0269 | H54-SG181.MS0269/L28-SK1 | H54-SG181.MS269 | 5 6 4 | 8 5 3 | 8 7 4 |
| | MS0270 | H54-SG181.MS0270/L28-SK1 | H54-SG181.MS270 | 5 6 5 | 8 5 4 | 8 7 4 |
| Val-Phe insertion | MS0271 | H54-SG181.MS0271/L28-SK1 | H54-SG181.MS271 | 5 6 6 | 8 5 5 | 8 7 4 |
| | MS0272 | H54-SG181.MS0272/L28-SK1 | H54-SG181.MS272 | 5 6 7 | 8 5 6 | 8 7 4 |
| | MS0273 | H54-SG181.MS0273/L28-SK1 | H54-SG181.MS273 | 5 6 8 | 8 5 7 | 8 7 4 |
| | MS0274 | H54-SG181.MS0274/L28-SK1 | H54-SG181.MS274 | 5 6 9 | 8 5 8 | 8 7 4 |
| | MS0275 | H54-SG181.MS0275/L28-SK1 | H54-SG181.MS275 | 5 7 0 | 8 5 9 | 8 7 4 |
| | MS0276 | H54-SG181.MS0276/L28-SK1 | H54-SG181.MS276 | 5 7 1 | 8 6 0 | 8 7 4 |
| | MS0277 | H54-SG181.MS0277/L28-SK1 | H54-SG181.MS277 | 5 7 2 | 8 6 1 | 8 7 4 |

**(Table 4-9) Modified antibodies comprising modified peptides**

| Modification type | Peptide name | Antibody name | Antibody name (another name) | Heavy chain full length SEQ ID NO | Heavy chain constant region SEQ ID NO | Light chain full length SEQ ID NO |
|---|---|---|---|---|---|---|
| | MS0278 | H54-SG181.MS0278/L28-SK1 | H54-SG181.MS278 | 5 7 3 | 8 6 2 | 8 7 4 |
| | MS0279 | H54-SG181.MS0279/L28-SK1 | H54-SG181.MS279 | 5 7 4 | 8 6 3 | 8 7 4 |
| | MS0280 | H54-SG181.MS0280/L28-SK1 | H54-SG181.MS280 | 5 7 5 | 8 6 4 | 8 7 4 |
| | MS0281 | H54-SG181.MS0281/L28-SK1 | H54-SG181.MS281 | 5 7 6 | 8 6 5 | 8 7 4 |
| | MS0282 | H54-SG181.MS0282/L28-SK1 | H54-SG181.MS282 | 5 7 7 | 8 6 6 | 8 7 4 |
| Ala-Ala-Ala insertion | MS0283 | H54-SG181.MS0283/L28-SK1 | H54-SG181.MS283 | 5 7 8 | 8 6 7 | 8 7 4 |
| | MS0284 | H54-SG181.MS0284/L28-SK1 | H54-SG181.MS284 | 5 7 9 | 8 6 8 | 8 7 4 |
| | MS0285 | H54-SG181.MS0285/L28-SK1 | H54-SG181.MS285 | 5 8 0 | 8 6 9 | 8 7 4 |
| | MS0286 | H54-SG181.MS0286/L28-SK1 | H54-SG181.MS286 | 5 8 1 | 8 7 0 | 8 7 4 |
| | MS0287 | H54-SG181.MS0287/L28-SK1 | H54-SG181.MS287 | 5 8 2 | 8 7 1 | 8 7 4 |
| | MS0288 | H54-SG181.MS0288/L28-SK1 | H54-SG181.MS288 | 5 8 3 | 8 7 2 | 8 7 4 |
| | MS0289 | H54-SG181.MS0289/L28-SK1 | H54-SG181.MS289 | 5 8 4 | 8 7 3 | 8 7 4 |

### Example 2-2. Measurement of the thermal denaturation midpoint temperature of modified antibodies comprising modified peptides

The antigen-binding and thermal denaturation midpoint temperature of the modified antibodies comprising modified peptides prepared in Example 2-1 were measured. This confirms that the modified peptides do not affect the antibody structure. The antigen-binding activity was evaluated as described below. The thermal denaturation midpoint temperature was measured using the Differential Scanning Fluorimetry (DSF) method (J Pharm Sci. 2013 Aug;102(8):2471-83). The results are shown in Tables 5-1 to 5-9.

### Example 2-3. Confirmation of antigen binding of modified antibodies comprising modified peptides

The binding activity of the prepared modified antibodies comprising modified peptides to human IL-6R was evaluated using Biacore^{™} T200 (Cytiva). Using Amine Coupling Kit (Cytiva), Protein G (CALBIOCHEM) or Anti-Human IgG (Fc) antibody (Cytiva) was immobilized on all flow cells of Series S Sensor Chip CM4 (Cytiva). For Protein G, the running buffer used was 50 mM sodium phosphate, 150 mM NaCl, 0.05 w/v% Tween 20, pH 7.4. For Anti-Human IgG (Fc) antibody, the running buffer used was 20 mM sodium phosphate, 150 mM NaCl, 0.05 w/v% Tween 20, pH 7.4. The evaluation was performed at 37°C. Each antibody was captured on the sensor chip through interaction with Protein G or Anti-Human IgG (Fc) antibody at the target value of 190-200 RU. Human IL-6R diluted with the running buffer was injected onto the antibody-captured sensor chip at 10 µL/min for 120 seconds, followed by the running buffer at 10 µL/min for 120 seconds. Then, to regenerate the sensor chip, for Protein G, 10 mM Gly-HCl pH 1.5 was injected at 30 µL/min for 30 seconds, followed by 25 mM NaOH at 30 µL/min for 30 seconds. For Anti-Human IgG (Fc) antibody, 3 M MgCl₂ was injected twice at 30 µL/min for 18 seconds. The above procedure was repeated using 0, 100, and 400 nM human IL-6R to obtain a sensorgram for each antibody. The human IL-6R used in this measurement was prepared by the technique described in WO2018097307 (Example 3-2). The dissociation constant (KD) of each antibody for human IL-6R was calculated using Biacore T200 Evaluation Software (Cytiva). Specifically, the sensorgram of each antibody was curve-fitted based on a 1:1 binding model to calculate the binding rate constant ka (1/Ms) and the dissociation rate constant kd (1/s), and the dissociation constant KD (M) was calculated from these values. The measurement results are shown in Tables 5-1 to 5-9. In these tables, the notation E used to indicate KD means "power of 10", for example, 4.74E-09 indicates 4.74 × 10⁻⁹.

It was confirmed that all of the modified antibodies comprising the modified polypeptides were expressed at the same level as the antibody before modification, maintained the lowest denaturation midpoint temperature 40°C higher than human body temperature, and bind to human IL-6R at the same level as the antibody before modification. Therefore, it was demonstrated that these modifications can be used without changing the properties of the antibodies.

**(Table 5-1) Concentration after purification, Tm value, and antigen-binding activity of modified antibodies**

| Modification type | Modified peptide | Name of modified antibody | Concentration after purification (mg/mL) | Lowest denaturation midpoint temperature (°C) | KD[M] |
|---|---|---|---|---|---|
| | Before modification | H54-SG181/L28-SK1 | 1.539 | 72.11 | 3.88E-09 |
| | MS0001 | H54-SG181.MS0001/L28-SK1 | 2.543 | 71.42 | 3.78E-09 |
| | MS0002 | H54-SG181.MS0002/L28-SK1 | 2.606 | 70.59 | 3.78E-09 |
| | MS0003 | H54-SG181.MS0003/L28-SK1 | 2.471 | 71.14 | 3.73E-09 |
| | MS0004 | H54-SG181.MS0004/L28-SK1 | 2.320 | 70.73 | 3.69E-09 |
| Ala substitution | MS0005 | H54-SG181.MS0005/L28-SK1 | 2.030 | 70.87 | 3.78E-09 |
| | MS0006 | H54-SG181.MS0006/L28-SK1 | 2.409 | 70.59 | 3.74E-09 |
| | MS0007 | H54-SG181.MS0007/L28-SK1 | 2.357 | 70.18 | 3.84E-09 |
| | MS0008 | H54-SG181.MS0008/L28-SK1 | 2.488 | 71.14 | 3.85E-09 |
| | MS0009 | H54-SG181.MS0009/L28-SK1 | 2.548 | 71.01 | 3.74E-09 |
| | MS0010 | H54-SG181.MS0010/L28-SK1 | 2.289 | 70.59 | 3.76E-09 |
| | MS0011 | H54-SG181.MS0011/L28-SK1 | 2.380 | 71.01 | 3.76E-09 |
| | MS0012 | H54-SG181.MS0012/L28-SK1 | 2.436 | 71.01 | 3.61E-09 |
| | MS0013 | H54-SG181.MS0013/L28-SK1 | 2.221 | 70.73 | 3.66E-09 |
| | MS0014 | H54-SG181.MS0014/L28-SK1 | 1.917 | 71.42 | 3.58E-09 |
| | MS0015 | H54-SG181.MS0015/L28-SK1 | 2.501 | 70.59 | 3.67E-09 |
| Ala insertion | MS0016 | H54-SG181.MS0016/L28-SK1 | 2.544 | 69.63 | 3.97E-09 |
| | MS0017 | H54-SG181.MS0017/L28-SK1 | 2.418 | 70.87 | 3.79E-09 |
| | MS0018 | H54-SG181.MS0018/L28-SK1 | 2.441 | 71.56 | 3.84E-09 |
| | MS0019 | H54-SG181.MS0019/L28-SK1 | 2.174 | 69.63 | 3.85E-09 |
| | MS0020 | H54-SG181.MS0020/L28-SK1 | 2.261 | 71.28 | 3.73E-09 |
| | MS0021 | H54-SG181.MS0021/L28-SK1 | 2.054 | 71.01 | 3.84E-09 |
| | MS0022 | H54-SG181.MS0022/L28-SK1 | 2.097 | 69.63 | 3.82E-09 |
| | MS0023 | H54-SG181.MS0023/L28-SK1 | 2.409 | 69.63 | 3.91E-09 |
| | MS0024 | H54-SG181.MS0024/L28-SK1 | 2.514 | 71.83 | 3.87E-09 |
| | MS0025 | H54-SG181.MS0025/L28-SK1 | 2.340 | 71.70 | 3.75E-09 |
| Ala-Ala insertion | MS0026 | H54-SG181.MS0026/L28-SK1 | 2.202 | 73.49 | 3.82E-09 |
| | MS0027 | H54-SG181.MS0027/L28-SK1 | 2.211 | 71.28 | 3.82E-09 |
| | MS0028 | H54-SG181.MS0028/L28-SK1 | 2.131 | 71.56 | 3.68E-09 |
| | MS0029 | H54-SG181.MS0029/L28-SK1 | 1.791 | 72.66 | 3.83E-09 |
| | MS0030 | H54-SG181.MS0030/L28-SK1 | 2.487 | 71.01 | 3.81E-09 |
| | MS0031 | H54-SG181.MS0031/L28-SK1 | 1.447 | 71.01 | 3.87E-09 |
| | MS0032 | H54-SG181.MS0032/L28-SK1 | 2.489 | 71.70 | 3.89E-09 |

**(Table 5-2) Concentration after purification, Tm value, and antigen-binding activity of modified antibodies**

| Modification type | Modified peptide | Name of modified antibody | Concentration after purification (mg/mL) | Lowest denaturation midpoint temperature (°C) | KD[M] |
|---|---|---|---|---|---|
| | MS0033 | H54-SG181.MS0033/L28-SK1 | 2.510 | 71.42 | 3.82E-09 |
| | MS0034 | H54-SG181.MS0034/L28-SK1 | 2.571 | 70.18 | 3.84E-09 |
| | MS0035 | H54-SG181.MS0035/L28-SK1 | 2.274 | 70.87 | 3.80E-09 |
| | MS0036 | H54-SG181.MS0036/L28-SK1 | 2.386 | 70.45 | 3.77E-09 |
| Ile substitution | MS0037 | H54-SG181.MS0037/L28-SK1 | 2.466 | 70.45 | 3.85E-09 |
| | MS0038 | H54-SG181.MS0038/L28-SK1 | 2.615 | 70.87 | 3.79E-09 |
| | MS0039 | H54-SG181.MS0039/L28-SK1 | 2.504 | 70.18 | 3.92E-09 |
| | MS0040 | H54-SG181.MS0040/L28-SK1 | 2.536 | 71.14 | 3.89E-09 |
| | MS0041 | H54-SG181.MS0041/L28-SK1 | 2.309 | 71.01 | 3.70E-09 |
| | MS0042 | H54-SG181.MS0042/L28-SK1 | 2.402 | 70.87 | 3.85E-09 |
| | MS0043 | H54-SG181.MS0043/L28-SK1 | 2.465 | 70.18 | 3.84E-09 |
| | MS0044 | H54-SG181.MS0044/L28-SK1 | 2.332 | 72.11 | 3.77E-09 |
| | MS0045 | H54-SG181.MS0045/L28-SK1 | 2.455 | 70.04 | 3.84E-09 |
| | MS0046 | H54-SG181.MS0046/L28-SK1 | 2.595 | 71.42 | 3.68E-09 |
| | MS0047 | H54-SG181.MS0047/L28-SK1 | 2.534 | 70.87 | 3.79E-09 |
| Ile insertion | MS0048 | H54-SG181.MS0048/L28-SK1 | 2.598 | 70.73 | 3.83E-09 |
| | MS0049 | H54-SG181.MS0049/L28-SK1 | 2.509 | 70.32 | 3.79E-09 |
| | MS0050 | H54-SG181.MS0050/L28-SK1 | 2.532 | 70.73 | 3.82E-09 |
| | MS0051 | H54-SG181.MS0051/L28-SK1 | 2.543 | 70.73 | 3.82E-09 |
| | MS0052 | H54-SG181.MS0052/L28-SK1 | 2.379 | 71.01 | 3.74E-09 |
| | MS0053 | H54-SG181.MS0053/L28-SK1 | 2.337 | 70.59 | 3.86E-09 |
| | MS0054 | H54-SG181.MS0054/L28-SK1 | 2.661 | 71.14 | 3.93E-09 |
| | MS0055 | H54-SG181.MS0055/L28-SK1 | 2.486 | 70.18 | 3.91E-09 |
| | MS0056 | H54-SG181.MS0056/L28-SK1 | 2.541 | 71.83 | 3.94E-09 |
| | MS0057 | H54-SG181.MS0057/L28-SK1 | 2.539 | 70.45 | 3.86E-09 |
| | MS0058 | H54-SG181.MS0058/L28-SK1 | 2.463 | 70.59 | 3.84E-09 |
| | MS0059 | H54-SG181.MS0059/L28-SK1 | 2.342 | 72.80 | 3.77E-09 |
| | MS0060 | H54-SG181.MS0060/L28-SK1 | 1.999 | 70.59 | 3.79E-09 |
| Ile-Ile insertion | MS0061 | H54-SG181.MS0061/L28-SK1 | 2.539 | 70.32 | 3.75E-09 |
| | MS0062 | H54-SG181.MS0062/L28-SK1 | 2.468 | 70.73 | 3.71E-09 |
| | MS0063 | H54-SG181.MS0063/L28-SK1 | 2.486 | 70.18 | 3.80E-09 |
| | MS0064 | H54-SG181.MS0064/L28-SK1 | 2.515 | 71.28 | 3.91E-09 |
| | MS0065 | H54-SG181.MS0065/L28-SK1 | 2.504 | 70.59 | 3.81E-09 |
| | MS0066 | H54-SG181.MS0066/L28-SK1 | 1.627 | 71.14 | 3.82E-09 |
| | MS0067 | H54-SG181.MS0067/L28-SK1 | 2.554 | 71.14 | 3.83E-09 |

**(Table 5-3) Concentration after purification, Tm value, and antigen-binding activity of modified antibodies**

| Modification type | Modified peptide | Name of modified antibody | Concentration after purification (mg/mL) | Lowest denaturation midpoint temperature (°C) | KD[M] |
|---|---|---|---|---|---|
| | MS0068 | H54-SG181.MS0068/L28-SK1 | 2.341 | 70.87 | 3.81E-09 |
| | MS0069 | H54-SG181.MS0069/L28-SK1 | 2.564 | 70.87 | 3.84E-09 |
| | MS0070 | H54-SG181.MS0070/L28-SK1 | 2.540 | 70.04 | 3.79E-09 |
| | MS0071 | H54-SG181.MS0071/L28-SK1 | 2.434 | 70.18 | 3.91E-09 |
| Val substitution | MS0072 | H54-SG181.MS0072/L28-SK1 | 1.852 | 71.56 | 3.89E-09 |
| | MS0073 | H54-SG181.MS0073/L28-SK1 | 2.363 | 70.45 | 3.73E-09 |
| | MS0074 | H54-SG181.MS0074/L28-SK1 | 2.428 | 70.59 | 3.89E-09 |
| | MS0075 | H54-SG181.MS0075/L28-SK1 | 2.389 | 68.80 | 3.86E-09 |
| | MS0076 | H54-SG181.MS0076/L28-SK1 | 2.507 | 70.73 | 3.79E-09 |
| | MS0077 | H54-SG181.MS0077/L28-SK1 | 2.378 | 70.87 | 3.86E-09 |
| | MS0078 | H54-SG181.MS0078/L28-SK1 | 2.561 | 70.87 | 3.83E-09 |
| | MS0079 | H54-SG181.MS0079/L28-SK1 | 2.499 | 69.90 | 3.86E-09 |
| | MS0080 | H54-SG181.MS0080/L28-SK1 | 2.516 | 70.87 | 3.82E-09 |
| | MS0081 | H54-SG181.MS0081/L28-SK1 | 2.514 | 71.42 | 3.70E-09 |
| Val insertion | MS0082 | H54-SG181.MS0082/L28-SK1 | 2.537 | 71.28 | 3.78E-09 |
| | MS0083 | H54-SG181.MS0083/L28-SK1 | 2.337 | 70.18 | 3.95E-09 |
| | MS0084 | H54-SG181.MS0084/L28-SK1 | 2.486 | 70.59 | 3.78E-09 |
| | MS0085 | H54-SG181.MS0085/L28-SK1 | 2.474 | 71.42 | 3.82E-09 |
| | MS0086 | H54-SG181.MS0086/L28-SK1 | 2.575 | 71.56 | 3.79E-09 |
| | MS0087 | H54-SG181.MS0087/L28-SK1 | 2.263 | 71.83 | 3.91E-09 |
| | MS0088 | H54-SG181.MS0088/L28-SK1 | 2.548 | 71.42 | 3.96E-09 |
| | MS0089 | H54-SG181.MS0089/L28-SK1 | 2.281 | 71.14 | 3.79E-09 |
| | MS0090 | H54-SG181.MS0090/L28-SK1 | 2.427 | 70.32 | 3.86E-09 |
| | MS0091 | H54-SG181.MS0091/L28-SK1 | 2.268 | 71.01 | 3.94E-09 |
| | MS0092 | H54-SG181.MS0092/L28-SK1 | 2.411 | 70.73 | 3.77E-09 |
| | MS0093 | H54-SG181.MS0093/L28-SK1 | 2.099 | 73.35 | 3.85E-09 |
| | MS0094 | H54-SG181.MS0094/L28-SK1 | 2.384 | 72.11 | 3.83E-09 |
| Val-Val insertion | MS0095 | H54-SG181.MS0095/L28-SK1 | 2.438 | 70.73 | 3.90E-09 |
| | MS0096 | H54-SG181.MS0096/L28-SK1 | 2.381 | 71.56 | 3.85E-09 |
| | MS0097 | H54-SG181.MS0097/L28-SK1 | 2.522 | 71.42 | 3.76E-09 |
| | MS0098 | H54-SG181.MS0098/L28-SK1 | 2.470 | 71.70 | 3.82E-09 |
| | MS0099 | H54-SG181.MS0099/L28-SK1 | 2.413 | 71.01 | 3.84E-09 |
| | MS0100 | H54-SG181.MS0100/L28-SK1 | 2.280 | 70.45 | 3.79E-09 |
| | MS0101 | H54-SG181.MS0101/L28-SK1 | 2.196 | 71.83 | 3.83E-09 |

**(Table 5-4) Concentration after purification, Tm value, and antigen-binding activity of modified antibodies**

| Modification type | Modified peptide | Name of modified antibody | Concentration after purification (mg/mL) | Lowest denaturation midpoint temperature (°C) | KD[M] |
|---|---|---|---|---|---|
| | MS0102 | H54-SG181.MS0102/L28-SK1 | 2.453 | 70.59 | 3.85E-09 |
| | MS0103 | H54-SG181.MS0103/L28-SK1 | 2.441 | 71.14 | 3.87E-09 |
| | MS0104 | H54-SG181.MS0104/L28-SK1 | 2.458 | 71.42 | 3.90E-09 |
| | MS0105 | H54-SG181.MS0105/L28-SK1 | 2.529 | 70.87 | 3.67E-09 |
| Leu substitution | MS0106 | H54-SG181.MS0106/L28-SK1 | 2.214 | 70.87 | 3.72E-09 |
| | MS0107 | H54-SG181.MS0107/L28-SK1 | 2.401 | 70.45 | 3.69E-09 |
| | MS0108 | H54-SG181.MS0108/L28-SK1 | 2.406 | 71.14 | 3.65E-09 |
| | MS0109 | H54-SG181.MS0109/L28-SK1 | 2.120 | 71.28 | 3.71E-09 |
| | MS0110 | H54-SG181.MS0110/L28-SK1 | 1.943 | 71.42 | 3.74E-09 |
| | MS0111 | H54-SG181.MS0111/L28-SK1 | 2.484 | 69.76 | 3.83E-09 |
| | MS0112 | H54-SG181.MS0112/L28-SK1 | 2.532 | 70.87 | 3.91E-09 |
| | MS0113 | H54-SG181.MS0113/L28-SK1 | 2.498 | 70.18 | 3.67E-09 |
| | MS0114 | H54-SG181.MS0114/L28-SK1 | 2.528 | 71.01 | 3.71E-09 |
| | MS0115 | H54-SG181.MS0115/L28-SK1 | 1.619 | 72.52 | 3.68E-09 |
| Leu insertion | MS0116 | H54-SG181.MS0116/L28-SK1 | 2.447 | 70.87 | 3.66E-09 |
| | MS0117 | H54-SG181.MS0117/L28-SK1 | 2.445 | 71.14 | 3.88E-09 |
| | MS0118 | H54-SG181.MS0118/L28-SK1 | 2.015 | 71.56 | 3.82E-09 |
| | MS0119 | H54-SG181.MS0119/L28-SK1 | 2.431 | 71.28 | 3.87E-09 |
| | MS0120 | H54-SG181.MS0120/L28-SK1 | 2.546 | 71.97 | 3.93E-09 |
| | MS0121 | H54-SG181.MS0121/L28-SK1 | 2.390 | 70.45 | 3.60E-09 |
| | MS0122 | H54-SG181.MS0122/L28-SK1 | 2.488 | 70.18 | 3.82E-09 |
| | MS0123 | H54-SG181.MS0123/L28-SK1 | 2.414 | 70.32 | 3.81E-09 |
| | MS0124 | H54-SG181.MS0124/L28-SK1 | 2.287 | 59.69 | 3.78E-09 |
| | MS0125 | H54-SG181.MS0125/L28-SK1 | 2.264 | 71.42 | 3.87E-09 |
| | MS0126 | H54-SG181.MS0126/L28-SK1 | 2.527 | 70.18 | 3.75E-09 |
| | MS0127 | H54-SG181.MS0127/L28-SK1 | 1.230 | 71.83 | 3.72E-09 |
| | MS0128 | H54-SG181.MS0128/L28-SK1 | 2.398 | 72.94 | 3.95E-09 |
| Leu-Leu insertion | MS0129 | H54-SG181.MS0129/L28-SK1 | 2.373 | 71.70 | 3.77E-09 |
| | MS0130 | H54-SG181.MS0130/L28-SK1 | 2.472 | 71.01 | 3.77E-09 |
| | MS0131 | H54-SG181.MS0131/L28-SK1 | 2.099 | 72.11 | 3.72E-09 |
| | MS0132 | H54-SG181.MS0132/L28-SK1 | 2.286 | 71.70 | 3.66E-09 |
| | MS0133 | H54-SG181.MS0133/L28-SK1 | 2.348 | 70.73 | 3.83E-09 |
| | MS0134 | H54-SG181.MS0134/L28-SK1 | 2.430 | 71.83 | 3.77E-09 |
| | MS0135 | H54-SG181.MS0135/L28-SK1 | 2.375 | 71.70 | 3.87E-09 |

**(Table 5-5) Concentration after purification, Tm value, and antigen-binding activity of modified antibodies**

| Modification type | Modified peptide | Name of modified antibody | Concentration after purification (mg/mL) | Lowest denaturation midpoint temperature (°C) | KD[M] |
|---|---|---|---|---|---|
| | MS0136 | H54-SG181.MS0136/L28-SK1 | 2.451 | 72.11 | 3.92E-09 |
| | MS0137 | H54-SG181.MS0137/L28-SK1 | 2.432 | 71.01 | 3.68E-09 |
| | MS0138 | H54-SG181.MS0138/L28-SK1 | 2.279 | 71.14 | 3.83E-09 |
| | MS0139 | H54-SG181.MS0139/L28-SK1 | 2.426 | 70.45 | 3.83E-09 |
| Phe substitution | MS0140 | H54-SG181.MS0140/L28-SK1 | 2.301 | 71.28 | 3.74E-09 |
| | MS0141 | H54-SG181.MS0141/L28-SK1 | 2.445 | 71.97 | 3.81E-09 |
| | MS0142 | H54-SG181.MS0142/L28-SK1 | 2.498 | 71.01 | 3.81E-09 |
| | MS0143 | H54-SG181.MS0143/L28-SK1 | 2.121 | 72.25 | 3.83E-09 |
| | MS0144 | H54-SG181.MS0144/L28-SK1 | 2.560 | 71.14 | 3.89E-09 |
| | MS0145 | H54-SG181.MS0145/L28-SK1 | 2.484 | 71.14 | 3.78E-09 |
| | MS0146 | H54-SG181.MS0146/L28-SK1 | 1.639 | 72.25 | 3.81E-09 |
| | MS0147 | H54-SG181.MS0147/L28-SK1 | 2.207 | 71.83 | 3.74E-09 |
| | MS0148 | H54-SG181.MS0148/L28-SK1 | 2.091 | 72.66 | 3.62E-09 |
| | MS0149 | H54-SG181.MS0149/L28-SK1 | 2.331 | 72.52 | 3.68E-09 |
| | MS0150 | H54-SG181.MS0150/L28-SK1 | 2.456 | 69.63 | 3.85E-09 |
| Phe insertion | MS0151 | H54-SG181.MS0151/L28-SK1 | 1.218 | 51.96 | 3.84E-09 |
| | MS0152 | H54-SG181.MS0152/L28-SK1 | 2.279 | 72.39 | 3.84E-09 |
| | MS0153 | H54-SG181.MS0153/L28-SK1 | 2.436 | 71.70 | 3.76E-09 |
| | MS0154 | H54-SG181.MS0154/L28-SK1 | 2.160 | 71.42 | 3.82E-09 |
| | MS0155 | H54-SG181.MS0155/L28-SK1 | 2.401 | 71.14 | 3.83E-09 |
| | MS0156 | H54-SG181.MS0156/L28-SK1 | 2.096 | 71.56 | 3.75E-09 |
| | MS0157 | H54-SG181.MS0157/L28-SK1 | 2.371 | 71.01 | 3.81E-09 |
| | MS0158 | H54-SG181.MS0158/L28-SK1 | 2.429 | 70.73 | 3.79E-09 |
| | MS0159 | H54-SG181.MS0159/L28-SK1 | 2.438 | 69.63 | 3.93E-09 |
| | MS0160 | H54-SG181.MS0160/L28-SK1 | 2.472 | 71.97 | 3.90E-09 |
| | MS0161 | H54-SG181.MS0161/L28-SK1 | 2.456 | 71.70 | 3.79E-09 |
| | MS0162 | H54-SG181.MS0162/L28-SK1 | 1.551 | 70.59 | 3.75E-09 |
| Phe-Phe insertion | MS0163 | H54-SG181.MS0163/L28-SK1 | 2.433 | 71.14 | 3.80E-09 |
| | MS0164 | H54-SG181.MS0164/L28-SK1 | 2.138 | 71.70 | 3.69E-09 |
| | MS0165 | H54-SG181.MS0165/L28-SK1 | 2.348 | 71.14 | 3.79E-09 |
| | MS0166 | H54-SG181.MS0166/L28-SK1 | 2.460 | 70.18 | 3.81E-09 |
| | MS0167 | H54-SG181.MS0167/L28-SK1 | 2.358 | 71.01 | 3.89E-09 |
| | MS0168 | H54-SG181.MS0168/L28-SK1 | 1.849 | 72.11 | 3.90E-09 |
| | MS0169 | H54-SG181.MS0169/L28-SK1 | 2.309 | 70.73 | 3.77E-09 |

**(Table 5-6) Concentration after purification, Tm value, and antigen-binding activity of modified antibodies**

| Modification type | Modified peptide | Name of modified antibody | Concentration after purification (mg/mL) | Lowest denaturation midpoint temperature (°C) | KD[M] |
|---|---|---|---|---|---|
| | MS0170 | H54-SG181.MS0170/L28-SK1 | 2.229 | 71.97 | 3.97E-09 |
| | MS0171 | H54-SG181.MS0171/L28-SK1 | 2.348 | 71.56 | 3.88E-09 |
| | MS0172 | H54-SG181.MS0172/L28-SK1 | 2.308 | 71.56 | 3.75E-09 |
| | MS0173 | H54-SG181.MS0173/L28-SK1 | 2.211 | 72.80 | 3.85E-09 |
| Ala-Ile insertion | MS0174 | H54-SG181.MS0174/L28-SK1 | 2.386 | 69.63 | 3.77E-09 |
| | MS0175 | H54-SG181.MS0175/L28-SK1 | 2.455 | 71.42 | 3.81E-09 |
| | MS0176 | H54-SG181.MS0176/L28-SK1 | 2.506 | 72.11 | 3.89E-09 |
| | MS0177 | H54-SG181.MS0177/L28-SK1 | 2.518 | 55.14 | 3.80E-09 |
| | MS0178 | H54-SG181.MS0178/L28-SK1 | 2.410 | 71.56 | 3.84E-09 |
| | MS0179 | H54-SG181.MS0179/L28-SK1 | 2.295 | 71.14 | 3.84E-09 |
| | MS0180 | H54-SG181.MS0180/L28-SK1 | 2.341 | 71.01 | 3.80E-09 |
| | MS0181 | H54-SG181.MS0181/L28-SK1 | 2.335 | 71.28 | 3.83E-09 |
| | MS0182 | H54-SG181.MS0182/L28-SK1 | 2.347 | 54.86 | 3.90E-09 |
| | MS0183 | H54-SG181.MS0183/L28-SK1 | 2.409 | 71.01 | 3.93E-09 |
| | MS0184 | H54-SG181.MS0184/L28-SK1 | 2.412 | 71.97 | 3.90E-09 |
| | MS0185 | H54-SG181.MS0185/L28-SK1 | 2.401 | 71.42 | 3.78E-09 |
| | MS0186 | H54-SG181.MS0186/L28-SK1 | 2.334 | 71.42 | 3.82E-09 |
| Ala-Val insertion | MS0187 | H54-SG181.MS0187/L28-SK1 | 2.232 | 71.42 | 3.82E-09 |
| | MS0188 | H54-SG181.MS0188/L28-SK1 | 2.321 | 71.97 | 3.78E-09 |
| | MS0189 | H54-SG181.MS0189/L28-SK1 | 2.117 | 71.14 | 3.82E-09 |
| | MS0190 | H54-SG181.MS0190/L28-SK1 | 2.185 | 72.11 | 3.81E-09 |
| | MS0191 | H54-SG181.MS0191/L28-SK1 | 2.303 | 70.73 | 3.89E-09 |
| | MS0192 | H54-SG181.MS0192/L28-SK1 | 2.341 | 71.01 | 3.83E-09 |
| | MS0193 | H54-SG181.MS0193/L28-SK1 | 2.433 | 70.73 | 3.74E-09 |
| | MS0194 | H54-SG181.MS0194/L28-SK1 | 2.457 | 51.13 | 3.91E-09 |
| | MS0195 | H54-SG181.MS0195/L28-SK1 | 1.599 | 70.87 | 3.82E-09 |
| | MS0196 | H54-SG181.MS0196/L28-SK1 | 1.485 | 71.70 | 3.67E-09 |
| | MS0197 | H54-SG181.MS0197/L28-SK1 | 1.292 | 71.01 | 3.73E-09 |
| | MS0198 | H54-SG181.MS0198/L28-SK1 | 1.593 | 72.52 | 3.74E-09 |
| Ala-Leu insertion | MS0199 | H54-SG181.MS0199/L28-SK1 | 1.564 | 72.39 | 3.71E-09 |
| | MS0200 | H54-SG181.MS0200/L28-SK1 | 2.378 | 70.18 | 3.81E-09 |
| | MS0201 | H54-SG181.MS0201/L28-SK1 | 2.381 | 70.45 | 3.69E-09 |
| | MS0202 | H54-SG181.MS0202/L28-SK1 | 2.285 | 70.45 | 3.76E-09 |
| | MS0203 | H54-SG181.MS0203/L28-SK1 | 2.367 | 70.04 | 3.81E-09 |
| | MS0204 | H54-SG181.MS0204/L28-SK1 | 2.230 | 70.32 | 3.69E-09 |
| | MS0205 | H54-SG181.MS0205/L28-SK1 | 2.199 | 70.32 | 3.78E-09 |

**(Table 5-7) Concentration after purification, Tm value, and antigen-binding activity of modified antibodies**

| Modification type | Modified peptide | Name of modified antibody | Concentration after purification (mg/mL) | Lowest denaturation midpoint temperature (°C) | KD[M] |
|---|---|---|---|---|---|
| | MS0206 | H54-SG181.MS0206/L28-SK1 | 1.901 | 71.70 | 3.79E-09 |
| | MS0207 | H54-SG181.MS0207/L28-SK1 | 1.398 | 71.14 | 3.82E-09 |
| | MS0208 | H54-SG181.MS0208/L28-SK1 | 2.319 | 69.49 | 3.83E-09 |
| | MS0209 | H54-SG181.MS0209/L28-SK1 | 2.404 | 69.49 | 3.72E-09 |
| | MS0210 | H54-SG181.MS0210/L28-SK1 | 2.381 | 71.70 | 3.75E-09 |
| Ala-Phe insertion | MS0211 | H54-SG181.MS0211/L28-SK1 | 2.261 | 70.04 | 3.75E-09 |
| | MS0212 | H54-SG181.MS0212/L28-SK1 | 2.314 | 70.32 | 3.68E-09 |
| | MS0213 | H54-SG181.MS0213/L28-SK1 | 2.287 | 70.18 | 3.78E-09 |
| | MS0214 | H54-SG181.MS0214/L28-SK1 | 2.202 | 69.35 | 3.67E-09 |
| | MS0215 | H54-SG181.MS0215/L28-SK1 | 2.314 | 70.73 | 3.83E-09 |
| | MS0216 | H54-SG181.MS0216/L28-SK1 | 2.361 | 69.35 | 3.82E-09 |
| | MS0217 | H54-SG181.MS0217/L28-SK1 | 2.339 | 70.18 | 3.74E-09 |
| | MS0218 | H54-SG181.MS0218/L28-SK1 | 2.422 | 69.63 | 3.79E-09 |
| | MS0219 | H54-SG181.MS0219/L28-SK1 | 2.273 | 69.63 | 3.84E-09 |
| | MS0220 | H54-SG181.MS0220/L28-SK1 | 2.262 | 69.90 | 3.69E-09 |
| | MS0221 | H54-SG181.MS0221/L28-SK1 | 2.375 | 70.18 | 3.77E-09 |
| | MS0222 | H54-SG181.MS0222/L28-SK1 | 2.420 | 70.18 | 3.75E-09 |
| Ile-Val insertion | MS0223 | H54-SG181.MS0223/L28-SK1 | 1.410 | 70.59 | 3.77E-09 |
| | MS0224 | H54-SG181.MS0224/L28-SK1 | 2.210 | 70.73 | 3.79E-09 |
| | MS0225 | H54-SG181.MS0225/L28-SK1 | 2.299 | 70.87 | 3.71E-09 |
| | MS0226 | H54-SG181.MS0226/L28-SK1 | 1.911 | 71.97 | 3.76E-09 |
| | MS0227 | H54-SG181.MS0227/L28-SK1 | 2.170 | 70.18 | 3.82E-09 |
| | MS0228 | H54-SG181.MS0228/L28-SK1 | 2.153 | 70.18 | 3.71E-09 |
| | MS0229 | H54-SG181.MS0229/L28-SK1 | 2.255 | 69.90 | 3.81E-09 |
| | MS0230 | H54-SG181.MS0230/L28-SK1 | 2.497 | 69.35 | 3.80E-09 |
| | MS0231 | H54-SG181.MS0231/L28-SK1 | 2.305 | 70.32 | 3.90E-09 |
| | MS0232 | H54-SG181.MS0232/L28-SK1 | 2.359 | 70.32 | 3.84E-09 |
| | MS0233 | H54-SG181.MS0233/L28-SK1 | 2.389 | 70.87 | 3.73E-09 |
| | MS0234 | H54-SG181.MS0234/L28-SK1 | 2.141 | 71.83 | 3.83E-09 |
| Ile-Leu insertion | MS0235 | H54-SG181.MS0235/L28-SK1 | 2.139 | 70.45 | 3.80E-09 |
| | MS0236 | H54-SG181.MS0236/L28-SK1 | 2.083 | 71.42 | 3.68E-09 |
| | MS0237 | H54-SG181.MS0237/L28-SK1 | 2.216 | 71.01 | 3.78E-09 |
| | MS0238 | H54-SG181.MS0238/L28-SK1 | 2.346 | 71.42 | 3.74E-09 |
| | MS0239 | H54-SG181.MS0239/L28-SK1 | 2.265 | 71.28 | 3.89E-09 |
| | MS0240 | H54-SG181.MS0240/L28-SK1 | 2.258 | 70.32 | 3.92E-09 |
| | MS0241 | H54-SG181.MS0241/L28-SK1 | 2.154 | 70.89 | 3.71E-09 |

**(Table 5-8) Concentration after purification, Tm value, and antigen-binding activity of modified antibodies**

| Modification type | Modified peptide | Name of modified antibody | Concentration after purification (mg/mL) | Lowest denaturation midpoint temperature (°C) | KD[M] |
|---|---|---|---|---|---|
| | MS0242 | H54-SG181.MS0242/L28-SK1 | 2.325 | 70.18 | 3.86E-09 |
| | MS0243 | H54-SG181.MS0243/L28-SK1 | 2.349 | 70.59 | 3.92E-09 |
| | MS0244 | H54-SG181.MS0244/L28-SK1 | 1.999 | 71.42 | 3.72E-09 |
| | MS0245 | H54-SG181.MS0245/L28-SK1 | 2.089 | 70.45 | 3.78E-09 |
| | MS0246 | H54-SG181.MS0246/L28-SK1 | 2.255 | 72.11 | 3.86E-09 |
| Ile-Phe insertion | MS0247 | H54-SG181.MS0247/L28-SK1 | 2.266 | 71.42 | 3.84E-09 |
| | MS0248 | H54-SG181.MS0248/L28-SK1 | 2.093 | 71.83 | 3.80E-09 |
| | MS0249 | H54-SG181.MS0249/L28-SK1 | 2.131 | 71.42 | 3.76E-09 |
| | MS0250 | H54-SG181.MS0250/L28-SK1 | 2.245 | 70.59 | 3.82E-09 |
| | MS0251 | H54-SG181.MS0251/L28-SK1 | 2.153 | 70.04 | 3.84E-09 |
| | MS0252 | H54-SG181.MS0252/L28-SK1 | 1.795 | 71.83 | 3.76E-09 |
| | MS0253 | H54-SG181.MS0253/L28-SK1 | 2.222 | 69.90 | 3.82E-09 |
| | MS0254 | H54-SG181.MS0254/L28-SK1 | 2.225 | 71.14 | 3.79E-09 |
| | MS0255 | H54-SG181.MS0255/L28-SK1 | 2.343 | 70.04 | 3.95E-09 |
| | MS0256 | H54-SG181.MS0256/L28-SK1 | 2.302 | 53.62 | 3.85E-09 |
| | MS0257 | H54-SG181.MS0257/L28-SK1 | 2.281 | 70.18 | 3.82E-09 |
| | MS0258 | H54-SG181.MS0258/L28-SK1 | 1.489 | 72.52 | 3.81E-09 |
| Val-Leu insertion | MS0259 | H54-SG181.MS0259/L28-SK1 | 2.309 | 70.73 | 3.83E-09 |
| | MS0260 | H54-SG181.MS0260/L28-SK1 | 2.123 | 71.01 | 3.71E-09 |
| | MS0261 | H54-SG181.MS0261/L28-SK1 | 2.344 | 69.63 | 3.83E-09 |
| | MS0262 | H54-SG181.MS0262/L28-SK1 | 2.281 | 71.71 | 3.60E-09 |
| | MS0263 | H54-SG181.MS0263/L28-SK1 | 2.230 | 70.04 | 3.85E-09 |
| | MS0264 | H54-SG181.MS0264/L28-SK1 | 1.583 | 70.45 | 3.88E-09 |
| | MS0265 | H54-SG181.MS0265/L28-SK1 | 2.118 | 69.90 | 3.81E-09 |
| | MS0266 | H54-SG181.MS0266/L28-SK1 | 2.259 | 70.32 | 3.89E-09 |
| | MS0267 | H54-SG181.MS0267/L28-SK1 | 2.218 | 69.90 | 3.91E-09 |
| | MS0268 | H54-SG181.MS0268/L28-SK1 | 2.249 | 71.01 | 3.77E-09 |
| | MS0269 | H54-SG181.MS0269/L28-SK1 | 2.098 | 71.56 | 3.81E-09 |
| | MS0270 | H54-SG181.MS0270/L28-SK1 | 2.351 | 69.21 | 3.78E-09 |
| Val-Phe insertion | MS0271 | H54-SG181.MS0271/L28-SK1 | 2.204 | 70.45 | 3.90E-09 |
| | MS0272 | H54-SG181.MS0272/L28-SK1 | 2.341 | 70.04 | 3.86E-09 |
| | MS0273 | H54-SG181.MS0273/L28-SK1 | 2.353 | 70.45 | 3.81E-09 |
| | MS0274 | H54-SG181.MS0274/L28-SK1 | 2.273 | 70.04 | 3.88E-09 |
| | MS0275 | H54-SG181.MS0275/L28-SK1 | 2.151 | 70.87 | 3.83E-09 |
| | MS0276 | H54-SG181.MS0276/L28-SK1 | 2.131 | 71.42 | 3.80E-09 |
| | MS0277 | H54-SG181.MS0277/L28-SK1 | 2.224 | 69.49 | 3.89E-09 |

**(Table 5-9) Concentration after purification, Tm value, and antigen-binding activity of modified antibodies**

| Modification type | Modified peptide | Name of modified antibody | Concentration after purification (mg/mL) | Lowest denaturation midpoint temperature (°C) | KD[M] |
|---|---|---|---|---|---|
| | MS0278 | H54-SG181.MS0278/L28-SK1 | 2.271 | 69.76 | 3.90E-09 |
| | MS0279 | H54-SG181.MS0279/L28-SK1 | 2.280 | 70.73 | 3.89E-09 |
| | MS0280 | H54-SG181.MS0280/L28-SK1 | 2.347 | 70.73 | 3.81E-09 |
| | MS0281 | H54-SG181.MS0281/L28-SK1 | 2.277 | 70.45 | 3.72E-09 |
| | MS0282 | H54-SG181.MS0282/L28-SK1 | 2.152 | 70.73 | 3.83E-09 |
| Ala-Ala-Ala insertion | MS0283 | H54-SG181.MS0283/L28-SK1 | 2.210 | 70.18 | 3.89E-09 |
| | MS0284 | H54-SG181.MS0284/L28-SK1 | 2.069 | 70.73 | 3.82E-09 |
| | MS0285 | H54-SG181.MS0285/L28-SK1 | 1.886 | 70.18 | 3.81E-09 |
| | MS0286 | H54-SG181.MS0286/L28-SK1 | 1.810 | 71.70 | 3.74E-09 |
| | MS0287 | H54-SG181.MS0287/L28-SK1 | 2.159 | 71.42 | 3.88E-09 |
| | MS0288 | H54-SG181.MS0288/L28-SK1 | 1.704 | 55.00 | 3.69E-09 |
| | MS0289 | H54-SG181.MS0289/L28-SK1 | 1.877 | 72.39 | 3.56E-09 |

### Example 3. Differential and simultaneous quantification of multiple antibodies in plasma using a high-performance liquid chromatography-coupled electrospray ionization mass spectrometer (LC/ESI-MS/MS)

### 3-1. Experimental method

### 3-1-1. Preparation of unmodified antibody and modified antibodies with alanine sub stitution/insertion

The antibody before modification H54-SG181/L28-SK1 in Tables 4-1 to 4-9, the modified antibodies shown in Table 6, and an antibody in which "V", "L", and "K" were labeled with stable isotopes in GPSVFPLAPSSK (SEQ ID NO: 5) comprised in the human IgG1 CH1 domain of the antibody before modification H54-SG181/L28-SK1 (internal standard), were synthesized.

**(Table 6) Alanine modified antibodies**

| Modified peptide corresponding to GPSVFPLAPSSK (SEQ ID NO: 5) of the human native IgG CH1 domain | | | | Modified antibody comprising the modified peptide in the left instead of GPSVFPLAPSSK (SEQ ID NO: 5) in the CH1 domain | |
|---|---|---|---|---|---|
| Modification type | Peptide name | Sequence | SEQ ID NO | Name | Name (another name) |
| | MS0001 | **A**PSVFPLAPSSK | 6 | H54-SG181.MS0001/L28-SK1 | H54-SG181.MS1 |
| | MS0002 | G**A**SVFPLAPSSK | 7 | H54-SG181.MS0002/L28-SK1 | H54-SG181.MS2 |
| | MS0003 | GP**A**VFPLAPSSK | 8 | H54-SG181.MS0003/L28-SK1 | H54-SG181.MS3 |
| | MS0004 | GPS**A**FPLAPSSK | 9 | H54-SG 181. MS0004/L28-SK1 | H54-SG181.MS4 |
| Ala substitution | MS0005 | GPSV**A**PLAPSSK | 1 0 | H54-SG181.MS0005/L28-SK1 | H54-SG181.MS5 |
| | MS0006 | GPSVF**A**LAPSSK | 1 1 | H54-SG181.MS0006/L28-SK1 | H54-SG181.MS6 |
| | MS0007 | GPSVFP**A**APSSK | 1 2 | H54-SG181.MS0007/L28-SK1 | H54-SG181.MS7 |
| | MS0008 | GPSVFPL**A**ASSK | 1 3 | H54-SG181.MS0008/L28-SK1 | H54-SG181.MS8 |
| | MS0009 | GPSVFPLAP**A**SK | 1 4 | H54-SG181.MS0009/L28-SK1 | H54-SG181.MS9 |
| | MS0010 | GPSVFPLAPS**A**K | 1 5 | H54-SG181.MS0010/L28-SK1 | H54-SG181.MS10 |
| | MS0011 | **A**GPSVFPLAPSSK | 1 6 | H54-SG181.MS0011/L28-SK1 | H54-SG181.MS11 |
| | MS0012 | G**A**PSVFPLAPSSK | 1 7 | H54-SG181.MS0012/L28-SK1 | H54-SG181.MS12 |
| | MS0013 | GP**A**SVFPLAPSSK | 1 8 | H54-SG181.MS0013/L28-SK1 | H54-SG181.MS13 |
| | MS0014 | GPS**A**VFPLAPSSK | 1 9 | H54-SG181.MS0014/L28-SK1 | H54-SG181.MS14 |
| Ala insertion | MS0015 | GPSV**A**FPLAPSSK | 2 0 | H54-SG181.MS0015/L28-SK1 | H54-SG181.MS15 |
| | MS0016 | GPSVF**A**PLAPSSK | 2 1 | H54-SG181.MS0016/L28-SK1 | H54-SG181.MS16 |
| | MS0017 | GPSVFP**A**LAPSSK | 2 2 | H54-SG181.MS0017/L28-SK1 | H54-SG181.MS17 |
| | MS0018 | GPSVFPL**A**APSSK | 2 3 | H54-SG181.MS0018/L28-SK1 | H54-SG181.MS18 |
| | MS0019 | GPSVFPLAP**A**SSK | 2 4 | H54-SG181.MS0019/L28-SK1 | H54-SG181.MS19 |
| | MS0020 | GPSVFPLAPS**A**SK | 2 5 | H54-SG181.MS0020/L28-SK1 | H54-SG181.MS20 |
| | MS0021 | GPSVFPLAPSS**A**K | 2 6 | H54-SG181.MS0021/L28-SK1 | H54-SG181.MS21 |
| | MS0022 | **AA**GPSVFPLAPSSK | 2 7 | H54-SG181.MS0022/L28-SK1 | H54-SG181.MS22 |
| | MS0023 | G**AA**PSVFPLAPSSK | 2 8 | H54-SG181.MS0023/L28-SK1 | H54-SG181.MS23 |
| | MS0024 | GP**AA**SVFPLAPSSK | 2 9 | H54-SG181.MS0024/L28-SK1 | H54-SG181.MS24 |
| | MS0025 | GPS**AA**VFPLAPSSK | 3 0 | H54-SG181.MS0025/L28-SK1 | H54-SG181.MS25 |
| Ala-Ala insertion | MS0026 | GPSV**AA**FPLAPSSK | 3 1 | H54-SG181.MS0026/L28-SK1 | H54-SG181.MS26 |
| | MS0027 | GPSVF**AA**PLAPSSK | 3 2 | H54-SG181.MS0027/L28-SK1 | H54-SG181.MS27 |
| | MS0028 | GPSVFP**AA**LAPSSK | 3 3 | H54-SG181.MS0028/L28-SK1 | H54-SG181.MS28 |
| | MS0029 | GPSVFPL**AA**APSSK | 3 4 | H54-SG181.MS0029/L28-SK1 | H54-SG181.MS29 |
| | MS0030 | GPSVFPLAP**AA**SSK | 3 5 | H54-SG181.MS0030/L28-SK1 | H54-SG181.MS30 |
| | MS0031 | GPSVFPLAPS**AA**SK | 3 6 | H54-SG181.MS0031/L28-SK1 | H54-SG181.MS31 |
| | MS0032 | GPSVFPLAPSS**AA**K | 3 7 | H54-SG181.MS0032/L28-SK1 | H54-SG181.MS32 |

### 3-1-2. Examination of the possibility of differential and simultaneous measurement of alanine-modified antibodies

The antibodies synthesized in 3-1-1 were subjected to the enzymatic treatment described below to generate GPSVFPLAPSSK (SEQ ID NO: 5) and the modified peptide fragments shown in Table 6, and combinations of precursor ions and product ions in selected reaction monitoring (SRM) were determined to examine the possibility of differential and simultaneous measurement of each peptide fragment. From the perspective of specificity (not to overlap with the measurement peak of a blank sample comprising no antibody), a product ion having a m/z value greater than the m/z value of the precursor ion was combined.

2 µL of each antibody sample, prepared to 50 µg/mL using 50 mmol/L ammonium bicarbonate, were suspended in 25 µL of 50 mmol/L ammonium bicarbonate containing 7.5 mol/L urea and 8 mmol/L dithiothreitol, and then shaken at 56°C for 45 minutes. 2 µL of 500 mmol/L iodoacetamide was then added, and mixtures were shaken at 37°C for 30 minutes in the dark. 160 µL of suspension buffer containing 0.625 µg/mL sequencing grade modified trypsin (Promega) was then added. After shaking at 37°C for 16 hours, 5 µL of 10% trifluoroacetic acid was added to prepare enzymatically digested samples. 5 µL of each enzymatically digested sample were used for analysis with LC/ESI-MS/MS.

In addition, samples prepared by adding each antibody sample to mouse plasma, or mouse plasma to which no antibody sample was added, were subjected to the same enzymatic treatment as above and used for analysis with LC/ESI-MS/MS.

### 3-1-3. Preparation of isoleucine-substituted and valine-substituted modified antibodies and examination of the possibility of differential and simultaneous measurement

The modified antibodies shown in Table 7 were synthesized, and the possibility of differential and simultaneous measurement of the modified peptide fragments shown in Table 7 comprised in each antibody was examined. The method of enzymatic treatment was the same as that described in 3-1-2, except that the concentration of each antibody sample was 25 µg/mL.

**(Table 7) Isoleucine-substituted and valine-substituted modified antibodies**

| Modified peptide corresponding to GPSVFPLAPSSK (SEQ ID NO: 5) of the human native IgG CH1 domain | | | | Modified antibody comprising the modified peptide in the left instead of GPSVFPLAPSSK (SEQ ID NO: 5) in the CH1 domain | |
|---|---|---|---|---|---|
| Modification type | Peptide name | Sequence | SEQ ID NO | Name | Name (another name) |
| | MS0033 | IPSVFPLAPSSK | 3 8 | H54-SG181.MS0033/L28-SK1 | H54-SG181.MS33 |
| | MS0034 | GISVFPLAPSSK | 3 9 | H54-SG181.MS0034/L28-SK1 | H54-SG181.MS34 |
| | MS0035 | GPIVFPLAPSSK | 4 0 | H54-SG181.MS0035/L28-SK1 | H54-SG181.MS35 |
| | MS0036 | GPSIFPLAPSSK | 4 1 | H54-SG181.MS0036/L28-SK1 | H54-SG181.MS36 |
| Ile substitution | MS0037 | GPSVIPLAPSSK | 4 2 | H54-SG181.MS0037/L28-SK1 | H54-SG181.MS37 |
| | MS0038 | GPSVFILAPSSK | 4 3 | H54-SG181.MS0038/L28-SK1 | H54-SG181.MS38 |
| | MS0039 | GPSVFPIAPSSK | 44 | H54-SG181.MS0039/L28-SK1 | H54-SG181.MS39 |
| | MS0040 | GPSVFPLIPSSK | 4 5 | H54-SG 181. MS0040/L28-SK1 | H54-SG181.MS40 |
| | MS0041 | GPSVFPLAISSK | 4 6 | H54-SG181.MS0041/L28-SK1 | H54-SG181.MS41 |
| | MS0042 | GPSVFPLAPISK | 4 7 | H54-SG181.MS0042/L28-SK1 | H54-SG181.MS42 |
| | MS0043 | GPSVFPLAPSIK | 48 | H54-SG181.MS0043/L28-SK1 | H54-SG181.MS43 |
| | MS0068 | VPSVFPLAPSSK | 7 3 | H54-SG181.MS0068/L28-SK1 | H54-SG181.MS68 |
| | MS0069 | GVSVFPLAPSSK | 7 4 | H54-SG181.MS0069/L28-SK1 | H54-SG181.MS69 |
| | MS0070 | GPVVFPLAPSSK | 7 5 | H54-SG181.MS0070/L28-SK1 | H54-SG181.MS70 |
| Val substitution | MS0071 | GPSVVPLAPSSK | 7 6 | H54-SG181.MS0071/L28-SK1 | H54-SG181.MS71 |
| | MS0072 | GPSVFVLAPSSK | 7 7 | H54-SG181.MS0072/L28-SK1 | H54-SG181.MS72 |
| | MS0073 | GPSVFPVAPSSK | 7 8 | H54-SG181.MS0073/L28-SK1 | H54-SG181.MS73 |
| | MS0074 | GPSVFPLVPSSK | 7 9 | H54-SG181.MS0074/L28-SK1 | H54-SG181.MS74 |
| | MS0075 | GPSVFPLAVSSK | 8 0 | H54-SG181.MS0075/L28-SK1 | H54-SG181.MS75 |
| | MS0076 | GPSVFPLAPVSK | 8 1 | H54-SG181.MS0076/L28-SK1 | H54-SG181.MS76 |
| | MS0077 | GPSVFPLAPSVK | 8 2 | H54-SG181.MS0077/L28-SK1 | H54-SG181.MS77 |

### 3-1-4. Plasma pharmacokinetic study of modified antibodies

To investigate pharmacokinetics (PK) of the modified antibodies in plasma, the following antibodies were administered at 1 mg/kg to C57BL/6J mice (6-8 weeks old, male) via the tail vein, by single administration or mixed administration. The administered antibodies were H54-SG181, H54-SG181.MS1, H54-SG181.MS2, H54-SG181.MS3, H54-SG181.MS5, H54-SG181.MS7, H54-SG181.MS8, H54-SG181.MS9, H54-SG181.MS12, H54-SG181.MS19, H54-SG181.MS22, and H54-SG181.M31. Blood was collected over time up to 28 days after administration, and centrifuged (12,000 rpm, 4°C, 5 min) to obtain plasma.

### 3-1-5. Plasma sample pretreatment

The concentrations of the modified antibodies in mouse plasma were measured by LC/ESI-MS/MS. 2 µL of each plasma sample were added to 50 µL of magnetic beads (Magnosphere MS300/Low Carboxyl, JSR) to which anti-Fc antibody was immobilized, as described in WO2019/112027, and mixtures were shaken at 25°C for 1.5 hours. The magnetic beads in the samples were washed three times with 0.2 mL of PBS containing 0.05% Tween 20, followed by another wash with 0.2 mL PBS. The magnetic beads were suspended in 25 µL of 50 mmol/L ammonium bicarbonate containing 7.5 mol/L urea, 8 mmol/L dithiothreitol, and 0.160 µg/mL of the internal standard, and then shaken at 56°C for 45 minutes. Then, 2 µL of 500 mmol/L iodoacetamide was added, and mixtures were shaken at 37°C for 30 minutes in the dark. Next, 160 µL of suspension buffer containing 0.625 µg/mL sequencing grade modified trypsin (Promega) was added. The samples were shaken at 37°C for 16 hours, and 5 µL of 10% trifluoroacetic acid was added to prepare enzymatically digested samples. 80 µL of each enzymatically digested sample were used for analysis with LC/ESI-MS/MS. The internal standard was as described in 3-1-1.

The concentrations of each antibody were calculated using internal calibration curves. To create the internal calibration curves, calibration curve samples were prepared by preparing a mixed standard stock solution in which antibodies used in the mixed administration in 3-1-4 were mixed, and serially diluting the mixed standard stock solution with mouse plasma to adjust the concentration of each antibody in mouse plasma to a range of 0.0200 to 5.12 µg/mL. The calibration curve samples were pretreated in the same way as the plasma samples described above. The internal calibration curves were generated by linear regression with a weighting of 1/x², using antibody concentration as the horizontal axis and peak area ratio (peak area of antibody sample / peak area of internal standard) as the vertical axis. As the result of creating calibration curves for the antibodies, it was shown that the lower limit of quantification (LLOQ) differs depending on the antibody: the LLOQs for antibodies including MS0002, MS0005, MS0007, MS0012, and MS0022 were 40 ng/ml, the LLOQs for antibodies including MS0008, MS0009, MS0019, and MS0031 and the unmodified antibody were 160 ng/ml, and the LLOQs for antibodies including MS0001 and MS0003 were 320 ng/ml.

In addition, plasma samples individually spiked with each antibody were prepared by individually adding a standard solution of each antibody to mouse plasma, so that the antibody concentration in mouse plasma would be adjusted to 1.28 µg/mL. The plasma samples individually spiked with each antibody were pretreated in the same way as the plasma samples described above.

### 3-1-6. LC/ESI-MS/MS analysis

LC/ESI-MS/MS was performed using a Xevo TQ-XS triple quadrupole instrument (Waters) coupled to an ACQUITY UPLC I-class (Waters). The LC and MS conditions for 3-1-4 are shown in Tables 8-1 and 8-2. GPSV^{*}FPL^{*}APSSK^{*} is an antibody in which "V", "L", and "K" in GPSVFPLAPSSK (SEQ ID NO: 5) are labeled with stable isotopes (internal standard, IS).

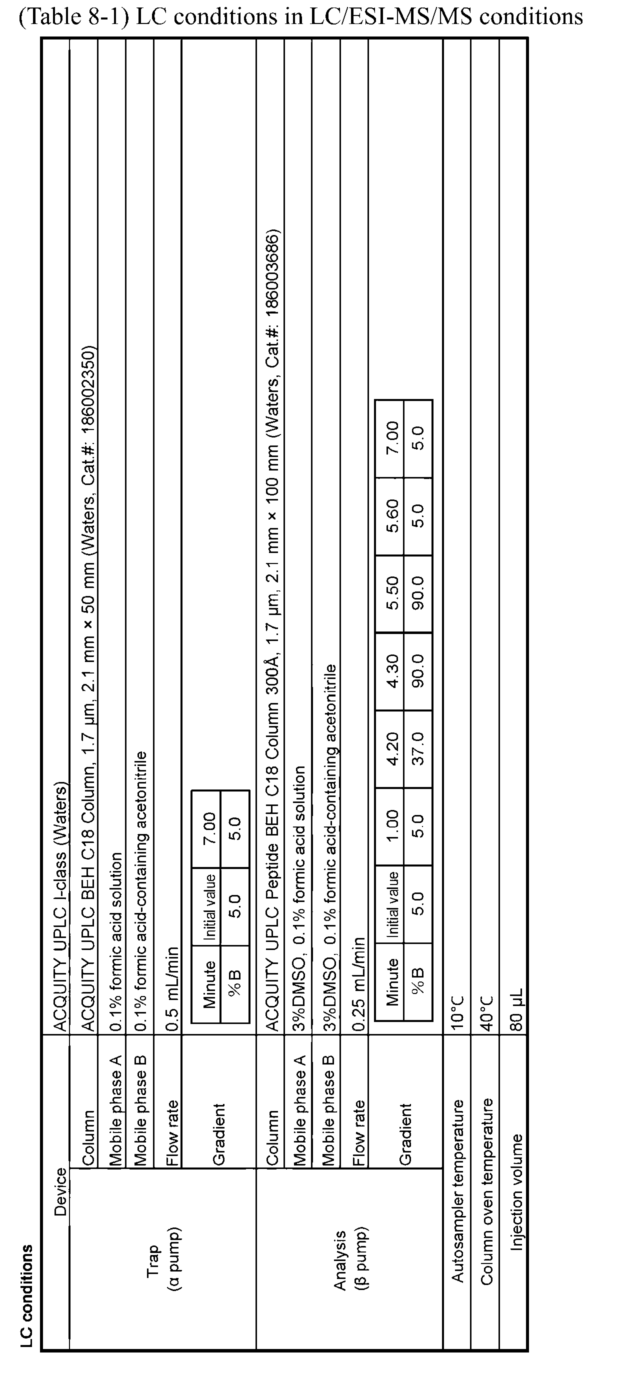

**(Table 8-2) MS/MS conditons in LC/ESI-MS/MS conditions**

| **MS** conditions | | | | | | | |
|---|---|---|---|---|---|---|---|
| Device | Xevo TQ-XS triple quadrupole instrument (Waters) | | | | | | |
| Ionization/polarity | ESI/positive | | | | | | |
| Ion source temperature | 150°C | | | | | | |
| | Name | Sequence of peptide fragment of precursor ion | Precursor ion **(*m*/*z*)** | Product ion **(*m*/*z*)** | Cone (V) | Collision (V) | y |
| | Unmodified | GPSVFPLAPSSK (SEQ ID NO: 5) | 593.8 | 699.4 | 35 | 21 | 7 |
| | MS0001 | **A**PSVFPLAPSSK (SEQ ID NO:6) | 600.8 | 699.4 | 35 | 21 | 7 |
| | MS0002 | G**A**SVFPLAPSSK (SEQ ID NO:7) | 580.8 | 699.4 | 35 | 20 | 7 |
| | MS0003 | GP**A**VFPLAPSSK (SEQ ID NO:8) | 585.8 | 699.4 | 35 | 21 | 7 |
| | MS0005 | GPSV**A**PLAPSSK(SEQ ID NO: 10) | 555.8 | 699.4 | 35 | 19 | 7 |
| | MS0007 | GPSVFP**A**APSSK(SEQ ID NO: 12) | 572.8 | 657.4 | 35 | 20 | 7 |
| | MS0008 | GPSVFPLA**A**SSK(SEQ ID NO:13) | 580.8 | 673.4 | 35 | 20 | 7 |
| MS parameters | MS0009 | GPSVFPLAP**A**SK (SEQ ID NO:14) | 585.8 | 683.4 | 35 | 21 | 7 |
| | MS0012 | G**A**PSVFPLAPSSK (SEQ ID NO: 17) | 629.3 | 699.4 | 35 | 22 | 7 |
| | MS0019 | GPSVFPLAP**A**SSK (SEQ ID NO: 24) | 629.3 | 770.4 | 35 | 22 | 8 |
| | MS0022 | **AA**GPSVFPLAPSSK (SEQ ID NO: 27) | 664.9 | 699.4 | 35 | 24 | 7 |
| | MS0031 | GPSVFPLAPS**AA**SK (SEQ ID NO: 36) | 664.9 | 841.5 | 35 | 24 | 9 |
| | IS | GPSV 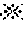 FPL 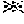 APSSK 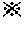 (SEQ ID NO: 5) | 603.9 | 713.4 | 35 | 21 | 7 |
| | | | | | | | |

### 3-2. Results and Discussion

### 3-2-1. Possibility of differential and simultaneous measurement of alanine-modified antibodies in buffer

Table 9 shows the SRM transitions set for the modified peptide fragments (alanine substituted/inserted modified peptides) shown in Table 6, and the peak intensity ratios of the modified peptides to the peptide fragment of GPSVFPLAPSSK (SEQ ID NO: 5) (unmodified peptide). Since most alanine substituted/inserted modified peptides showed peak intensities comparable to that of the unmodified peptide, antibodies comprising any of the modified peptides were considered to be applicable as candidates of multiple antibodies that are to be simultaneously administered. Among these candidates, peptides with different SRM transitions, MS0001, MS0002, MS0003, MS0005, MS0007, MS0008, MS0009, MS0012, MS0019, MS0022, and MS0031 (abbreviations), were selected as peptide sequences that may be simultaneously and differentially measured, and subjected to [3-1-4. Plasma pharmacokinetic study of modified antibodies] using antibodies comprising these peptides instead of GPSVFPLAPSSK (SEQ ID NO: 5). Considering the influence of isotope peaks, abbreviated name MS0002 was selected from peptide sequences that differ by only one m/z from each other (abbreviated name: MS0002 and MS0004), but MS0004 can be selected instead of MS0002. Furthermore, when using high-resolution MS that can distinguish between multiple peptides that differ by only one m/z, MS0004 can be selected along with MS0002.

LC/ESI-MS/MS analysis of mouse plasma samples to which antibodies comprising MS0001, MS0002, MS0003, MS0005, MS0007, MS0008, MS0009, MS0012, MS0019, MS0022, or MS0031 instead of GPSVFPLAPSSK (SEQ ID NO: 5) had been added confirmed that none of these peptides overlapped with the measurement peak of mouse plasma that comprises no antibody.

**(Table 9) SRM transitions of alanine-substituted/inserted modified peptides and peak intensity ratios of modified peptides to unmodified peptide**

| Ala-substituted modified peptides | | | | | |
|---|---|---|---|---|---|
| Peptide name | Sequence | SEQ ID NO: | Precursor ion **(*m*/*z*)** | Product ion **(*m*/*z*)** | Peak intensity of modified peptide/ peak intensity of unmodified peptide |
| Unmodified | GPSVFPLAPSSK | 5 | 593.8 | 699.4 | |
| **MS0001** | **A**PSVFPLAPSSK | 6 | 600.8 | 699.4 | **0.619** |
| **MS0002** | G**A**SVFPLAPSSK | 7 | 580.8 | 699.4 | **1.68** |
| **MS0003** | GP**A**VFPLAPSSK | 8 | 585.8 | 699.4 | **0.743** |
| MS0004 | GPS**A**FPLAPSSK | 9 | 579.8 | 699.4 | 1.27 |
| **MS0005** | GPSV**A**PLAPSSK | 1 0 | 555.8 | 699.4 | **1.13** |
| MS0006 | GPSVF**A**LAPSSK | 1 1 | 580.8 | 673.4 | 0.204 |
| **MS0007** | GPSVFP**A**APSSK | 1 2 | 572.8 | 657.4 | **1.23** |
| **MS0008** | GPSVFPL**A**ASSK | 1 3 | 580.8 | 673.4 | **0.631** |
| **MS0009** | GPSVFPLAP**A**SK | 1 4 | 585.8 | 683.4 | **0.766** |
| MS0010 | GPSVFPLAPS**A**K | 1 5 | 585.8 | 683.4 | 0.668 |

| Ala-inserted modified peptides | | | | | |
|---|---|---|---|---|---|
| Peptide name | Sequence | SEQ ID NO: | Precursor ion **(*m*/*z*)** | Product ion **(*m*/*z*)** | Peak intensity of modified peptide/ peak intensity of unmodified peptide |
| Unmodified | GPSVFPLAPSSK | 5 | 593.8 | 699.4 | - |
| MS0011 | **A**GPSVFPLAPSSK | 1 6 | 629.3 | 699.4 | 0.744 |
| **MS0012** | G**A**PSVFPLAPSSK | 1 7 | 629.3 | 699.4 | **0.700** |
| MS0013 | GP**A**SVFPLAPSSK | 1 8 | 629.3 | 699.4 | 0.379 |
| MS0014 | GPS**A**VFPLAPSSK | 1 9 | 629.3 | 699.4 | 0.490 |
| MS0015 | GPSV**A**FPLAPSSK | 2 0 | 629.3 | 699.4 | 0.373 |
| MS0016 | GPSVF**A**PLAPSSK | 2 1 | 629.3 | 699.4 | 0.496 |
| MS0017 | GPSVFP**A**LAPSSK | 2 2 | 629.3 | 770.4 | 0.640 |
| MS0018 | GPSVFPL**A**APSSK | 23 | 629.3 | 770.4 | 0.452 |
| **MS0019** | GPSVFPLAP**A**SSK | 24 | 629.3 | 770.4 | **0.714** |
| MS0020 | GPSVFPLAPS**A**SK | 25 | 629.3 | 770.4 | 0.550 |
| MS0021 | GPSVFPLAPSS**A**K | 26 | 629.3 | 770.4 | 0.665 |

| Ala-Ala-inserted modified peptides | | | | | |
|---|---|---|---|---|---|
| Peptide name | Sequence | SEQ ID NO: | Precursor ion **(*m*/*z*)** | Product ion **(*m*/*z*)** | Peak intensity of modified peptide/ peak intensity of unmodified peptide |
| Unmodified | GPSVFPLAPSSK | 5 | 593.8 | 699.4 | - |
| **MS0022** | **AA**GPSVFPLAPSSK | 2 7 | 664.9 | 699.4 | **1.75** |
| MS0023 | G**AA**PSVFPLAPSSK | 2 8 | 664.9 | 699.4 | 1.47 |
| MS0024 | GP**AA**SVFPLAPSSK | 2 9 | 664.9 | 699.4 | 0.368 |
| MS0025 | GPS**AA**VFPLAPSSK | 30 | 664.9 | 699.4 | 0.363 |
| MS0026 | GPSV**AA**FPLAPSSK | 3 1 | 664.9 | 699.4 | 0.670 |
| MS0027 | GPSVF**AA**PLAPSSK | 32 | 664.9 | 699.4 | 0.792 |
| MS0028 | GPSVFP**AA**LAPSSK | 33 | 664.9 | 841.5 | 0.753 |
| MS0029 | GPSVFPL**AA**APSSK | 34 | 664.9 | 841.5 | 0.708 |
| MS0030 | GPSVFPLAP**AA**SSK | 3 5 | 664.9 | 841.5 | 0.925 |
| **MS0031** | GPSVFPLAPS**AA**SK | 3 6 | 664.9 | 841.5 | **1.04** |
| MS0032 | GPSVFPLAPSS**AA**K | 37 | 664.9 | 841.5 | 1.14 |

### 3-2-2. Possibility of differential and simultaneous measurement of isoleucine-substituted and valine-substituted modified antibodies

Table 10 shows the SRM transitions set for the modified peptide fragments (isoleucine-substituted and valine-substituted modified peptides) shown in Table 7, and the peak intensity ratios of the modified peptides to the peptide fragment GPSVFPLAPSSK (SEQ ID NO: 5) (unmodified peptide). Isoleucine-substituted and valine-substituted modified peptides also showed peak intensities roughly equivalent to that of the unmodified peptide. A plurality of modified peptides with SRM transitions different from those of the peptides selected in 3-2-1 (MS0033, MS0035, MS0037, MS0040, MS0041, MS0042, MS0068, MS0070, MS0071, MS0073, MS0074, MS0075, and MS0077) were found, and thus, it was considered that further adding antibodies comprising these modified peptides would enable the differential and simultaneous measurement of more antibodies.

**(Table 10) SRM transitions of isoleucine-substituted and valine-substituted modified peptides and peak intensity ratios of modified peptides to unmodified peptide**

| **Isoleucine-substituted modified peptides** | | | | | |
|---|---|---|---|---|---|
| **Peptide name** | **Sequence** | **SEQ ID NO:** | **Precursor ion (*m*/*z*)** | **Product ion (*m*/*z*)** | **Peak intensity of modified peptide/ peak intensity of unmodified peptide** |
| Unmodified | GPSVFPLAPSSK | 5 | 593.8 | 699.4 | - |
| MS0033 | IPSVFPLAPSSK | 3 8 | 621.9 | 699.4 | 0.291 |
| MS0034 | G**I**SVFPLAPSSK | 3 9 | 601.8 | 699.4 | 1.26 |
| MS0035 | GP**I**VFPLAPSSK | 4 0 | 606.9 | 699.4 | 0.502 |
| MS0036 | GPS**I**FPLAPSSK | 4 1 | 600.8 | 699.4 | 0.706 |
| MS0037 | GPSV**I**PLAPSSK | 4 2 | 576.8 | 699.4 | 2.35 |
| MS0038 | GPSVF**I**LAPSSK | 4 3 | 601.8 | 862.5 | 0.729 |
| MS0039 | GPSVFP**I**APSSK | 4 4 | 593.8 | 699.4 | 1.74 |
| MS0040 | GPSVFPL**I**PSSK | 4 5 | 614.9 | 741.5 | 0.408 |
| MS0041 | GPSVFPLA**I**SSK | 4 6 | 601.8 | 715.4 | 0.675 |
| MS0042 | GPSVFPLAP**I**SK | 4 7 | 606.9 | 725.5 | 0.831 |
| MS0043 | GPSVFPLAPS**I**K | 4 8 | 606.9 | 725.5 | 0.624 |

| **Valine-substituted modified peptides** | | | | | |
|---|---|---|---|---|---|
| **Peptide name** | **Sequence** | **SEQ ID NO:** | **Precursor ion (*m*/*z*)** | **Product ion (*m*/*z*)** | **Peak intensity of modified peptide/ peak intensity of unmodified peptide** |
| Unmodified | GPSVFPLAPSSK | 5 | 593.8 | 699.4 | - |
| MS0068 | **V**PSVFPLAPSSK | 7 3 | 614.9 | 699.4 | 0.234 |
| MS0069 | G**V**SVFPLAPSSK | 7 4 | 594.8 | 699.4 | 1.19 |
| MS0070 | GP**V**VFPLAPSSK | 7 5 | 599.8 | 699.4 | 0.431 |
| MS0071 | GPS**V**VPLAPSSK | 7 6 | 569.8 | 699.4 | 2.49 |
| MS0072 | GPSVF**V**LAPSSK | 7 7 | 594.8 | 848.5 | 0.274 |
| MS0073 | GPSVFP**V**APSSK | 7 8 | 586.8 | 685.4 | 0.718 |
| MS0074 | GPSVFPL**V**PSSK | 7 9 | 607.8 | 727.4 | 0.403 |
| MS0075 | GPSVFPLA**V**SSK | 8 0 | 594.8 | 701.4 | 0.385 |
| MS0077 | GPSVFPLAPS**V**K | 8 2 | 599.8 | 711.4 | 0.421 |

### 3-2-3. Pharmacokinetic test

### 3-2-3-1. Analysis

The internal calibration curves of each modified antibody are shown in Figures 1-1 to 1-12. Good linearity was observed for all measured antibodies (correlation coefficient: r > 0.979).

Five minutes after mixed administration of the antibodies, blood was drawn from the mice and plasma was obtained as described in 3-1-4. The plasma sample was then subjected to the pretreatment described in 3-1-5 and the LC/ESI-MS/MS analysis described in 3-1-6, and the SRM chromatogram of each peptide obtained from the sample are shown in Figures 2-1 to 2-4. As shown in Figures 2-1 to 2-4, peaks of each peptide were detected in the mouse blood collected five minutes after the mixed administration, and all peaks had good shapes.

Figures 3A-1 to 3L-3 show the SRM chromatograms resulting from the LC/ESI-MS/MS analysis of the plasma samples individually spiked with each antibody prepared in 3-1-5. As shown in Figures 3A-1 to 3L-3, the plasma samples individually spiked with each antibody showed peaks only in the SRM transition which is set for the peptide derived from the added antibody, and showed no peaks in the SRM transition which is set for the peptide derived from the antibody which was not added, demonstrating that the peptides did not interfere with each other in the LC/ESI-MS/MS measurement.

### 3-2-3-2. PK profile

### 3-2-3-2-1. Single administration

The plasma antibody concentration-time curves are shown in Figures 4-1 to 4-6. For all antibodies, the PK profiles of the modified molecules were similar to that of the original molecule, H54-SG181, and there were no significant differences among antibodies.

The PK parameters of each antibody molecule at single administration, calculated using Phoenix WinNonlin (Certara), are shown in Table 11. The PK parameters of the modified molecules were equivalent to the PK parameters of the original molecule, H54-SG181, and there were no significant differences among antibodies.

**(Table 11) PK parameters of each molecule at single administration**

| **Antibody** | **T_{1/2} day** | **AUC day*ng/mL** | **Clearance Vdₛₛ mL/day/kg mL/kg** | |
|---|---|---|---|---|
| H54-SG181 | 31.1 | 483370 | 2.17 | 91.8 |
| H54-SG181.MS2 | 30.5 | 439466 | 2.51 | 97.7 |
| H54-SG181.MS5 | 22.9 | 373554 | 2.7 | 87.2 |
| H54-SG181.MS7 | 39.3 | 555000 | 1.82 | 103.4 |
| H54-SG181.MS12 | 22.4 | 354447 | 2.87 | 92 |
| H54-SG181.MS22 | 21 | 381213 | 2.77 | 80 |

### 3-2-3-2-1. Mixed administration

The plasma antibody concentration-time curves are shown in Figure 5. For all antibodies, the PK profiles of the modified molecules were similar to that of the original molecule, H54-SG181. Furthermore, the PK profiles of each molecule at mixed administration were similar to the PK profiles at single administration.

The PK parameters of the antibody at mixed administration, calculated using Phoenix WinNonlin (Certara), are shown in Table 12. The PK parameters of the modified molecules were equivalent to the PK parameters of the original molecule, H54-SG181. In addition, the PK parameters at mixed administration were comparable to the PK parameters at single administration shown in Table 11.

**(Table 12) PK parameters at mixed administration**

| **Antibody** | **T_{1/2} day** | **AUC day*ng/mL** | **Clearance mL/day/kg** | **Vdₛₛ mL/kg** |
|---|---|---|---|---|
| H54-SG181 | 20.7 | 370388 | 3.08 | 80.3 |
| H54-SG181.MS1 | 14.9 | 238090 | 4.31 | 88.8 |
| H54-SG181.MS2 | 20.7 | 391096 | 2.61 | 77.8 |
| H54-SG181.MS3 | 18.4 | 390306 | 2.57 | 72 |
| H54-SG181.MS5 | 15.2 | 273917 | 3.65 | 81.6 |
| H54-SG181.MS7 | 20.1 | 319555 | 3.23 | 93 |
| H54-SG181.MS8 | 19 | 402556 | 2.6 | 68.7 |
| H54-SG181.MS9 | 19.6 | 356967 | 2.87 | 81.6 |
| H54-SG181.MS12 | 15.6 | 282333 | 3.55 | 82 |
| H54-SG181.MS19 | 19.3 | 356540 | 2.92 | 84.3 |
| H54-SG181.MS22 | 16.2 | 280494 | 3.57 | 87.2 |
| H54-SG181.MS31 | 21.1 | 300474 | 3.48 | 100 |

The foregoing disclosure is described in detail with examples and illustrations for the purpose of facilitating clear understanding, but the descriptions and illustrations herein should not be construed as limiting the scope of the disclosure.

### [Industrial Applicability]

By using the modified antigen-binding molecules of the present application, it is possible to distinguish each of multiple types of modified antigen-binding molecules or distinguish between unmodified and modified antigen-binding molecule and measure their pharmacokinetics and tissue distribution. For example, when an antibody comprising a modified peptide portion in place of a peptide portion for mass spectrometry in a humanized antibody or human antibody is administered to an animal, it is possible to measure its pharmacokinetics and tissue distribution while distinguishing the administered antibody from an antibody that does not comprise the modified peptide portion. Furthermore, simultaneous administration of multiple types of antibodies each comprising a modified peptide portion to a single animal allows simultaneous evaluation of the pharmacokinetics of multiple types of antibodies that bind to the same antigen or multiple types of antibodies that have the same CDR sequences/variable region sequences. This helps to reduce the number of experimental animals.

Furthermore, while the conventional ligand binding assay (LBA)-based simultaneous pharmacokinetic evaluation of multiple drugs requires each administered antibody drug to bind to a different antigen, the present method allows simultaneous pharmacokinetic evaluation of multiple antibodies against the same antigen.

## Claims

1. An antigen-binding molecule comprising an antigen-binding domain, a CL domain, and a non-native human IgG CH1 domain, wherein the amino acid sequence between positions 121 and 133 according to the EU index in the non-native human IgG CH1 domain is different from a corresponding sequence in a native human IgG of the same isotype as the non-native human IgG.

2. The antigen-binding molecule of claim 1, wherein the amino acid sequence between positions 121 and 133 according to the EU index in the non-native human IgG CH1 domain has any one of the following features:
(1) an amino acid at any one or more sites of positions 122 to 128 and positions 130 to 132 is Ala;
(2) an amino acid at any one or more sites of positions 122 to 132 is Ile;
(3) an amino acid at any one or more sites of positions 122 to 124 and positions 126 to 132 is Val;
(4) an amino acid at any one or more sites of positions 122 to 127 and positions 129 to 132 is Leu;
(5) an amino acid at any one or more sites of positions 122 to 125 and positions 127 to 132 is Phe;
(6) Ala, Ile, Val, Leu, Phe, Ala-Ala, Ile-Ile, Val-Val, Leu-Leu, Phe-Phe, Ala-Ile, Ala-Val, Ala-Leu, Ala-Phe, Ile-Val, Ile-Leu, Ile-Phe, Val-Leu, Val-Phe, Leu-Phe, or Ala-Ala-Ala has been inserted at one or more sites selected from a site between positions 121 and 122, a site between positions 122 and 123, a site between positions 123 and 124, a site between positions 124 and 125, a site between positions 125 and 126, a site between positions 126 and 127, a site between positions 127 and 128, a site between positions 128 and 129, a site between positions 129 and 130, a site between positions 130 and 131, a site between positions 131 and 132, and a site between positions 132 and 133.

3. A method for detecting and/or quantifying an antigen-binding molecule in a biological sample, comprising:
(a) treating a biological sample containing an antigen-binding molecule with a digestive enzyme to generate a peptide from the antigen-binding molecule, wherein
the antigen-binding molecule comprises an antigen-binding domain, a CL domain, and a non-native human IgG CH1 domain,
an amino acid sequence between positions 121 and 133 according to the EU index in the non-native human IgG CH1 domain is different from a corresponding sequence in a native human IgG of the same isotype as the non-native human IgG, and
the peptide consists of the amino acid sequence between positions 121 and 133 according to the EU index in the non-native human IgG CH1 domain;
(b) analyzing the biological sample treated in (a) by mass spectrometry to detect the peptide; and
(c) detecting and/or quantifying the antigen-binding molecule based on the result of the analysis of (b).

4. A method for detecting and/or quantifying each of a plurality of types of antigen-binding molecules in a biological sample, comprising:
(a) treating a biological sample containing a plurality of types of antigen-binding molecules with a digestive enzyme to generate a plurality of types of peptides from the plurality of types of antigen-binding molecules, wherein
the plurality of types of antigen-binding molecules comprise antigen-binding molecules comprising an antigen-binding domain, a CL domain, and a native or non-native human IgG CH1 domain,
the amino acid sequence between positions 121 and 133 according to the EU index in the human IgG CH1 domain of the plurality of types of antigen-binding molecules is different among the plurality of types of antigen-binding molecules,
the plurality of types of peptides consist of an amino acid sequence between positions 121 and 133 according to the EU index in a native or non-native human IgG CH1 domain, and the amino acid sequence is different among the plurality of types of peptides;
(b) analyzing the biological sample treated in (a) by mass spectrometry to detect each of the plurality of types of peptides; and
(c) detecting and/or quantifying each of the plurality of types of antigen-binding molecules based on the result of the analysis of (b).

5. The method of claim 4, wherein the plurality of types of peptides comprise:
(I) two or more types of peptides which consist of an amino acid sequence between positions 121 and 133 according to the EU index in a non-native human IgG CH1 domain, wherein the amino acid sequence is different from a corresponding sequence in a native human IgG of the same isotype as the non-native human IgG, or
(II) a peptide consisting of an amino acid sequence between positions 121 and 133 according to the EU index in a native human IgG CH1 domain, and one or more types of peptides consisting of an amino acid sequence between positions 121 and 133 according to the EU index in a non-native human IgG CH1 domain, wherein the amino acid sequence is different from a corresponding sequence in a native human IgG of the same isotype as the non-native human IgG.

6. A method for designing, selecting, or producing a modified antigen-binding molecule comprising an artificially modified peptide portion that is detectable by mass spectrometry, wherein the method comprises:
(I) identifying a peptide which is obtained after treating one antigen-binding molecule with a digestive enzyme and detectable by mass spectrometry;
(II) designing an artificially modified peptide by any of the following:
(II-1) deleting or substituting one or more amino acid residues in the peptide identified in (I) with other amino acid residues;
(II-2) inserting one or more amino acid residues into the peptide identified in (I);
(II-3) adding an amino acid residue to the N-terminus or C-terminus of the peptide identified in (I);
(II-4) a combination of (II-1) and (II-2);
(II-5) a combination of (II-1) and (II-3);
(II-6) a combination of (II-2) and (II-3);
(II-7) a combination of (II-1), (II-2), and (II-3);
(III) performing the modification of (II) on the peptide portion identified in (I) in the antigen-binding molecule of (I) to produce a modified antigen-binding molecule;
(IV) selecting the modified antigen-binding molecule produced in (III) if the modified antigen-binding molecule satisfies any of the following:
(IV-1) the Tm value of the modified antigen-binding molecule is 50°C or higher;
(IV-2) the antigen-binding activity of the modified antigen-binding molecule is 10% or more of the antigen-binding activity of the antigen-binding molecule of (I); and
(IV-3) both (IV-1) and (IV-2) above; and
(V) selecting the modified antigen-binding molecule selected in (IV) if a portion contained in the modified antigen-binding molecule that corresponds to the peptide designed in (II) is detectable by mass spectrometry after treating a biological sample containing the modified antigen-binding molecule with the digestive enzyme of (I).

7. The method of claim 6, wherein, in (II-2), the amino acid residues inserted are Ala, Ile, Val, Leu, Phe, Ala-Ala, Ile-Ile, Val-Val, Leu-Leu, Phe-Phe, Ala-Ile, Ala-Val, Ala-Leu, Ala-Phe, Ile-Val, Ile-Leu, Ile-Phe, Val-Leu, Val-Phe, Leu-Phe, or Ala-Ala-Ala.

8. A modified antigen-binding molecule which is designed, selected, or produced by the method of claim 6 or 7.

9. A peptide consisting of an amino acid sequence between positions 121 and 133 according to the EU index in a non-native human IgG CH1 domain, wherein the amino acid sequence is different from a corresponding sequence in a native human IgG of the same isotype as the non-native human IgG.

10. A set comprising two or more types of peptides consisting of an amino acid sequence between positions 121 and 133 according to the EU index in a native or non-native human IgG CH1 domain, wherein the amino acid sequence is different among the two or more types of peptides.

11. The set of claim 10, wherein the set comprises two or more types of peptides consisting of an amino acid sequence between positions 121 and 133 according to the EU index in a non-native human IgG CH1 domain, wherein the amino acid sequence between positions 121 and 133 according to the EU index in the non-native human IgG CH1 domain is selected from the group consisting of SEQ ID NOs: 6-294 and 1565-1833.

12. The set of claim 10, wherein the set comprises a peptide consisting of an amino acid sequence between positions 121 and 133 according to the EU index in a native human IgG CH1 domain, and one or more types of peptides consisting of an amino acid sequence between positions 121 and 133 according to the EU index in a non-native human IgG CH1 domain, wherein
when the amino acid sequence between positions 121 and 133 according to the EU index in the native human IgG CH1 domain is SEQ ID NO: 5, the amino acid sequence between positions 121 and 133 according to the EU index in the non-native human IgG CH1 domain is selected from the group consisting of SEQ ID NOs: 6-294, and
when the amino acid sequence between positions 121 and 133 according to the EU index in the native human IgG CH1 domain is SEQ ID NO: 945, the amino acid sequence between positions 121 and 133 according to the EU index in the non-native human IgG CH1 domain is selected from the group consisting of SEQ ID NOs: 1565-1833.

13. The set of claim 10, which is for use in a method for detecting each of a plurality of types of peptides derived from a plurality of types of antigen-binding molecules in a biological sample by mass spectrometry, or a method for detecting and/or quantifying each of a plurality of types of antigen-binding molecules in a biological sample.

14. A method for administering to an individual or a subject a mixture of a plurality of types of antigen-binding molecules each of which comprise a respective peptide of the set of any one of claims 10 to 13.

15. A method for selecting an antigen-binding molecule, comprising:
(a) detecting and/or quantifying each of a plurality of types of antigen-binding molecules in a biological sample derived from an individual or a subject that a mixture of the plurality of types of antigen-binding molecules has been administered to, wherein the plurality of types of antigen-binding molecules each of which comprise a respective peptide of the set of any one of claims 10 to 13; and
(b) selecting an antigen-binding molecule based on the result of the detection and/or quantification of (a).
